# EUROPEAN PATENT SPECIFICATION

(11) **EP 3 548 084 B1**
(45) Date of publication and mention of the grant of the patent: **23.08.2023**
(21) Application number: 17825663.2
(22) Date of filing: 01.12.2017
(51) Int. Cl.: A61K 39/395, A61K 39/44, A61K 39/00, A61P 35/00

(54) **METHODS FOR DETERMINING CAR-T CELLS DOSING**
VERFAHREN ZUR FESTLEGUNG DER DOSIERUNG VON CAR-T-ZELLEN
MÉTHODES POUR DÉTERMINER LE DOSAGE DE CÉLLULES CAR-T

(30) Priority: 03.12.2016 US 201662429738 P; 02.06.2017 US 201762514765 P; 05.06.2017 US 201762515523 P
(43) Date of publication of application: 09.10.2019
(60) Divisional application: 23185364.9
(73) Proprietor: Juno Therapeutics, Inc., Seattle, WA 98109 (US)
(72) Inventor: LI, He, Seattle, WA 98109-4703 (US); ALBERTSON, Tina, Seattle, WA 98109 (US); HEIPEL, Mark, D., Seattle, WA 98109 (US); SUTHERLAND, Claire, L., Seattle, WA 98109 (US)
(74) Representative: Carpmaels & Ransford LLP
(86) International application number: PCT/US2017/064364
(87) International publication number: WO 2018/102787

(56) References cited:
- WO-A1-2012/062596
- WO-A1-2014/011984
- WO-A1-2016/064929
- WO-A1-2017/165571
- US-A1- 2016 185 861
- GARFALL ALFRED L ET AL: "Posterior Reversible Encephalopathy Syndrome (PRES) after Infusion of Anti-Bcma CAR T Cells (CART-BCMA) for Multiple Myeloma: Successful Treatment with Cyclophosphamide", BLOOD; 58TH ANNUAL MEETING AND EXPOSITION OF THE AMERICAN-SOCIETY-OF-HEMATOLOGY (ASH), AMERICAN SOCIETY OF HEMATOLOGY, US; SAN DIEGO, CA, USA , vol. 128, no. 22 2 December 2016 (2016-12-02), page 5702, XP009503773, ISSN: 0006-4971 Retrieved from the Internet: URL:www.bloodjournal.org/content/128/22/57 02
- BATLEVI C L ET AL: "Novel immunotherapies in lymphoid malignancies", NATURE REVIEWS CLINICAL ONCOLOGY 20160101 NATURE PUBLISHING GROUP GBR, vol. 13, no. 1, 1 January 2016 (2016-01-01), pages 25-40, XP009503771, ISSN: 1759-4774
- BRANDON R. SHANK ET AL: "Chimeric Antigen Receptor T Cells in Hematologic Malignancies", PHARMACOTHERAPY : THE JOURNAL OF HUMAN PHARMACOLOGY AND DRUG THERAPY, vol. 37, no. 3, 1 March 2017 (2017-03-01), pages 334-345, XP055471941, US ISSN: 0277-0008, DOI: 10.1002/phar.1900
- M. L. DAVILA ET AL: "Efficacy and Toxicity Management of 19-28z CAR T Cell Therapy in B Cell Acute Lymphoblastic Leukemia", SCIENCE TRANSLATIONAL MEDICINE, vol. 6, no. 224, 19 February 2014 (2014-02-19), pages 224ra25-224ra25, XP055234425, US ISSN: 1946-6234, DOI: 10.1126/scitranslmed.3008226
- MAUDE SHANNON L ET AL: "Managing cytokine release syndrome associated with novel T cell-engaging therapies.", CANCER JOURNAL (SUDBURY, MASS.), vol. 20, no. 2, March 2014 (2014-03), pages 119-122, XP002780801, ISSN: 1540-336X
- MARCO L. DAVILA ET AL: "CD19-Targeted T Cells for Hematologic Malignancies : Clinical Experience to Date", CANCER JOURNAL, vol. 21, no. 6, 1 January 2015 (2015-01-01), pages 470-474, XP055377589, US ISSN: 1528-9117, DOI: 10.1097/PPO.0000000000000153
- JENNIFER N. BRUDNO ET AL: "Toxicities of chimeric antigen receptor T cells: recognition and management", BLOOD, vol. 127, no. 26, 20 May 2016 (2016-05-20) , pages 3321-3330, XP055442400, US ISSN: 0006-4971, DOI: 10.1182/blood-2016-04-703751
- FREY NOELLE V ET AL: "Cytokine release syndrome with novel therapeutics for acute lymphoblastic leukemia", HEMATOLOGY / THE EDUCATION PROGRAM OF THE AMERICAN SOCIETY OF HEMATOLOGY, AMERICAN SOCIETY OF HEMATOLOGY, US, vol. 2016, no. 1, 2 December 2016 (2016-12-02), pages 567-572, XP008184859, ISSN: 1520-4383, DOI: 10.1182/ASHEDUCATION-2016.1.567
- R. J. BRENTJENS ET AL: "CD19-Targeted T Cells Rapidly Induce Molecular Remissions in Adults with Chemotherapy-Refractory Acute Lymphoblastic Leukemia", SCIENCE TRANSLATIONAL MEDICINE, vol. 5, no. 177, 20 March 2013 (2013-03-20), pages 177ra38-177ra38, XP055234457, US ISSN: 1946-6234, DOI: 10.1126/scitranslmed.3005930
- D. T. Teachey ET AL: "Identification of Predictive Biomarkers for Cytokine Release Syndrome after Chimeric Antigen Receptor T-cell Therapy for Acute Lymphoblastic Leukemia", CANCER DISCOVERY, vol. 6, no. 6, 13 April 2016 (2016-04-13), pages 664-679, XP055314474, US ISSN: 2159-8274, DOI: 10.1158/2159-8290.CD-16-0040
- Shu-Lin Yu ET AL: "Serum lactate dehydrogenase predicts prognosis and correlates with systemic inflammatory response in patients with advanced pancreatic cancer after gemcitabine-based chemotherapy", Scientific Reports, vol. 7, no. 1, 27 March 2017 (2017-03-27), XP055704394, DOI: 10.1038/srep45194
- J Dick ET AL: "Use of LDH and autoimmune side effects to predict response to ipilimumab treatment", Immunotherapy, vol. 8, no. 9, 1 September 2016 (2016-09-01), pages 1033-1044, XP055704483, GB ISSN: 1750-743X, DOI: 10.2217/imt-2016-0083
- C. Lv ET AL: "Serum Lactate Dehydrogenase as a Predictor of Outcome in Posterior Reversible Encephalopathy Syndrome: Imperative to Unify", American Journal of Neuroradiology, vol. 36, no. 4, 22 January 2015 (2015-01-22), pages E29-E30, XP055704398, US ISSN: 0195-6108, DOI: 10.3174/ajnr.A4243

## Description

### Cross-Reference to Related Applications

This application claims priority from U.S. provisional application No. 62/429,738, filed December 3, 2016, entitled "METHODS FOR DETERMINING DOSING IN CELL THERAPY," U.S. provisional application No. 62/514,765, filed June 2, 2017, entitled "METHODS FOR DETERMINING DOSING IN CELL THERAPY," and U.S. provisional application No. 62/515,523, filed June 5, 2017, entitled "METHODS FOR DETERMINING DOSING IN CELL THERAPY".

### Sequence Listing

The present application is being filed along with a Sequence Listing in electronic format. The Sequence Listing is provided as a file entitled 735042009240SeqList.txt, created November 29, 2017, which is 34 kilobytes in size.

### Field

The present invention relates to an agent that is capable of decreasing expansion or proliferation of genetically engineered T cells expressing a chimeric antigen receptor (CAR) in a subject for use in a method of modulating activity of engineered T cells expressing a CAR in treating cancer. Also described herein are methods which in some instances include determining a therapeutic range and/or window for dosing, for example, based on the estimated probabilities of risk of developing a toxicity and estimated probabilities of a treatment outcome or response, such as treatment, reduction nor amelioration of a sign or symptom thereof, or degree or durability thereof, following administration of the cell therapy or engineered cells.

### Background

Various approaches are available for immunotherapy, for example, adoptive cell therapy methods involving administering T cells, such as those expressing genetically engineered antigen receptors, such as CARs. In some aspects, available methods may not be entirely satisfactory. There is a need for additional strategies for immunotherapy and adoptive cell therapy, e.g., strategies to enhance persistence, activity and/or proliferation of administered cells and responses and strategies for modulating T cell phenotype. Described herein are methods, cells, compositions, articles of manufacture, and systems to address such needs.

WO2017/165571 describes early intervention methods to prevent or ameliorate toxicity caused by or due to a therapy, such as an immunotherapy or a cell therapy.

### Summary

The present invention is set out in the appended claims. In particular, the present invention provides an agent that is capable of decreasing expansion or proliferation of genetically engineered T cells expressing a chimeric antigen receptor (CAR) in a subject for use in a method of modulating activity of engineered T cells expressing a CAR in treating cancer, wherein the method comprises:
administering the agent to the subject;
wherein the subject is one in which the level, amount or concentration of a volumetric measure of tumor burden or an inflammatory marker in a sample from the subject is at or above a threshold level;
wherein the sample is obtained from the subject prior to receiving administration of genetically engineered T cells expressing a CAR;
wherein the volumetric measure is a sum of the products of diameters (SPD) and the threshold value is greater than or equal to 50 cm²;
wherein the inflammatory marker is lactate dehydrogenase (LDH) and the threshold value is greater than or equal to 500 units per liter; and
wherein the agent is a steroid.

The invention also provides an agent that is capable of decreasing expansion or proliferation of genetically engineered T cells expressing a chimeric antigen receptor (CAR) in a subject and genetically engineered cells comprising T cells expressing a CAR for use in a method of modulating activity of engineered T cells expressing a CAR and treating cancer, wherein the engineered T cells are administered to treat cancer and the agent is administered to modulate activity of the engineered T cells, and wherein the method comprises:
a) administering the agent to the subject; and
b) administering a dose of genetically engineered T cells expressing a CAR,
wherein:
(i) the subject is one in which the level, amount or concentration of a volumetric measure of tumor burden or an inflammatory marker in a sample from the subject is at or above a threshold level;
(ii) the sample is obtained from the subject prior to receiving administration of genetically engineered T cells expressing a CAR;
(iii) the volumetric measure is a sum of the products of diameters (SPD) and the threshold value is greater than or equal to 50 cm²;
(iv) the inflammatory marker is lactate dehydrogenase (LDH) and the threshold value is greater than or equal to 500 units per liter; and
(v) the agent is a steroid.

Described herein as part of the disclosure are methods of dosing or treating a subject, which in some aspects involve administering to the subject a dose of engineered cells, such as those engineered with a chimeric antigen receptor (CAR), and/or assessing and/or administering further agent(s) to subjects having been administered such engineered cells. In some instances, the dose administered is within a therapeutic range and/or window and/or is sufficient to achieve an overall or peak amount or number of engineered cells, e.g., CAR+ cells, in a sample or tissue or bodily fluid of the subject, such as in the blood of the subject, within a specified range, such as within a specified or determined therapeutic range, optionally within or over a certain period of time following administration. In some instances, the therapeutic range is determined based upon or relates to probabilities, such as estimated probabilities, e.g., probability of response and/or probability or risk of developing a sign or symptom of a toxicity, such as a severe and/or grade 3 or higher toxicity, such as neurotoxicity (NT), e.g., a grade 3 or higher toxicity.

In some instances, the administering involves administration of a sub-optimal or reduced or low dose of cells which in some aspects is insufficient to be within or achieve or result within a therapeutic range and/or window and/or is insufficient to achieve an overall or peak amount or number of engineered cells, e.g., CAR+ cells, in a sample or tissue or bodily fluid of the subject, such as in the blood of the subject, within a specified range, such as within a specified or determined therapeutic range, optionally within or over a certain period of time following administration. In some instances, the described methods further include administering a compound to the subject other than or in addition to the engineered cells. In some instances, such agent may be an agent known or suspected of being capable of enhancing or increasing the likelihood, degree, rapidity, or level of expansion, persistence and/or exposure of the subject to the engineered cells, such as the CAR+ cells. In some instances, the agent(s) increases or promotes expansion of the cells in vivo, and/or is capable of resulting in levels, degree or rapidity of expansion, peak levels, AUC, or other measure of the cells in the subject, such as CAR+ cells, expansion is within the therapeutic range and/or window. In some of any such instances, the therapeutic range in some aspects is determined based upon or relates to probabilities, such as estimated probabilities, e.g., probability of response and/or probability or risk of developing a sign or symptom of a toxicity, such as a severe and/or grade 3 or higher toxicity, such as neurotoxicity (NT), e.g., a grade 3 or higher toxicity.

In some instances, the methods involve, e.g., subsequent to the administration, to the subject the cell therapy or engineered cells; monitoring levels of engineered or other cells in a sample of the subject such as a blood or blood-derived samples (such as peak CAR cells in the blood), optionally over time, for example, to assess whether the cells are within a therapeutic range and/or window. In some instances, if the cells are not within a therapeutic range or window, the described methods include an administration to the subject, such as administering a compound to enhance expansion or exposure to the engineered cells such as to enhance CAR+ cell expansion in vivo, e.g., such that the peak CAR+ expansion and/or levels and/or exposure and/or AUC is within the therapeutic or desired range.

In some of any such instances, the level of engineered, e.g., CAR+, cells in the sample is determined as the number of the cells, e.g., CAR+ cells, per microliter of the sample; in some instances, the peak level is the highest such measurement following, optionally over a specified period of time following, administration of the cells or cell therapy to the subject.

In some of any such instances, the therapeutic range is a range in which the estimated probability of a toxicity or toxic outcome or sign or symptom thereof, such as a severe toxicity and/or a neurotoxicity (NT) or CRS, is less than 20%, less than 15%, less than 10% or less than 5%; in some aspects, the probability is based on a probability curve, e.g., based on outcomes of subjects treated with or administered the cell therapy and/or cells engineered to express the recombinant receptor. In some instances, the estimated probability of achieving a treatment response, effect, amelioration or treatment is greater than 20 %, 25%, 30 %, 40 %, 45 %, 50 %, 55 %, 60 %, 65%, 70%, 75%, 80%, 85%, 90%, 95% or more.

In some of any such instances, the toxicity is a neurotoxicity and/or is severe toxicity and/or is grade 3-5 neurotoxicity.

In some instances, the response or indicator of response is a marrow response or an outcome measured in bone marrow of the subject. In some cases, the presence or absence of the marrow response is or is determined by flow cytometry and/or IgH sequencing and/or indicates or is a reduction or elimination of cells of the disease or condition in a sample of the subject, optionally an organ, tissue or fluid of the subject, such as a lymph node, bone marrow, tumor site, blood or other sample, of the subject.

In some of any such instances, the disease or condition is a cancer. In some aspects, the cancer is selected from the group consisting of sarcomas, carcinomas, lymphomas, non-Hodgkin lymphomas (NHLs), diffuse large B cell lymphoma (DLBCL), leukemia, CLL, ALL, AML and myeloma. In some cases, the cancer is a pancreatic cancer, bladder cancer, colorectal cancer, breast cancer, prostate cancer, renal cancer, hepatocellular cancer, lung cancer, ovarian cancer, cervical cancer, pancreatic cancer, rectal cancer, thyroid cancer, uterine cancer, gastric cancer, esophageal cancer, head and neck cancer, melanoma, neuroendocrine cancers, CNS cancers, brain tumors, bone cancer, or soft tissue sarcoma.

In some of any such instances, the chimeric antigen receptor (CAR) contains an extracellular antigen-recognition domain that specifically binds to the antigen and an intracellular signaling domain comprising an ITAM. In some aspects, the intracellular signaling domain contains an intracellular domain of a CD3-zeta (CD3ζ) chain. In some instances, the chimeric antigen receptor (CAR) further comprises a costimulatory signaling region. In some cases, the costimulatory signaling region comprises a signaling domain of CD28 or 4-1BB. In some instances, the costimulatory domain is a domain of CD28. In some instances, the costimulatory domain is a domain of 4-1BB.

In some of any such instances, the CAR specifically recognizes or binds an antigen selected from among antigens expressed by B cells, ROR1, B cell maturation antigen (BCMA), tEGFR, Her2, Ll-CAM, CD19, CD20, CD22, mesothelin, CEA, and hepatitis B surface antigen, anti-folate receptor, CD23, CD24, CD30, CD33, CD38, CD44, EGFR, EGP-2, EGP-4, EPHa2, ErbB2, 3, or 4, erbB dimers, EGFR vIII, FBP, FCRL5, FCRH5, GPRC5D, fetal acethycholine e receptor, GD2, GD3, HMW-MAA, IL-22R-alpha, IL-13R-alpha2, kdr, kappa light chain, Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, EGP2, EGP40, TAG72, B7-H6, IL-13 receptor a2 (IL-13Ra2), CA9, GD3, HMW-MAA, CD171, G250/CAIX, HLA-AI MAGE Al, HLA-A2 NY-ESO-1, PSCA, folate receptor-a, CD44v6, CD44v7/8, avb6 integrin, 8H9, NCAM, VEGF receptors, 5T4, Foetal AchR, NKG2D ligands, CD44v6, dual antigen, and an antigen associated with a universal tag, a cancer-testes antigen, mesothelin, MUC1, MUC16, PSCA, NKG2D Ligands, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, CCL-1, CD138, and a pathogen-specific antigen.

In some of any such instances, the cells are T cells. In some cases, the T cells are CD4+ or CD8+.

Also described herein but not claimed are articles of manufacture and compositions, such as those containing the cells and instructions for administration such as according to the methods and uses of any of the instances described herein.

Described here but not claimed are methods of treatment including administering to a subject having a disease or condition, a dose of genetically engineered cells comprising T cells expressing a chimeric antigen receptor (CAR) for treating the disease or condition, after administering the dose of genetically engineered cells, monitoring CAR+ T cells in the blood of the subject to assess if the cells are within a therapeutic range, and if the genetically engineered cells are not within the therapeutic range, administering an agent to the subject capable of modulating, optionally increasing or decreasing, CAR+ T cell expansion or proliferation, in the subject, wherein the therapeutic range is: (i) based upon the range of peak CD3+ CAR+ T cells, or a CD8+CAR+ T cell subset thereof, in the blood among one or more subjects previously treated with the genetically engineered cells that is associated with an estimated probability of response of greater than or greater than about 65%, 70%, 75%, 80%, 85%, 90%, and an estimated probability of a toxicity of less than or about 30%; or (ii) peak CD3+CAR+ T cells in the blood, following administration of the genetically engineered cells, that is between or between about 10 cells per microliter and 500 cells per microliter; or (iii) peak CD8+CAR+ T cells in the blood, following administration of the genetically engineered cells, that is between or between about 2 cells per microliter and 200 cells per microliter.

Disclosed herein but not claimed are methods of treatment including monitoring, in the blood of a subject, the presence of genetically engineered cells containing T cells expressing a chimeric antigen receptor (CAR) to assess if the cells are within a therapeutic range, wherein the subject has been previously administered a dose of the genetically engineered cells for treating a disease or condition; and if the genetically engineered cells are not within the therapeutic range, administering an agent to the subject capable of modulating, optionally increasing or decreasing, CAR+ T cell expansion or proliferation, in the subject, wherein the therapeutic range is: (i) based upon the range of peak CD3+ CAR+ T cells, or a CD8+CAR+ T cell subset thereof, in the blood among one or more subjects previously treated with the genetically engineered cells that is associated with an estimated probability of response of greater than or greater than about 65%, 70%, 75%, 80%, 85%, 90%, and an estimated probability of a toxicity of less than or about 30%, 25%, 20%, 15%, 10%, 5%; or (ii) peak CD3+CAR+ T cells in the blood, following administration of the genetically engineered cells, that is between or between about 10 cells per microliter and 500 cells per microliter; or (iii) peak CD8+CAR+ T cells in the blood, following administration of the genetically engineered cells, that is between or between about 2 cells per microliter and 200 cells per microliter. In some instances, if the peak number of CAR+ T cells in the blood of the subject is less than the lowest number of peak CAR+ T cells in the therapeutic range, an agent is administered to the subject that is capable of increasing CAR+ T cell expansion or proliferation. In some cases, the agent is capable of CAR-specific expansion.

In some instances, the agent is an anti-idiotype antibody or antigen-binding fragment thereof specific to the CAR, an immune checkpoint inhibitor, a modulator of a metabolic pathway, an adenosine receptor antagonist, a kinase inhibitor, an anti-TGFβ antibody or an anti-TGFβR antibody or a cytokine.

In some instances, if the peak number of CAR+ T cells in the blood of the subject is greater than the highest number of peak CAR+ T cells in the therapeutic range, an agent is administered to the subject that is capable of decreasing CAR+ T cell expansion or proliferation. In some examples, the agent is a steroid. In some cases, the steroid is a corticosteroid. In some instances, the steroid is dexamethasone or methylprednisolone.

In some of any such instances, the steroid is administered in an amount that is between or between about 1.0 mg and about 40 mg, between or between about 1.0 mg and about 20 mg, between or between about 2.0 mg and about 20 mg, between or between about 5.0 mg and about 25.0 mg, between or between about 10 mg and about 20 mg dexamethasone or equivalent thereof, each inclusive.

In some of any such instances, the subject is monitored for CAR+ T cells in the blood at a time that is at least 8 days, 9 days, 10 days, 11 days, 12 days, 13 days, 14 days, 15 days, 16 days, 17 days, 18 days, 19 days, 20 days or 21 days after initiation of administration of the genetically engineered cells. In some instances, the subject is monitored for CAR+ T cells in the blood at a time that is between or between about 11 to 22 says, 12 to 18 days or 14 to 16 days, each inclusive, after initiation of administration of the genetically engineered cells.

In some instances, the agent is administered at a time that is greater than or greater than about 8 days, 9 days, 10 days, 11 days, 12 days, 13 days, 14 days, 15 days, 16 days, 17 days, 18 days, 19 days, 20 days or 21 days after initiation of administration of the genetically engineered cells. In some instances, the agent is administered at a time that is between or between about 11 to 22 says, 12 to 18 days or 14 to 16 days, each inclusive, after initiation of administration of the genetically engineered cells.

Described herein but not claimed are methods of modulating activity of engineered cells, the method including selecting a subject in which the level, amount or concentration of a volumetric measure of tumor burden or an inflammatory marker in a sample from the subject is at or above a threshold level, wherein the sample does not contain genetically engineered T cells expressing a chimeric antigen receptor (CAR) and/or is obtained from the subject prior to receiving administration of genetically engineered T cells expressing a CAR; and administering to the selected subject an agent that is capable of decreasing expansion or proliferation of genetically engineered T cells expressing a CAR.

Provided herein as part of the claimed invention is an agent that is capable of decreasing expansion or proliferation of genetically engineered T cells expressing a chimeric antigen receptor (CAR) in a subject for use in a method of modulating activity of engineered T cells expressing a CAR in treating cancer, the method including administering the agent to the subject, wherein the subject is one in which the level, amount or concentration of a volumetric measure of tumor burden or an inflammatory marker in a sample from the subject is at or above a threshold level, wherein the sample is obtained from the subject prior to receiving administration of genetically engineered T cells expressing a CAR, wherein the volumetric measure is a sum of the products of diameters (SPD) and the threshold value is greater than or equal to 50 cm², wherein the inflammatory marker is lactate dehydrogenase (LDH) and the threshold value is greater than or equal to 500 units per liter, and wherein the agent is a steroid.

In some embodiments, the agent is administered prior to or concurrently with initiation of administration of the genetically engineered cells T cells expressing a chimeric antigen receptor. In some cases, the method further includes administering a dose of the genetically engineered T cells expressing a CAR.

The genetically engineered T cells expressing a CAR are for treating cancer.

In some embodiments, prior to administering the agent, the selected subject is at risk of developing a toxicity following administration of the genetically engineered cells. In some embodiments, the administration of the agent is sufficient to achieve peak CAR+ T cells in a therapeutic range in the subject, or in a majority of selected subjects so treated by the method or in greater than 75%, 80%, 85%, 90%, 95% of the selected subjects so treated by the method.

In some aspects, the therapeutic range is based upon the range of peak CD3+ CAR+ T cells, or a CD8+CAR+ T cell subset thereof, in the blood among one or more subjects previously treated with the genetically engineered cells that is associated with an estimated probability of response of greater than or greater than about 65%, 70%, 75%, 80%, 85%, 90%, and an estimated probability of a toxicity of less than or about 30%, 25%, 20%, 15%, 10%, 5%; or peak CD3+CAR+ T cells in the blood, following administration of the genetically engineered cells, that is between or between about 10 cells per microliter and 500 cells per microliter; or peak CD8+CAR+ T cells in the blood, following administration of the genetically engineered cells, that is between or between about 2 cells per microliter and 200 cells per microliter.

In the claimed invention, the volumetric measure of tumor burden is a sum of the products of diameters (SPD). Alternative volumetric measures for tumor burden include longest tumor diameters (LD), sum of longest tumor diameters (SLD), tumor volume, necrosis volume, necrosis-tumor ratio (NTR), peritumoral edema (PTE), and edema-tumor ratio (ETR). In some embodiments, the volumetric measure is measured using computed tomography (CT), positron emission tomography (PET), and/or magnetic resonance imaging (MRI) of the subject.

An inflammatory marker in a sample from the subject is measured. In the claimed invention, the inflammatory marker is lactate dehydrogenase (LDH). Alternative inflammatory markers include C-reactive protein (CRP), erythrocyte sedimentation rate (ESR), albumin, ferritin, β2 microglobulin (β2-M), a cytokine or a chemokine. In some instances of the disclosure described herein, the inflammatory marker is a cytokine or a chemokine that is IL-7, IL15, MIP-1alpha or TNF-alpha. In some instances, the cytokine or chemokine is associated with macrophage or monocyte activation. In some embodiments, the sample is or contains a blood sample, plasma sample, or serum sample. In some cases, the inflammatory marker is assessed using a colorimetric assay or an immunoassay. In some cases, the inflammatory marker is assessed using an immunoassay and the immunoassay is selected from enzyme-linked immunosorbent assay (ELISA), enzyme immunoassay (EIA), radioimmunoassay (RIA), surface plasmon resonance (SPR), Western Blot, Lateral flow assay, immunohistochemistry, protein array or immuno-PCR (iPCR).

In some instances of the disclosure described herein, the threshold value is a value that is within 25%, within 20%, within 15%, within 10%, or within 5% above the average value of the volumetric measure or inflammatory marker and/or is within a standard deviation above the average value of the volumetric measure or the inflammatory marker in a plurality of control subjects; is above the highest value of the volumetric measure or inflammatory marker, optionally within 50%, within 25%, within 20%, within 15%, within 10%, or within 5% above such highest fold change, measured in at least one subject from among a plurality of control subjects; and/or is above the highest value of the volumetric measure or inflammatory marker as measured among more than 75%, 80%, 85%, 90%, 95%, or 98% of subjects from a plurality of control subjects.

In some instances of the disclosure, the plurality of control subjects are a group of subjects prior to receiving a dose of the genetically engineered cells, wherein each of the control subjects of the group exhibited a peak CAR+ T cells in the blood greater than the highest peak CAR+ T cells in the therapeutic range; each of the control subjects of the group went on to develop at toxicity, optionally a neurotoxicity or cytokine release syndrome (CRS), a grade 2 or grade 3 or higher neurotoxicity or a grade 3 or higher CRS, after receiving a dose of the engineered cells for treating the same disease or condition; each of the control subjects of the group did not develop a response, optionally a complete response (CR) or partial response (PR), following administration of the dose of genetically engineered cells; and/or each of the control subjects of the group did not develop a durable response, optionally for at or about or greater than or about 3 months or at or about or greater than or about 6 months, following administration of the dose of genetically engineered cells.

In the claimed invention, the volumetric measure is SPD and the threshold value is greater than or equal to 50 per cm². In some embodiments, the threshold value is or is about 60 per cm², or is or is about 70 per cm². Alternative threshold values include 30 or about 30 per cm², or 40 or about 40 per cm².

In the claimed invention, the inflammatory marker is LDH and the threshold value is greater than or equal to 500 units per liter. In some embodiments, the threshold value is or is about 600 units per liter. Alternative threshold values include 300 or about 300 units per liter, or 400 or about 400 units per liter.

The agent is a steroid. In some instances, the steroid is a corticosteroid. In some examples, the steroid is dexamethasone or methylprednisolone. In some of any such embodiments, the steroid is administered in an amount that is between or between about 1.0 mg and about 40 mg, between or between about 1.0 mg and about 20 mg, between or between about 2.0 mg and about 20 mg, between or between about 5.0 mg and about 25.0 mg, between or between about 10 mg and about 20 mg dexamethasone or equivalent thereof, each inclusive. In some embodiments, the volumetric measure or inflammatory marker is measured in the subject within 1 day, 2 days, 3 days, 4 days, 6 days, 8 days, 12 days, 16 days, 20 days, 24 days, 28 days or more prior to initiation of administration of the genetically engineered cells.

Described but not claimed are methods of dosing a subject, the method includes administering to a subject having a disease or condition, a dose of genetically engineered cells including T cells expressing a chimeric antigen receptor (CAR), wherein the dose contains a number of the genetically engineered cells that is sufficient to achieve peak CAR+ cells in the blood within a determined therapeutic range in the subject, or in a majority of subjects so treated by the method or in greater than 75%, 80%, 85%, 90%, 95% of the subjects so treated by the method, wherein the therapeutic range is: (i) based upon the range of peak CD3+ CAR+ T cells, or a CD8+CAR+ T cell subset thereof, in the blood among one or more subjects previously treated with the genetically engineered cells that is associated with an estimated probability of response of greater than or greater than about 65%, 70%, 75%, 80%, 85%, 90%, and an estimated probability of a toxicity of less than or about 30%, 25%, 20%, 15%, 10%, 5%; or (ii) peak CD3+CAR+ T cells in the blood, following administration of the genetically engineered cells, that is between or between about 10 cells per microliter and 500 cells per microliter; or (iii) peak CD8+CAR+ T cells in the blood, following administration of the genetically engineered cells, that is between or between about 2 cells per microliter and 200 cells per microliter.

In some of any such instances, the dose of genetically engineered cells contains from or from about 1 × 10⁵ to 5 × 10⁸ total CAR-expressing T cells, 1 × 10⁶ to 2.5 × 10⁸ total CAR-expressing T cells, 5 × 10⁶ to 1 × 10⁸ total CAR-expressing T cells, 1 × 10⁷ to 2.5 × 10⁸ total CAR-expressing T cells, 5 × 10⁷ to 1 × 10⁸ total CAR-expressing T cells, each inclusive. In some instances, the dose of genetically engineered cells contains at least or at least about 1 × 10⁵ CAR-expressing cells, at least or at least about 2.5 × 10⁵ CAR-expressing cells, at least or at least about 5 × 10⁵ CAR-expressing cells, at least or at least about 1 × 10⁶ CAR-expressing cells, at least or at least about 2.5 × 10⁶ CAR-expressing cells, at least or at least about 5 × 10⁶ CAR-expressing cells, at least or at least about 1 × 10⁷ CAR-expressing cells, at least or at least about 2.5 × 10⁷ CAR-expressing cells, at least or at least about 5 × 10⁷ CAR-expressing cells, at least or at least about 1 × 10⁸ CAR-expressing cells, at least or at least about 2.5 × 10⁸ CAR-expressing cells, or at least or at least about 5 × 10⁸ CAR-expressing cells.

Described but not claimed are methods of dosing a subject, the method including administering to a subject having a disease or condition, a sub-optimal dose of genetically engineered cells including T cells engineered with a chimeric antigen receptor (CAR), wherein the dose contains a number of the genetically engineered cells that is insufficient to achieve peak CAR+ cells in the blood within a determined therapeutic range in the subject, or in a majority of subjects so treated by the method or in greater than 75%, 80%, 85%, 90%, 95% of the subjects so treated by the method; and subsequent to administering the genetically engineered cells, administering an agent to enhance CAR+ cell expansion or proliferation in the subject to achieve peak CAR+ T cells in the blood within the therapeutic range, wherein the therapeutic range is: (i) based upon the range of peak CD3+ CAR+ T cells, or a CD8+CAR+ T cell subset thereof, in the blood among one or more subjects previously treated with the genetically engineered cells that is associated with an estimated probability of response of greater than or greater than about 65%, 70%, 75%, 80%, 85%, 90% and an estimated probability of a toxicity of less than or about 30%, 25%, 20%, 15%, 10%, 5%; or (ii) peak CD3+CAR+ T cells in the blood, following administration of the genetically engineered cells, that is between or between about 10 cells per microliter and 500 cells per microliter; or (iii) peak CD8+CAR+ T cells in the blood, following administration of the genetically engineered cells, that is between or between about 2 cells per microliter and 200 cells per microliter.

In some instances, after administering the dose of genetically engineered cells, the method includes monitoring the CAR+ T cells in the blood of the subject. In some instances, following administration of the agent, the method achieves an increased frequency of peak CAR+ cells in the blood within a determined therapeutic range in the subject, compared to a method involving administration of the same dose of genetically engineered cells but without the agent; or peak CAR+ cells in the blood within a determined therapeutic range in the subject, or in a majority of subjects so treated by the method or in greater than 75%, 80%, 85%, 90%, 95% of the subjects so treated by the method.

In some instances, the dose of genetically engineered cells is less than or less than about 1 × 10⁷ CAR-expressing cells, less than or less than about 5 × 10⁶ CAR-expressing cells, less than or less than about 2.5 × 10⁶ CAR-expressing cells, less than or less than about 1 × 10⁶ CAR-expressing cells, less than or less than about 5 × 10⁵ CAR-expressing cells, less than or less than about 2.5 × 10⁵ CAR-expressing cells, less than or less than about 1 × 10⁵ CAR-expressing cells.

In some instances, the agent is capable of increasing expansion of the CAR+ T cells, optionally CAR-specific expansion. In some cases, the agent is an anti-idiotype antibody or antigen-binding fragment thereof specific to the CAR, an immune checkpoint inhibitor, a modulator of a metabolic pathway, an adenosine receptor antagonist, a kinase inhibitor, an anti-TGFβ antibody or an anti-TGFβR antibody or a cytokine.

In some of any such instances, among a plurality of subjects treated, the method achieves an increase in the percentage of subjects achieving a durable response, optionally a complete response (CR) or objective response (OR) or a partial response (PR), optionally that is durable for at or greater than 3 months or at or greater than 6 months, compared to a method that does not contain administering the agent. In some examples, the increase is greater than or greater than about 1.2-fold, 1.5-fold, 2-fold, 3-fold, 4-fold, 5-fold, 10-fold or more. In some instances, at least 15%, at least 20%, at least 25%, at least 30%, at least 35%, at least 40 % or at least 50% of subjects treated according to the method achieve a complete response (CR) that is durable for at or greater than 3 months or at or greater than 6 months; and/or at least 25%, at least 30%, at least 40%, at least 50%, at least 60% or at least 70% of the subjects treated according to the method achieve objective response (OR) that is durable for at or greater than 3 months or at or greater than 6 months.

In some instances, greater than or greater than about 50%, greater than or greater than about 60 %, greater than or greater than about 70 %, or greater than or greater than about 80 % of the subjects treated according to the method do not exhibit a grade 3 or greater cytokine release syndrome (CRS) and/or do not exhibit a grade 2 or greater or grade 3 or greater neurotoxicity; or greater than or greater than about 40 %, greater than or greater than about 50 % or greater than or greater than about 55 % of the subjects treated according to the method do not exhibit any neurotoxicity or CRS.

In some of any such instances, peak CAR+ T cells is determined as the number of CAR+ T cells per microliter in the blood of the subject. In some instances, the therapeutic range is the range in which the estimated probability of toxicity is less than 20%, less than 15%, less than 10% or less than 5% and the estimated probability of achieving a response is greater than 65%, 70%, 75%, 80%, 85%, 90%, 95% or more.

In some instances, the probability of toxicity is based on a toxicity selected from any neurotoxicity or cytokine release syndrome (CRS); severe toxicity or grade 3 or higher toxicity; severe CRS or a grade 3 or higher CRS; or severe neurotoxicity, grade 2 or higher neurotoxicity or grade 3 or higher neurotoxicity. In some instances, the probability of a toxicity is based on the probability of a severe toxicity or a grade 3 or higher toxicity. In some cases, the severe toxicity is grade 3-5 neurotoxicity.

In some instances, the probability of response is based on a response that is a complete response (CR), an objective response (OR) or a partial response (PR), optionally wherein the response is durable, optionally durable for at or at least 3 months or at or at least 6 months. In some instances, the response is a marrow response as determined based on assessment of the presence of a malignant immunoglobulin heavy chain locus (IGH) ad/or an index clone in the bone marrow of the subject. In some cases, the malignant IGH and/or index clone is assessed by flow cytometry or IgH sequencing.

Described but not claimed is a method of assessing likelihood of a durable response, the method including detecting, in a biological sample from a subject, peak levels of one or more inflammatory marker and/or peak levels of genetically engineered cells including T cells expressing a chimeric antigen receptor (CAR), wherein the subject has been previously administered a dose of the genetically engineered cells for treating a disease or condition; and comparing, individually, the peak levels to a threshold value, thereby determining a likelihood that a subject will achieve a durable response to the administration of the genetically engineered cells.

In some instances, the subject is likely to achieve a durable response if the peak levels of the one or more inflammatory marker is below a threshold value and the subject is not likely to achieve a durable response if the peak levels of the one or more inflammatory marker is above a threshold value; or the subject is likely to achieve a durable response if the peak level of the genetically engineered cells is within a therapeutic range between a lower threshold value and an upper threshold value and the subject is not likely to achieve a durable response if the peak level of the genetically engineered cells is below the lower threshold value or is above the upper threshold value.

In some instances, if the subject is determined not likely to achieve a durable response, further including selecting a subject for treatment with a therapeutic agent or with an alternative therapeutic treatment other than the genetically engineered cells. In some aspects, if the subject is determined as not likely to achieve a durable response, further including administering a therapeutic agent or an alternative therapeutic treatment other than the genetically engineered cells.

Described but not claimed is a method of treatment including selecting a subject having received administration of genetically engineered cells including T cells expressing a chimeric antigen receptor (CAR) in which peak levels of one or more inflammatory markers in a sample from the subject is above a threshold value; and/or peak level of T cells including a chimeric antigen receptor (CAR) in a sample from the subject is below a lower threshold value or is above an upper threshold value; and administering to the subject a therapeutic agent or alternative therapeutic treatment other than the genetically engineered cells.

In some instances, the response is a complete response (CR), objective response (OR) or partial response (PR). In some cases, the response is durable for at or greater than 3 months, 4 months, 5 months, or 6 months.

In some instances, the peak levels are assessed and/or the sample is obtained from the subject at a time that is at least 8 days, 9 days, 10 days, 11 days, 12 days, 13 days, 14 days, 15 days, 16 days, 17 days, 18 days, 19 days, 20 days or 21 days after initiation of administration of the genetically engineered cells. In some instances, the peak levels are assessed and/or the sample is obtained from the subject at a time that is between or between about 11 to 22 days, 12 to 18 days or 14 to 16 days, each inclusive, after initiation of administration of the genetically engineered cells.

In some instances, the peak level is a peak level of one or more inflammatory marker and the inflammatory marker is selected from C reactive protein (CRP), IL-2, IL-6, IL-10, IL-15, TNF-alpha, MIP-1alpha, MIP-1beta, MCP-1, CXCL10 or CCL13. In some instances, the peak level of one or more inflammatory marker is assessed and the threshold value is within 25%, within 20%, within 15%, within 10% or within 5% and/or is within a standard deviation of the median or mean of the peak level of the inflammatory marker as determined among a group of control subjects having received administration of the genetically engineered cells, wherein each of the subjects of the group did not achieve a durable response, optionally a CR and/or PR, optionally at or greater than 3 months or 6 months following administration of the genetically engineered cells. In some instances, the control subjects exhibited stable disease (SD) or progressive disease (PD) following administration of the genetically engineered cells, optionally at or greater than 3 months or 6 months following administration of the genetically engineered cells.

In some instances, the peak level is a peak level of CAR+ T cells, or a CD8+ T cell subset thereof. In some instances, the lower threshold value and upper threshold value is the lower and upper end, respectively, of a therapeutic range of peak CD3+ CAR+ T cells, or a CD8+CAR+ T cell subset thereof, in the blood among one or more subjects previously treated with the genetically engineered cells that is associated with an estimated probability of response of greater than or greater than about 65%, 70%, 75%, 80%, 85%, 90% and an estimated probability of a toxicity of less than or about 30%, 25%, 20%, 15%, 10%, 5%.

In some instances, the therapeutic range is the range in which the estimated probability of toxicity is less than 20%, less than 15%, less than 10% or less than 5% and the estimated probability of achieving a response is greater than 65%, 70%, 75%, 80%, 85%, 90%, 95% or more. In some cases, the probability of toxicity is based on a toxicity selected from any neurotoxicity or cytokine release syndrome (CRS); severe toxicity or grade 3 or higher toxicity; severe CRS or a grade 3 or higher CRS; or severe neurotoxicity, grade 2 or higher neurotoxicity or grade 3 or higher neurotoxicity. In some instances, the probability of response is based on a response that is a complete response (CR), an objective response (OR) or a partial response (PR), optionally wherein the response is durable, optionally durable for at or at least 3 months or at or at least 6 months.

In some instances, peak CAR+ T cells is determined as the number of CAR+ T cells per microliter in the blood of the subject. In some instances, the upper threshold value is between or between about 300 cells per microliter and 1000 cells per microliter or 400 cells per microliter and 600 cells per microliter, or is about 300 cells per microliter, 400 cells per microliter, 500 cells per microliter, 600 cells per microliter, 700 cells per microliter, 800 cells per microliter, 900 cells per microliter or 1000 cells per microliter; or the lower threshold value is less than or less than about 10 cells per microliter, 9 cells per microliter, 8 cells per microliter, 7 cells per microliter, 6 cells per microliter, 5 cells per microliter, 4 cells per microliter, 3 cells per microliter, 2 cells per microliter or 1 cell per microliter.

In some instances, the sample is a blood sample or plasma sample. In some instances, the method is carried out ex vivo.

In some instances, the peak level of CAR+ T cells is below a lower threshold value and the therapeutic agent is an agent that is capable of decreasing CAR+ T cell expansion or proliferation. In some cases, the agent is a steroid. In some cases, the steroid is a corticosteroid. In some examples, the steroid is dexamethasone or methylprednisolone. In some instances, the steroid is administered in an amount that is between or between about 1.0 mg and about 40 mg, between or between about 1.0 mg and about 20 mg, between or between about 2.0 mg and about 20 mg, between or between about 5.0 mg and about 25.0 mg, between or between about 10 mg and about 20 mg dexamethasone or equivalent thereof, each inclusive.

In some instances, the peak level of CAR+ T cells is above the upper threshold value and the therapeutic agent is an agent that is capable of increasing expansion of the CAR+ T cells, optionally CAR-specific expansion.

In some instances, the agent is an anti-idiotype antibody or antigen-binding fragment thereof specific to the CAR, an immune checkpoint inhibitor, a modulator of a metabolic pathway, an adenosine receptor antagonist, a kinase inhibitor, an anti-TGFβ antibody or an anti-TGFβR antibody or a cytokine.

In some of any such instances, the disease or condition is a cancer. In some cases, the cancer is a B cell malignancy. In some examples, the cancer is selected from the group consisting of sarcomas, carcinomas, lymphomas, non-Hodgkin lymphomas (NHLs), diffuse large B cell lymphoma (DLBCL), leukemia, CLL, ALL, AML and myeloma. In some instances, the cancer is a pancreatic cancer, bladder cancer, colorectal cancer, breast cancer, prostate cancer, renal cancer, hepatocellular cancer, lung cancer, ovarian cancer, cervical cancer, pancreatic cancer, rectal cancer, thyroid cancer, uterine cancer, gastric cancer, esophageal cancer, head and neck cancer, melanoma, neuroendocrine cancers, CNS cancers, brain tumors, bone cancer, or soft tissue sarcoma.

In some instances, the subject is a human.

In some instances, the CAR specifically binds to an antigen associated with a disease or condition and/or expressed in cells associated with the disease or condition. In some examples, the antigen is selected from among 5T4, 8H9, avb6 integrin, B7-H6, B cell maturation antigen (BCMA), CA9, a cancer-testes antigen, carbonic anhydrase 9 (CAIX), CCL-1, CD19, CD20, CD22, CEA, hepatitis B surface antigen, CD23, CD24, CD30, CD33, CD38, CD44, CD44v6, CD44v7/8, CD123, CD138, CD171, carcinoembryonic antigen (CEA), CE7, a cyclin, cyclin A2, c-Met, dual antigen, EGFR, epithelial glycoprotein 2 (EPG-2), epithelial glycoprotein 40 (EPG-40), EPHa2, ephrinB2, erb-B2, erb-B3, erb-B4, erbB dimers, EGFR vIII, estrogen receptor, Fetal AchR, folate receptor alpha, folate binding protein (FBP), FCRL5, FCRH5, fetal acetylcholine receptor, G250/CAIX, GD2, GD3, gp100, Her2/neu (receptor tyrosine kinase erbB2), HMW-MAA, IL-22R-alpha, IL-13 receptor alpha 2 (IL-13Ra2), kinase insert domain receptor (kdr), kappa light chain, Lewis Y, L1-cell adhesion molecule (L1-CAM), Melanoma-associated antigen (MAGE)-A1, MAGE-A3, MAGE-A6, MART-1, mesothelin, murine CMV, mucin 1 (MUC1), MUC16, NCAM, NKG2D, NKG2D ligands, NY-ESO-1, O-acetylated GD2 (OGD2), oncofetal antigen, Preferentially expressed antigen of melanoma (PRAME), PSCA, progesterone receptor, survivin, ROR1, TAG72, tEGFR, VEGF receptors, VEGF-R2, Wilms Tumor 1 (WT-1), a pathogen-specific antigen.

In some instances, the chimeric antigen receptor (CAR) contains an extracellular antigen-recognition domain that specifically binds to the antigen and an intracellular signaling domain containing an ITAM. In some cases, the intracellular signaling domain contains an intracellular domain of a CD3-zeta (CD3ζ) chain. In some instances, the chimeric antigen receptor (CAR) further contains a costimulatory signaling region. In some aspects, the costimulatory signaling region contains a signaling domain of CD28 or 4-1BB. In some instances, the costimulatory domain is a domain of4-1BB.

In some instances, the cells are T cells. In some cases, the T cells are CD4+ or CD8+. In some examples, the T cells are primary T cells obtained from a subject. In some of any such instances, the cells of the genetically engineered cells are autologous to the subject. In some instances, the cells are allogeneic to the subject.

Also described but not claimed are kits containing a composition containing genetically engineered cells including T cells expressing a chimeric antigen receptor (CAR) and instructions for administering a dose of the cells to a subject following or based on the results of assessing if peak CAR+ T cells are within a therapeutic range, wherein the therapeutic range is: (i) based upon the range of peak CD3+ CAR+ T cells, or a CD8+CAR+ T cell subset thereof, in the blood among one or more subjects previously treated with the genetically engineered cells that is associated with an estimated probability of response of greater than or greater than about 65% and an estimated probability of a toxicity of less than or about 30%; or (ii) peak CD3+CAR+ T cells in the blood, following administration of the genetically engineered cells, that is between or between about 10 cells per microliter and 500 cells per microliter; or (iii) peak CD8+CAR+ T cells in the blood, following administration of the genetically engineered cells, that is between or between about 2 cells per microliter and 200 cells per microliter. In some instances, the instructions specify that if the genetically engineered cells are not within the therapeutic range, administering an agent to the subject capable of modulating, optionally increasing or decreasing, CAR+ T cell expansion or proliferation, in the subject. In some instances, the kit further contains the agent.

Described but not claimed are kits containing an agent capable of modulating, optionally increasing or decreasing, expansion or proliferation of genetically engineered cells including CAR+ T cells in a subject, and instructions for administering the agent to a subject, said subject having been administered the genetically engineered cells, based on results of assessing if peak CAR+ T cells are within a therapeutic range, wherein the therapeutic range is (i) based upon the range of peak CD3+ CAR+ T cells, or a CD8+CAR+ T cell subset thereof, in the blood among one or more subjects previously treated with the genetically engineered cells that is associated with an estimated probability of response of greater than or greater than about 65% and an estimated probability of a toxicity of less than or about 30%; or (ii) peak CD3+CAR+ T cells in the blood, following administration of the genetically engineered cells, that is between or between about 10 cells per microliter and 500 cells per microliter; or (iii) peak CD8+CAR+ T cells in the blood, following administration of the genetically engineered cells, that is between or between about 2 cells per microliter and 200 cells per microliter. In some instances, the instructions specify that if the peak number of CAR+ T cells in the blood of the subject is less than the lowest number of peak CAR+ T cells in the therapeutic range, an agent is administered to the subject that is capable of increasing CAR+ T cell expansion or proliferation. In some instances, the agent is capable of CAR-specific expansion.

In some instances, the agent is an anti-idiotype antibody or antigen-binding fragment thereof specific to the CAR, an immune checkpoint inhibitor, a modulator of a metabolic pathway, an adenosine receptor antagonist, a kinase inhibitor, an anti-TGFβ antibody or an anti-TGFβR antibody or a cytokine. In some instances, if the peak number of CAR+ T cells in the blood of the subject is greater than the highest number of peak CAR+ T cells in the therapeutic range, an agent is administered to the subject that is capable of decreasing CAR+ T cell expansion or proliferation.

Described but not claimed are kits containing an agent capable of decreasing expansion or proliferation of genetically engineered cells including CAR+ T cells in a subject, and instructions for assessing a subject the level, amount or concentration of a volumetric measure of tumor burden or an inflammatory marker in a sample from the subject and administering to the subject the agent if the level, amount or concentration is at or above a threshold level, wherein the sample does not contain genetically engineered T cells expressing a chimeric antigen receptor (CAR) and/or is obtained from the subject prior to receiving administration of genetically engineered T cells expressing a CAR. In some instances, the volumetric measure is a sum of the products of diameters (SPD), longest tumor diameters (LD), sum of longest tumor diameters (SLD), tumor volume, necrosis volume, necrosis-tumor ratio (NTR), peritumoral edema (PTE), and edema-tumor ratio (ETR). In some cases, the volumetric measure is a sum of the products of diameter (SPD).

In some instances, the inflammatory marker is C-reactive protein (CRP), erythrocyte sedimentation rate (ESR), albumin, ferritin, β2 microglobulin (β2-M), lactate dehydrogenase (LDH), a cytokine or a chemokine. In some examples, the inflammatory marker is LDH.

In some instances, the agent is a steroid. In some cases, the steroid is a corticosteroid. In some examples, the steroid is dexamethasone or methylprednisolone. In some instances, the steroid is formulated for administration in an amount that is between or between about 1.0 mg and about 40 mg, between or between about 1.0 mg and about 20 mg, between or between about 2.0 mg and about 20 mg, between or between about 5.0 mg and about 25.0 mg, between or between about 10 mg and about 20 mg dexamethasone or equivalent thereof, each inclusive.

In some instances, the CAR specifically binds to an antigen associated with a disease or condition and/or expressed in cells associated with the disease or condition. In some instances, the genetically engineered cells include T cells, optionally CD4+ or CD8+ T cells.

Also described but not claimed are articles of manufacture containing any of the kits according to the disclosure.

### Brief Description of the Drawings

**FIG. 1** shows an estimated probability curve of response and an estimated probability of developing Grade 3-5 neurotoxicity constructed based on the number of CD4+/truncated receptor+ or CD8+/truncated receptor+ CAR-T cells in the blood.
**FIG. 2A** shows the number of CD3⁺/CAR⁺ T cells in peripheral blood measured at certain time points post-infusion for subjects grouped by best overall response.
**FIGS. 2B-2D** show CD3⁺/CAR⁺ T cells, CD4⁺/CAR⁺ T, and CD8⁺/CAR⁺ T cell levels in peripheral blood measured at certain time points post-infusion for subjects who achieved a response, grouped by continued response at 3 months.
**FIG. 3** shows the percentage of subjects who experienced laboratory abnormalities and treatment-emergent adverse events (TEAEs) that occurred in ≥20% of subjects. ^{∗}: One Grade 5 AE of multi-organ failure unrelated to study treatment and due to progression of lymphoma; †: One Grade 5 AE of diffuse alveolar damage, investigator assessed as related to fludarabine, cyclophosphamide, and CAR T cell therapy, occurred on day 23 in a subject who refused mechanical ventilation for progressive respiratory failure while neutropenic on growth factors and broad spectrum antibiotics and antifungals
**FIG. 4** is a Kaplan meier curve depicting observed time to onset of CRS and neurotoxicity.
**FIG. 5A** and **FIG. 5B** depict response rates among subgroups of treated subjects.
**FIG. 6A** and **FIG. 6B** show the duration of response (CR/PR, CR or PR) and overall survival in the full and core cohort of subjects.
**FIG. 7A** shows the pharmacokinetics of the CAR⁺ T cells in peripheral blood at various time points post-treatment at different dose levels.
**FIG. 7B** shows the pharmacokinetics of the CAR⁺ T cells in peripheral blood at various time points post-treatment between responders and nonresponders.
**FIG. 7C** shows the pharmacokinetics of the CAR⁺ T cells in peripheral blood at various time points post-treatment in subjects that did or did not develop any neurotoxicity.
**FIG. 8** shows levels of analytes measured in the serum of subjects prior to administration of the CAR+ T cells and correlation to the development of neurotoxicity.
**FIG. 9** shows a graph plotting progression-free time (months) and indicating best overall response and response durability, and individual clinical outcomes observed over time in individual subjects within a Full cohort and a Core cohort of NHL subjects treated with an anti-CD19 cell therapy containing CAR-T-expressing CD4+ and CD8+ T cells. ^{a} : Patients achieved BOR at month 1 except where otherwise noted; ^{b} :Complete resolution of CNS involvement by lymphoma observed in 2 patients; ^{c} : One patient re-expanded after biopsy upon disease progression.

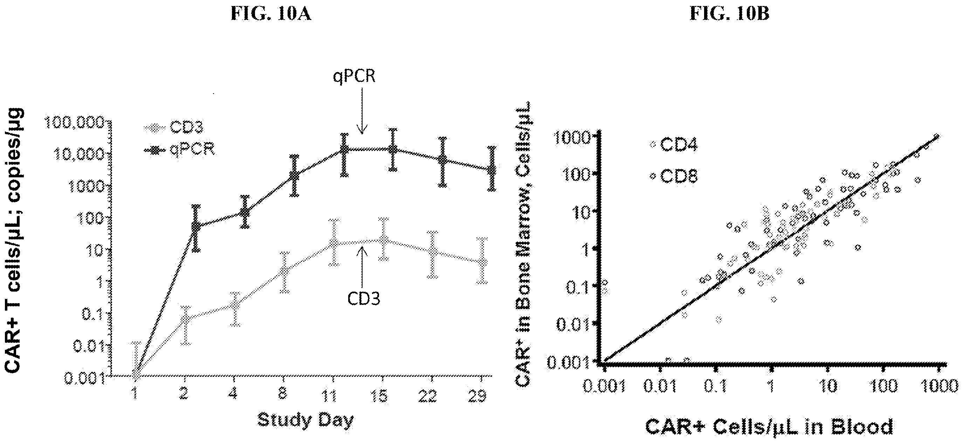

**FIG. 10A** depicts the median (±quartiles) number of CAR-expressing CD3+ cells/µL blood, assessed by flow cytometry using an antibody specific for a truncated receptor (CD3, circle; N=87); or median (±quartiles) number of copies integrated CAR transgene/µg genomic DNA, assessed by quantitative polymerase chain reaction (qPCR) using primers specific for a woodchuck hepatitis virus post-transcriptional regulatory element (WPRE) present in the vector encoding the CAR (qPCR, square; N=85) in blood samples from 87 subjects that have been administered anti-CD19 CAR-expressing cells. The cutoff for CAR+ cell detection in flow cytometry was set at ≥ 25 events in the CAR+ gate, and limit of detection for qPCR was ≥ 12.5 copies of CAR transgene per µg of genomic DNA.
**FIG. 10B** depicts the relative numbers of CD4⁺ and CD8⁺CAR-expressing cells/µL in blood and bone marrow samples from 67 subjects that have been administered anti-CD19 CAR-expressing cells, on day 11 ± 3 days. The line represents the line of unity and is not a regression line.
**FIGS. 11A** and **11B** depict the median (±quartiles) area under the curve between days 0 and 28 (AUC₀₋₂₈; **FIG. 11A**) and maximum serum concentration (Cₘₐₓ; CAR⁺ cells/µL blood; **FIG. 11B**) of CD4+ and CD8+ CAR+ cells in subject subgroups with diffuse large B-cell lymphoma de novo or transformed from indolent lymphoma (DLBCL, NOS; N=27), transformed follicular lymphoma (tFL; N=10), DLBCL transformed from marginal zone lymphoma or chronic lymphocytic leukemia (tMZL/tCLL; N=4), or mantle cell lymphoma (MCL; N-5), who have received CAR-expressing T cells at DL1.
**FIGS. 12A** and **12B** depict the median (±quartiles) area under the curve between days 0 and 28 (AUC₀₋₂₈; **FIG. 12A**) and maximum serum concentration (Cₘₐₓ; CAR⁺ cells/µL blood; **FIG. 12B**) of CD3+, CD4+ and CD8+ CAR+ cells in subjects who have received CAR+ cells at DL1 or DL2.
**FIGS. 13A-13D** depict the median (±quartiles) number of CAR-expressing CD4+ and CD8+ CAR⁺ cells/µL blood over time, in subjects that developed cytokine release syndrome (any CRS) compared to subjects that have not developed CRS (no CRS) (CD4+: **FIG. 13A****;** CD8+: **FIG. 13B**) or in subjects that developed neurotoxicity (any NT) compared to subjects that have not developed NT (no NT) (CD4+: **FIG. 13C****;** CD8+: **FIG. 13D****).**
**FIG. 14** depicts the number of peak CD3⁺ CAR⁺ cells/µL (CD3+ Cₘₐₓ) in subjects grouped by subjects who had the best overall response (BOR) of CR, PR or PD.
**FIG. 15A** depicts pre-lymphodepletion blood analyte levels in serum samples from subjects that exhibited high CAR+ cell expansion (CD3+ Cₘₐₓ > 500) and subjects that exhibited low CAR+ cell expansion (CD3+ Cₘₐₓ < 500).
**FIG. 15B** depicts the peak blood analyte levels in serum samples from subjects that exhibited high CAR+ cell expansion (CD3+ Cₘₐₓ > 500) and subjects that exhibited low CAR+ cell expansion (CD3+ Cₘₐₓ < 500).
**FIG. 16** depicts a plot depicting pre-lymphodepletion SPD (cm²) against AUC₀₋₂₈ (cells^{∗}day/µL) of CD3+ CAR+ cells, for individual subjects administered DL1 or DL2 of CAR+ cells.
**FIGS. 17A** and **17B** depict pre-lymphodepletion blood analyte levels in serum samples from subjects that developed cytokine release syndrome (CRS grade 1-4) compared to subjects that have not developed CRS (CRS grade 0) (**FIG. 17A**) or in subjects that developed neurotoxicity (NT grade 0) compared to subjects that have not developed NT (NT grade 1-4) (**FIG. 17B**)**.** The units were: Ferritin and D-dimer (µg/L); CRP (mg/L) and cytokines (pg/mL).
**FIG. 18** depicts the assessment of pre-lymphodepletion patient parameter sum of product dimensions (SPD; cm²), indicative of tumor burden, and lactate dehydrogenase (LDH; U/L) level, in subjects that developed cytokine release syndrome (any CRS) compared to subjects that have not developed CRS (no CRS) or in subjects that developed neurotoxicity (any NT) compared to subjects that have not developed NT (no NT).

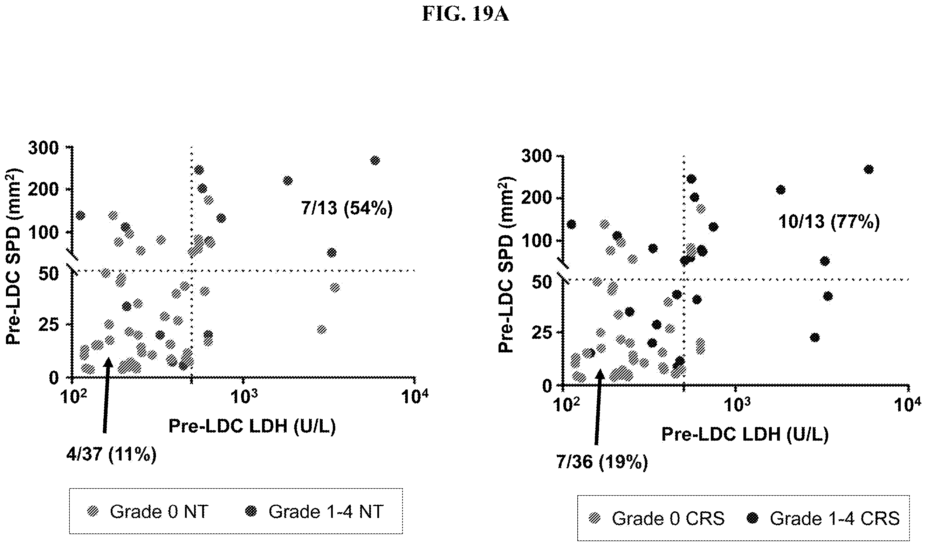

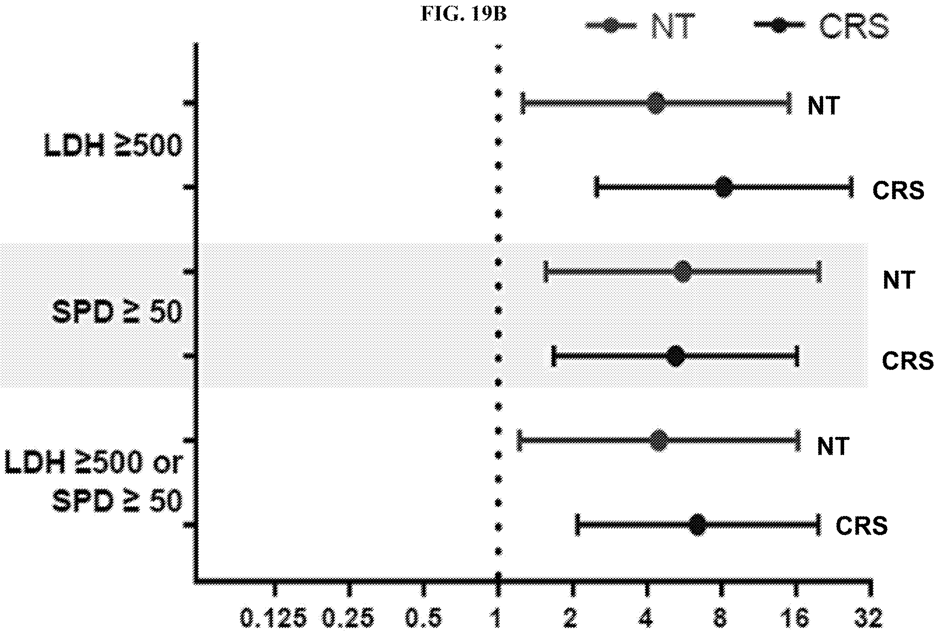

**FIG. 19A** is a plot depicting pre-lymphodepletion SPD (cm²) against pre-lymphodepletion LDH (U/L) levels, in individuals that have developed neurotoxicity (Grade 1-4 NT) or subjects that have not developed NT (Grade 0 NT) (left panel), and in individuals that have developed CRS (Grade 1-4 CRS) or subjects that have not developed CRS (Grade 0 CRS) (right panel). Dotted lines represent levels of SPD (50 cm² or higher) or LDH (500 or higher) that is associated with higher rates of CRS or NT. **FIG. 19B** depicts the odds ratio estimates for developing CRS or NT based on the levels of SPD (50 cm² or higher) or LDH (500 or higher), with 95% confidence intervals (CI).
**FIG. 20** depicts pre-lymphodepletion tumor burden parameter (SPD) and blood analyte levels in for subjects that had a durable response at 3 months versus for subjects that did not have a response at 3 months. The units were: Ferritin and D-dimer (µg/L); CRP and SAA-1 (mg/L) and cytokines (pg/mL).
**FIGS. 21A** and **21B** depict peak blood analyte levels in serum samples from subjects that developed cytokine release syndrome (any CRS) compared to subjects that have not developed CRS (no CRS) (**FIG. 21A**) or in subjects that developed neurotoxicity (any NT) compared to subjects that have not developed NT (no NT) (**FIG. 21B**)**.** The units were: CRP (mg/L), SAA-1 (mg/L) and cytokines (pg/mL).
**FIG. 22A** depicts peak blood analyte levels in serum samples from subjects that had a best overall response (BOR) of complete response (CR) or partial response (PR) (N=57) compared to levels in subjects that had stable disease (SD) or progressive disease (PD) (N=17). **FIG. 22B** depicts peak blood analyte levels in serum samples from subjects that had a 3-month response of SD/PD (N=31), compared to subjects who had a 3-month response CR/PR (N=35). The units were: CRP (mg/L), SAA-1 (mg/L) and cytokines (pg/mL).
**FIGS. 23A-23C** depict estimated probability curves for response, toxicity and durable response outcomes, based on the maximum serum concentration of CD3+ (**FIG. 23A**), CD4+ (**FIG. 23B**) or CD8+ (**FIG. 23C**) CAR-expressing cells (Cₘₐₓ; cells/µL blood). The estimated probability curves for overall response rate (ORR; including subjects with complete response (CR) and partial response (PR)), 3-month response (M3 response; including CR and PR at month 3 after administration), any NT, any CRS, Grade 3-4 NT, Grade 3-5 NT or Grade 2-5 CRS.

### Detailed Description

Embodiments of the invention are described in some of the instances and aspects below.

### I. METHOD FOR DETERMINING THERAPEUTIC DOSAGE RANGE

Among the instances described herein as part of the disclosure are methods, uses, compositions and articles of manufacture involving and related to the administration of cell therapies such as those including engineered cells to subjects having or suspected of having a disease or condition, such as those specifically recognized by the cells of the therapy. The described instances in some aspects relate to dosing a subject, e.g., administering a particular dose of the cell therapy to the subject, such as administering a dose that is or is suspected of being within a therapeutic dosage range and/or window, which generally is a range and/or window that achieves or is likely to achieve a desired level of the engineered cells in a sample, fluid, tissue, organ or location of the subject.

Adoptive cell therapies (including those involving the administration of cells expressing chimeric receptors specific for a disease or disorder of interest, such as chimeric antigen receptors (CARs) and/or other recombinant antigen receptors, as well as other adoptive immune cell and adoptive T cell therapies) can be effective in the treatment of cancer and other diseases and disorders. In certain contexts, available approaches to adoptive cell therapy may not always be entirely satisfactory. In some contexts, optimal response to therapy can depend on the ability of the administered cells to recognize and bind to a target, *e.g.,* target antigen, to traffic, localize to and successfully enter appropriate sites within the subject, tumors, and environments thereof, to become activated, expand, to exert various effector functions, including cytotoxic killing and secretion of various factors such as cytokines, to persist, including long-term, to differentiate, transition or engage in reprogramming into certain phenotypic states (such as effector, long-lived memory, less-differentiated, and effector states), to provide effective and robust recall responses following clearance and re-exposure to target ligand or antigen, and avoid or reduce exhaustion, anergy, terminal differentiation, and/or differentiation into a suppressive state.

In some aspects, the therapeutic effect of adoptive cell therapy may be limited by the development of toxicity in the subject to whom such cells are administered, which toxicity in some cases can be severe, at certain doses or exposure of administered cells. In some cases, while a higher dose of such cells can increase the therapeutic effect, for example, by increasing exposure to the cells such as by promoting expansion and/or persistence, they may also result in an even greater risk of developing a toxicity or a more severe toxicity. Also, in some cases, subjects with a higher disease burden also may be at a greater risk for developing a toxicity or a more severe toxicity. Certain available methods for dosing subjects cell therapy may not always be entirely satisfactory. Increasing a dose of cells or promoting expansion or proliferation of administered cells in the subject can be related to higher response rates, but also an increase in development of toxicity.

The described methods offer advantages over available approaches in determining the dose of the cell therapy. The described methods permit administering a dose to a subject that is or is suspected of being within a therapeutic dosage range and/or window, which generally is a range and/or window that achieves or is likely to achieve a desired level of the engineered cells in the subject. The described methods permit dosing of cells that can achieve or can be associated with associated with a high or specified desired degree of likelihood of a treatment outcome such as a favorable outcome or response and/or a durable response or outcome, and also associated with a relatively low or minimized or desired degree of likelihood of risk of developing a toxic outcome or toxicity following administration to the subject of the cell therapy. The described methods also offer advantages over available approaches by permitting modulation, modification and/or alteration of cell therapy if the subject is determined to be not likely to achieve a response and/or a durable response, thereby optimizing the response without substantially increasing the risk of toxicity. In some instances, pharmacokinetic parameters, patient attributes, tumor burden and/or expression of biomarkers, such as inflammatory markers can be used to determine likelihood of response and/or any need for modulating, modifying or altering the therapy, to achieve greater response or more durable response, without substantially increasing the risk of toxicity.

In some instances, the therapeutic dosage range and/or window achieves or is likely to achieve a desired level of the engineered cells, e.g., CAR T cells, that, in some aspects, is a peak level, which generally refers to the maximum number, concentration or percentage of the cells observed or measured in the relevant sample, fluid, tissue, organ or other location following treatment or within a certain period following treatment. In some aspects, the level may be a number, concentration or percentage (such as number of the cells per weight or volume or area or total cell number) or exposure of the subject or tissue or organ or fluid or location to the cells, at a given time or over a period of time. In some aspects, the level is an area under the curve (AUC) with respect to a plot of the number or percentage or other readout of the relevant cells in the tissue or sample or fluid or organ or other location, over a given period of time following treatment or administration of the cells or initiation thereof.

In some examples, the level is expressed as CAR+ cell concentration (e.g., cells/µl) in the blood, AUC of a curve of CAR+ cells/volume (e.g., CAR+ cells/microliter) over a period of time, maximum or peak CAR+ cells/volume (e.g., CAR+ cells/microliter) in the blood following treatment, or CAR+ cells/microliter of blood at day 1, 2, 3, 4, 5, 6, 7, 8, 9, 10, 11, 12, 13, 14, 15, 16, 17, 18, 19, 20 or 21, or week 1, 2, 3, 4, 5, 6, 7, 8, 9, 10, 11, or 12 or more post-treatment or initiation thereof. In some instances, the desired level is within, or is a level within, a determined therapeutic range.

In some instances, the therapeutic range is a therapeutic range and/or window associated with a high or specified desired degree of likelihood of a treatment outcome such as a favorable outcome or response and/or a durable response or outcome, and also associated with a relatively low or minimized or desired degree of likelihood of risk of developing a toxic outcome or toxicity following administration to the subject of the cell therapy, e.g., the engineered cells. In some aspects, the toxicity or toxic outcome is cytokine release syndrome (CRS) or neurotoxicity (NT). In some aspects, the toxicity or toxic outcome is any CRS or grade 1 or higher CRS or any neurotoxicity or grade 1 or higher neurotoxicity. In some aspects, the toxicity or toxic outcome is severe CRS or grade 3 or higher CRS or severe neurotoxicity or grade 3 or higher neurotoxicity. In some cases, risk of toxicity is correlated to disease burden, dose of cells, expansion of cells, and the pharmacokinetic (PK) of the cells, e.g., cell exposure or peak cell concentration. Yet, at the same time to maximize response, in some cases, a higher or greater dose of cells, exposure of cells or peak concentration of cells is required. In some aspects, however, it is found herein that probability of durable response, e.g., response that persists after a period of time from initiation of therapy, can increase with higher or greater dose of cells, exposure of cells or peak concentration of cells, up to a certain dose, exposure or concentration; then can decrease. It is found herein, from probability curves for toxicity (e.g. CRS or neurotoxicity, severe CRS or severe severe neurotoxicty) and response (e.g. marrow response) and/or durable response generated from a population of subjects treated with CAR+ T cells, that there is a therapeutic range and/or window, e.g. widest range between curves, at which a dose can be determined to maximize estimated probability of response or durable response and minimize estimated risk of toxicity. In some instances, such probability curves can be used in methods to choose or to determine a dose of cells to administer to a subject. In some instances, such probability curves can be used in methods to modify the dose of cells and/or to modulate the expansion and/or activity of cells, e.g., by administering an agent and/or intervention that affects cell expansion, activity and/or function.

In some instances, the described methods include administering to the subject a dose of cells engineered with a chimeric antigen receptor (CAR), wherein the dose is sufficient to achieve peak CAR+ cells/µl within a determined therapeutic range and/or an exposure (e.g., AUC) within a determined therapeutic range, wherein the therapeutic range is determined based upon the estimated probability of a response outcome (e.g. marrow response) and/or durable response, e.g., response at 3 months, and the estimated probability of a toxic outcome (e.g. grade 3-5 neurotoxicity).

In some instances, the estimated probability is determined from a probability curve generated based on results or outcomes from a population of subjects, such as at least 10, 25, 50, 100, 150, 300, 400, 500 or more subjects. In some instances, the population of subjects is diseased subjects, such as subjects having a disease or condition, such as a tumor or cancer. In some instances, the population of subjects is or includes subjects that are likely to or are candidates or who are or have been receiving treatment with genetically engineered cells, e.g. CAR-T cells, for treating the disease or condition. In some instances, the subject has a sarcoma, a carcinoma or a lymphoma, optionally a non-Hodgkin lymphomas (NHLs), diffuse large B cell lymphoma (DLBCL), leukemia, chronic lymphocytic leukemia (CLL) , acute lymphoblastic leukemia (ALL), acute myeloid leukemia (AML) and myeloma. In some instances, the subject has CLL. In some instances, a first probability curve is generated for risk of a toxic outcome (e.g., CRS or neurotoxicity, such as grade 3-5 neurotoxicity) and a second probability curve is generated for a response outcome (e.g. marrow response). In some instances, a first probability curve is generated for risk of a toxic outcome (e.g., CRS or neurotoxicity, such as grade 3-5 neurotoxicity) and a second probability curve is generated for durable response outcome. In some instances, the probability curves are transformed or provided as a Sigmoidal curve.

In some instances, the estimated probability of toxicity (e.g. CRS or neurotoxicity, such as grade 3-5 CRS or neurotoxicity) and/or estimated probability of response (e.g. marrow response) or durable response (e.g., response at 3 months) is correlated to peak CAR+ cell concentration (cells/µl) in a biological sample, such as in blood. In some instances, the CAR+ cells are or comprise T cells, e.g., are or comprise CD3+ T cells. In some instances, the T cells are CD4+ or CD8+ T cells. In some instances, the administered composition comprises CD4+ and CD8+ CAR+ T cells and the probability curves are generated separately for the CD4+ cells and for the CD8+ cells and/or for CD3+ cells.

In some instances, the described methods include a method of dosing a subject comprising administering to the subject a dose of cells engineered with a recombinant receptor, such as an antigen receptor, e.g. chimeric antigen receptor (CAR), wherein the dose is sufficient to achieve peak CAR+ cells/µl within a determined therapeutic range, wherein the therapeutic range is determined based upon the estimated probability of a response outcome (e.g. marrow response) and/or durable response (e.g., response at 3 months) and the estimated probability of a toxic outcome (e.g. CRS or neurotoxicity, such as grade 3-5 neurotoxicity). In some instances, the estimated probability of causing toxicity is less than 35%, less than 30%, less than 25%, less than 20%, less than 15%, less than 10% or less than 5% on the toxicity probability curve. In some instances, the estimated probability of achieving a response is greater than 65%, 70%, 75%, 80%, 85%, 90%, 95% or more. In some instances, the estimated probability of achieving a durable response, e.g., a response at 3 months, is greater than 65%, 70%, 75%, 80%, 85%, 90%, 95% or more. In some instances, the toxicity is CRS, such as any CRS, such as grade 1 or higher CRS, or neurotoxicity, such as any neurotoxicity, such as grade 1 or higher neurotoxicity. In some instances, the severe toxicity is severe CRS or grade 3 or higher CRS or severe neurotoxicity or grade 3 or higher neurotoxicity. In some cases, the response is a marrow response. In some instances, response is assessed using IgH deep sequencing. In some instances, the toxicity outcome is severe neurotoxicity or grade 3 or higher neurotoxicity, such as grade 3-5 neurotoxicity.

Also described is a method of dosing by administering, to a subject having a disease or condition (e.g. tumor or cancer), a dose of cells, and monitoring the subject post-infusion for peak CAR+ cells/µl, such as at one or more various time points, e.g. at or about or greater than 3 days, 7 days, 14 days, 28 days, 2 months, 3 months, 4 months, 5 months, 6 months, 1 year, 2 years or more after infusion with the cell therapy, or AUC over time, such as up to one or more time point after administration, e.g., up to or up to about or greater than 3 days, 7 days, 14 days, 28 days, 2 months, 3 months, 4 months, 5 months, 6 months, 1 year, 2 years or more after infusion with the cell therapy. In some instances, the method can include determining or assessing the probability the peak CAR+ cells/µl are in the therapeutic range, such as determined form a toxicity probability curve and/or response probability curve and/or a durable response probability curve. In some instances, if the peak CAR+ cells/µl or AUC is not in the therapeutic range, the method further involves administering a compound or agent to enhance or boost CAR+ cell expansion *in vivo* such that the peak CAR+ expansion is within the therapeutic range, such as determined by the described methods and/or to reduce, inhibit, prevent and/or delay CAR+ T cell activity and/or expansion.

Also described is a method of dosing, to a subject having a disease or condition (e.g. tumor or cancer), by administering to the subject a sub-optimal dose of cells, wherein the dose is insufficient to achieve peak CAR+ cells/µl within a determined therapeutic range. In some instances, the method further involves administering a compound or agent to enhance or boost CAR+ cell expansion *in vivo* such that the peak CAR+ expansion is within the therapeutic range, such as determined by the described methods.

In some instances, the method further involves administering to the subject a second dose of cells based on the response and toxicity probability curves for peak CD3+, CD4+ and/or CD8+ CAR+T cell concentration (cells/µl) and/or AUC, e.g., peak CD8+ CAR+ T cell concentrations. In some instances, the method further involves administering to the subject a tumor microenvironment (TME) targeting agent based on the response and toxicity probability curves for peak CD3+, CD4+ and/or CD8+ CAR+T cell concentrations (cells/µl) and/or AUC, e.g., peak CD8+ CAR+ T cell concentrations. In some aspects, the method allows the selection of a dosing range that achieves a more durable response and/or remission. Also described are methods that involve assessing, determining or monitoring pharmacokinetic parameters, such as maximum (peak) plasma concentration (Cₘₐₓ) and area under the curve (*i.e.* the area under the curve generated by plotting time versus plasma concentration of the therapeutic agent CAR+ T cells; AUC) of administered cells in the subject. In some instances, such assessments can be used to determine whether the administered cells are within a therapeutic range or window. In some instances, such assessments can be used as an indicator to modulate, modify and/or alter therapy, e.g., by administering agents capable of modulating the expansion, proliferation and/or activity of the administered CAR+ T cells, administer additional and/or modified doses, and/or administer alternative therapy. In some instances, also described are methods of administering a therapeutic agent accordingly. In some instances, such assessments can be used to monitor the progress of the therapy and/or to assess the effect of modulated therapy. In some instances, such measurements can be used to assess the likelihood of a response or a durable response.

Also described are methods that involve assessing, determining or monitoring other parameters, such as patient attributes, tumor burden and/or expression of biomarkers, such as inflammatory markers. In some instances, the assessment can be performed using a sample from the subject obtained prior to administration of the cell therapy or initiation thereof. In some instances, the assessment can be performed using a sample from the subject obtained after administration of the cell therapy or initiation thereof. In some instances, such assessments can be used to determine whether the administered cells are likely to be, or is likely to correlate with or associate with being, within a therapeutic range or window. In some instances, such assessments can be used as an indicator to modulate, modify and/or alter therapy, e.g., by administering agents capable of modulating the expansion, proliferation and/or activity of the administered CAR+ T cells, administer additional and/or modified doses, and/or administer alternative therapy. In some instances, also described are methods of administering a therapeutic agent accordingly. In some instances, such assessments can be used to monitor the progress of the therapy and/or to assess the effect of modulated therapy. In some instances, such measurements can be used to assess the likelihood of a response or a durable response.

### II. TOXICITY AND RESPONSE PROBABILITY CURVES

In some instances, probability curves from a population of subjects as described are generated and correlated with the risk of toxic outcome (e.g. CRS or neurotoxicity, e.g., grade 3-5 neurotoxicity) or response (e.g. marrow response), and/or durability of response (e.g., month 3 response). In some instances, the information regarding toxic outcome and response outcome as described above are combined and/or correlated with data collected regarding peak cell levels and/or concentrations, or exposure (e.g., AUC) in the subject. In some instances, the information about toxic outcome and response outcome are collected from a cohort of subjects, each correlated with cell level data (*e.g.,* peak number or concentration of CAR+ T cells), and independently assessed. In some instances, for example, the toxic outcome data are collected and assessed with CAR+ cell numbers to construct a toxicity probability curve. In some cases, the response outcome data, including data for durable response outcomes, are collected and assessed with CAR+ cell numbers to construct a response probability curve and/or a durable response probability curve.

In some instances, the resulting toxicity and response and/or durable response probability curves can be jointly assessed, such as assessed in parallel or at around the same time or substantially the same time, to inform the dosing decisions or adaptive treatments of subjects.

In some instances, toxic outcome and response outcome are used to construct an estimated probability curve of response and an estimated probability of developing toxicity based on the number, concentration and/or exposure of CAR+ T cells in the blood. In some cases, the estimated probability of achieving a response is greater than 65%, 70%, 75%, 80%, 85%, 90%, 95% or more. In some cases, the estimated probability of achieving a durable response, e.g., a 3- or 6-month durable response, is greater than 65%, 70%, 75%, 80%, 85%, 90%, 95% or more. In some cases, the estimated probability of causing or resulting in toxicity is less than 35%, less than 30%, less than 25%, less than 20%, less than 15%, less than 10% or less than 5% on the toxicity probability curve.

In some instances, the methods involve administering a sufficient number or dose of cells to achieve a peak CAR+ cell concentration in the subject that is within a determined target therapeutic range or window. In some instances, the methods involve administering a sufficient number or dose of cells to achieve a peak CAR+ cell concentration in a majority of subjects so treated by the method, or greater than or greater than about 50%, 60%, 70%, 75%, 80%, 85%, 90% or 95% or more, such as greater than 75% of the subjects so treated by the method, is within a determined target therapeutic range or window. In the described methods, one or more therapeutic outcomes or events associated with toxicity (toxic outcome) and one or more therapeutic outcomes or events associated with efficacy (response outcome, including durable response outcome) of the therapeutic agent is assessed and dosing decisions are made in accord with the described methods. In some instances, the information regarding toxic outcome and response outcome are combined and/or correlated with data collected regarding peak cell levels, concentrations and/or exposure in the subject. In some instances, the information about toxic outcome and response outcome are collected from a cohort of subjects, each correlated with cell level data, and independently assessed. In some instances, for example, the toxic outcome data are collected and assessed to construct a toxicity probability curve and the response outcome data are collected and assessed to construct a response probability curve. In some instances, durable response outcome data (e.g., durable response at 3, 6, 9 or 12 months) are collected and assessed to construct a durable response probability curve.

In some instances, the toxicity and response probability curves can be jointly assessed, such as assessed in parallel or at around the same time or substantially the same time, to inform the dosing decisions or adaptive treatments of subjects.

In some instances, the toxic outcome and response outcome are monitored at a time at which a toxicity outcome and a response outcome are present. The particular time at which such outcome may be present will depend on the particular therapeutic agent and is known to a skilled artisan, such as a physician or clinician, or is within the level of such a skilled artisan to determine. In some instances, the time at which a toxic outcome or response outcome is assessed is within or within about a period of time in which a symptom of toxicity or efficacy is detectable in a subject or at such time in which an adverse outcome associated with non-response or toxicity is not detectable in the subject. In some instances, the time period is near or substantially near to when the toxic outcome and/or response outcome has peaked in the subject. In some instances, the time period includes time required for assessing durability of response, e.g., durable response at 3, 6, 9 or 12 months after first administration of the cells.

In some instances, the toxic outcome or response outcome can be assessed in the subject at a time that is within or about within 120 days after initiation of the first dose of the therapeutic agent to the subject, within or within about 90 days after initiation of the first dose, within or within about 60 days after initiation of the first dose of the therapeutic agent or within or within about 30 days after initiation of the first dose to a subject. In some instances, the toxic outcome or response can be assessed in the subject within or within about 6 days, 12 days, 16 days, 20 days, 24 days, 28 days, 32 days, 36 days, 40 days, 44 days, 48 days, 52 days, 56 days, 60 days, 64 days, 68 days, 72 days, 76 days, 80 days, 84 days, 88 days, 92 days, 96 days or 100 days after initiation of the first dose to a subject.

In some instances, the toxic outcome or response outcome is present or can be assessed or monitored at such time period where only a single dose of the therapeutic agent is administered. In the context of adoptive cell therapy, administration of a given "dose" encompasses administration of the given amount or number of cells as a single composition and/or single uninterrupted administration, e.g., as a single injection or continuous infusion, and also encompasses administration of the given amount or number of cells as a split dose, provided in multiple individual compositions or infusions, over a specified period of time, which is no more than 3 days. Thus, in some contexts, the first dose is a single or continuous administration of the specified number of cells, given or initiated at a single point in time. In some contexts, however, the first dose is administered in multiple injections or infusions over a period of no more than three days, such as once a day for three days or for two days or by multiple infusions over a single day period.

The term "split dose" refers to a dose that is split so that it is administered over more than one day. This type of dosing is encompassed by the present methods and is considered to be a single dose.

As used herein, "first dose" is used to describe the timing of a given dose, which, in some cases can be the only dose or can be followed by one or more repeat or additional doses. The term does not necessarily imply that the subject has never before received a dose of a therapeutic agent even that the subject has not before received a dose of the same or substantially the same therapeutic agent.

In some instances, the toxic outcome or response outcome is present and/or can be assessed or monitored at such time period that is after a first cycle of administration of the therapeutic agent, after a second cycle of administration of the therapeutic agent, after a third cycle of administration of the therapeutic agent, or after a fourth cycle of administration of the therapeutic agent. In some instances, a cycle of administration can be a repeated schedule of a dosing regimen that is repeated over successive administrations. In some instances, a schedule of administration can be daily, every other day, or once a week for one week, two weeks, three weeks or four weeks (e.g. 28 days).

In some instances, the toxic outcome and response outcome can be assessed by monitoring one or more symptoms or events associated with a toxic outcome and one or more symptoms or events associated with a response outcome. In some instances, the disease or condition is a tumor or cancer.

### A. Toxicity Outcome

In some instances, a toxic outcome in a subject to administration of a therapeutic agent (e.g. CAR T-cells) can be assessed or monitored. In some instances, the toxic outcome is or is associated with the presence of a toxic event, such as cytokine release syndrome (CRS), severe CRS (sCRS), macrophage activation syndrome, tumor lysis syndrome, fever of at least at or about 38 degrees Celsius for three or more days and a plasma level of C-reactive protein (CRP) of at least at or about 20 mg/dL, neurotoxicity and/or severe neurotoxicity. In some instances, the toxic outcome is a sign, or symptom, particular signs, and symptoms and/or quantities or degrees thereof which presence or absence may specify a particular extent, severity or level of toxicity in a subject. It is within the level of a skilled artisan to specify or determine a particular sign, symptom and/or quantities or degrees thereof that are related to an undesired toxic outcome of a therapeutic agent (e.g. CAR- T cells).

In some instances, the toxic outcome is an indicator associated with the toxic event. In some instances, the toxic outcome is the presence or absence of one or more biomarkers or the presence of absence of a level of one or more biomarkers. In some instances, the biomarker is a molecule present in the serum or other bodily fluid or tissue indicative of cytokine-release syndrome (CRS), severe CRS or CRS-related outcomes. In some instances, the biomarker is a molecule present in the serum or other bodily fluid or tissue indicative of neurotoxicity or severe neurotoxicity.

In some instances, the subject exhibits toxicity or a toxic outcome if a toxic event, such as CRS-related outcomes, e.g. if a serum level of an indicator of CRS or other biochemical indicator of the toxicity is more than at or about 10 times, more than at or about 15 times, more than at or about 20 times, more than at or about 25 times, more than at or about 50 times, more than at or about 75 times, more than at or about 100 times, more than at or about 125 times, more than at or about 150 times, more than at or about 200 times, or more than at or about 250 times the baseline or pre-treatment level, such as the serum level of the indicator immediately prior to administration of the first dose of the therapeutic agent.

In some aspects, the toxic outcome is or is associated with or indicative of cytokine release syndrome (CRS) or severe CRS (sCRS). CRS, e.g., sCRS, can occur in some cases following adoptive T cell therapy and administration to subjects of other biological products. See Davila et al., Sci Transl Med 6, 224ra25 (2014); Brentjens et al., Sci. Transl. Med. 5, 177ra38 (2013); Grupp et al., N. Engl. J. Med. 368, 1509-1518 (2013); and Kochenderfer et al., Blood 119, 2709-2720 (2012); Xu et al., Cancer Letters 343 (2014) 172-78.

Typically, CRS is caused by an exaggerated systemic immune response mediated by, for example, T cells, B cells, NK cells, monocytes, and/or macrophages. Such cells may release a large amount of inflammatory mediators such as cytokines and chemokines. Cytokines may trigger an acute inflammatory response and/or induce endothelial organ damage, which may result in microvascular leakage, heart failure, or death. Severe, life-threatening CRS can lead to pulmonary infiltration and lung injury, renal failure, or disseminated intravascular coagulation. Other severe, life-threatening toxicities can include cardiac toxicity, respiratory distress, neurologic toxicity and/or hepatic failure.

In the context of administering CAR-expressing cells, CRS typically occurs 6-20 days after infusion of cells that express a CAR. See Xu et al., Cancer Letters 343 (2014) 172-78. In some cases, CRS occurs less than 6 days or more than 20 days after CAR T cell infusion. The incidence and timing of CRS may be related to baseline cytokine levels or tumor burden at the time of infusion. Commonly, CRS involves elevated serum levels of interferon (IFN)-γ, tumor necrosis factor (TNF)-α, and/or interleukin (II)-2. Other cytokines that may be rapidly induced in CRS are IL-1β, IL-6, IL-8, and IL-10.

Exemplary signs or symptoms associated with CRS include fever, rigors, chills, hypotension, dyspnea, acute respiratory distress syndrome (ARDS), encephalopathy, aspartate transaminase (AST)/alanine transaminase (ALT) elevation, renal failure, cardiac disorders, hypoxia, neurologic disturbances, and death. Neurological complications include delirium, seizure-like activity, confusion, word-finding difficulty, aphasia, and/or becoming obtunded. Other CRS-related signs or outcomes include fatigue, nausea, headache, seizure, tachycardia, myalgias, rash, acute vascular leak syndrome, liver function impairment, and renal failure. In some aspects, CRS is associated with an increase in one or more factors such as serum-ferritin, d-dimer, aminotransferases, lactate dehydrogenase and triglycerides, or with hypofibrinogenemia or hepatosplenomegaly.

In some instances, signs or symptoms associated with CRS include one or more of: persistent fever, e.g., fever of a specified temperature, e.g., greater than at or about 38 degrees Celsius, for two or more, e.g., three or more, e.g., four or more days or for at least three consecutive days; fever greater than at or about 38 degrees Celsius; elevation of cytokines (e.g. IFNγ or IL-6); and/or at least one clinical sign of toxicity, such as hypotension (e.g., as measured by at least one intravenous vasoactive pressor); hypoxia (e.g., plasma oxygen (POz) levels of less than at or about 90 %); and/or one or more neurologic disorders (including mental status changes, obtundation, and seizures).

Exemplary CRS-related outcomes include increased or high serum levels of one or more factors, including cytokines and chemokines and other factors associated with CRS. Exemplary outcomes further include increases in synthesis or secretion of one or more of such factors. Such synthesis or secretion can be by the T cell or a cell that interacts with the T cell, such as an innate immune cell or B cell.

In some instances, one or more inflammatory markers, e.g., cytokines or chemokines are monitored before, during, or after CAR treatment. In some aspects, the one or more cytokines or chemokines include IFN-γ, TNF-α, IL-2, IL-1β, IL-6, IL-7, IL-8, IL-10, IL-12, sIL-2Rα, granulocyte macrophage colony stimulating factor (GM-CSF), or macrophage inflammatory protein (MIP). In some instances, IFN-γ, TNF-α, and IL-6 are monitored.

In some instances, the presence of one or more biomarkers is indicative of the grade of, severity or extent of a toxic event, such as CRS or neurotoxicity. In some instances, the toxic outcome is a particular grade, severity or extent of a toxic event, such as a particular grade, severity or extent of CRS or neurotoxicity. In some instances, the presence of a toxic event about a certain grade, severity or extent can be a dose-limiting toxicity. In some instances, the absence of a toxic event or the presence of a toxic event below a certain grade, severity or extent can indicate the absence of a dose-limiting toxicity.

CRS criteria that appear to correlate with the onset of CRS to predict which patients are more likely to be at risk for developing sCRS have been developed (see Davilla et al. Science translational medicine. 2014;6(224):224ra25). Factors include fevers, hypoxia, hypotension, neurologic changes, elevated serum levels of inflammatory cytokines whose treatment-induced elevation can correlate well with both pretreatment tumor burden and sCRS symptoms. Other guidelines on the diagnosis and management of CRS are known (see e.g., Lee et al, Blood. 2014;124(2):188-95). In some instances, the criteria reflective of CRS grade are those detailed in **Table 1** below.

| **Table 1:** | **Exemplary Grading Criteria for CRS** | |
|---|---|---|
| **Grade** | | **Description of Symptoms** |
| 1 | | Not life-threatening, require only symptomatic treatment such as antipyretics and anti-emetics (e.g., fever, nausea, fatigue, headache, myalgias, malaise) |
| Mild | | |
| 2 | | Require and respond to moderate intervention: |
| Moderate | | • Oxygen requirement < 40%, or |
| | | • Hypotension responsive to fluids or low dose of a single vasepresser, or |
| | | • Grade 2 organ toxicity (by CTCAE v4.0) |
| 3 | | Require and respond to aggressive intervention: |
| Severe | | • Oxygen requirement ≥ 40%, or |
| | | • Hypotension requiring high dose of a single vasepresser (e.g., norepinephrine ≥ 20 µg/kg/min, dopamine ≥ 10 µg/kg/min, phenylephrine ≥ 200 µg/kg/min, or epinephrine ≥ 10 µg/kg/min), or |
| | | • Hypotension requiring multiple vasopressors (e.g., vasopressin + one of the above agents, or combination vasopressors equivalent to ≥ 20 µg/kg/min norepinephrine), or |
| | | • Grade 3 organ toxicity or Grade 4 transaminitis (by CTCAE v4.0) |
| 4 | | Life-threatening: |
| Life-threatening | | • Requirement for ventilator support, or |
| | | • Grade 4 organ toxicity (excluding transaminitis) |
| 5 | | Death |
| Fatal | | |

In some instances, the toxic outcome is severe CRS. In some instances, the toxic outcome is the absence of severe CRS (e.g. moderate or mild CRS). In some instances, severe CRS includes CRS with a grade of 3 or greater, such as set forth in **Table 1.** In some instances, severe CRS includes CRS with a grade of 2 or higher, such as grades 2, 3, 4 or 5 CRS.

In some instances, the level of the toxic outcome, e.g. the CRS-related outcome, e.g. the serum level of an indicator of CRS, is measured by ELISA. In some instances, fever and/or levels of C-reactive protein (CRP) can be measured. In some instances, subjects with a fever and a CRP ≥ 15 mg/dL may be considered high-risk for developing severe CRS. In some instances, the CRS-associated serum factors or CRS-related outcomes include an increase in the level and/or concentration of inflammatory cytokines and/or chemokines, including Flt-3L, fracktalkine, granulocyte macrophage colony stimulating factor (GM-CSF), interleukin-1 beta (IL-1β), IL-2, IL-5, IL-6, IL-7, IL-8, IL-10, IL-12, interferon gamma (IFN-γ), macrophage inflammatory protein (MIP)-1, MIP-1, sIL-2Rα, or tumor necrosis factor alpha (TNFα). In some instances, the factor or outcome includes C reactive protein (CRP). In addition to being an early and easily measurable risk factor for CRS, CRP also is a marker for cell expansion. In some instances, subjects that are measured to have high levels of CRP, such as ≥ 15 mg/dL, have CRS. In some instances, subjects that are measured to have high levels of CRP do not have CRS. In some instances, a measure of CRS includes a measure of CRP and another factor indicative of CRS.

In some aspects, the toxic outcome is or is associated with neurotoxicity. In some instances, signs or symptoms associated with a clinical risk of neurotoxicity include confusion, delirium, expressive aphasia, obtundation, myoclonus, lethargy, altered mental status, convulsions, seizure-like activity, seizures (optionally as confirmed by electroencephalogram (EEG)), elevated levels of beta amyloid (Aβ), elevated levels of glutamate, and elevated levels of oxygen radicals. In some instances, neurotoxicity is graded based on severity (e.g., using a Grade 1-5 scale (see, e.g., Guido Cavaletti & Paola Marmiroli Nature Reviews Neurology 6, 657-666 (December 2010); National Cancer Institute-Common Toxicity Criteria version 4.03 (NCI-CTCAE v4.03). In some instances, a subject is deemed to develop "severe neurotoxicity" in response to or secondary to administration of a cell therapy or dose of cells thereof, if, following administration, the subject displays symptoms that limit self-care (e.g. bathing, dressing and undressing, feeding, using the toilet, taking medications) from among: 1) symptoms of peripheral motor neuropathy, including inflammation or degeneration of the peripheral motor nerves; 2) symptoms of peripheral sensory neuropathy, including inflammation or degeneration of the peripheral sensory nerves, dysesthesia, such as distortion of sensory perception, resulting in an abnormal and unpleasant sensation, neuralgia, such as intense painful sensation along a nerve or a group of nerves, and/or paresthesia, such as functional disturbances of sensory neurons resulting in abnormal cutaneous sensations of tingling, numbness, pressure, cold and warmth in the absence of stimulus. In some instances, severe neurotoxicity includes neurotoxicity with a grade of 3 or greater, such as set forth in **Table 2.** In some instances, severe neurotoxicity includes neurotoxicity with a grade of 2 or higher, such as grades 2, 3, 4 or 5 neurotoxicity.

| **Table 2:** | **Exemplary Grading Criteria for neurotoxicity** | |
|---|---|---|
| **Grade** | | **Description of Symptoms** |
| 1 | | Mild or asymptomatic symptoms |

| Asymptomatic or Mild | | |
|---|---|---|
| 2 | | Presence of symptoms that limit instrumental activities of daily living (ADL), such as preparing meals, shopping for groceries or clothes, using the telephone, managing money |
| Moderate | | |
| 3 | | Presence of symptoms that limit self-care ADL, such as bathing, dressing and undressing, feeding self, using the toilet, taking medications |
| Severe | | |
| 4 | | Symptoms that are life-threatening, requiring urgent intervention |
| Life-threatening | | |
| 5 | | Death |
| Fatal | | |

In some instances, the toxic outcome is a dose-limiting toxicity. In some instances, the toxic outcome is the absence of a dose-limiting toxicity. In some instances, a dose-limiting toxicity (DLT) is defined as any grade 3 or higher toxicity as assessed by any known or published guidelines for assessing the particular toxicity, such as any described above and including the National Cancer Institute (NCI) Common Terminology Criteria for Adverse Events (CTCAE) version 4.0.

### B. Response Outcome

In some instances, a response outcome in a subject to administration of a therapeutic agent can be monitored or assessed. In some instances, the response outcome is no response. In some instances, the response outcome is a partial response. In some instances, the response outcome is a complete response (CR). In some instances, response outcome is assessed by monitoring the disease burden in the subject. In some instances, the presence of no response, a partial response or a clinical or complete response can be assessed.

In some instances, a partial response (PR) or complete response (CR) is one in which the therapeutic agent reduces or prevents the expansion or burden of the disease or condition in the subject. For example, where the disease or condition is a tumor, reduced disease burden exists or is present if there is a reduction in the tumor size, bulk, metastasis, percentage of blasts in the bone marrow or molecularly detectable cancer and/or an improvement prognosis or survival or other symptom associated with tumor burden compared to prior to treatment with the therapeutic agent (e.g. CAR T cells).

In some instances, the administration effectively treats the subject despite the subject having become resistant to another therapy. In some instances, at least 35%, at least 40 % or at least 50% of subjects treated according to the method achieve complete response (CR); and/or at least 50%, at least 60% or at least 70% of the subjects treated according to the method achieve objective response rate (ORR). In some instances, at least or about at least 50% of subjects, at least or about at least 60% of the subjects, at least or about at least 70% of the subjects, at least or about at least 80% of the subjects or at least or about at least 90% of the subjects treated according to the method achieve CR and/or achieve an objective response (OR). In some instances, criteria assessed for effective treatment includes overall response rate or objective response rate (ORR), complete response (CR), duration of response (DOR), progression-free survival (PFS), and/or overall survival (OS).

In some instances, at least 40% or at least 50 % of subjects treated according to the methods described herein achieve complete remission (CR), exhibit progression-free survival (PFS) and/or overall survival (OS) of greater than at or about 3 months, 6 months or 12 months or greater than 13 months or approximately 14 months; on average, subjects treated according to the method exhibit a median PFS or OS of greater than at or about 6 months, 12 months, or 18 months; and/or the subject exhibits PFS or OS following therapy for at least at or about 6, 12, 18 or more months.

In some aspects, response rates in subjects, such as subjects with NHL, are based on the Lugano criteria. (Cheson et al., (2014) JCO 32(27):3059-3067; Johnson et al., (2015) Radiology 2:323-338; Cheson, B.D. (2015) Chin Clin Oncol 4(1):5). In some aspects, response assessment utilizes any of clinical, hematologic, and/or molecular methods. In some aspects, response assessed using the Lugano criteria involves the use of positron emission tomography (PET)-computed tomography (CT) and/or CT as appropriate. PET-CT evaluations may further comprise the use of fluorodeoxyglucose (FDG) for FDG-avid lymphomas. In some aspects, where PET-CT will be used to assess response in FDG-avid histologies, a 5-point scale may be used. In some respects, the 5-point scale comprises the following criteria: 1, no uptake above background; 2, uptake ≤ mediastinum; 3, uptake > mediastinum but ≤ liver; 4, uptake moderately > liver; 5, uptake markedly higher than liver and/or new lesions; X, new areas of uptake unlikely to be related to lymphoma.

In some aspects, a complete response as described using the Lugano criteria involves a complete metabolic response and a complete radiologic response at various measureable sites. In some aspects, these sites include lymph nodes and extralymphatic sites, wherein a CR is described as a score of 1, 2, or 3 with or without a residual mass on the 5-point scale, when PET-CT is used. In some aspects, in Waldeyer's ring or extranodal sites with high physiologic uptake or with activation within spleen or marrow (e.g., with chemotherapy or myeloid colony-stimulating factors), uptake may be greater than normal mediastinum and/or liver. In this circumstance, complete metabolic response may be inferred if uptake at sites of initial involvement is no greater than surrounding normal tissue even if the tissue has high physiologic uptake. In some aspects, response is assessed in the lymph nodes using CT, wherein a CR is described as no extralymphatic sites of disease and target nodes/nodal masses must regress to ≤ 1.5 cm in longest transverse diameter of a lesion (LDi). Further sites of assessment include the bone marrow wherein PET-CT-based assessment should indicate a lack of evidence of FDG-avid disease in marrow and a CT-based assessment should indicate a normal morphology, which if indeterminate should be IHC negative. Further sites may include assessment of organ enlargement, which should regress to normal. In some aspects, nonmeasured lesions and new lesions are assessed, which in the case of CR should be absent (Cheson et al., (2014) JCO 32(27):3059-3067; Johnson et al., (2015) Radiology 2:323-338; Cheson, B.D. (2015) Chin Clin Oncol 4(1):5).

In some aspects, a partial response (PR) as described using the Lugano criteria involves a partial metabolic and/or radiological response at various measureable sites. In some aspects, these sites include lymph nodes and extralymphatic sites, wherein a PR is described as a score of 4 or 5 with reduced uptake compared with baseline and residual mass(es) of any size, when PET-CT is used. At interim, such findings can indicate responding disease. At the end of treatment, such findings can indicate residual disease. In some aspects, response is assessed in the lymph nodes using CT, wherein a PR is described as ≥50% decrease in sum of product dimensions (SPD) of up to 6 target measureable nodes and extranodal sites. If a lesion is too small to measure on CT, 5 mm × 5 mm is assigned as the default value; if the lesion is no longer visible, the value is 0 mm × 0 mm; for a node >5 mm × 5 mm, but smaller than normal, actual measurements are used for calculation. Further sites of assessment include the bone marrow wherein PET-CT-based assessment should indicate residual uptake higher than uptake in normal marrow but reduced compared with baseline (diffuse uptake compatible with reactive changes from chemotherapy allowed). In some aspects, if there are persistent focal changes in the marrow in the context of a nodal response, consideration should be given to further evaluation with MRI or biopsy, or an interval scan. In some aspects, further sites may include assessment of organ enlargement, where the spleen must have regressed by >50% in length beyond normal. In some aspects, nonmeasured lesions and new lesions are assessed, which in the case of PR should be absent/normal, regressed, but no increase. No response/stable disease (SD) or progressive disease (PD) can also be measured using PET-CT and/or CT based assessments. (Cheson et al., (2014) JCO 32(27):3059-3067; Johnson et al., (2015) Radiology 2:323-338; Cheson, B.D. (2015) Chin Clin Oncol 4(1):5).

In some respects, progression-free survival (PFS) is described as the length of time during and after the treatment of a disease, such as cancer, that a subject lives with the disease but it does not get worse. In some aspects, objective response (OR) is described as a measurable response. In some aspects, objective response rate (ORR) is described as the proportion of patients who achieved CR or PR. In some aspects, overall survival (OS) is described as the length of time from either the date of diagnosis or the start of treatment for a disease, such as cancer, that subjects diagnosed with the disease are still alive. In some aspects, event-free survival (EFS) is described as the length of time after treatment for a cancer ends that the subject remains free of certain complications or events that the treatment was intended to prevent or delay. These events may include the return of the cancer or the onset of certain symptoms, such as bone pain from cancer that has spread to the bone, or death.

In some instances, the measure of duration of response (DOR) includes the time from documentation of tumor response to disease progression. In some instances, the parameter for assessing response can include durable response, e.g., response that persists after a period of time from initiation of therapy and/or long-lasting positive response to therapy. In some instances, durable response is indicated by the response rate at approximately 1, 2, 3, 4, 5, 6, 7, 8, 9, 10, 11, 12, 18 or 24 months after initiation of therapy. In some instances, the response is durable for greater than 3 months or greater than 6 months. In some instances, durable response is response measured at month 3 after administration of therapy, e.g., a 3-month response. In some instances, durable response is response measured at month 6 after administration of therapy, e.g., a 6-month response.

In some aspects, the RECIST criteria is used to determine objective tumor response; in some aspects, in solid tumors. (Eisenhauer et al., European Journal of Cancer 45 (2009) 228-247.) In some aspects, the RECIST criteria is used to determine objective tumor response for target lesions. In some respects, a complete response as determined using RECIST criteria is described as the disappearance of all target lesions and any pathological lymph nodes (whether target or non-target) must have reduction in short axis to <10 mm. In other aspects, a partial response as determined using RECIST criteria is described as at least a 30% decrease in the sum of diameters of target lesions, taking as reference the baseline sum diameters. In other aspects, progressive disease (PD) is described as at least a 20% increase in the sum of diameters of target lesions, taking as reference the smallest sum on study (this includes the baseline sum if that is the smallest on study). In addition to the relative increase of 20%, the sum must also demonstrate an absolute increase of at least 5 mm (in some aspects the appearance of one or more new lesions is also considered progression). In other aspects, stable disease (SD) is described as neither sufficient shrinkage to qualify for PR nor sufficient increase to qualify for PD, taking as reference the smallest sum diameters while on study.

In some instances, the disease or condition is a tumor and a reduction in disease burden is a reduction in tumor size. In some instances, the disease burden reduction is indicated by a reduction in one or more factors, such as load or number of disease cells in the subject or fluid or organ or tissue thereof, the mass or volume of a tumor, or the degree or extent of metastases. In some instances, disease burden, e.g. tumor burden, can be assessed or monitored for the extent of morphological disease and/or minimal residual disease.

In some instances, the burden of a disease or condition in the subject is detected, assessed, or measured. Disease burden may be detected in some aspects by detecting the total number of disease or disease-associated cells, e.g., tumor cells, in the subject, or in an organ, tissue, or bodily fluid of the subject, such as blood or serum. In some instances, disease burden, e.g. tumor burden, is assessed by measuring the mass of a solid tumor and/or the number or extent of metastases. In some aspects, survival of the subject, survival within a certain time period, extent of survival, presence or duration of event-free or symptom-free survival, or relapse-free survival, is assessed. In some instances, any symptom of the disease or condition is assessed. In some instances, the measure of disease or condition burden is specified.

In some instances, disease burden can encompass a total number of cells of the disease in the subject or in an organ, tissue, or bodily fluid of the subject, such as the organ or tissue of the tumor or another location, e.g., which would indicate metastasis. For example, tumor cells may be detected and/or quantified in the blood or bone marrow in the context of certain hematological malignancies. Disease burden can include, in some instances, the mass of a tumor, the number or extent of metastases and/or the percentage of blast cells present in the bone marrow.

In some instances, a subject has leukemia. The extent of disease burden can be determined by assessment of residual leukemia in blood or bone marrow.

In some aspects, response rates in subjects, such as subjects with chronic lymphocytic leukemia (CLL), are based on the International Workshop on Chronic Lymphocytic Leukemia (IWCLL) response criteria (Hallek, et al., Blood 2008, Jun 15; 111(12): 5446-5456). In some aspects, these criteria are described as follows: complete remission (CR), which in some aspects requires the absence of peripheral blood clonal lymphocytes by immunophenotyping, absence of lymphadenopathy, absence of hepatomegaly or splenomegaly, absence of constitutional symptoms and satisfactory blood counts; complete remission with incomplete marrow recovery (CRi), which in some aspects is described as CR above, but without normal blood counts; partial remission (PR), which in some aspects is described as ≥ 50% fall in lymphocyte count, ≥ 50% reduction in lymphadenopathy or ≥ 50% reduction in liver or spleen, together with improvement in peripheral blood counts; progressive disease (PD), which in some aspects is described as ≥ 50% rise in lymphocyte count to > 5 ×10⁹/L, ≥ 50% increase in lymphadenopathy, ≥ 50% increase in liver or spleen size, Richter's transformation, or new cytopenias due to CLL; and stable disease, which in some aspects is described as not meeting criteria for CR, CRi, PR or PD.

In some instances, the subjects exhibits a CR or OR if, within 1 month of the administration of the dose of cells, lymph nodes in the subject are less than at or about 20 mm in size, less than at or about 10 mm in size or less than at or about 10 mm in size.

In some instances, an index clone of the CLL is not detected in the bone marrow of the subject (or in the bone marrow of greater than 50%, 60%, 70%, 80%, 90% or more of the subjects treated according to the methods. In some instances, an index clone of the CLL is assessed by IgH deep sequencing. In some instances, the index clone is not detected at a time that is at or about or at least at or about 1, 2, 3, 4, 5, 6, 12, 18 or 24 months following the administration of the cells.

In some instances, a response outcome exists if there is a reduction in the percent of blasts in the bone marrow compared to the percent of blasts in the bone marrow prior to treatment with the therapeutic agent. In some instances, reduction of disease burden exists if there is a decrease or reduction of at least or at least about 20%, 30%, 40%, 50%, 60%, 70%, 80%, 90%, 95% or more in the number or percentage of blasts in the bone marrow compared to the number or percent of blasts in the bone marrow prior to treatment.

In some instances, the subject exhibits a response if the subject does not exhibit morphologic disease (non-morphological disease) or does not exhibit substantial morphologic disease. In some instances, a subject exhibits morphologic disease if there are greater than or equal to 5% blasts in the bone marrow, for example, as detected by light microscopy. In some instances, a subject exhibits complete or clinical remission if there are less than 5% blasts in the bone marrow.

In some instances, a subject exhibits reduced or decreased disease burden if they exhibited morphological disease prior to treatment and exhibit complete remission (e.g., fewer than 5% blasts in bone marrow) with or without molecular disease (e.g., minimum residual disease (MRD) that is molecularly detectable, e.g., as detected by flow cytometry or quantitative PCR) after treatment. In some instances, a subject exhibits reduced or decreased disease burden if they exhibited molecular disease prior to treatment and do not exhibit molecular disease after treatment.

In some instances, a subject may exhibit complete remission, but a small proportion of morphologically undetectable (by light microscopy techniques) residual leukemic cells are present. A subject is said to exhibit minimum residual disease (MRD) if the subject exhibits less than 5% blasts in the bone marrow and exhibits molecularly detectable cancer. In some instances, molecularly detectable cancer can be assessed using any of a variety of molecular techniques that permit sensitive detection of a small number of cells. In some aspects, such techniques include PCR assays, which can determine unique Ig/T-cell receptor gene rearrangements or fusion transcripts produced by chromosome translocations. In some instances, flow cytometry can be used to identify cancer cell based on leukemia-specific immunophenotypes. In some instances, molecular detection of cancer can detect as few as 1 leukemia or blast cell in 100,000 normal cells or 1 leukemia or blast cell in 10,000 normal cells. In some instances, a subject exhibits MRD that is molecularly detectable if at least or greater than 1 leukemia cell in 100,000 cells is detected, such as by PCR or flow cytometry.

In some instances, the disease burden of a subject is molecularly undetectable or MRD⁻, such that, in some cases, no leukemia cells are able to be detected in the subject using PCR or flow cytometry techniques.

In some instances the response outcome is the absence of a CR or the presence of a complete response in which the subject achieves or exhibits minimal residual disease or molecular detectable disease status. In some instances, the response outcome is the presence of a CR with molecularly detectable disease or the presence of a CR without molecularly detectable disease. In some instances, subjects are assessed for disease burden using methods as described herein, such as methods that assess blasts in bone marrow or molecular disease by flow cytometry or qPCR methods.

In some instances of the methods described herein, response is determined by complete remission or complete response (CR) and/or objective response (OR); and/or the subject exhibits CR, OR, lymph nodes of less than at or about 20 mm in size, within 1 month of the administration of the dose of cells; and/or an index clone of the disease or condition, such as the CLL or NHL, is not detected in the bone marrow of the subject (or in the bone marrow of greater than 50 % of subjects treated according to the methods), optionally as assessed by IgH deep sequencing, optionally at a time that is at or about or at least at or about 1, 2, 3, 4, 5, 6, 12, 18, or 24 months following the administration of the cell dose.

### C. Determining Pharmacokinetics (PK) of Engineered Cells, e.g. Peak Cell Levels

In some instances, the method includes assessment of the exposure, number, concentration, persistence and proliferation of the T cells, *e.g.,* T cells administered for the T cell based therapy. In some instances, the exposure, or prolonged expansion and/or persistence of the cells, and/or changes in cell phenotypes or functional activity of the cells, *e.g.,* cells administered for immunotherapy, *e.g*. T cell therapy, in the methods described herein, can be measured by assessing the characteristics of the T cells *in vitro* or *ex vivo.* In some instances, such assays can be used to determine or confirm the function of the T cells used for the immunotherapy, e.g. T cell therapy, before or after administering the cell therapy described herein.

In some aspects, the exposure, number, concentration, persistence and proliferation relate to pharmacokinetic parameters. In some cases, pharmacokinetics can be assessed by measuring such parameters as the maximum (peak) plasma concentration (Cₘₐₓ), the peak time (*i.e.* when maximum plasma concentration (Cₘₐₓ) occurs; Tₘₐₓ), the minimum plasma concentration (*i.e.* the minimum plasma concentration between doses of a therapeutic agent, e.g., CAR+ T cells; Cₘᵢₙ), the elimination half-life (T_{1/2}) and area under the curve (*i.e.* the area under the curve generated by plotting time versus plasma concentration of the therapeutic agent CAR+ T cells; AUC), following administration. The concentration of a particular therapeutic agent, e.g., CAR+ T cells, in the plasma following administration can be measured using any method known in the art suitable for assessing concentrations of the therapeutic agents, e.g., CAR+ T cells, in samples of blood, or any methods described herein. For example, nucleic acid-based methods, such as quantitative PCR (qPCR) or flow cytometry-based methods, or other assays, such as an immunoassay, ELISA, or chromatography/mass spectrometry-based assays can be used.

In some instances, the pharmacokinetics (PK) of administered cells, e.g., CAR⁺ T cell composition, are determined to assess the availability, e.g., bioavailability, of the administered cells. In some instances, the determined pharmacokinetic parameters of the administered cells include maximum (peak) plasma concentrations (Cₘₐₓ), such as Cₘₐₓ of CD3⁺ CAR⁺ cells, CD4⁺ CAR⁺ cells and or CD8⁺ CAR⁺ T cells; the time point at which Cₘₐₓ is achieved (Tₘₐₓ), such as the Tₘₐₓ of CD3⁺ CAR⁺ cells, CD4⁺ CAR⁺ cells and or CD8⁺ CAR⁺ T cells, and or area under the curve (AUC), such as the AUC₀₋₂₈, of CD3⁺ CAR⁺ cells, CD4⁺ CAR⁺ cells and or CD8⁺ CAR⁺ T cells. In some instances, the pharmacokinetic parameter is peak CD3⁺ CAR⁺ T cell concentration (Cₘₐₓ CD3⁺ CAR⁺ T cells), or CD8⁺ CAR⁺ T cell concentration (Cₘₐₓ CD8⁺ CAR⁺ T cells). In some instances, the pharmacokinetic parameter is AUC₀₋₂₈, of CD3⁺ CAR⁺ T cells, (AUC0-28 CD3⁺ CAR⁺ T cells), or AUC₀₋₂₈, of CD8⁺ CAR⁺ T cells, (AUC0-28 CD8⁺ CAR⁺ T cells),

In some instances, "exposure" can refer to the body exposure of a therapeutic agent, e.g., CAR+ T cells in the plasma (blood or serum) after administration of the therapeutic agent over a certain period of time. In some instances exposure can be set forth as the area under the therapeutic agent concentration-time curve (AUC) as determined by pharmacokinetic analysis after administration of a dose of the therapeutic agent, e.g., CAR+ T cells. In some cases, the AUC is expressed in cells^{∗}days/µL, for cells administered in cell therapy, or in corresponding units thereof. In some instances, the AUC is measured as an average AUC in a patient population, such as a sample patient population, *e.g.*, the average AUC from one or more patient(s). In some instances, systemic exposure refers to the area under the curve (AUC) within a certain period of time, e.g., from day 0 to day 1, 2, 3, 4, 5, 6, 7, 8, 9, 10, 11, 12, 13, 14, 21, 28 days or more, or week 1, 2, 3, 4, 5, 6, 7, 8, 9, 10, 11, 12, 13, 14, 15 or more, or month 1, 2, 3, 4, 5, 6, 7, 8, 9, 10, 11, 12, 18, 24, 48 or more. In some instances, the AUC is measured as an AUC from day 0 to day 28 (AUC₀₋₂₈) after administration of the therapeutic agent, e.g., CAR+ T cells, including all measured data and data extrapolated from measured pharmacokinetic (PK) parameters, such as an average AUC from a patient population, such as a sample patient population. In some instances, to determine exposure over time, e.g., AUC for a certain period of time, such as AUC₀₋₂₈, a therapeutic agent concentration-time curve is generated, using multiple measurements or assessment of parameters, e.g., cell concentrations, over time, e.g., measurements taken every 1, 2, 3, 4, 5, 6, 7, 8, 9, 10, 11, 12, 13, 14, 21 or 28 days or more.

In some instances, the presence and/or amount of cells expressing the recombinant receptor (*e.g.*, CAR-expressing cells administered for T cell based therapy) in the subject following the administration of the T cells and before, during and/or after the administration of the therapy is detected. In some aspects, nucleic acid-based methods, such as quantitative PCR (qPCR), is used to assess the quantity of cells expressing the recombinant receptor (*e.g.*, CAR-expressing cells administered for T cell based therapy) in the blood or serum or organ or tissue sample (*e.g.*, disease site, *e.g.*, tumor sample) of the subject. In some aspects, persistence is quantified as copies of DNA or plasmid encoding the receptor, *e.g.*, CAR, per microgram of DNA, or as the number of receptor-expressing, *e.g.*, CAR-expressing, cells per microliter of the sample, *e.g.*, of blood or serum, or per total number of peripheral blood mononuclear cells (PBMCs) or white blood cells or T cells per microliter of the sample. In some instances, the primers or probe used for qPCR or other nucleic acid-based methods are specific for binding, recognizing and/or amplifying nucleic acids encoding the recombinant receptor, and/or other components or elements of the plasmid and/or vector, including regulatory elements, e.g., promoters, transcriptional and/or post-transcriptional regulatory elements or response elements, or markers, e.g., surrogate markers. In some instances, the primers can be specific for regulatory elements, such as the woodchuck hepatitis virus post-transcriptional regulatory element (WPRE).

In some instances, the cells are detected in the subject at or at least at 4, 14, 15, 27, or 28 days following the administration of the T cells, *e.g.*, CAR-expressing T cells. In some aspects, the cells are detected at or at least at 2, 4, or 6 weeks following, or 3, 6, or 12, 18, or 24, or 30 or 36 months, or 1, 2, 3, 4, 5, or more years, following the administration of the T cells, e.g., CAR-expressing T cells.

In some instances, the peak levels and/or AUC are assessed and/or the sample is obtained from the subject at a time that is at least 8 days, 9 days, 10 days, 11 days, 12 days, 13 days, 14 days, 15 days, 16 days, 17 days, 18 days, 19 days, 20 days or 21 days after initiation of administration of the genetically engineered cells. In some instances the peak levels and/or AUC are assessed and/or the sample is obtained from the subject at a time that is between or between about 11 to 22 days, 12 to 18 days or 14 to 16 days, each inclusive, after initiation of administration of the genetically engineered cells.

The exposure, *e.g.,* number or concentration of cells, *e.g.* T cells administered for T cell therapy, indicative of expansion and/or persistence, may be stated in terms of maximum numbers or concentration of the cells to which the subject is exposed, duration of detectable cells or cells above a certain number or percentage, area under the curve (AUC) for number or concentration of cells over time, and/or combinations thereof and indicators thereof. Such outcomes may be assessed using known methods, such as qPCR to detect copy number of nucleic acid encoding the recombinant receptor compared to total amount of nucleic acid or DNA in the particular sample, *e.g.*, blood, serum, plasma or tissue, such as a tumor sample, and/or flow cytometric assays detecting cells expressing the receptor generally using antibodies specific for the receptors. Cell-based assays may also be used to detect the number or percentage or concentration of functional cells, such as cells capable of binding to and/or neutralizing and/or inducing responses, *e.g.*, cytotoxic responses, against cells of the disease or condition or expressing the antigen recognized by the receptor.

In some aspects, increased exposure of the subject to the cells includes increased expansion of the cells. In some instances, the receptor expressing cells, *e.g.* CAR-expressing cells, expand in the subject following administration of the T cells, *e.g.*, CAR-expressing T cells.

In some instances, cells expressing the receptor are detectable in the serum, plasma, blood or tissue, *e.g.,* tumor sample, of the subject, *e.g.,* by a specified method, such as qPCR or flow cytometry-based detection method, at least 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 or more days following administration of the T cells, *e.g.*, CAR-expressing T cells , for at least at or about 2, 3, 4, 5, 6, 7, 8, 9, 10, 11, 12, 13, 14, 15, 16, 17, 18, 19, 20, 21, 22, 23, or 24 or more weeks following the administration of the T cells, *e.g.*, CAR-expressing T cells.

In some aspects, at least about 1 × 10², at least about 1 × 10³, at least about 1 × 10⁴, at least about 1 × 10⁵, or at least about 1 × 10⁶ or at least about 5 × 10⁶ or at least about 1 × 10⁷ or at least about 5 × 10⁷ or at least about 1 × 10⁸ recombinant receptor-expressing, *e.g.,* CAR-expressing cells, and/or at least 10, 25, 50, 100, 200, 300, 400, or 500, or 1000 receptor-expressing cells per microliter, e.g., at least 10 per microliter, are detectable or are present in the subject or fluid, plasma, serum, tissue, or compartment thereof, such as in the blood, *e.g.,* peripheral blood, or disease site, *e.g.*, tumor, thereof. In some instances, such a number or concentration of cells is detectable in the subject for at least about 20 days, at least about 40 days, or at least about 60 days, or at least about 3, 4, 5, 6, 7, 8, 9, 10, 11, or 12 months, or at least 2 or 3 years, following administration of the T cells, *e.g.*, CAR-expressing T cells. Such cell numbers may be as detected by flow cytometry-based or quantitative PCR-based methods and extrapolation to total cell numbers using known methods. *See, e.g.,* Brentjens et al., Sci Transl Med. 2013 5(177), Park et al, Molecular Therapy 15(4):825-833 (2007), Savoldo et al., JCI 121(5):1822-1826 (2011), Davila et al., (2013) PLoS ONE 8(4):e61338, Davila et al., Oncoimmunology 1(9):1577-1583 (2012), Lamers, Blood 2011 117:72-82, Jensen et al., Biol Blood Marrow Transplant 2010 September; 16(9): 1245-1256, Brentjens et al., Blood 2011 118(18):4817-4828.

In some aspects, the copy number of nucleic acid encoding the recombinant receptor, *e.g.*, vector copy number, per 100 cells, for example in the peripheral blood or bone marrow or other compartment, as measured by immunohistochemistry, PCR, and/or flow cytometry, is at least 0.01, at least 0.1, at least 1, or at least 10, at about 1 week, about 2 weeks, about 3 weeks, about 4 weeks, about 5 weeks, or at least about 6 weeks, or at least about 2, 3, 4, 5, 6, 7, 8. 9, 10, 11, or 12 months or at least 2 or 3 years following administration of the cells, e.g., CAR-expressing T cells. In some instances, the copy number of the vector expressing the receptor, *e.g.* CAR, per microgram of genomic DNA is at least 100, at least 1000, at least 5000, or at least 10,000, or at least 15,000 or at least 20,000 at a time about 1 week, about 2 weeks, about 3 weeks, or at least about 4 weeks following administration of the T cells, e.g., CAR-expressing T cells or at least 2, 3, 4, 5, 6, 7, 8, 9, 10, 11, or 12 months or at least 2 or 3 years following such administration.

In some aspects, the receptor, *e.g.* CAR, expressed by the cells, is detectable by quantitative PCR (qPCR) or by flow cytometry in the subject, plasma, serum, blood, tissue and/or disease site thereof, *e.g.*, tumor site, at a time that is at least about 3 months, at least about 6 months, at least about 12 months, at least about 1 year, at least about 2 years, at least about 3 years, or more than 3 years, following the administration of the cells, *e.g.*, following the initiation of the administration of the T cells. In some instances, the area under the curve (AUC) for concentration of receptor- (*e.g.*, CAR-) expressing cells in a fluid, plasma, serum, blood, tissue, organ and/or disease site, e.g. tumor site, of the subject over time following the administration of the T cells, *e.g.*, CAR-expressing T cells, is measured.

Also described are methods of assessing likelihood of a response or a durable response. In some instances, the methods involve detecting, in a biological sample from a subject, peak levels of one or more inflammatory marker and/or peak levels of genetically engineered cells comprising T cells expressing a chimeric antigen receptor (CAR), wherein the subject has been previously administered a dose of the genetically engineered cells for treating a disease or condition. In some instances, the methods involve comparing, individually, the peak levels to a threshold value, thereby determining a likelihood that a subject will achieve a durable response to the administration of the genetically engineered cells.

In some instances, the subject is likely to achieve a response or a durable response if the peak levels of the one or more inflammatory marker is below a threshold value and the subject is not likely to achieve a durable response if the peak levels of the one or more inflammatory marker is above a threshold value. In some instances, the subject is likely to achieve a durable response if the peak level of the genetically engineered cells is within a therapeutic range between a lower threshold value and an upper threshold value and the subject is not likely to achieve a durable response if the peak level of the genetically engineered cells is below the lower threshold value or is above the upper threshold value.

### III.METHOD OF TREATMENT

In some instances, described are methods of treatment. In some instances, the methods include administering an immunotherapy and/or a cell therapy. In some instances, the methods involve administration of genetically engineered cells, e.g., cells engineered to express a recombinant receptor such as a chimeric antigen receptor (CAR). In some instances, the methods include administering a dose of cells, e.g., CAR+ expressing cells, to a subject such that the cells are within a target therapeutic range or window. In some instances, whether the cells in the subject is within a target therapeutic range or window can be determined or assessed by monitoring parameters, e.g., pharmacokinetic parameters, such as peak cell concentration (Cₘₐₓ). In some aspects, the described methods also include a method of determining a dose of a subject, or a method of dosing a subject, based on an assessment of the parameters, e.g., pharmacokinetic parameters, such as peak cell concentration (Cₘₐₓ), patient attributes and/or biomarkers.

In some instances, a dose of cells expressing a recombinant receptor are administered to a subject to treat or prevent diseases, conditions, and disorders, including cancers. In some instances, the cells, populations, and compositions are administered to a subject or patient having the particular disease or condition to be treated, e.g., via adoptive cell therapy, such as adoptive T cell therapy. In some instances, cells and compositions, such as engineered compositions and end-of-production compositions following incubation and/or other processing steps, are administered to a subject, such as a subject having or at risk for the disease or condition. In some aspects, the methods thereby treat, e.g., ameliorate one or more symptom of, the disease or condition, such as by lessening tumor burden in a cancer expressing an antigen recognized by an engineered T cell.

Methods for administration of cells for adoptive cell therapy are known and may be used in connection with the described methods and compositions. For example, adoptive T cell therapy methods are described, e.g., in US Patent Application Publication No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

The disease or condition that is treated can be any in which expression of an antigen is associated with and/or involved in the etiology of a disease condition or disorder, e.g. causes, exacerbates or otherwise is involved in such disease, condition, or disorder. Exemplary diseases and conditions can include diseases or conditions associated with malignancy or transformation of cells (e.g. cancer), autoimmune or inflammatory disease, or an infectious disease, e.g. caused by a bacterial, viral or other pathogen. Exemplary antigens, which include antigens associated with various diseases and conditions that can be treated, are described above. In particular instances, the chimeric antigen receptor or transgenic TCR specifically binds to an antigen associated with the disease or condition.

Among the diseases, conditions, and disorders are tumors, including solid tumors, hematologic malignancies, and melanomas, and including localized and metastatic tumors, infectious diseases, such as infection with a virus or other pathogen, e.g., HIV, HCV, HBV, CMV, and parasitic disease, and autoimmune and inflammatory diseases. In some instances, the disease or condition is a tumor, cancer, malignancy, neoplasm, or other proliferative disease or disorder. Such diseases include but are not limited to leukemia, lymphoma, e.g., chronic lymphocytic leukemia (CLL), acute-lymphoblastic leukemia (ALL), non-Hodgkin's lymphoma, acute myeloid leukemia, multiple myeloma, refractory follicular lymphoma, mantle cell lymphoma, indolent B cell lymphoma, B cell malignancies, cancers of the colon, lung, liver, breast, prostate, ovarian, skin, melanoma, bone, and brain cancer, ovarian cancer, epithelial cancers, renal cell carcinoma, pancreatic adenocarcinoma, Hodgkin lymphoma, cervical carcinoma, colorectal cancer, glioblastoma, neuroblastoma, Ewing sarcoma, medulloblastoma, osteosarcoma, synovial sarcoma, and/or mesothelioma. In some instances, the subject has acute-lymphoblastic leukemia (ALL). In some instances, the subject has non-Hodgkin's lymphoma.

In some instances, the disease or condition is an infectious disease or condition, such as, but not limited to, viral, retroviral, bacterial, and protozoal infections, immunodeficiency, Cytomegalovirus (CMV), Epstein-Barr virus (EBV), adenovirus, BK polyomavirus. In some instances, the disease or condition is an autoimmune or inflammatory disease or condition, such as arthritis, e.g., rheumatoid arthritis (RA), Type I diabetes, systemic lupus erythematosus (SLE), inflammatory bowel disease, psoriasis, scleroderma, autoimmune thyroid disease, Grave's disease, Crohn's disease, multiple sclerosis, asthma, and/or a disease or condition associated with transplant.

In some instances, the antigen associated with the disease or disorder is selected from the group consisting of αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD138, CD171, epidermal growth factor protein (EGFR), truncated epidermal growth factor protein (tEGFR), type III epidermal growth factor receptor mutation (EGFR vIII), epithelial glycoprotein 2 (EPG-2), epithelial glycoprotein 40 (EPG-40), ephrinB2, ephrine receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), G Protein Coupled Receptor 5D (GPCR5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Ra), IL-13 receptor alpha 2 (IL-13Ra2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, mesothelin, c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), vascular endothelial growth factor receptor (VEGFR), vascular endothelial growth factor receptor 2 (VEGFR2), Wilms Tumor 1 (WT-1), a pathogen-specific antigen, or an antigen associated with a universal tag, and/or biotinylated molecules, and/or molecules expressed by HIV, HCV, HBV or other pathogens. Antigens targeted by the receptors in some instances include antigens associated with a B cell malignancy, such as any of a number of known B cell marker. In some instances, the antigen is or includes CD20, CD19, CD22, ROR1, CD45, CD21, CD5, CD33, Igkappa, Iglambda, CD79a, CD79b or CD30.

In some instances, the antigen associated with the disease or disorder is selected from the group consisting of orphan tyrosine kinase receptor ROR1, tEGFR, Her2, Ll-CAM, CD19, CD20, CD22, mesothelin, CEA, and hepatitis B surface antigen, anti-folate receptor, CD23, CD24, CD30, CD33, CD38, CD44, EGFR, EGP-2, EGP-4, 0EPHa2, ErbB2, 3, or 4, FBP, fetal acethycholine e receptor, GD2, GD3, HMW-MAA, IL-22R-alpha, IL-13R-alpha2, kdr, kappa light chain, Lewis Y, L1-cell adhesion molecule, MAGE-A1, mesothelin, MUC1, MUC16, PSCA, NKG2D Ligands, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, CS-1, c-Met, GD-2, and MAGE A3, CE7, Wilms Tumor 1 (WT-1), a cyclin, such as cyclin A1 (CCNA1), and/or biotinylated molecules, and/or molecules expressed by HIV, HCV, HBV or other pathogens.

In some instances, the cell therapy, e.g., adoptive T cell therapy, is carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, following isolation and processing are administered to the same subject.

In some instances, the cell therapy, e.g., adoptive T cell therapy, is carried out by allogeneic transfer, in which the cells are isolated and/or otherwise prepared from a subject other than a subject who is to receive or who ultimately receives the cell therapy, e.g., a first subject. In such instances, the cells then are administered to a different subject, e.g., a second subject, of the same species. In some instances, the first and second subjects are genetically identical. In some instances, the first and second subjects are genetically similar. In some instances, the second subject expresses the same HLA class or supertype as the first subject.

The cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some instances, they are administered by parenteral, intrapulmonary, and intranasal, and, if desired for local treatment, intralesional administration. Parenteral infusions include intramuscular, intravenous, intraarterial, intraperitoneal, or subcutaneous administration. In some instances, a given dose is administered by a single bolus administration of the cells. In some instances, it is administered by multiple bolus administrations of the cells, for example, over a period of no more than 3 days, or by continuous infusion administration of the cells.

For the prevention or treatment of disease, the appropriate dosage may depend on the type of disease to be treated, the type of cells or recombinant receptors, the severity and course of the disease, whether the cells are administered for preventive or therapeutic purposes, previous therapy, the subject's clinical history and response to the cells, and the discretion of the attending physician. The compositions and cells are in some instances suitably administered to the subject at one time or over a series of treatments.

In some instances, the cells are administered as part of a combination treatment, such as simultaneously with or sequentially with, in any order, another therapeutic intervention, such as an antibody or engineered cell or receptor or agent, such as a cytotoxic or therapeutic agent. The cells in some instances are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some instances, the cells are administered prior to the one or more additional therapeutic agents. In some instances, the cells are administered after the one or more additional therapeutic agents. In some instances, the one or more additional agents include a cytokine, such as IL-2, for example, to enhance persistence. In some instances, the methods comprise administration of a chemotherapeutic agent.

In some instances, the methods comprise administration of a chemotherapeutic agent, e.g., a conditioning chemotherapeutic agent, for example, to reduce tumor burden prior to the administration.

Preconditioning subjects with immunodepleting (e.g., lymphodepleting) therapies in some aspects can improve the effects of adoptive cell therapy (ACT).

Thus, in some instances, the methods include administering a preconditioning agent, such as a lymphodepleting or chemotherapeutic agent, such as cyclophosphamide, fludarabine, or combinations thereof, to a subject prior to the initiation of the cell therapy. For example, the subject may be administered a preconditioning agent at least 2 days prior, such as at least 3, 4, 5, 6, or 7 days prior, to the initiation of the cell therapy. In some instances, the subject is administered a preconditioning agent no more than 7 days prior, such as no more than 6, 5, 4, 3, or 2 days prior, to the initiation of the cell therapy.

In some instances, the subject is preconditioned with cyclophosphamide at a dose between or between about 20 mg/kg and 100 mg/kg, such as between or between about 40 mg/kg and 80 mg/kg. In some aspects, the subject is preconditioned with or with about 60 mg/kg of cyclophosphamide. In some instances, the cyclophosphamide can be administered in a single dose or can be administered in a plurality of doses, such as given daily, every other day or every three days. In some instances, the cyclophosphamide is administered once daily for one or two days.

In some instances, where the lymphodepleting agent comprises fludarabine, the subject is administered fludarabine at a dose between or between about 1 mg/m² and 100 mg/m², such as between or between about 10 mg/m² and 75 mg/m², 15 mg/m² and 50 mg/m², 20 mg/m² and 30 mg/m², or 24 mg/m² and 26 mg/m². In some instances, the subject is administered 25 mg/m² of fludarabine. In some instances, the fludarabine can be administered in a single dose or can be administered in a plurality of doses, such as given daily, every other day or every three days. In some instances, fludarabine is administered daily, such as for 1-5 days, for example, for 3 to 5 days.

In some instances, the lymphodepleting agent comprises a combination of agents, such as a combination of cyclophosphamide and fludarabine. Thus, the combination of agents may include cyclophosphamide at any dose or administration schedule, such as those described above, and fludarabine at any dose or administration schedule, such as those described above. For example, in some aspects, the subject is administered 60 mg/kg (∼2 g/m²) of cyclophosphamide and 3 to 5 doses of 25 mg/m² fludarabine prior to the first or subsequent dose.

Following administration of the cells, the biological activity of the engineered cell populations in some instances is measured, e.g., by any of a number of known methods. Parameters to assess include specific binding of an engineered or natural T cell or other immune cell to antigen, in vivo, e.g., by imaging, or ex vivo, e.g., by ELISA or flow cytometry. In certain instances, the ability of the engineered cells to destroy target cells can be measured using any suitable method known in the art, such as cytotoxicity assays described in, for example, Kochenderfer et al., J. Immunotherapy, 32(7): 689-702 (2009), and Herman et al. J. Immunological Methods, 285(1): 25-40 (2004). In certain instances, the biological activity of the cells is measured by assaying expression and/or secretion of one or more cytokines, such as CD107a, IFNγ, IL-2, and TNF. In some aspects the biological activity is measured by assessing clinical outcome, such as reduction in tumor burden or load.

In certain instances, the engineered cells are further modified in any number of ways, such that their therapeutic or prophylactic efficacy is increased. For example, the engineered CAR or TCR expressed by the population can be conjugated either directly or indirectly through a linker to a targeting moiety. The practice of conjugating compounds, e.g., the CAR or TCR, to targeting moieties is known in the art. See, for instance, Wadwa et al., J. Drug Targeting 3: 1 1 1 (1995), and U.S. Patent 5,087,616.

### A. Dosing

In some instances, the subject is administered a dose that achieves or is likely to achieve the therapeutic range and/or window of CAR+ T cells. The method, in some instances, involves administering a dose of cells in an amount that is or is likely to achieve a peak CAR+ cell number in the blood within a range in which the peak CAR+ cell numbers have less than a certain estimated probability of causing toxicity. The method, in some instances, involves administering a dose of cells in an amount that is or is likely to achieve a peak CAR+ cell number in the blood within a range in which the peak CAR+ cell numbers have more than a certain estimated probability of causing response or durable response. In some cases, the amount of cells is an amount effective to treat the disease or condition, such as therapeutically effective or prophylactically effective amount. In some cases, the estimated probability of achieving a response is greater than 65%, 70%, 75%, 80%, 85%, 90%, 95% or more. In some cases, the estimated probability of causing toxicity is less than 35%, less than 30%, less than 25%, less than 20%, less than 15%, less than 10% or less than 5% on the toxicity probability curve. In some instances, the dose of cells is both above the desired estimated probability of achieving a response and below the desired estimated probability of causing toxicity.

In some instances, the amount or dose of cells that is administered is based upon assessment of parameters, e.g., pharmacokinetic parameters, and estimated probability of response and/or toxicity, e.g., as described in Section II.

In some instances, the methods involve administering a sufficient number or dose of cells to achieve a peak CAR+ cell concentration in the subject that is within a determined target therapeutic range or window. In some instances, the methods involve administering a sufficient number or dose of cells to achieve a peak CAR+ cell concentration in a majority of subjects so treated by the method, or greater than or greater than about 50%, 60%, 70%, 75%, 80%, 85%, 90% or 95% or more, such as greater than 75% of the subjects so treated by the method, is within a determined target therapeutic range or window.

In some instances, the therapeutic window or range is determined as described above, e.g., in Section II. In some instances, the therapeutic range is based upon the range of peak CD3+ CAR+ T cells, or a CD8+CAR+ T cell subset thereof, in the blood among one or more subjects previously treated with the genetically engineered cells that is associated with an estimated probability of response of greater than or greater than about 65%, 70%, 75%, 80%, 85%, 90%, 95% or more, and an estimated probability of a toxicity of less than or about 30%, 25%, 20%, 15%, 10%, 5% or less.

In some instances, the therapeutic window or range is determined based on specific range of numbers and/or concentrations of cells, e.g., CD3+, CD4+ or CD8+ T cells. In some instances, an exemplary peak CD3+CAR+ T cell concentration in the blood that can achieve a therapeutic window, is or includes between approximately 1, 2, 3, 4, 5, 6, 7, 8, 9, 10, 15, 20, 20, 30, 40, 50 CD3+ CAR+ T cells per microliter in the blood and approximately 200, 300, 400, 500, 600, 700 or 750, CD3+ CAR+ T cells per microliter in the blood. In some instances, an exemplary peak CD8+CAR+ T cell concentration in the blood that can achieve a therapeutic window, is or includes between approximately 1, 2, 3, 4, 5, 6, 7, 8, 9, 10, 15, 20, 20, 30, 40, 50 CD8+ CAR+ T cells per microliter in the blood and approximately 200, 300, 400, 500, 600, 700 or 750, CD8+ CAR+ T cells per microliter in the blood.

In some instances, the target therapeutic range or window is a peak CD3+CAR+ T cell concentration of between or between about 10 cells per microliter and 500 cells per microliter in the blood following administration. In some instances, the target therapeutic range or window is a peak CD8+CAR+ T cell concentration of between or between about 2 cells per microliter and 200 cells per microliter in the blood following administration.

In some instances, described are methods of dosing a subject that involves administering, to a subject having a disease or condition, a dose of genetically engineered cells comprising T cells expressing a chimeric antigen receptor (CAR), wherein the dose comprises a number of the genetically engineered cells that is sufficient to achieve peak CAR+ cells in the blood within a determined therapeutic range in the subject, or in a majority of subjects so treated by the method or in greater than 75% of the subjects so treated by the method, wherein the therapeutic range is: (i) based upon the range of peak CD3+ CAR+ T cells, or a CD8+CAR+ T cell subset thereof, in the blood among one or more subjects previously treated with the genetically engineered cells that is associated with an estimated probability of response of greater than or greater than about 65% and an estimated probability of a toxicity of less than or about 30%; or (ii) peak CD3+CAR+ T cells in the blood, following administration of the genetically engineered cells, that is between or between about 10 cells per microliter and 500 cells per microliter; or (iii) peak CD8+CAR+ T cells in the blood, following administration of the genetically engineered cells, that is between or between about 2 cells per microliter and 200 cells per microliter.

In some instances, described are methods of dosing a subject that involves (a) administering, to a subject having a disease or condition, a sub-optimal dose of genetically engineered cells comprising T cells engineered with a chimeric antigen receptor (CAR), wherein the dose comprises a number of the genetically engineered cells that is insufficient to achieve peak CAR+ cells in the blood within a determined therapeutic range in the subject, or in a majority of subjects so treated by the method or in greater than 75% of the subjects so treated by the method; and (b) subsequent to administering the genetically engineered cells, administering an agent to enhance CAR+ cell expansion or proliferation in the subject to achieve peak CAR+ T cells in the blood within the therapeutic range, wherein the therapeutic range is: (i) based upon the range of peak CD3+ CAR+ T cells, or a CD8+CAR+ T cell subset thereof, in the blood among one or more subjects previously treated with the genetically engineered cells that is associated with an estimated probability of response of greater than or greater than about 65% and an estimated probability of a toxicity of less than or about 30%; or (ii) peak CD3+CAR+ T cells in the blood, following administration of the genetically engineered cells, that is between or between about 10 cells per microliter and 500 cells per microliter; or (iii) peak CD8+CAR+ T cells in the blood, following administration of the genetically engineered cells, that is between or between about 2 cells per microliter and 200 cells per microliter. In some instances, the subject is administered a dose that can achieve the target therapeutic range or window. In some instances, the dose is less than or less than about 1 × 10⁷ CAR-expressing cells, less than or less than about 5 x 10⁶ CAR-expressing cells, less than or less than about 2.5 x 10⁶ CAR-expressing cells, less than or less than about 1 × 10⁶ CAR-expressing cells, less than or less than about 5 x 10⁵ CAR-expressing cells, less than or less than about 2.5 x 10⁵ CAR-expressing cells, less than or less than about 1 × 10⁵ CAR-expressing cells.

In the context of adoptive cell therapy, administration of a given "dose" encompasses administration of the given amount or number of cells as a single composition and/or single uninterrupted administration, e.g., as a single injection or continuous infusion, and also encompasses administration of the given amount or number of cells as a split dose, provided in multiple individual compositions or infusions, over a specified period of time, which is no more than 3 days. Thus, in some contexts, the dose is a single or continuous administration of the specified number of cells, given or initiated at a single point in time. In some contexts, however, the dose is administered in multiple injections or infusions over a period of no more than three days, such as once a day for three days or for two days or by multiple infusions over a single day period.

Thus, in some aspects, the cells of the dose are administered in a single pharmaceutical composition. In some instances, the cells of the dose are administered in a plurality of compositions, collectively containing the cells of the first dose.

The term "split dose" refers to a dose that is split so that it is administered over more than one day. This type of dosing is encompassed by the present methods and is considered to be a single dose.

Thus, the dose in some aspects may be administered as a split dose. For example, in some instances, the dose may be administered to the subject over 2 days or over 3 days. Exemplary methods for split dosing include administering 25% of the dose on the first day and administering the remaining 75% of the dose on the second day. In other instances, 33% of the first dose may be administered on the first day and the remaining 67% administered on the second day. In some aspects, 10% of the dose is administered on the first day, 30% of the dose is administered on the second day, and 60% of the dose is administered on the third day. In some instances, the split dose is not spread over more than 3 days.

In some instances, cells of the dose may be administered by administration of a plurality of compositions or solutions, such as a first and a second, optionally more, each containing some cells of the dose. In some aspects, the plurality of compositions, each containing a different population and/or sub-types of cells, are administered separately or independently, optionally within a certain period of time. For example, the populations or sub-types of cells can include CD8⁺ and CD4⁺ T cells, respectively, and/or CD8+- and CD4+-enriched populations, respectively, e.g., CD4+ and/or CD8+ T cells each individually including cells genetically engineered to express the recombinant receptor. In some instances, the administration of the dose comprises administration of a first composition comprising a dose of CD8+ T cells or a dose of CD4+ T cells and administration of a second composition comprising the other of the dose of CD4+ T cells and the CD8+ T cells.

In some instances, the administration of the composition or dose, e.g., administration of the plurality of cell compositions, involves administration of the cell compositions separately. In some aspects, the separate administrations are carried out simultaneously, or sequentially, in any order. In some instances, the dose comprises a first composition and a second composition, and the first composition and second composition are administered 0 to 12 hours apart, 0 to 6 hours apart or 0 to 2 hours apart. In some instances, the initiation of administration of the first composition and the initiation of administration of the second composition are carried out no more than 2 hours, no more than 1 hour, or no more than 30 minutes apart, no more than 15 minutes, no more than 10 minutes or no more than 5 minutes apart. In some instances, the initiation and/or completion of administration of the first composition and the completion and/or initiation of administration of the second composition are carried out no more than 2 hours, no more than 1 hour, or no more than 30 minutes apart, no more than 15 minutes, no more than 10 minutes or no more than 5 minutes apart.

In some composition, the first composition, e.g., first composition of the dose, comprises CD4+ T cells. In some composition, the first composition, e.g., first composition of the dose, comprises CD8+ T cells. In some instances, the first composition is administered prior to the second composition.

In some instances, the dose or composition of cells includes a defined or target ratio of CD4+ cells expressing a recombinant receptor to CD8+ cells expressing a recombinant receptor and/or of CD4+ cells to CD8+ cells, which ratio optionally is approximately 1:1 or is between approximately 1:3 and approximately 3:1, such as approximately 1:1. In some aspects, the administration of a composition or dose with the target or desired ratio of different cell populations (such as CD4+:CD8+ ratio or CAR+CD4+:CAR+CD8+ ratio, e.g., 1:1) involves the administration of a cell composition containing one of the populations and then administration of a separate cell composition comprising the other of the populations, where the administration is at or approximately at the target or desired ratio.

In some instances, one or more consecutive or subsequent dose of cells can be administered to the subject. In some instances, the consecutive or subsequent dose of cells is administered greater than or greater than about 7 days, 14 days, 21 days, 28 days or 35 days after initiation of administration of the first dose of cells. The consecutive or subsequent dose of cells can be more than, approximately the same as, or less than the first dose. In some instances, administration of the T cell therapy, such as administration of the first and/or second dose of cells, can be repeated.

In some instances, a dose of cells is administered to subjects in accord with the described methods. In some instances, the size or timing of the doses is determined as a function of the particular disease or condition in the subject. It is within the level of a skilled artisan to empirically determine the size or timing of the doses for a particular disease. Dosages may vary depending on attributes particular to the disease or disorder and/or patient and/or other treatments.

In some aspects, the time between the administration of the first dose and the administration of the consecutive dose is about 9 to about 35 days, about 14 to about 28 days, or 15 to 27 days. In some instances, the administration of the consecutive dose is at a time point more than about 14 days after and less than about 28 days after the administration of the first dose. In some aspects, the time between the first and consecutive dose is about 21 days. In some instances, an additional dose or doses, e.g. consecutive doses, are administered following administration of the consecutive dose. In some aspects, the additional consecutive dose or doses are administered at least about 14 and less than about 28 days following administration of a prior dose. In some instances, the additional dose is administered less than about 14 days following the prior dose, for example, 4, 5, 6, 7, 8, 9, 10, 11, 12, or 13 days after the prior dose. In some instances, no dose is administered less than about 14 days following the prior dose and/or no dose is administered more than about 28 days after the prior dose.

In some instances, the dose of cells, e.g., recombinant receptor-expressing cells, comprises two doses (e.g., a double dose), comprising a first dose of the T cells and a consecutive dose of the T cells, wherein one or both of the first dose and the second dose comprises administration of the split dose of T cells.

In certain instances, the cells, or individual populations of sub-types of cells, are administered to the subject at a range of about 0.1 million to about 100 billion cells and/or that amount of cells per kilogram of body weight of the subject, such as, *e.g.,* 0.1 million to about 50 billion cells (*e.g.,* about 5 million cells, about 25 million cells, about 500 million cells, about 1 billion cells, about 5 billion cells, about 20 billion cells, about 30 billion cells, about 40 billion cells, or a range defined by any two of the foregoing values), 1 million to about 50 billion cells (*e.g.,* about 5 million cells, about 25 million cells, about 500 million cells, about 1 billion cells, about 5 billion cells, about 20 billion cells, about 30 billion cells, about 40 billion cells, or a range defined by any two of the foregoing values), such as about 10 million to about 100 billion cells (*e.g.,* about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or a range defined by any two of the foregoing values), and in some cases about 100 million cells to about 50 billion cells (*e.g.,* about 120 million cells, about 250 million cells, about 350 million cells, about 450 million cells, about 650 million cells, about 800 million cells, about 900 million cells, about 3 billion cells, about 30 billion cells, about 45 billion cells) or any value in between these ranges and/or per kilogram of body weight of the subject. Dosages may vary depending on attributes particular to the disease or disorder and/or patient and/or other treatments. In some instances, such values refer to numbers of recombinant receptor-expressing cells; in other instances, they refer to number of T cells or PBMCs or total cells administered.

In some instances, the cell therapy comprises administration of a dose comprising a number of cells that is at least or at least about or is or is about 0.1 × 10⁶ cells/kg body weight of the subject, 0.2 × 10⁶ cells/kg, 0.3 × 10⁶ cells/kg, 0.4 × 10⁶ cells/kg, 0.5 × 10⁶ cells/kg, 1 × 10⁶ cell/kg, 2.0 × 10⁶ cells/kg, 3 × 10⁶ cells/kg or 5 × 10⁶ cells/kg.

In some instances, the cell therapy comprises administration of a dose comprising a number of cells is between or between about 0.1 × 10⁶ cells/kg body weight of the subject and 1.0 × 10⁷ cells/kg, between or between about 0.5 × 10⁶ cells/kg and 5 × 10⁶ cells/kg, between or between about 0.5 × 10⁶ cells/kg and 3 × 10⁶ cells/kg, between or between about 0.5 × 10⁶ cells/kg and 2 × 10⁶ cells/kg, between or between about 0.5 × 10⁶ cells/kg and 1 × 10⁶ cell/kg, between or between about 1.0 × 10⁶ cells/kg body weight of the subject and 5 × 10⁶ cells/kg, between or between about 1.0 × 10⁶ cells/kg and 3 × 10⁶ cells/kg, between or between about 1.0 × 10⁶ cells/kg and 2 × 10⁶ cells/kg, between or between about 2.0 × 10⁶ cells/kg body weight of the subject and 5 × 10⁶ cells/kg, between or between about 2.0 × 10⁶ cells/kg and 3 × 10⁶ cells/kg, or between or between about 3.0 × 10⁶ cells/kg body weight of the subject and 5 × 10⁶ cells/kg, each inclusive.

In some instances, the dose of cells comprises between at or about 2 x 10⁵ of the cells/kg and at or about 2 x 10⁶ of the cells/kg, such as between at or about 4 x 10⁵ of the cells/kg and at or about 1 x 10⁶ of the cells/kg or between at or about 6 x 10⁵ of the cells/kg and at or about 8 x 10⁵ of the cells/kg. In some instances, the dose of cells comprises no more than 2 x 10⁵ of the cells (*e.g.* antigen-expressing, such as CAR-expressing cells) per kilogram body weight of the subject (cells/kg), such as no more than at or about 3 x 10⁵ cells/kg, no more than at or about 4 x 10⁵ cells/kg, no more than at or about 5 x 10⁵ cells/kg, no more than at or about 6 x 10⁵ cells/kg, no more than at or about 7 x 10⁵ cells/kg, no more than at or about 8 x 10⁵ cells/kg, nor more than at or about 9 x 10⁵ cells/kg, no more than at or about 1 × 10⁶ cells/kg, or no more than at or about 2 x 10⁶ cells/kg. In some instances, the dose of cells comprises at least or at least about or at or about 2 × 10⁵ of the cells (*e.g.* antigen-expressing, such as CAR-expressing cells) per kilogram body weight of the subject (cells/kg), such as at least or at least about or at or about 3 x 10⁵ cells/kg, at least or at least about or at or about 4 x 10⁵ cells/kg, at least or at least about or at or about 5 x 10⁵ cells/kg, at least or at least about or at or about 6 x 10⁵ cells/kg, at least or at least about or at or about 7 x 10⁵ cells/kg, at least or at least about or at or about 8 x 10⁵ cells/kg, at least or at least about or at or about 9 x 10⁵ cells/kg, at least or at least about or at or about 1 × 10⁶ cells/kg, or at least or at least about or at or about 2 x 10⁶ cells/kg.

In some instances, the cells are administered at a desired dosage, which in some aspects includes a desired dose or number of cells or cell type(s) and/or a desired ratio of cell types. Thus, the dosage of cells in some instances is based on a total number of cells (or number per kg body weight) and a desired ratio of the individual populations or sub-types, such as the CD4+ to CD8+ ratio. In some instances, the dosage of cells is based on a desired total number (or number per kg of body weight) of cells in the individual populations or of individual cell types. In some instances, the dosage is based on a combination of such features, such as a desired number of total cells, desired ratio, and desired total number of cells in the individual populations.

In some instances, the populations or sub-types of cells, such as CD8⁺ and CD4⁺ T cells, are administered at or within a tolerated difference of a desired dose of total cells, such as a desired dose of T cells. In some aspects, the desired dose is a desired number of cells or a desired number of cells per unit of body weight of the subject to whom the cells are administered, *e.g.,* cells/kg. In some aspects, the desired dose is at or above a minimum number of cells or minimum number of cells per unit of body weight. In some aspects, among the total cells, administered at the desired dose, the individual populations or sub-types are present at or near a desired output ratio (such as CD4⁺ to CD8⁺ ratio), *e.g.,* within a certain tolerated difference or error of such a ratio.

In some instances, the cells are administered at or within a tolerated difference of a desired dose of one or more of the individual populations or sub-types of cells, such as a desired dose of CD4+ cells and/or a desired dose of CD8+ cells. In some aspects, the desired dose is a desired number of cells of the sub-type or population, or a desired number of such cells per unit of body weight of the subject to whom the cells are administered, *e.g.,* cells/kg. In some aspects, the desired dose is at or above a minimum number of cells of the population or sub-type, or minimum number of cells of the population or sub-type per unit of body weight.

Thus, in some instances, the dosage is based on a desired fixed dose of total cells and a desired ratio, and/or based on a desired fixed dose of one or more, *e.g.,* each, of the individual sub-types or sub-populations. Thus, in some instances, the dosage is based on a desired fixed or minimum dose of T cells and a desired ratio of CD4⁺ to CD8⁺ cells, and/or is based on a desired fixed or minimum dose of CD4⁺ and/or CD8⁺ cells.

In some instances, the cells are administered at or within a tolerated range of a desired output ratio of multiple cell populations or sub-types, such as CD4+ and CD8+ cells or sub-types. In some aspects, the desired ratio can be a specific ratio or can be a range of ratios. for example, in some instances, the desired ratio (*e.g.,* ratio of CD4⁺ to CD8⁺ cells) is between at or about 5:1 and at or about 5:1 (or greater than about 1:5 and less than about 5:1), or between at or about 1:3 and at or about 3:1 (or greater than about 1:3 and less than about 3:1), such as between at or about 2:1 and at or about 1:5 (or greater than about 1:5 and less than about 2:1, such as at or about 5:1, 4.5:1, 4:1, 3.5:1, 3:1, 2.5:1, 2:1, 1.9:1, 1.8:1, 1.7:1, 1.6:1, 1.5:1, 1.4:1, 1.3:1, 1.2:1, 1.1:1, 1:1, 1:1.1, 1:1.2, 1:1.3, 1:1.4, 1:1.5, 1:1.6, 1:1.7, 1:1.8, 1:1.9: 1:2, 1:2.5, 1:3, 1:3.5, 1:4, 1:4.5, or 1:5. In some aspects, the tolerated difference is within about 1%, about 2%, about 3%, about 4% about 5%, about 10%, about 15%, about 20%, about 25%, about 30%, about 35%, about 40%, about 45%, about 50% of the desired ratio, including any value in between these ranges.

In particular instances, the numbers and/or concentrations of cells refer to the number of recombinant receptor (*e.g.,* CAR)-expressing cells. In other instances, the numbers and/or concentrations of cells refer to the number or concentration of all cells, T cells, or peripheral blood mononuclear cells (PBMCs) administered.

In some instances, for example, where the subject is a human, the dose includes fewer than about 5 x 10⁸ total recombinant receptor (e.g., CAR)-expressing cells, T cells, or peripheral blood mononuclear cells (PBMCs), e.g., in the range of about 1 × 10⁶ to 5 x 10⁸ such cells, such as 2 x 10⁶, 5 x 10⁶, 1 × 10⁷, 5 x 10⁷, 1 × 10⁸, or 5 x 10⁸ total such cells, or the range between any two of the foregoing values.

In some instances, the dose of genetically engineered cells comprises from or from about 1 × 10⁵ to 5 x 10⁸ total CAR-expressing T cells, 1 × 10⁵ to 2.5 x 10⁸ total CAR-expressing T cells, 1 × 10⁵ to 1 × 10⁸ total CAR-expressing T cells, 1 × 10⁵ to 5 x 10⁷ total CAR-expressing T cells, 1 × 10⁵ to 2.5 x 10⁷ total CAR-expressing T cells, 1 × 10⁵ to 1 × 10⁷ total CAR-expressing T cells, 1 × 10⁵ to 5 x 10⁶ total CAR-expressing T cells, 1 × 10⁵ to 2.5 x 10⁶ total CAR-expressing T cells, 1 × 10⁵ to 1 × 10⁶ total CAR-expressing T cells, 1 × 10⁶ to 5 x 10⁸ total CAR-expressing T cells, 1 × 10⁶ to 2.5 x 10⁸ total CAR-expressing T cells, 1 × 10⁶ to 1 × 10⁸ total CAR-expressing T cells, 1 × 10⁶ to 5 x 10⁷ total CAR-expressing T cells, 1 × 10⁶ to 2.5 x 10⁷ total CAR-expressing T cells, 1 × 10⁶ to 1 × 10⁷ total CAR-expressing T cells, 1 × 10⁶ to 5 x 10⁶ total CAR-expressing T cells, 1 × 10⁶ to 2.5 x 10⁶ total CAR-expressing T cells, 2.5 x 10⁶ to 5 x 10⁸ total CAR-expressing T cells, 2.5 x 10⁶ to 2.5 x 10⁸ total CAR-expressing T cells, 2.5 x 10⁶ to 1 × 10⁸ total CAR-expressing T cells, 2.5 × 10⁶ to 5 x 10⁷ total CAR-expressing T cells, 2.5 x 10⁶ to 2.5 x 10⁷ total CAR-expressing T cells, 2.5 x 10⁶ to 1 × 10⁷ total CAR-expressing T cells, 2.5 x 10⁶ to 5 x 10⁶ total CAR-expressing T cells, 5 x 10⁶ to 5 x 10⁸ total CAR-expressing T cells, 5 x 10⁶ to 2.5 x 10⁸ total CAR-expressing T cells, 5 x 10⁶ to 1 × 10⁸ total CAR-expressing T cells, 5 x 10⁶ to 5 x 10⁷ total CAR-expressing T cells, 5 x 10⁶ to 2.5 x 10⁷ total CAR-expressing T cells, 5 x 10⁶ to 1 x 10⁷ total CAR-expressing T cells, 1 x 10⁷ to 5 x 10⁸ total CAR-expressing T cells, 1 x 10⁷ to 2.5 x 10⁸ total CAR-expressing T cells, 1 x 10⁷ to 1 x 10⁸ total CAR-expressing T cells, 1 x 10⁷ to 5 x 10⁷ total CAR-expressing T cells, 1 x 10⁷ to 2.5 x 10⁷ total CAR-expressing T cells, 2.5 x 10⁷ to 5 x 10⁸ total CAR-expressing T cells, 2.5 x 10⁷ to 2.5 x 10⁸ total CAR-expressing T cells, 2.5 x 10⁷ to 1 x 10⁸ total CAR-expressing T cells, 2.5 x 10⁷ to 5 x 10⁷ total CAR-expressing T cells, 5 x 10⁷ to 5 x 10⁸ total CAR-expressing T cells, 5 x 10⁷ to 2.5 x 10⁸ total CAR-expressing T cells, 5 x 10⁷ to 1 x 10⁸ total CAR-expressing T cells, 1 x 10⁸ to 5 x 10⁸ total CAR-expressing T cells, 1 x 10⁸ to 2.5 x 10⁸ total CAR-expressing T cells, or 2.5 x 10⁸ to 5 x 10⁸ total CAR-expressing T cells.

In some instances, the dose of genetically engineered cells comprises at least or at least about 1 x 10⁵ CAR-expressing cells, at least or at least about 2.5 x 10⁵ CAR-expressing cells, at least or at least about 5 x 10⁵ CAR-expressing cells, at least or at least about 1 x 10⁶ CAR-expressing cells, at least or at least about 2.5 x 10⁶ CAR-expressing cells, at least or at least about 5 x 10⁶ CAR-expressing cells, at least or at least about 1 x 10⁷ CAR-expressing cells, at least or at least about 2.5 x 10⁷ CAR-expressing cells, at least or at least about 5 x 10⁷ CAR-expressing cells, at least or at least about 1 x 10⁸ CAR-expressing cells, at least or at least about 2.5 x 10⁸ CAR-expressing cells, or at least or at least about 5 x 10⁸ CAR-expressing cells.

In some instances, the cell therapy comprises administration of a dose comprising a number of cell from or from about 1 x 10⁵ to 5 x 10⁸ total recombinant receptor-expressing cells, total T cells, or total peripheral blood mononuclear cells (PBMCs), from or from about 5 x 10⁵ to 1 x 10⁷ total recombinant receptor-expressing cells, total T cells, or total peripheral blood mononuclear cells (PBMCs) or from or from about 1 x 10⁶ to 1 x 10⁷ total recombinant receptor-expressing cells, total T cells, or total peripheral blood mononuclear cells (PBMCs), each inclusive. In some instances, the cell therapy comprises administration of a dose of cells comprising a number of cells at least or at least about 1 x 10⁵ total recombinant receptor-expressing cells, total T cells, or total peripheral blood mononuclear cells (PBMCs), such at least or at least 1 x 10⁶, at least or at least about 1 x 10⁷, at least or at least about 1 x 10⁸ of such cells. In some instances, the number is with reference to the total number of CD3+ or CD8+, in some cases also recombinant receptor-expressing (e.g. CAR+) cells. In some instances, the cell therapy comprises administration of a dose comprising a number of cell from or from about 1 x 10⁵ to 5 x 10⁸ CD3+ or CD8+ total T cells or CD3+ or CD8+ recombinant receptor-expressing cells, from or from about 5 x 10⁵ to 1 x 10⁷ CD3+ or CD8+ total T cells or CD3+ or CD8+ recombinant receptor-expressing cells, or from or from about 1 x 10⁶ to 1 x 10⁷ CD3+ or CD8+ total T cells or CD3+ or CD8+recombinant receptor-expressing cells, each inclusive. In some instances, the cell therapy comprises administration of a dose comprising a number of cell from or from about 1 x 10⁵ to 5 x 10⁸ total CD3+/CAR+ or CD8+/CAR+ cells, from or from about 5 x 10⁵ to 1 x 10⁷ total CD3+/CAR+ or CD8+/CAR+ cells, or from or from about 1 x 10⁶ to 1 x 10⁷ total CD3+/CAR+ or CD8+/CAR+ cells, each inclusive.

In some instances, the T cells of the dose include CD4+ T cells, CD8+ T cells or CD4+ and CD8+ T cells.

In some instances, for example, where the subject is human, the CD8+ T cells of the dose, including in a dose including CD4+ and CD8+ T cells, includes between about 1 x 10⁶ and 5 x 10⁸ total recombinant receptor (e.g., CAR)-expressing CD8+cells, e.g., in the range of about 5 x 10⁶ to 1 x 10⁸ such cells, such cells 1 x 10⁷, 2.5 x 10⁷, 5 x 10⁷, 7.5 x 10⁷, 1 x 10⁸, or 5 x 10⁸ total such cells, or the range between any two of the foregoing values. In some instances, the patient is administered multiple doses, and each of the doses or the total dose can be within any of the foregoing values. In some instances, the dose of cells comprises the administration of from or from about 1 x 10⁷ to 0.75 x 10⁸ total recombinant receptor-expressing CD8+ T cells, 1 x 10⁷ to 2.5 x 10⁷ total recombinant receptor-expressing CD8+ T cells, from or from about 1 x 10⁷ to 0.75 x 10⁸ total recombinant receptor-expressing CD8+ T cells, each inclusive. In some instances, the dose of cells comprises the administration of or about 1 x 10', 2.5 x 10⁷, 5 x 10⁷ 7.5 x 10⁷, 1 x 10⁸, or 5 x 10⁸ total recombinant receptor-expressing CD8+ T cells.

In some instances, the dose of cells, e.g., recombinant receptor-expressing T cells, is administered to the subject as a single dose or is administered only one time within a period of two weeks, one month, three months, six months, 1 year or more.

In some aspects, the size of the dose is determined based on one or more criteria such as response of the subject to prior treatment, *e.g.* chemotherapy, disease burden in the subject, such as tumor load, bulk, size, or degree, extent, or type of metastasis, stage, and/or likelihood or incidence of the subject developing toxic outcomes, *e.g.,* CRS, macrophage activation syndrome, tumor lysis syndrome, neurotoxicity, and/or a host immune response against the cells and/or recombinant receptors being administered.

### IV.METHODS OF MONITORING, ASSESSMENT AND MODULATING THERAPY

In some instances, described are methods of treatment. In some instances, the methods include administering an immunotherapy and/or a cell therapy. In some instances, the methods involve administration of genetically engineered cells, e.g., cells engineered to express a recombinant receptor such as a chimeric antigen receptor (CAR). In some instances, the methods include administering a dose of cells, e.g., CAR+ expressing cells, to a subject such that the cells are within a target therapeutic range or window. In some instances, the methods also involve monitoring parameters, e.g., pharmacokinetic parameters, such as peak cell concentration (Cmax), to determine whether the cells in the subject is within the therapeutic range or window. In some instances, if the cells are not within the therapeutic range or window, the treatment can be modified, e.g., by administering additional doses, altering subsequent or additional doses, and/or by administering an agent that can modulate CAR+ T cell expansion, proliferation and/or activity. In some aspects, the described methods also include a method of determining a dose of a subject, or a method of dosing a subject, based on an assessment of the parameters, e.g., pharmacokinetic parameters, such as peak cell concentration (Cmax), patient attributes and/or biomarkers.

In some aspects, described are methods of modulating a therapy, e.g., a cell therapy such as a T cell therapy with recombinant receptor-expressing cells. In some instances, the cell therapy is modulated by administering to the subject receiving cell therapy an agent to the subject capable of modulating CAR+ T cell expansion, proliferation, expansion, survival, activity and/or function,, e.g., increases or decreases CAR+ T cell expansion, proliferation, survival and/or activity.

In some instances, the agent is administered after assessment of pharmacokinetic parameters, e.g., peak CAR+ T cell concentration, exposure (e.g., AUC) and/or cell level or concentration. In some instances, the agent is administered after assessment of other parameters, such as patient attributes, factors, characteristics and/or expression of biomarkers, that is associated with and/or correlated with pharmacokinetic parameters, response, durable response and/or development of toxicity.

In some instances, described are methods of treatment that involves administering, to a subject having a disease or condition, a dose of genetically engineered cells comprising T cells expressing a recombinant receptor, such as a chimeric antigen receptor (CAR) for treating the disease or condition. In some instances, the method involves after administering the dose of genetically engineered cells, monitoring pharmacokinetic parameters, e.g., CAR+ T cells, in the blood of the subject to assess if the cells are within a therapeutic range or window. In some instances, the method involves administering an agent to the subject capable of modulating, optionally increasing or decreasing, CAR+ T cell expansion, proliferation and/or activity, in the subject if the genetically engineered cells are not within the therapeutic range.

In some instances, also described are methods of treatment that involves monitoring, in the blood of a subject, the presence of genetically engineered cells comprising T cells expressing a chimeric antigen receptor (CAR) to assess if the cells are within a therapeutic range, wherein the subject has been previously administered a dose of the genetically engineered cells for treating a disease or condition. In some instances, the methods also involve administering an agent capable of modulating, optionally increasing or decreasing, CAR+ T cell expansion, proliferation and/or activity, in the subject if the genetically engineered cells are not within the therapeutic range.

In some aspects, if the peak number of CAR+ T cells in the blood of the subject is less than the lowest number of peak CAR+ T cells in the therapeutic range, an agent is administered to the subject that is capable of increasing CAR+ T cell expansion or proliferation. In some aspects, if the peak number of CAR+ T cells in the blood of the subject is greater than the highest number of peak CAR+ T cells in the therapeutic range, an agent is administered to the subject that is capable of decreasing CAR+ T cell expansion or proliferation.

In some instances, also described are methods of modulating activity of engineered cells. In some instances, the methods involves assessing the level, amount or concentration of a parameter, such as a volumetric measure of tumor burden or an inflammatory marker, in a sample from the subject is at or above a threshold level. In some instances, the sample does not comprise genetically engineered T cells expressing a chimeric antigen receptor (CAR) and/or is obtained from the subject prior to receiving administration of genetically engineered T cells expressing a CAR. In some instances, a subject is selected for administration of an agent capable of decreasing expansion or proliferation of genetically engineered T cells expressing a CAR. In some instances, the agent capable of decreasing expansion or proliferation of genetically engineered T cells expressing a CAR is administered to the subject.

In some instances, also described are methods of modulating activity of engineered cells, that involves administering to a subject an agent capable of decreasing expansion or proliferation of genetically engineered T cells expressing a chimeric antigen receptor (CAR) in a subject, wherein the subject is one in which the level, amount or concentration of a parameter, e.g., a volumetric measure of tumor burden or an inflammatory marker in a sample from the subject is at or above a threshold level.

In some instances, the described methods involve administration of a genetically engineered cell, e.g., a T cell engineered to express a recombinant receptor, e.g., CAR. In some instances, an agent capable of modulating modulating, e.g., increasing or decreasing, CAR+ T cell expansion, proliferation and/or activity, is administered prior to or concurrently with initiation of administration of a dose of genetically engineered cells comprising T cells expressing a chimeric antigen receptor. In some aspects, prior to administering the agent, the selected subject is at risk of developing a toxicity following administration of the genetically engineered cells. In some instances, the administration of the agent is sufficient to achieve peak CAR+ T cells in a therapeutic range or window in the subject. In some instances, the administration of the agent is sufficient to achieve peak CAR+ T cell concentrations, in the blood in a majority of subjects so treated by the method, or greater than or greater than about 50%, 60%, 70%, 75%, 80%, 85%, 90% or 95% or more, such as greater than 75% of the subjects so treated by the method, is within a determined target therapeutic range or window.

In some instances, also described are methods of dosing a subject. In some instances, the methods involve administering, to a subject having a disease or condition, a sub-optimal dose of genetically engineered cells comprising T cells engineered with a chimeric antigen receptor (CAR), wherein the dose comprises a number of the genetically engineered cells that is insufficient to achieve peak CAR+ cells in the blood within a determined therapeutic range in the subject, or in a majority of subjects so treated by the method or in greater than 75% of the subjects so treated by the method. In some instances, the methods involve administering an agent to enhance CAR+ cell expansion or proliferation in the subject to achieve peak CAR+ T cells in the blood within the therapeutic range or window, subsequent to administering the genetically engineered cells. In some instances, the dose of genetically engineered cells is less than or less than about 1 x 10⁷ CAR-expressing cells, less than or less than about 5 x 10⁶ CAR-expressing cells, less than or less than about 2.5 x 10⁶ CAR-expressing cells, less than or less than about 1 x 10⁶ CAR-expressing cells, less than or less than about 5 x 10⁵ CAR-expressing cells, less than or less than about 2.5 x 10⁵ CAR-expressing cells, less than or less than about 1 x 10⁵ CAR-expressing cells.

In some instances, following administration of the agent, the method achieves an increased frequency of peak CAR+ cells in the blood within a determined therapeutic range in the subject, compared to a method involving administration of the same dose of genetically engineered cells but without the agent; or peak CAR+ cells in the blood within a determined therapeutic range in the subject, or in a majority of subjects so treated by the method or in greater than 75% of the subjects so treated by the method.

In some instances, the therapeutic range or window is determined as described herein, e.g., in Section II or elsewhere. In some instances, the therapeutic range is based upon the range of peak CD3+ CAR+ T cells, or a CD8+CAR+ T cell subset thereof, in the blood among one or more subjects previously treated with the genetically engineered cells that is associated with an estimated probability of response of greater than or greater than about 65%, 70%, 75%, 80%, 85%, 90%, 95% or more, and an estimated probability of a toxicity of less than or about 30%, 25%, 20%, 15%, 10%, 5% or less.

In some instances, the therapeutic window or range is determined based on specific range of numbers and/or concentrations of cells, e.g., CD3+, CD4+ or CD8+ T cells. In some instances, an exemplary peak CD3+CAR+ T cell concentration in the blood that can achieve a therapeutic window, is or includes between approximately 1, 2, 3, 4, 5, 6, 7, 8, 9, 10, 15, 20, 20, 30, 40, 50 CD3+ CAR+ T cells per microliter in the blood and approximately 200, 300, 400, 500, 600, 700 or 750, CD3+ CAR+ T cells per microliter in the blood. In some instances, an exemplary peak CD8+CAR+ T cell concentration in the blood that can achieve a therapeutic window, is or includes between approximately 1, 2, 3, 4, 5, 6, 7, 8, 9, 10, 15, 20, 20, 30, 40, 50 CD8+ CAR+ T cells per microliter in the blood and approximately 200, 300, 400, 500, 600, 700 or 750, CD8+ CAR+ T cells per microliter in the blood.

In some instances, the methods also involve monitoring the CAR+ T cells in the blood of the subject after administering the dose of genetically engineered cells.

In some instances, the subject is monitored for CAR+ T cells in the blood at a time that is at least 8 days, 9 days, 10 days, 11 days, 12 days, 13 days, 14 days, 15 days, 16 days, 17 days, 18 days, 19 days, 20 days or 21 days after initiation of administration of the genetically engineered cells. In some instances, the subject is monitored for CAR+ T cells in the blood at a time that is between or between about 11 to 22 says, 12 to 18 days or 14 to 16 days, each inclusive, after initiation of administration of the genetically engineered cells.

Also described are methods of assessing likelihood of a response or a durable response, and methods of administering a therapeutic agent accordingly. In some instances, the methods involve detecting, in a biological sample from a subject, peak levels of one or more inflammatory marker and/or peak levels of genetically engineered cells comprising T cells expressing a chimeric antigen receptor (CAR), wherein the subject has been previously administered a dose of the genetically engineered cells for treating a disease or condition. In some instances, the methods involve comparing, individually, the peak levels to a threshold value, thereby determining a likelihood that a subject will achieve a durable response to the administration of the genetically engineered cells.

In some instances, the subject is likely to achieve a response or a durable response if the peak levels of the one or more inflammatory marker is below a threshold value and the subject is not likely to achieve a durable response if the peak levels of the one or more inflammatory marker are above a threshold value. In some instances, the subject is likely to achieve a durable response if the peak level of the genetically engineered cells is within a therapeutic range between a lower threshold value and an upper threshold value and the subject is not likely to achieve a durable response if the peak level of the genetically engineered cells is below the lower threshold value or is above the upper threshold value.

In some instances, the threshold value is a value that: is within 25%, within 20%, within 15%, within 10%, or within 5% above the average value of the volumetric measure or inflammatory marker and/or is within a standard deviation above the average value of the volumetric measure or the inflammatory marker in a plurality of control subjects. In some instances, the threshold value is a value that: is above the highest value of the volumetric measure or inflammatory marker, optionally within 50%, within 25%, within 20%, within 15%, within 10%, or within 5% above such highest fold change, measured in at least one subject from among a plurality of control subjects. In some instances, the threshold value is a value that: is above the highest value of the volumetric measure or inflammatory marker as measured among more than 75%, 80%, 85%, 90%, or 95%, or 98% of subjects from a plurality of control subjects. In some instances, the plurality of control subjects are a group of subjects prior to receiving a dose of the genetically engineered cells, wherein: each of the control subjects of the group exhibited a peak CAR+ T cells in the blood greater than the highest peak CAR+ T cells in the therapeutic range; each of the control subjects of the group went on to develop at toxicity, optionally a neurotoxicity or cytokine release syndrome (CRS), a grade 2 or grade 3 or higher neurotoxicity or a grade 3 or higher CRS, after receiving a dose of the engineered cells for treating the same disease or condition; each of the control subjects of the group did not develop a response, optionally a complete response (CR) or partial response (PR), following administration of the dose of genetically engineered cells; and/or each of the control subjects of the group did not develop a durable response, optionally for at or about or greater than or about 3 months or at or about or greater than or about 6 months, following administration of the dose of genetically engineered cells.

In some instances, the methods also involve administering an agent or an alternative therapy, based on the assessment of the likelihood of achieving a response or a durable response. In some instances, if the subject is determined not likely to achieve a response or durable response, the subject is selected for treatment with a therapeutic agent or with an alternative therapeutic treatment other than the genetically engineered cells. In some instances, if the subject is determined not likely to achieve a response or durable response, a therapeutic agent or an alternative therapeutic treatment other than the genetically engineered cells is administered to the subject.

In some instances, also described are methods of treatment that involves selecting a subject for administration of a therapeutic agent and/or alternative therapeutic treatment. In some instances, the methods involve selecting a subject having received administration of genetically engineered cells comprising T cells expressing a chimeric antigen receptor (CAR) in which: peak levels of one or more inflammatory markers in a sample from the subject is above a threshold value; and/or peak level of T cells comprising a chimeric antigen receptor (CAR) in a sample from the subject is below a lower threshold value or is above an upper threshold value.

In some instances, the response is a complete response (CR), objective response (OR) or partial response (PR). In some instances, the response is durable for at or greater than 3 months, 4 months, 5 months, or 6 months.

In some instances, the peak levels are assessed and/or the sample is obtained from the subject at a time that is at least 8 days, 9 days, 10 days, 11 days, 12 days, 13 days, 14 days, 15 days, 16 days, 17 days, 18 days, 19 days, 20 days or 21 days after initiation of administration of the genetically engineered cells. In some instances, the peak levels are assessed and/or the sample is obtained from the subject at a time that is between or between about 11 to 22 days, 12 to 18 days or 14 to 16 days, each inclusive, after initiation of administration of the genetically engineered cells.

In some instances, the peak level is or includes the peak level of one or more inflammatory markers, e.g., C reactive protein (CRP), IL-2, IL-6, IL-10, IL-15, TNF-alpha, MIP-1alpha, MIP-1beta, MCP-1, CXCL10 or CCL13.

In some instances, the peak level of one or more inflammatory marker is assessed and the threshold value is within 25%, within 20%, within 15%, within 10% or within 5% and/or is within a standard deviation of the median or mean of the peak level of the inflammatory marker as determined among a group of control subjects having received administration of the genetically engineered cells, wherein each of the subjects of the group did not achieve a durable response, optionally a CR and/or PR, optionally at or greater than 3 months or 6 months following administration of the genetically engineered cells. In some instances, the control subjects exhibited stable disease (SD) or progressive disease (PD) following administration of the genetically engineered cells, optionally at or greater than 3 months or 6 months following administration of the genetically engineered cells. In some instances, the peak level is a peak level of CAR+ T cells, or a CD8+ T cell subset thereof.

In some instances, the lower threshold value and upper threshold value is the lower and upper end, respectively, of a therapeutic range of peak CD3+ CAR+ T cells, or a CD8+CAR+ T cell subset thereof, in the blood among one or more subjects previously treated with the genetically engineered cells that is associated with an estimated probability of response of greater than or greater than about 65% and an estimated probability of a toxicity of less than or about 30%. In some instances, the therapeutic range is the range in which the estimated probability of toxicity is less than 20%, less than 15%, less than 10% or less than 5% and the estimated probability of achieving a response is greater than 65%, 70%, 75%, 80%, 85%, 90%, 95% or more.

In some instances, the probability of response is based on a response that is a complete response (CR), an objective response (OR) or a partial response (PR), optionally wherein the response is durable, optionally durable for at or at least 3 months or at or at least 6 months.

In some instances, peak CAR+ T cells is determined as the number of CAR+ T cells per microliter in the blood of the subject. In some instances, the upper threshold value is between or between about 300 cells per microliter and 1000 cells per microliter or 400 cells per microliter and 600 cells per microliter, or is about 300 cells per microliter, 400 cells per microliter, 500 cells per microliter, 600 cells per microliter, 700 cells per microliter, 800 cells per microliter, 900 cells per microliter or 1000 cells per microliter; or the lower threshold value is less than or less than about 10 cells per microliter, 9 cells per microliter, 8 cells per microliter, 7 cells per microliter, 6 cells per microliter, 5 cells per microliter, 4 cells per microliter, 3 cells per microliter, 2 cells per microliter or 1 cell per microliter.

In some instances of the methods described herein, among a plurality of subjects treated, the method achieves an increase in the percentage of subjects achieving a durable response, optionally a complete response (CR) or objective response (OR) or a partial response (PR), optionally that is durable for at or greater than 3 months or at or greater than 6 months, compared to a method that does not comprise administering the agent. In some instances, the increase is greater than or greater than about 1.2-fold, 1.5-fold, 2-fold, 3-fold, 4-fold, 5-fold, 10-fold or more.

In some instances, at least 15%, at least 20%, at least 25%, at least 30%, at least 35%, at least 40 % or at least 50% of subjects treated according to the method achieve a complete response (CR) that is durable for at or greater than 3 months or at or greater than 6 months; and/or at least 25%, at least 30%, at least 40%, at least 50%, at least 60% or at least 70% of the subjects treated according to the method achieve objective response (OR) that is durable for at or greater than 3 months or at or greater than 6 months. In some instances, greater than or greater than about 50%, greater than or greater than about 60 %, greater than or greater than about 70 %, or greater than or greater than about 80 % of the subjects treated according to the method do not exhibit a grade 3 or greater cytokine release syndrome (CRS) and/or do not exhibit a grade 2 or greater or grade 3 or greater neurotoxicity; or greater than or greater than about 40 %, greater than or greater than about 50 % or greater than or greater than about 55 % of the subjects treated according to the method do not exhibit any neurotoxicity or CRS.

In some instances, the parameters, such as attributes, factors, characteristic of the patient and/or the disease or condition, and/or expression of biomarkers, are assessed prior to administration of the therapy, e.g., cell therapy. In some instances, the parameters, such as attributes, factors, characteristic of the patient and/or the disease or condition, and/or expression of biomarkers, are assessed after administration of the therapy, e.g., cell therapy. In some instances, the parameters include levels or measurements, e.g., peak levels, of attributes, factors, characteristic of the patient and/or the disease or condition, and/or expression of biomarkers, that can be assessed after administration of the therapy, e.g., cell therapy.

In some instances, the parameter is SPD and in some cases, development of toxicity, e.g., CRS or NT, is correlated with the SPD value that is above a threshold value. In some instances, the volumetric measure is SPD, and the threshold value is or is about 30 per cm²,is or is about 40 per cm², is or is about 50 per cm², is or is about 60 per cm², or is or is about 70 per cm². In some instances, the volumetric measure is SPD and the threshold value is or is about 30 per cm²,is or is about 40 per cm², is or is about 50 per cm², is or is about 60 per cm², or is or is about 70 per cm².

In some instances, the parameter is LDH and in some cases, development of toxicity, e.g., CRS or NT, is correlated with the LDH value that is above a threshold value. In some instances, the inflammatory marker is LDH and the threshold value is or is about 300 units per liter, is or is about 400 units per liter, is or is about 500 units per liter or is or is about 600 units per liter.

### A. Pharmacokinetic Parameters

In some cases, the described instances involve administering an agent capable of modulating CAR+ T cell expansion, proliferation, and/or activity to the subject, based on assessment of pharmacokinetic (PK) parameters. In some instances, the pharmacokinetic parameters include any of those described herein, e.g., in Section II.C. In some instances, the pharmacokinetic parameters include maximum (peak) plasma concentration (Cₘₐₓ), the peak time (*i.e.* when maximum plasma concentration (Cₘₐₓ) occurs; Tₘₐₓ), the minimum plasma concentration (*i.e.* the minimum plasma concentration between doses of a therapeutic agent, e.g., CAR+ T cells; Cₘᵢₙ), the elimination half-life (T_{1/2}) and area under the curve (*i.e.* the area under the curve generated by plotting time versus plasma concentration of the therapeutic agent CAR+ T cells; AUC), following administration.

In some instances, if the assessed pharmacokinetic parameters indicate that the dose of cells administered is not within or falls outside a therapeutic range and/or window, the subject can be administered an agent capable of modulating CAR+ T cell expansion, proliferation, and/or activity to the subject. In some instances, the therapeutic range and/or window is any described herein and/or is associated with any pharmacokinetic parameters described herein.

In some instances, if a pharmacokinetic parameter, e.g., peak number of CAR+ T cells in the blood of the subject, is less than the lowest number of the pharmacokinetic parameter, e.g., peak number of CAR+ T cells in the blood of the subject in the therapeutic range, an agent is administered to the subject that increases CAR+ T cell expansion, proliferation, and/or activity.

In some instances, if a pharmacokinetic parameter, e.g., peak number of CAR+ T cells in the blood of the subject, is more than the highest number of the pharmacokinetic parameter, e.g., peak number of CAR+ T cells in the blood of the subject in the therapeutic range, an agent is administered to the subject that decreases CAR+ T cell expansion, proliferation, and/or activity.

In some instances, the agent is administered after assessment of pharmacokinetic parameters, e.g., peak CAR+ T cell concentration, exposure (e.g., AUC) and/or cell level or concentration.

In some aspects, the described instances involve assessing and/or monitoring pharmacokinetic parameters, e.g., number or concentration of CAR+ T cells in the blood. In some instances, the methods involve monitoring CAR+ T cell numbers and/or concentration in the blood of the subject to assess if the cells are within a therapeutic range and/or window. In some instances, the methods involve administering an agent to the subject capable of modulating CAR+ T cell expansion, optionally increasing or decreasing CAR+ T cell expansion, in the subject, if the subjects are not within the therapeutic range.

In some instances, the therapeutic range and/or window is determined and/or based upon any criteria based on the assessment of the parameters described herein. In some instances, the therapeutic range and/or window is based upon the range of peak CD3+ CAR+ T cells, or a CD8+CAR+ T cell subset thereof, in the blood among one or more subjects previously treated with the genetically engineered cells that is associated with an estimated probability of response of greater than or greater than about 65% and an estimated probability of a toxicity of less than or about 30%; or peak CD3+CAR+ T cells in the blood, following administration of the genetically engineered cells, that is between or between about 10 cells per microliter and 500 cells per microliter; or peak CD8+CAR+ T cells in the blood, following administration of the genetically engineered cells, that is between or between about 2 cells per microliter and 200 cells per microliter.

### B. Patient Attributes and Biomarkers

In some cases, the described instances involve assessing parameters, such as attributes, factors, characteristic of the patient and/or the disease or condition, and/or expression of biomarkers. In some instances, the assessed parameters are associated with and/or correlated with pharmacokinetic parameters, response, durable response and/or development of toxicity. In some instances, the parameters include patient factors or patient attributes. In some instances, the parameters include attributes, factors, characteristic of the disease or condition. In some instances, the parameters are assessed prior to treatment, e.g., prior to administration of the cell therapy. In some instances, the parameters are assessed after treatment, e.g., after administration of one or more doses of the cell therapy.

In some instances, the parameter is or includes pharmacokinetic parameters, e.g., maximum (peak) plasma concentration (Cₘₐₓ), the peak time (*i.e.* when maximum plasma concentration (Cₘₐₓ) occurs; Tₘₐₓ), the minimum plasma concentration (*i.e.* the minimum plasma concentration between doses of a therapeutic agent, e.g., CAR+ T cells; Cₘᵢₙ), the elimination half-life (T_{1/2}) and area under the curve (*i.e.* the area under the curve generated by plotting time versus plasma concentration of the therapeutic agent CAR+ T cells; AUC; such as AUC₀₋₂₈).

In some instances, the parameter is or includes one or more factors indicative of the state of the patient and/or the disease or condition of the patient. In some instances, the parameter is indicative of tumor burden. In some instances, the factor indicative of tumor burden is a volumetric measure of tumor(s). In some instances, the volumetric measure is a measure of the lesion(s), such as the tumor size, tumor diameter, tumor volume, tumor mass, tumor load or bulk, tumor-related edema, tumor-related necrosis, and/or number or extent of metastases. In some instances, the volumetric measure of tumor is a bidimensional measure. For example, in some instances, the area of lesion(s) are calculated as the product of the longest diameter and the longest perpendicular diameter of all measurable tumors. In some cases, the volumetric measure of tumor is a unidimensional measure. In some cases, the size of measurable lesions is assessed as the longest diameter. In some instances, the sum of the products of diameters (SPD), longest tumor diameters (LD), sum of longest tumor diameters (SLD), necrosis, tumor volume, necrosis volume, necrosis-tumor ratio (NTR), peritumoral edema (PTE), and edema-tumor ratio (ETR) is measured.

Exemplary methods for measuring and assessing tumor burden include those described in, e.g., Carceller et al., Pediatr Blood Cancer. (2016) 63(8): 1400-1406 and Eisenhauer et al., Eur J Cancer. (2009) 45(2):228-247. In some instances, the volumetric is a sum of the products of diameters (SPD) measured by determining the sum of the products of the largest perpendicular diameters of all measurable tumors. In some aspects, the tumor or lesion are measured in one dimension with the longest diameter (LD) and/or by determining the sum of longest tumor diameters (SLD) of all measurable lesions. In some instances, the volumetric measure of tumor is a volumetric quantification of tumor necrosis, such as necrosis volume and/or necrosis-tumor ratio (NTR), see Monsky et al., Anticancer Res. (2012) 32(11): 4951-4961. In some aspects, the volumetric measure of tumor is a volumetric quantification of tumor-related edema, such as peritumoral edema (PTE) and/or edema-tumor ratio (ETR). In some instances, measuring can be performed using imaging techniques such as computed tomography (CT), positron emission tomography (PET), and/or magnetic resonance imaging (MRI) of the subj ect.

In some instances, the volumetric measure is SPD and in some cases, development of toxicity, e.g., CRS or NT, is correlated with the SPD value that is above a threshold value. In some instances, the volumetric measure is SPD, and the threshold value is or is about 30 per cm²,is or is about 40 per cm², is or is about 50 per cm², is or is about 60 per cm², or is or is about 70 per cm². In some instances, the volumetric measure is SPD and the threshold value is or is about 30 per cm²,is or is about 40 per cm², is or is about 50 per cm², is or is about 60 per cm², or is or is about 70 per cm².

In some instances, the volumetric measure of tumor is determined at a screening session, such as a routine assessment or blood draw to confirm and/or identify the condition or disease in the subject.

In some aspects, the parameter, e.g., measurements of tumor burden, correlates to and/or is associated with pharmacokinetic parameters. In some instances, the parameter, including pharmacokinetic parameters, is associated with response and/or durable response, and/or a risk for developing toxicity, e.g., CRS or neurotoxicity (NT).

In some instances, the parameter is or includes at least one or a panel of biomarkers. In some instances, expression and/or presence of the biomarker is associated with and/or correlated with pharmacokinetic parameters, response, durable response and/or development of toxicity. In some instances, the parameter is compared to a particular reference value, *e.g.,* those associated with response and/or durable response, and/or a risk for developing toxicity, e.g., CRS or neurotoxicity (NT). In some instances, the methods also involve administering an agent capable of modulating CAR+ T cell expansion, proliferation, and/or activity, to the subject, based on the assessment of patient factors and/or biomarkers.

In some aspects, the instances involve obtaining a biological sample for detecting the parameter and/or assessing the presence of and/or or detecting the parameter. In some instances, the biological sample is obtained generally within 4 hours to 12 months of administration of the cell therapy, or a first administration or dose thereof, or after the initiation of any of the foregoing, such as generally within or within about 1, 2, 3, 4, 5, 6, 7, 8, 9, 10, 11, 12, 13, 14, 21, 28, 30, 60 or 90 or more days, 2, 3, 4, 5, 6, 7, 8, 9, 10, 11, 12, 13, 14, 15 or more weeks, or 1, 2, 3, 4, 5, 6, 7, 8, 9, 10, 11, 12, 18, 24, 48 or more months, after administration of the cell therapy, or a first administration or dose thereof, or after the initiation of any of the foregoing. In some instances, the parameter is assessed or measured in a subject prior to administration of the cell therapy or soon after administration of the cell therapy, or a first administration or dose thereof, or after the initiation of any of the foregoing , such as generally within 4 hours to 3 days of administration of the cell therapy, or a first administration or dose thereof, or after the initiation of any of the foregoing, such as generally within or about 1 day, 2 days or 3 days after administration of the cell therapy, or a first administration or dose thereof, or after the initiation of any of the foregoing. In some instances, the parameter is assessed or measured. In some instances, the parameter is assessed generally within 4 hours to 12 months of administration of the cell therapy, or a first administration or dose thereof, or after the initiation of any of the foregoing, such as generally within or within about 1, 2, 3, 4, 5, 6, 7, 8, 9, 10, 11, 12, 13, 14, 21, 28, 30, 60 or 90 or more days, 2, 3, 4, 5, 6, 7, 8, 9, 10, 11, 12, 13, 14, 15 or more weeks, or 1, 2, 3, 4, 5, 6, 7, 8, 9, 10, 11, 12, 18, 24, 48 or more months, after administration of the cell therapy, or a first administration or dose thereof, or after the initiation of any of the foregoing.

In some aspects, the parameter, e.g., patient factor and/or biomarker, correlates to and/or is associated with pharmacokinetic parameters. In some instances, the parameter, including pharmacokinetic parameters, is associated with response and/or durable response, and/or a risk for developing toxicity, e.g., CRS or neurotoxicity (NT).

In some instances, the parameter is a biomarker. In some instances, the parameter is or includes the expression of the biomarker and/or the number, concentration, and/or percentage of cells that express a particular biomarker. In some instances, the parameter includes biomarkers or each biomarker in a panel that comprises a plurality of biomarkers. In some instances, the biomarker is or comprises a cytokine and/or other serum or blood factor, such as any as described herein. In some instances, the biomarker or each biomarker in a panel is a cytokine, which, in some cases, can be a chemokine. In some instances, the biomarkers or each biomarker in a panel comprises a soluble receptor. In some instances, the biomarkers or each biomarker in a panel comprises a soluble serum protein. Exemplary biomarkers or panel of biomarkers is described herein.

In some instances, the parameter is or includes levels and/or concentrations of a blood analyte. In some instances, the parameter is or includes levels and/or concentrations of an inflammatory marker. In some instances, the the blood analyte and/or inflammatory marker is or includes levels and/or concentrations of interleukin-7 (IL-7), IL-15, macrophage inflammatory protein (MIP-1α). In some instances, the blood analyte and/or inflammatory marker is or includes levels and/or concentrations of IL-6, IL-10, IL-16, interferon gamma (IFN-γ), tumor necrosis factor alpha (TNF-α), MIP-1α, MIP-1β, Monocyte chemoattractant protein-1 (MCP-1), and C-X-C motif chemokine 10 (CXCL10). In some instances, the blood analyte and/or inflammatory marker is or includes levels and/or concentrations of ferritin, C-reactive protein (CRP), D-dimer (fibrin degradation product), IL-6, IL-10, IL-15, IL-16, TNF-α, MIP-1α, and MIP-1β. In some instances, the blood analyte and/or inflammatory marker is or includes levels and/or concentrations of LDH, Ferritin, CRP, IL-6, IL-8, IL-10, TNF-α, IFN- α2, MCP-1 and/or MIP-1β. In some instances, the blood analyte and/or inflammatory marker is or includes levels and/or concentrations of CRP, Serum Amyloid A1 (SAA-1), IL-2, IL-6, IL-10, IL-15, TNF-α, MIP-1α, MIP-1β, MCP-1, CXCL10 and C-C Motif Chemokine Ligand 13 (CCL13). In some instances, the blood analyte and/or inflammatory marker is or includes levels and/or concentrations of LDH, ferritin, CRP, D-dimer, SAA-1, IL-6, IL-10, IL-15, IL-16, TNF-α, IFN-γ and/or MIP-1α.

In some instances, an inflammatory marker is or includes the level or presence of C-reactive protein (CRP), erythrocyte sedimentation rate (ESR), albumin, ferritin, β2 microglobulin (β2-M), or lactate dehydrogenase (LDH) is detected and assessed. In some instances, the inflammatory marker is assessed using an immune assay. For example, an enzyme-linked immunosorbent assay (ELISA), enzyme immunoassay (EIA), radioimmunoassay (RIA), surface plasmon resonance (SPR), Western Blot, Lateral flow assay, immunohistochemistry, protein array or immuno-PCR (iPCR) can be used to detect the inflammatory marker. In some instances, using the articles of manufacture include detecting an inflammatory marker indicative of tumor burden. In some cases, the assaying or assessing of an inflammatory marker is using flow cytometry. In some cases, the reagent is a soluble protein that binds the inflammatory marker. In some example, the reagent is a protein that binds C-reactive protein (CRP), erythrocyte sedimentation rate (ESR), albumin, ferritin, β2 microglobulin (β2-M), or lactate dehydrogenase (LDH).

In some instances, the biomarker, e.g., inflammatory marker is or includes C-reactive protein (CRP). In some instances, CRP is assessed using an in vitro enzyme-linked immunosorbent assay to obtain a quantitative measurement of human CRP from a sample such as serum, plasma, or blood. In some examples, CRP is detected using a human Enzyme- Linked Immunosorbent Assay (ELISA). In some instances, the biomarker, e.g. inflammatory marker is or includes erythrocyte sedimentation rate (ESR). In some instances, ESR is assessed by measuring the distance (in millimeters per hour) that red cells have fallen after separating from the plasma in a vertical pipette or tube. In some instances the biomarker is or includes albumin. In some aspects, albumin is assessed using a colorimetric test or an in vitro enzyme-linked immunosorbent assay. In some examples, albumin is detected using a human Enzyme- Linked Immunosorbent Assay (ELISA). In some instances, the biomarker, e.g., inflammatory marker is or includes ferritin or β2 microglobulin. In some instances, ferritin or β2 microglobulin is assessed using an immunoassay or detected using an ELISA. In some aspects, the biomarker, e.g., inflammatory marker is or includes lactate dehydrogenase (LDH), and LDH is assessed using a colorimetric test or an in vitro enzyme-linked immunosorbent assay.

In some instances, the parameter is LDH and in some cases, development of toxicity, e.g., CRS or NT, is correlated with the LDH value that is above a threshold value. In some instances, the inflammatory marker is LDH and the threshold value is or is about 300 units per liter, is or is about 400 units per liter, is or is about 500 units per liter or is or is about 600 units per liter.

In some instances, the one or more biomarkers include two or more biomarkers, e.g., cytokines, such as inflammatory cytokines, and/or patient attributes, e.g., tumor burden and/or expression of inflammatory markers. In some aspects, the two or more biomarkers are measured simultaneously from the same sample. In other aspects, the two or more biomarkers are measured or sequentially from the same sample or from different samples from the subject.

In some instances, the level, amount, concentration or other parameter of the biomarker or the panel of biomarkers are indicative of pharmacokinetic parameters of the cells, e.g., maximum (peak) plasma concentration (Cₘₐₓ), the peak time (*i.e.* when maximum plasma concentration (Cₘₐₓ) occurs; Tₘₐₓ), the minimum plasma concentration (*i.e.* the minimum plasma concentration between doses of a therapeutic agent, e.g., CAR+ T cells; Cₘᵢₙ), the elimination half-life (T_{1/2}) and area under the curve (*i.e.* the area under the curve generated by plotting time versus plasma concentration of the therapeutic agent CAR+ T cells; AUC; such as AUC₀₋₂₈). In some instances, the level, amount, concentration of the biomarker or the panel of biomarkers are indicative of the risk of developing a toxicity, e.g., neurotoxicity, such as severe neurotoxicity and/or CRS, such as sCRS. In some instances, the level, amount, concentration of the biomarker or the panel of biomarkers are indicative of, correlate with and/or associate with the likelihood and/or probability of response, e.g., objective response (OR), complete response (CR) or partial response (PR), or durable response, e.g., 3-month response.

In some instances, the parameter is or includes levels, concentrations and/or numbers pf C-reactive protein (CRP), erythrocyte sedimentation rate (ESR), albumin, ferritin, β2 microglobulin (β2-M), lactate dehydrogenase (LDH) and/or is an inflammatory cytokine. In some instances, the inflammatory marker is LDH. In some instances, the level, concentration and/or number of LDH is a surrogate for disease burden, e.g., for tumors or cancers, and may be useful for potential neurotoxicity risk assessment and/or risk-adapted dosing or adjustment of treatment of certain subjects. In some aspects, LDH levels may be assessed alone and/or in combination with another pre-treatment parameter, such as another measure or indicator of disease burden, such as a volumetric tumor measurement such as sum of product dimensions (SPD) or other CT-based or MRI-based volumetric measurement of disease burden, such as any described herein. In some aspects, one or more parameters indicative of disease burden are assessed, and in some contexts may indicate the presence, absence or degree of risk of developing neurotoxicity following the T cell therapy. In some aspects, the one or more parameters include LDH and/or a volumetric tumor measurement. In some instances, the parameter is SPD and/or LDH.

In some instances, the parameter is a patient attribute, factor and/or characteristic. In some instances, the parameter is a pre-treatment measurement, e.g., a baseline measurement, a pre-infusion measurement and/or a pre-lymphodepletion measurement. In some instances, the parameter is assessed before treatment, e.g., before administration of the cell therapy, or a first administration or dose thereof, or after the initiation of any of the foregoing. and/or lymphodepletion prior to cell therapy. In some instances, the parameter is assessed prior to lymphodepletion.

In some instances, the pre-treatment measurement is or includes the level and/or concentration of C-reactive protein (CRP), D-dimer (fibrin degradation product), Ferritin, IFN-α2, IFN-γ, IL-6, IL-7, IL-8, IL-10, IL-15, IL-16, lactate dehydrogenase (LDH), macrophage inflammatory protein (MIP-1α), MIP-1β, MCP-1, SAA-1 and/or TNF-α.

In some instances, higher or lower pre-treatment measurement of one or more of the parameters is correlated to and/or is associated with higher or lower pharmacokinetic parameters, e.g., Cₘₐₓ or AUC, of CAR+ T cells and/or higher or lower rate and/or incidence of toxicity, e.g., CRS or NT, such as severe CRS or severe NT. In some instances, higher or lower pre-treatment measurement of one or more of the parameters is correlated to and/or is associated with higher or lower response, e.g., ORR including CR and PR, and/or higher or lower durability of response, e.g., 3-month response.

In some instances, higher pre-treatment measurement of one or more of the parameters is correlated to and/or is associated with higher pharmacokinetic parameters, e.g., Cₘₐₓ or AUC, of CAR+ T cells and/or higher rate and/or incidence of toxicity, e.g., CRS or NT, such as severe CRS or severe NT.

In some instances, the parameter is or includes a post-treatment measurement, e.g., a peak or maximum measurement after administration of the therapy, e.g., cell therapy, and/or a post-infusion measurement and/or measurement after administration of the cell therapy, or a first administration or dose thereof, or after the initiation of any of the foregoing. In some instances, the peak measurement is or includes the peak or maximum value within a period of time after a certain amount of time after administration of the cell therapy and/or initiation thereof, such as within or within about 1, 2, 3, 4, 5, 6, 7, 8, 9, 10, 11, 12, 13, 14, 21, 28, 30, 60 or 90 or more days, 2, 3, 4, 5, 6, 7, 8, 9, 10, 11, 12, 13, 14, 15 or more weeks, or 1, 2, 3, 4, 5, 6, 7, 8, 9, 10, 11, 12, 18, 24, 48 or more months, after administration of the cell therapy, or a first administration or dose thereof, or after the initiation of any of the foregoing.

In some instances, the parameter is or includes peak level and/or concentration of inflammatory markers, including cytokines or chemokines. In some instances, lower peak measurements of one or more of the parameters is correlated to and/or is associated with higher pharmacokinetic parameters, e.g., Cₘₐₓ or AUC, of CAR+ T cells and/or higher rate and/or incidence of toxicity, e.g., CRS or NT, such as severe CRS or severe NT. In some instances, lower pre-treatment measurement of one or more of the parameters is correlated to and/or is associated with response, e.g., ORR including CR and PR, and/or lower durability of response, e.g., 3-month response.

In some instances, the parameter is or includes peak level and/or concentration of inflammatory markers, including cytokines or chemokines. In some instances, the parameter is or includes peak level and/or concentration of biomarkers, including C-C Motif Chemokine Ligand 13 (CCL13), C-reactive protein (CRP), C-X-C motif chemokine 10 (CXCL10), IL-2, IL-5, IL-6, IL-7, IL-8, IL-10, IL-15, IL-16, interferon gamma (IFN-γ), Lymphotoxin-alpha (LT-α), Monocyte chemoattractant protein-1 (MCP-1), macrophage inflammatory protein 1 alpha (MIP-1α), MIP-1β, Serum Amyloid A1 (SAA-1), Transforming growth factor beta (TGF-β) and tumor necrosis factor alpha (TNF-α). In some instances, higher peak levels and/or concentrations of one or more of the parameters is correlated to and/or is associated with higher rate and/or incidence of toxicity, e.g., CRS or NT, such as severe CRS or severe NT. In some instances, lower peak levels and/or concentrations is correlated to and/or is associated with higher response, e.g., ORR including CR and PR, and/or higher durability of response, e.g., 3-month response.

In some instances, the threshold value is a value that: is within 25%, within 20%, within 15%, within 10%, or within 5% above the average value of the volumetric measure or inflammatory marker and/or is within a standard deviation above the average value of the volumetric measure or the inflammatory marker in a plurality of control subjects. In some instances, the threshold value is a value that: is above the highest value of the volumetric measure or inflammatory marker, optionally within 50%, within 25%, within 20%, within 15%, within 10%, or within 5% above such highest fold change, measured in at least one subject from among a plurality of control subjects. In some instances, the threshold value is a value that: is above the highest value of the volumetric measure or inflammatory marker as measured among more than 75%, 80%, 85%, 90%, or 95%, or 98% of subjects from a plurality of control subjects. In some instances, the plurality of control subjects are a group of subjects prior to receiving a dose of the genetically engineered cells, wherein: each of the control subjects of the group exhibited a peak CAR+ T cells in the blood greater than the highest peak CAR+ T cells in the therapeutic range; each of the control subjects of the group went on to develop at toxicity, optionally a neurotoxicity or cytokine release syndrome (CRS), a grade 2 or grade 3 or higher neurotoxicity or a grade 3 or higher CRS, after receiving a dose of the engineered cells for treating the same disease or condition; each of the control subjects of the group did not develop a response, optionally a complete response (CR) or partial response (PR), following administration of the dose of genetically engineered cells; and/or each of the control subjects of the group did not develop a durable response, optionally for at or about or greater than or about 3 months or at or about or greater than or about 6 months, following administration of the dose of genetically engineered cells.

### C. Reagents for Measuring

In some instances, the parameter, e.g., patient factor, biomarker, inflammatory marker and/or cytokine, is detected using one or more reagent(s) capable of detecting or that is specific for the parameter. In some instances, also described are kits and articles of manufacture, for detection or assessment of the parameters and/or for modulating the therapy, e.g., cell therapy.

In some instances, instructions for using the reagent to assay a biological sample from a subject that is a candidate for treatment, optionally with a cell therapy, said cell therapy optionally including a dose or composition of genetically engineered cells expressing a recombinant receptor, are also described. In some instances of using the articles of manufacture, the level or presence of C-C Motif Chemokine Ligand 13 (CCL13), C-reactive protein (CRP), C-X-C motif chemokine 10 (CXCL10), D-dimer (fibrin degradation product), ferritin, IFN-α2, interleukin-2 (IL-2), IL-10, IL-15, IL-16, IL-6, IL-7, IL-8, interferon gamma (IFN-γ), lactate dehydrogenase (LDH), macrophage inflammatory protein (MIP-1α), MIP-1β, Monocyte chemoattractant protein-1 (MCP-1), SAA-1, Serum Amyloid A1 (SAA-1), tumor necrosis factor alpha (TNF-α), is detected and assessed. In some instances of using the articles of manufacture, the level or presence of C-reactive protein (CRP), erythrocyte sedimentation rate (ESR), albumin, ferritin, β2 microglobulin (β2-M), or lactate dehydrogenase (LDH) is detected and assessed. Also described are methods of detecting and assessing one or more patient attributes, factors and/or biomarkers indicative of tumor burden.

In some instances, measuring the value of the one or more parameters, e.g., biomarkers, comprises contacting a reagent capable of directly or indirectly detecting the analyte with the biological sample and determining the presence or absence, level, amount or concentration of the analyte in the biological sample. In some instances, the one or more parameters, e.g., biomarkers, is C-C Motif Chemokine Ligand 13 (CCL13), C-reactive protein (CRP), C-X-C motif chemokine 10 (CXCL10), D-dimer (fibrin degradation product), ferritin, IFN-α2, interleukin-2 (IL-2), IL-10, IL-15, IL-16, IL-6, IL-7, IL-8, interferon gamma (IFN-γ), lactate dehydrogenase (LDH), macrophage inflammatory protein (MIP-1α), MIP-1β, Monocyte chemoattractant protein-1 (MCP-1), SAA-1, Serum Amyloid A1 (SAA-1), tumor necrosis factor alpha (TNF-α), is detected and assessed. In some instances of using the articles of manufacture, the level or presence of C-reactive protein (CRP), erythrocyte sedimentation rate (ESR), albumin, ferritin, β2 microglobulin (β2-M), or lactate dehydrogenase (LDH). In some instances, the one or more parameters, e.g., biomarkers, is or includes LDH.

In some aspects, the reagent is a binding molecule that specifically binds to the biomarker. For example, in some instances, the reagent is an antibody or an antigen-binding fragment thereof. In some instances, the reagent is or includes a substrate or binding partner of the biomarker.

In some instances, the presence, absence or level, amount, concentration and/or other measure of LDH is detected or determined in a sample. Various methods of detecting or determining LDH are known. For example, an assay which measures LDH conversion of lactate to pyruvate through NAD+ reduction to NADH can be used to detect LDH in the sample. In some instances, the sample is contacted with lactate in the presence of coenzyme NAD which, as a measure of LDH in the sample, results in NADH that is then oxidized in the presence of an electron transfer agent. In some instances, the NADH interacts with a probe or dye precursor that is detectable by measuring absorption in a visible light range. In some examples, diaphorase uses the NADH to reduce tetrazolium salt (INT) to a red formazan product and the product is measured. Therefore, in some instances, the amount of colored product formed is directly proportional to the LDH activity in the sample.

In some instances, the patient attributes, factors and/or biomarkers is assessed using an immune assay. For example, an enzyme-linked immunosorbent assay (ELISA), enzyme immunoassay (EIA), radioimmunoassay (RIA), surface plasmon resonance (SPR), Western Blot, Lateral flow assay, immunohistochemistry, protein array or immuno-PCR (iPCR) can be used to detect the patient attributes, factors and/or biomarkers. In some instances, using the articles of manufacture include detecting patient attributes, factors and/or biomarkers indicative of tumor burden. In some cases, the assaying or assessing of an patient attributes, factors and/or biomarkers is using flow cytometry. In some cases, the reagent is a soluble protein that binds the patient attributes, factors and/or biomarkers. In some example, the reagent is a protein that binds C-reactive protein (CRP), erythrocyte sedimentation rate (ESR), albumin, ferritin, β2 microglobulin (β2-M), or lactate dehydrogenase (LDH).

In some instances, C-reactive protein (CRP) is assessed using an in vitro enzyme-linked immunosorbent assay to obtain a quantitative measurement of human CRP from a sample such as serum, plasma, or blood. In some examples, CRP is detected using a human Enzyme-Linked Immunosorbent Assay (ELISA). In some instances, erythrocyte sedimentation rate (ESR) is assessed by measuring the distance (in millimeters per hour) that red cells have fallen after separating from the plasma in a vertical pipette or tube. In some aspects, albumin is assessed using a colorimetric test or an in vitro enzyme-linked immunosorbent assay. In some examples, albumin is detected using a human Enzyme- Linked Immunosorbent Assay (ELISA). In some instances, ferritin or β2 microglobulin is assessed using an immunoassay or detected using an ELISA. In some aspects, lactate dehydrogenase (LDH) is assessed using a colorimetric test or an in vitro enzyme-linked immunosorbent assay.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, single chain antibody fragments, including single chain variable fragments (scFv), and single domain antibodies (e.g., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, e.g., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD.

Among the described antibodies are antibody fragments. An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (*e.g.* scFv); and multispecific antibodies formed from antibody fragments. In particular instances, the antibodies are single-chain antibody fragments comprising a variable heavy chain region and/or a variable light chain region, such as scFvs.

Single-domain antibodies are antibody fragments comprising all or a portion of the heavy chain variable domain or all or a portion of the light chain variable domain of an antibody. In certain instances, a single-domain antibody is a human single-domain antibody.

Antibody fragments can be made by various techniques, including but not limited to proteolytic digestion of an intact antibody as well as production by recombinant host cells. In some instances, the antibodies are recombinantly produced fragments, such as fragments comprising arrangements that do not occur naturally, such as those with two or more antibody regions or chains joined by synthetic linkers, e.g., peptide linkers, and/or that are may not be produced by enzyme digestion of a naturally-occurring intact antibody. In some aspects, the antibody fragments are scFvs.

A "humanized" antibody is an antibody in which all or substantially all CDR amino acid residues are derived from non-human CDRs and all or substantially all FR amino acid residues are derived from human FRs. A humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some instances, some FR residues in a humanized antibody are substituted with corresponding residues from a non-human antibody (e.g., the antibody from which the CDR residues are derived), e.g., to restore or improve antibody specificity or affinity.

Among the described antibodies are human antibodies. A "human antibody" is an antibody with an amino acid sequence corresponding to that of an antibody produced by a human or a human cell, or non-human source that utilizes human antibody repertoires or other human antibody-encoding sequences, including human antibody libraries. The term excludes humanized forms of non-human antibodies comprising non-human antigen-binding regions, such as those in which all or substantially all CDRs are non-human.

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic animals, the endogenous immunoglobulin loci have generally been inactivated. Human antibodies also may be derived from human antibody libraries, including phage display and cell-free libraries, containing antibody-encoding sequences derived from a human repertoire.

Among the described antibodies are monoclonal antibodies, including monoclonal antibody fragments. The term "monoclonal antibody" as used herein refers to an antibody obtained from or within a population of substantially homogeneous antibodies, i.e., the individual antibodies comprising the population are identical, except for possible variants containing naturally occurring mutations or arising during production of a monoclonal antibody preparation, such variants generally being present in minor amounts. In contrast to polyclonal antibody preparations, which typically include different antibodies directed against different epitopes, each monoclonal antibody of a monoclonal antibody preparation is directed against a single epitope on an antigen. The term is not to be construed as requiring production of the antibody by any particular method. A monoclonal antibody may be made by a variety of techniques, including but not limited to generation from a hybridoma, recombinant DNA methods, phage-display and other antibody display methods.

Also described are antibody immunoconjugates comprising an antibody against biomarker attached to a label, which can generate a detectable signal, indirectly or directly. These antibody immunoconjugates can be used for research or diagnostic applications. The label is preferably capable of producing, either directly or indirectly, a detectable signal. For example, the label may be radio-opaque or a radioisotope, such as ³H, ¹⁴C, ³²P, ³⁵S, ¹²³I, ¹²⁵I, ¹³¹I; a fluorescent (fluorophore) or chemiluminescent (chromophore) compound, such as fluorescein isothiocyanate, rhodamine or luciferin; an enzyme, such as alkaline phosphatase,β-galactosidase or horseradish peroxidase; an imaging agent; or a metal ion. In some instances, the label is a radioactive atom for scintigraphic studies, for example ⁹⁹Tc or ¹²³I, or a spin label for nuclear magnetic resonance (NMR) imaging (also known as magnetic resonance imaging, MRI), such as zirconium-89, iodine-123, iodine-131, indium-111, fluorine-19, carbon-13, nitrogen-15, oxygen-17, gadolinium, manganese or iron. Zirconium-89 may be complexed to various metal chelating agents and conjugated to antibodies, *e.g.,* for PET imaging (WO 2011/056983).

In some instances, the antibody immunoconjugate is detectable indirectly. For example, a secondary antibody that is specific for the antibody against the marker expressed on a population of myeloid cells immunoconjugate and contains a detectable label can be used to detect the antibody immunoconjugate.

In some instances, antibodies capable of detecting or that is specific the patient attributes, factors and/or biomarkers described herein may be identified, screened for, or characterized for their physical/chemical properties and/or biological activities by various known assays. In one aspect, the antibody is tested for its antigen binding activity, e.g., by known methods such as an immunoassay, ELISA, Western blotting, and/or flow cytometric assays, including cell-based binding assays.

### D. Samples

In certain instances, one or more patient attributes, factors and/or biomarkers are measured, assessed, and/or determined in one or more samples obtained at two or more time points to determine a fold change in the factor indicative of disease burden. In particular instances, the sample is a biological sample that is taken, collected, and/or obtained from a subject. In certain instances, the subject has a disease or condition and/or is suspected of having a disease or condition. In some instances, subject has received, will receive, or is a candidate to receive a therapy. In some instances, the therapy is an administration of a cell therapy. In particular instances, the therapy is an immunotherapy. In certain instances, the cell therapy treats and/or is capable of treating the disease or condition. In some instances, the therapy is a cell therapy that contains one or more engineered cells. In some instances, the engineered cells express a recombinant receptor. In particular instances, the recombinant receptor is a CAR. In particular instances, the sample is taken, collected, and/or obtained from a subject who has been, who will be, or is a candidate to be administered a therapy. In particular instances, the sample is taken, collected, and/or obtained prior to treatment or administration with the therapy, e.g., the cell therapy.

In some instances, the sample does not comprise genetically engineered T cells expressing a chimeric antigen receptor (CAR) and/or is obtained from the subject prior to receiving administration of genetically engineered T cells expressing a CAR.

In particular instances, the sample is taken, collected, and/or obtained from a subject who has been, who will be, or is a candidate to be administered a therapy. In particular instances, the sample is taken, collected, and/or obtained prior to treatment or administration with the therapy, e.g., the cell therapy. In accord with methods, kits and articles of manufacture described herein, the sample can be assessed for one or more patient attributes, factors and/or biomarkerss that is associated with and/or correlate to toxicity or risk of toxicity. Exemplary patient attributes, factors and/or biomarkerss associated with and/or correlated with a risk of developing toxicity and/or response that may be detected in a sample collected or obtained from a subject prior to receiving an immunotherapy include C-reactive protein (CRP), erythrocyte sedimentation rate (ESR), albumin, ferritin, β2 microglobulin (β2-M), or lactate dehydrogenase (LDH). In some instances, the patient attributes, factors and/or biomarkers associated with and/or correlated with a risk of developing toxicity and/or response that may be detected in a sample collected or obtained from a subject prior to or after receiving an immunotherapy include C-C Motif Chemokine Ligand 13 (CCL13), C-reactive protein (CRP), C-X-C motif chemokine 10 (CXCL10), D-dimer (fibrin degradation product), ferritin, IFN-α2, interleukin-2 (IL-2), IL-10, IL-15, IL-16, IL-6, IL-7, IL-8, interferon gamma (IFN-γ), lactate dehydrogenase (LDH), macrophage inflammatory protein (MIP-1α), MIP-1β, Monocyte chemoattractant protein-1 (MCP-1), SAA-1, Serum Amyloid A1 (SAA-1), tumor necrosis factor alpha (TNF-α). Thus, in some aspects, the described methods relate to identifying subjects, prior to receiving an immunotherapy, such as a cell therapy (e.g. CAR-T cells), who may achieve pharmacokinetic parameters within a therapeutic window or range. In some instances, the described methods relate to identifying subjects, prior to or after receiving an immunotherapy or cell therapy, for modulating the immunotherapy or cell therapy, e.g., by administration of agent to the subject capable of modulating, optionally increasing or decreasing, CAR+ T cell expansion, proliferation, and/or activity, As described elsewhere herein, the methods can be used to determine if the subject should be closely monitored following the administration of the immunotherapy, is a candidate for outpatient therapy or should receive treatment of the therapy in a hospital setting and/or is a candidate for receiving an agent capable of modulating CAR+ T cell expansion and/or proliferation and/or an intervention of preventing, treating or ameliorating a risk of a toxicity.

In some instances, the sample is taken, collected, and/or obtained from a subject that has or is suspected of having a condition or disease. In some instances, the subject has or is suspected of having a cancer or proliferative disease. In particular instances, the subject has a disease or condition, or is suspected of having a disease or condition, that is associated with an antigen and/or is associated with diseased cells that express the antigen. In some instances, the disease or condition, e.g., a cancer or proliferative disorder, is associated with αvβ6 integrin (avb6 integrin), B cell maturation antigen (BCMA), B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD138, CD171, epidermal growth factor protein (EGFR), truncated epidermal growth factor protein (tEGFR), type III epidermal growth factor receptor mutation (EGFR vIII), epithelial glycoprotein 2 (EPG-2), epithelial glycoprotein 40 (EPG-40), ephrinB2, ephrine receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, fetal acetylcholine receptor, ganglioside GD2, O-acetylated GD2 (OGD2), ganglioside GD3, G Protein Coupled Receptor 5D (GPCR5D), glycoprotein 100 (gp100), Her2/neu (receptor tyrosine kinase erbB2), Her3 (erb-B3), Her4 (erb-B4), erbB dimers, human high molecular weight-melanoma-associated antigen (HMW-MAA), hepatitis B surface antigen, Human leukocyte antigen A1 (HLA-AI), human leukocyte antigen A2 (HLA-A2), IL-22 receptor alpha(IL-22Ra), IL-13 receptor alpha 2 (IL-13Ra2), kinase insert domain receptor (kdr), kappa light chain, L1 cell adhesion molecule (L1CAM), CE7 epitope of L1-CAM, Leucine Rich Repeat Containing 8 Family Member A (LRRC8A), Lewis Y, melanoma-associated antigen (MAGE)-A1, MAGE-A3, MAGE-A6, mesothelin, c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, preferentially expressed antigen of melanoma (PRAME), progesterone receptor, a prostate specific antigen, prostate stem cell antigen (PSCA), prostate specific membrane antigen (PSMA), receptor tyrosine kinase like orphan receptor 1 (RORl), survivin, Trophoblast glycoprotein (TPBG also known as 5T4), tumor-associated glycoprotein 72 (TAG72), vascular endothelial growth factor receptor (VEGFR), vascular endothelial growth factor receptor 2 (VEGFR2), Wilms tumor 1 (WT-1), and/or a pathogen-specific antigen. In certain instances, the subject has a disease or condition, or is suspected of having a disease or condition, that is associated with CD19 and/or is associated with diseased cells that express CD19.

In some instances, the sample is taken, collected, and/or obtained from a subject that has or is suspected of having a cancer or proliferative disease that is a B cell malignancy or hematological malignancy. In some instances, the cancer or proliferative disease is a myeloma, *e.g.,* a multiple myeloma (MM), a lymphoma or a leukemia, lymphoblastic leukemia (ALL), non-Hodgkin's lymphoma (NHL), chronic lymphocytic leukemia (CLL), a diffuse large B-cell lymphoma (DLBCL), and/or acute myeloid leukemia (AML). In some instances, the cancer or proliferative disorder is ALL. In some instances, the subject has, or is suspected of having ALL. In some instances, the ALL is adult ALL. In particular instances, the ALL is pediatric ALL.

In particular instances, two or more samples are obtained, collected, or taken from the subject prior to administration of the therapy. In certain instances, the sample is a biological sample. In certain instances, the sample is a blood sample, plasma sample, or serum sample. In certain instances, the sample is a tissue sample. In some instances, the sample is a biopsy. In some instances, the sample is obtained from the subject at a screening session, such as a routine assessment or blood draw to confirm and/or identify the condition or disease in the subject.

### E. Agents for Modulating Cell Expansion and Activity

In some aspects, described are methods for modulating the expansion, proliferation and/or activity of the administered cells, e.g., CAR+ T cells, based on assessment and/or determination of the parameters, e.g., pharmacokinetic parameters and/or other parameters such as patient attributes and/or expression of a biomarker. In some instances, the method involves administering agents that modulate, such as increase or decrease, the expansion, proliferation and/or activity of the administered cells, e.g., CAR+ T cells, depending on the determination of the parameters. In some instances, an agent is administered if the genetically engineered cells are not within the therapeutic range based on assessment of the parameters, e.g., pharmacokinetic parameters, such as maximum or peak CAR+ cell concentration. In some instances, the agent is an agent that increases, augments or boosts the proliferation and/or expansion of the CAR+ T cells. In some instances, the agent is an agent that decreases, reduces, and/or dampens the proliferation and/or expansion of the CAR+ T cells.

In some instances, the agent can be administered sequentially, intermittently, or at the same time as or in the same composition as the therapy, such as cells for adoptive cell therapy. In some instances, the agent is administered before, simultaneously with, intermittently with, during, during the course of or after administration of the cells, *e.g.,* cells expressing a recombinant receptor, *e.g.* CAR. In some instances, such agents include agents that modulate the cell expansion and/or activity of the administered cells, *e.g.,* immune cells, such as T cells. In some instances, such agents include agents that reduce or decrease the expansion and/or proliferation of the cell expansion and/or activity of the administered cells, *e.g.,* immune cells, such as T cells.

In some instances, the agent is administered at a time that is greater than or greater than about 8 days, 9 days, 10 days, 11 days, 12 days, 13 days, 14 days, 15 days, 16 days, 17 days, 18 days, 19 days, 20 days or 21 days after initiation of administration of the genetically engineered cells. In some instances, the agent is administered at a time that is between or between about 11 to 22 says, 12 to 18 days or 14 to 16 days, each inclusive, after initiation of administration of the genetically engineered cells.

In some instances, the agent is administered at a time as described herein and in accord with the described methods, and/or with the described articles of manufacture or compositions. In some instances, the agent is administered at a time that is within, such as less than or no more than, 3, 4, 5, 6, 7, 8, 9 or 10 days after initiation of the immunotherapy and/or cell therapy. In some instances, the agent is administered within or within about 1 day, 2 days or 3 days after initiation of administration of the immunotherapy and/or cell therapy.

In some cases, the agent or therapy or intervention, is administered alone or is administered as part of a composition or formulation, such as a pharmaceutical composition or formulation, as described herein. Thus, the agent alone or as part of a pharmaceutical composition can be administered intravenously or orally, or by any other acceptable known route of administration or as described herein.

### 1. Agents for Augmenting or Enhancing Cell Expansion

In some instances, the methods include methods involving the combined administration, *e.g.* simultaneous or sequential administration, with a drug or agent capable of augmenting, boosting or enhancing the expansion, proliferation, survival and/or efficacy of the administered cells, *e.g.,* recombinant receptor expressing cells. In some instances, such agent is administered to achieve a peak CAR+ T cell expansion in the therapeutic range. In some instances, the dose of administered cells is sub-optimal and the combined administration of the agent boosts or augments expansion to achieve peak CAR+ T cells in the blood in the therapeutic range. In some instances, the method includes administering a dose of cells and monitoring the peak CAR+ T cells in the blood to ensure that the therapeutic range is maintained or achieved and, if it is not, administering an agent or compound to boost or augment the therapeutic dose. In some instances, low or limited expansion of cells, e.g., at low pharmacokinetic parameters such as low maximum CAR+ T cell concentration (Cₘₐₓ), tumor suppression effect may be limited.

In some instances, the agent is administered before, during, during the course of or after administration of the cells, *e.g.,* cells expressing a recombinant receptor, *e.g.* CAR. In some instances, such agents include agents that specifically augment, boost or enhance the expansion, proliferation, survival and/or efficacy of the engineered cells by virtue of specifically modulating the transgene, *e.g.,* transgene encoding a recombinant receptor. In some instances, such agents include agents that modulate the cell expansion and/or activity of the administered cells, *e.g.,* immune cells, such as T cells.

In some instances, the administered cell, *e.g.,* cells engineered to express a recombinant receptor, are modified to augment, boost or enhance the expansion, proliferation, survival and/or efficacy of the administered cells. In some instances, the administered cell, *e.g.,* cells engineered to express a recombinant receptor, are modified such that the expansion, proliferation, survival and/or efficacy of the engineered cells can be regulated and/or controlled, such as by administration of an agent. In some instances, the agent minimize the effects of inhibitory factors that suppress the proliferation, expansion and/or survival of the engineered cells *in vivo.*

In some instances, the additional agent is a small molecule, a peptide, a polypeptide, an antibody or antigen-binding fragment thereof, an antibody mimetic, an aptamer or a nucleic acid molecule (*e.g.* siRNA), a lipid, a polysaccharide or any combination thereof. In some instances, the additional agent is an inhibitor or an activator of a particular factor, molecule, receptor, function and/or enzyme. In some instances, the additional agent is an agonist or an antagonist of a particular factor, molecule, receptor, function and/or enzyme. In some instances, the additional agent is an analog or a derivative of one or more factors and/or metabolites. In some instances, the additional agent is a protein or polypeptide. In some instances, the additional agent is a cell, *e.g.,* an engineered cell, such as an additional dose of the same engineered cell that was administered and/or a different engineered cell.

In some instances, the agent is capable of transgene-specific expansion. In some instances, exemplary methods or agents for transgene-specific expansion include endogenous antigen exposure, vaccination, anti-idiotype antibodies or antigen-binding fragment thereof and/or regulatable recombinant receptor. For example, in some instances, methods for transgene-specific expansion include vaccination methods. In some instances, the agent is a peptide vaccine or a cell-based vaccine, *e.g.* cells engineered to express a particular antigen recognized by the recombinant receptor (see, *e.g.,* WO 2016/069647, WO 2011/066048, US 2016/0304624, US Pat. No. 9,476,028 and Hailemichael and Overwijk, Int J Biochem Cell Biol. (2014) 53: 46-50). In some instances, the methods for transgene-specific expansion include administering anti-idiotype antibodies. Anti-idiotype antibodies, including antigen-binding fragments thereof, specifically recognizes, is specifically targeted to, and/or specifically binds to an idiotope of an antibody or an antigen binding fragment thereof, *e.g.,* the antigen-binding domain of a recombinant receptor such as a chimeric antigen receptor (CAR). An idiotope is any single antigenic determinant or epitope within the variable portion of an antibody. In some instances, the anti-idiotype antibodies or antigen-binding fragments thereof are agonists and/or exhibit specific activity to stimulate cells expressing a particular antibody including conjugates or recombinant receptors containing the same or an antigen-binding fragment thereof (see, *e.g.,* U.S. Pat. Publication Nos. US 2016/0096902; US 2016/0068601; US 2014/0322183; US 2015/0175711; US 2015/283178; U.S. Pat. No. 9,102,760; Jena et al. PloS one (2013) 8(3):e57838; Long et al., Nature Medicine (2015) 21(6):581-590; Lee et al., The Lancet (2015) 385(9967):517-528; Zhao et al., PloS One (2014) 9(5):e96697; Leung et al., MAbs. (2015) 7(1):66-76).

In some instances, the methods include modulating the expansion of the engineered cells, for example, by inhibiting negative regulator of proliferation, expansion and/or activation of administered cells, *e.g.,* engineered immune cells. In particular environment in the body of the subject administered cells expressing the recombinant receptor, can encounter an environment that represses or suppresses the growth, proliferation, expansion and/or survival of the cells, *e.g.* immunosuppressive environment. For example, immunosuppressive environments can contain immunosuppressive cytokines, regulatory modulators and co-inhibitory receptors. In some instances, an additional agent can be used to modulate the expansion of the administered cells, *e.g.,* overcome suppressive environments.

In some instances, the additional agent includes an immunomodulatory agent, immune checkpoint inhibitor, modulators of metabolic pathways, adenosine pathway or adenosine receptor antagonist or agonist and modulators of signaling pathways, *e.g.,* kinase inhibitors.

In some instances, the additional agent is an immunomodulatory agent, such as an immune checkpoint inhibitor. In some examples, the additional agent increases, enhances or augments the expansion and/or proliferation of the administered cells and thereby increases, enhances or augments the immune response by blocking an immune checkpoint protein (i.e., immune checkpoint inhibitor). In some instances, the additional agent is an agent that enhances the activity of the engineered cell, *e.g.,* a recombinant receptor-expressing cell, is a molecule that inhibits an immune inhibitory molecule or an immune checkpoint molecule. Examples of immune inhibitory molecules include PD-1, PD-L1, CTLA4, TEVI3, CEACAM (*e.g.,* CEACAM-1, CEACAM-3 and/or CEACAM-5), LAG3, VISTA, BTLA, TIGIT, LAIR1, CD160, 2B4 and TGFβR. In some instances, the immune checkpoint inhibitor can be an antibody directed against an immune checkpoint protein, such as an antibody directed against cytotoxic T-lymphocyte antigen 4 (CTLA4 or CD152), programmed cell death protein 1 (PD-1), or programmed cell death protein 1 ligand 1 (PD-L1) (see, *e.g.,* Pardoll, Nat Rev Cancer. 2012 Mar. 22; 12(4):252-264).

Immune checkpoint inhibitors include any agent that blocks or inhibits in a statistically significant manner, the inhibitory pathways of the immune system. Such inhibitors may include small molecule inhibitors or may include antibodies, or antigen binding fragments thereof, that bind to and block or inhibit immune checkpoint receptors, ligands and/or receptor-ligand interaction. In some instances, modulation, enhancement and/or stimulation of particular receptors can overcome immune checkpoint pathway components. Illustrative immune checkpoint molecules that may be targeted for blocking, inhibition, modulation, enhancement and/or stimulation include, but are not limited to, PD-1 (CD279), PD-L1 (CD274, B7-H1), PDL2 (CD273, B7-DC), CTLA-4, LAG-3 (CD223), TIM-3, 4-1BB (CD137), 4-1BBL (CD137L), GITR (TNFRSF18, AITR), CD40, OX40 (CD134, TNFRSF4), CXCR2, tumor associated antigens (TAA), B7-H3, B7-H4, BTLA, HVEM, GAL9, B7H3, B7H4, VISTA, KIR, 2B4 (belongs to the CD2 family of molecules and is expressed on all NK, γδ, and memory CD8+ (αβ) T cells), CD160 (also referred to as BY55), CGEN-15049, CEACAM (e.g., CEACAM-1, CEACAM-3 and/or CEACAM-5), TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and a transforming growth factor receptor (TGFR; e.g., TGFR beta). Immune checkpoint inhibitors include antibodies, or antigen binding fragments thereof, or other binding proteins, that bind to and block or inhibit and/or enhance or stimulate the activity of one or more of any of the said molecules.

Exemplary immune checkpoint inhibitors include Tremelimumab (CTLA-4 blocking antibody, also known as ticilimumab, CP-675,206), anti-OX40, PD-L1 monoclonal antibody (Anti-B7-H1; MEDI4736), MK-3475 (PD-1 blocker), nivolumab (anti-PD-1 antibody), CT-011 (anti-PD-1 antibody), BY55 monoclonal antibody, AMP224 (anti-PD-L1 antibody), BMS-936559 (anti-PD-L1 antibody), MPLDL3280A (anti-PD-L1 antibody), MSB0010718C (anti-PD-L1 antibody) and ipilimumab (anti-CTLA-4 antibody, also known as Yervoy^{®}, MDX-010 and MDX-101). Exemplary of immunomodulatory antibodies include, but are not limited to, Daclizumab (Zenapax), Bevacizumab (Avastin ^{®}), Basiliximab, Ipilimumab, Nivolumab, pembrolizumab, MPDL3280A, Pidilizumab (CT-011), MK-3475, BMS-936559, MPDL3280A (Atezolizumab), tremelimumab, IMP321, BMS-986016, LAG525, urelumab, PF-05082566, TRX518, MK-4166, dacetuzumab (SGN-40), lucatumumab (HCD122), SEA-CD40, CP-870, CP-893, MEDI6469, MEDI6383, MOXR0916, AMP-224, MSB0010718C (Avelumab), MEDI4736, PDR001, rHIgM12B7, Ulocuplumab, BKT140, Varlilumab (CDX-1127), ARGX-110, MGA271, lirilumab (BMS-986015, IPH2101), IPH2201, ARGX-115, Emactuzumab, CC-90002 and MNRP1685A or an antibody-binding fragment thereof. Other exemplary immunomodulators include, e.g., afutuzumab (available from Roche^{®}); pegfilgrastim (Neulasta^{®}); lenalidomide (CC-5013, Revlimid^{®}); thalidomide (Thalomid^{®}), actimid (CC4047); and IRX-2 (mixture of human cytokines including interleukin 1, interleukin 2, and interferon gamma., CAS 951209-71-5, available from IRX Therapeutics).

In some instances, the agent includes a molecule that decreases the regulatory T cell (Treg) population. Methods that decrease the number of (e.g., deplete) Treg cells are known in the art and include, e.g., CD25 depletion, cyclophosphamide administration, and modulating Glucocorticoid-induced TNFR family related gene (GITR) function. GITR is a member of the TNFR superfamily that is upregulated on activated T cells, which enhances the immune system. Reducing the number of Treg cells in a subject prior to apheresis or prior to administration of engineered cells, e.g., CAR-expressing cells, can reduce the number of unwanted immune cells (e.g., Tregs) in the tumor microenvironment and reduces the subject's risk of relapse. In some instances, the agent includes a molecule targeting GITR and/or modulating GITR functions, such as a GITR agonist and/or a GITR antibody that depletes regulatory T cells (Tregs). In some instances, the agent includes cyclophosphamide. In some instances, the GITR binding molecule and/or molecule modulating GITR function (e.g., GITR agonist and/or Treg depleting GITR antibodies) is administered prior to the engineered cells, e.g., CAR-expressing cells. For example, in some instances, the GITR agonist can be administered prior to apheresis of the cells. In some instances, cyclophosphamide is administered to the subject prior to administration (e.g., infusion or re-infusion) of the engineered cells, e.g., CAR-expressing cells or prior to apheresis of the cells. In some instances, cyclophosphamide and an anti-GITR antibody are administered to the subject prior to administration (e.g., infusion or re-infusion) of the engineered cells, e.g., CAR-expressing cells or prior to apheresis of the cells.

In some instances, the agent is a GITR agonist. Exemplary GITR agonists include, e.g., GITR fusion proteins and anti-GITR antibodies (e.g., bivalent anti-GITR antibodies) such as, e.g., a GITR fusion protein described in U.S. Patent No. 6,111,090, European Patent No. 090505B1, U.S Patent No. 8,586,023, PCT Publication Nos.: WO 2010/003118 and 2011/090754, or an anti-GITR antibody described, e.g., in U.S. Patent No. 7,025,962, European Patent No. 1947183B1, U.S. Patent No. 7,812,135, U.S. Patent No. 8,388,967, U.S. Patent No. 8,591,886, European Patent No. EP 1866339, PCT Publication No. WO 2011/028683, PCT Publication No. WO 2013/039954, PCT Publication No. WO2005/007190, PCT Publication No. WO 2007/133822, PCT Publication No. WO2005/055808, PCT Publication No. WO 99/40196, PCT Publication No. WO 2001/03720, PCT Publication No. WO99/20758, PCT Publication No. WO2006/083289, PCT Publication No. WO 2005/115451, U.S. Patent No. 7,618,632, and PCT Publication No. WO 2011/051726. An exemplary anti-GITR antibody is TRX518.

In some instances, the agent is a structural or functional analog or derivative of thalidomide and/or an inhibitor of E3 ubiquitin ligase. In some instances, the immunomodulatory agent binds to cereblon (CRBN). In some instances, the immunomodulatory agent binds to the CRBN E3 ubiquitin-ligase complex. In some instances, the immunomodulatory agent binds to CRBN and the CRBN E3 ubiquitin-ligase complex. In some instances, the immunomodulatory agent up-regulates the protein or gene expression of CRBN. In some aspects, CRBN is the substrate adaptor for the CRL4^{CRBN} E3 ubiquitin ligase, and modulates the specificity of the enzyme. In some instances, binding to CRB or the CRBN E3 ubiquitin ligase complex inhibits E3 ubiquitin ligase activity. In some instances, the immunomodulatory agent induces the ubiqutination of KZF1 (Ikaros) and IKZF3 (Aiolos) and/or induces degradation of IKZF1 (Ikaros) and IKZF3 (Aiolos). In some instances, the immunomodulatory agent induces the ubiquitination of casein kinase 1A1 (CK1α) by the CRL4^{CRBN} E3 ubiquitin ligase. In some instances, the ubiquitination of CK1α results in CK1α degradation.

In some instances, the agent is an inhibitor of the Ikaros (IKZF1) transcription factor. In some instances, the agent enhances ubiquitination of Ikaros. In some instances, the agent enhances the degradation of Ikaros. In some instances, the agent down-regulates the protein or gene expression of Ikaros. In some instances, administration of the agent causes a decrease in Ikaros protein levels.

In some instances, the agent is an inhibitor of the Aiolos (IKZF3) transcription factor. In some instances, the agent enhances ubiquitination of Aiolos. In some instances, the agent enhances the degradation of Aiolos. In some instances, the agent down-regulates the protein or gene expression of Aiolos. In some instances, administration of the agent causes a decrease in Aiolos protein levels.

In some instances, the agent is thalidomide (2-(2,6-dioxopiperidin-3-yl)-1H-isoindole-1,3(2H)-dione) or an analog or derivative of thalidomide. In certain instances, a thalidomide derivative includes structural variants of thalidomide that have a similar biological activity. Exemplary thalidomide derivatives include, but are not limited to lenalidomide (REVLIMMUNOMODULATORY COMPOUND^{™}; Celgene Corporation), pomalidomide (also known as ACTIMMUNOMODULATORY COMPOUND^{™} or POMALYST^{™} (Celgene Corporation)), CC-1088, CDC-501, and CDC- 801, and the compounds disclosed in U.S. Pat. Nos. 5,712,291; 7,320,991; and 8,716,315; U.S. Appl. No. 2016/0313300; and PCT Pub. Nos. WO 2002/068414 and WO 2008/154252.

In some instances, the agent is 1-oxo- and 1,3 dioxo-2-(2,6-dioxopiperldin-3-yl) isoindolines substituted with amino in the benzo ring as described in U.S. Pat. No. 5,635,517.

In some instances, the agent is a compound that belongs to a class of isoindole-immunomodulatory compounds disclosed in U.S. Pat. No. 7,091,353, U.S. Patent Publication No. 2003/0045552, and International Application No. PCT/USOI/50401 (International Publication No. WO02/059106). For example, in some instances, the agent is [2-(2,6-dioxo-piperidin-3-yl)-1,3-dioxo-2,3-dihydro-1H-isoindol-4-ylmethyl]-amide; (2-(2,6-dioxo-piperidin-3-yl)-1,3-dioxo-2,3-dihydro-1H-isoindol-4-ylmethyl)-carbamic acid tert-butyl ester; 4-(aminomethyl)-2-(2,6-dioxo(3-piperidyl))-isoindoline-1,3-dione; N-(2-(2,6-dioxo-piperidin-3-yl)-1,3-dioxo-2,3-dihydro-1H-isoindol-4-ylmethyl)-acetamide; N-{(2-(2,6-dioxo(3-piperidyl)-1,3-dioxoisoindolin-4-yl)methyl}cyclopropyl-carboxamide; 2-chloro-N-{(2-(2,6-dioxo(3-piperidyl))-1,3-dioxoisoindolin-4-yl)methyl}acetamide; N-(2-(2,6-dioxo(3-piperidyl))-1,3-dioxoisoindolin-4-yl)-3-pyridylcarboxamide; 3-{1-oxo-4-(benzylamino)isoindolin-2-yl}piperidine-2,6-dione; 2-(2,6-dioxo(3-piperidyl))-4-(benzylamino)isoindoline-1,3-dione; N-{(2-(2,6-dioxo(3-piperidyl))-1,3-dioxoisoindolin-4-yl)methyl}propanamide; N-{(2-(2,6-dioxo(3-piperidyl))-1,3-dioxoisoindolin-4-yl)methyl}-3-pyridylcarboxamide; N-{(2-(2,6-dioxo(3-piperidyl))-1,3-dioxoisoindolin-4-yl)methyl}heptanamide; N-{(2-(2,6-dioxo(3-piperidyl))-1,3-dioxoisoindolin-4-yl)methyl}-2-furylcarboxamide; {N-(2-(2,6-dioxo(3-piperidyl))-1,3-dioxoisoindolin-4-yl)carbamoyl}methyl acetate; N-(2-(2,6-dioxo(3-piperidyl))-1,3-dioxoisoindolin-4-yl)pentanamide; N-(2-(2,6-dioxo(3-piperidyl))-1,3-dioxoisoindolin-4-yl)-2-thienylcarboxamide; N- f [2-(2,6-dioxo(3-piperidyl))-1,3-dioxoisoindolin-4-yl]methyl}(butylamino)carboxamide; N-{[2-(2,6-dioxo(3-piperidyl))-1,3-dioxoisoindolin-4-yl]methyl}(octylamino)carboxamide; or N-{[2-(2,6-dioxo(3-piperidyl))-1,3-dioxoisoindolin-4-yl]methyl}(benzylamino)carboxamide.

In some instances, the agent is lenalidomide, pomalidomide, avadomide, a stereoisomer of lenalidomide, pomalidomide, avadomide or a pharmaceutically acceptable salt, solvate, hydrate, co-crystal, clathrate, or polymorph thereof. In some instances, the immunomodulatory compound is lenalidomide, a stereoisomer of lenalidomide or a pharmaceutically acceptable salt, solvate, hydrate, co-crystal, clathrate, or polymorph thereof. In some instances, the immunomodulatory compound is lenalidomide, or ((RS)-3-(4-Amino-1-oxo-1,3-dihydro-2H-isoindol-2-yl)piperidine-2,6-dione).

In some instances, the methods include contacting the cells expressing the recombinant receptor with an agent that inhibits inhibitory cell surface receptors, *e.g.,* transforming growth factor beta receptor (TGFβR). In some instances, administered cells, *e.g.,* recombinant receptor expressing cells, can be engineered to resist the effects of immunosuppressive cytokines that can inhibit their effector functions (see, *e.g.,* Foster et al., J Immunother. (2008) 31:500-505; Bollard et al., Molecular Therapy. (2012) 20:S22; Bendle et al., J. Immunol. (2013) 191(6):3232-3239). In some instances, the additional agent is an anti-TGFβ antibody or an anti-TGFβR antibody (see, *e.g.,* WO 2011/109789).

In some instances, the additional agent modulates the metabolism, signaling and/or transport of immunosuppressive factors, *e.g.,* adenosine. In some instances, the additional agent is an inhibitor of extracellular adenosine or adenosine receptor, or an agent that causes a reduction or a decrease of extracellular adenosine levels, such as an agent that prevents the formation of, degrades, renders inactive, and/or decreases extracellular adenosine. In some instances, the additional agent is an adenosine receptor antagonist such as the A2a, A2b and/or A3 receptor. In some instances, the antagonist is a peptide, or a pepidomimetic, that binds the adenosine receptor but does not trigger a G1 protein dependent intracellular pathway. Exemplary adenosine receptor antagonists are described in U.S. Pat. Nos. 5,565,566; 5,545,627, 5,981,524; 5,861,405; 6,066,642; 6,326,390; 5,670,501; 6,117,998; 6,232,297; 5,786,360; 5,424,297; 6,313,131, 5,504,090; and 6,322,771; and Jacobson and Gao, Nat Rev Drug Discov. (2006) 5(3): 247-264.

In some instances, the agent is an A2 receptor (A2R) antagonist, such as an A2a antagonist. Exemplary A2R antagonists include KW6002 (istradefyline), SCH58261, caffeine, paraxanthine, 3,7-dimethyl-1-propargylxanthine (DMPX), 8-(m-chlorostyryl) caffeine (CSC), MSX-2, MSX-3, MSX-4, CGS-15943, ZM-241385, SCH-442416, preladenant, vipadenant (BII014), V2006, ST-1535, SYN-115, PSB-1115, ZM241365, FSPTP, and an inhibitory nucleic acid targeting A2R expression, e.g., siRNA or shRNA, or any antibodies or antigen-binding fragment thereof that targets an A2R. In some instances, the agent is an A2R antagonist described in, e.g., Ohta et al., Proc Natl Acad Sci U S A (2006) 103:13132-13137; Jin et al., Cancer Res. (2010) 70(6):2245-2255; Leone et al., Computational and Structural Biotechnology Journal (2015) 13:265-272; Beavis et al., Proc Natl Acad Sci U S A (2013) 110:14711-14716; and Pinna, A., Expert Opin Investig Drugs (2009) 18:1619-1631; Sitkovsky et al., Cancer Immunol Res (2014) 2(7):598-605; US 8,080,554; US 8,716,301; US 20140056922; WO2008/147482; US 8,883,500; US 20140377240; WO02/055083; US 7,141,575; US 7,405,219; US 8,883,500; US 8,450,329 and US 8,987,279).

In some instances, the methods include administering additional agents that are immunostimulatory. In some instances, the additional agent can generally promote the proliferation, expansion, survival and/or efficacy of immune cells. In some instances, the additional agent can specifically promote administered cells, *e.g.,* recombinant receptor-expressing cells. In some instances, the additional agent is a cytokine. In some instances, the additional agent is a ligand.

In some instances, the additional agent is an immunostimulatory ligand, *e.g.,* CD40L. In some instances, the additional agent is a cytokine, *e.g.,* IL-2, IL-3, IL-6, IL-11, IL-7, IL-12, IL-15, IL-21, granulocyte macrophage colony stimulating factor (GM-CSF), alpha, beta or gamma interferon (IFN) and erythropoietin (EPO). In some instances, the agent is a cytokine. In some instances, the immunomodulatory agent is a cytokine or is an agent that induces increased expression of a cytokine in the tumor microenvironment. Cytokines have important functions related to T cell expansion, differentiation, survival, and homeostasis. Cytokines that can be administered to the subject receiving the cells and/or compositions described herein include one or more of IL-2, IL-4, IL-7, IL-9, IL-15, IL-18, and IL-21. In some instances, the cytokine administered is IL-7, IL-15, or IL-21, or a combination thereof. In some instances, administration of the cytokine to the subject that has sub-optimal response to the administration of the engineered cells, e.g., CAR-expressing cells improves efficacy and/or anti-tumor activity of the administered cells, e.g., CAR-expressing cells.

In some instances, the agent is an inhibitor of hypoxia inducible factor 1 alpha (HIF-1α) signaling. Exemplary inhibitors of HIF-1α include digoxin, acriflavine, sirtuin-7 and ganetespib.

In some instances, the agent includes a protein tyrosine phosphatase inhibitor, e.g., a protein tyrosine phosphatase inhibitor described herein. In some instances, the protein tyrosine phosphatase inhibitor is an SHP-1 inhibitor, e.g., an SHP-1 inhibitor described herein, such as, e.g., sodium stibogluconate. In some instances, the protein tyrosine phosphatase inhibitor is an SHP-2 inhibitor, e.g., an SHP-2 inhibitor described herein.

In some aspects, the method results in at least a 2-fold, at least a 4-fold, at least a 10-fold, or at least a 20-fold increase in copies of nucleic acid encoding the recombinant receptor, *e.g.,* CAR, per microgram of DNA, *e.g.,* in the serum, plasma, blood or tissue, *e.g.,* tumor sample, of the subject. (from old section, move to method of modulation section)

In some aspects, the method results in high *in vivo* proliferation of the administered cells, for example, as measured by flow cytometry. In some aspects, high peak proportions of the cells are detected. For example, in some instances, at a peak or maximum level or concentration following the administration of the T cells, *e.g.,* CAR-expressing T cells, in the blood or disease-site of the subject or white blood cell fraction thereof, *e.g.,* PBMC fraction or T cell fraction, at least about 10%, at least about 20%, at least about 30%, at least about 40%, at least about 50%, at least about 60%, at least about 70%, at least about 80%, or at least about 90% of the cells express the recombinant receptor, *e.g.,* the CAR.

In some instances, the method results in a maximum concentration, in the blood or serum or other bodily fluid or organ or tissue of the subject, of at least 100, 500, 1000, 1500, 2000, 5000, 10,000 or 15,000 copies of or nucleic acid encoding the receptor, *e.g.,* the CAR, per microgram of DNA, or at least 0.1, 0.2, 0.3, 0.4, 0.5, 0.6, 0.7, 0.8, or 0.9 receptor-expressing, *e.g.,* CAR-expressing cells per total number of peripheral blood mononuclear cells (PBMCs), total number of mononuclear cells, total number of T cells, or total number of microliters of the blood or serum or other bodily fluid or organ or tissue of the subject. In some instances, the cells expressing the receptor are detected as at least 10, 20, 30, 40, 50, or 60 % of total PBMCs in the blood of the subject, and/or at such a level for at least 1, 2, 3, 4, 5, 6, 7, 8, 9, 10, 11, 12, 24, 36, 48, or 52 weeks following the T cells, e.g., CAR-expressing T cells or for 1, 2, 3, 4, or 5, or more years following such administration.

### 2. Agents for Reducing Cell Expansion

In some instances, the described methods and articles of manufacture can be used in connection with, or involve or include, one or more agents or treatments capable of modulating, e.g., increasing or decreasing, CAR+ T cell expansion, proliferation, and/or activity. In some instances, the agent is capable of reducing, decreasing, and/or dampening CAR+ T cell expansion and/or proliferation. In some instances, expansion and/or proliferation of CAR+ T cells above a certain threshold value, or high expression of certain biomarkers, such as inflammatory markers, can be associated with a reduced response and/or reduced durable response. In some instances, if the administered cells in the subject are determined to have very high or excessive expansion, or if the subject is determined to express biomarkers associated with very high expansion or excessive expansion, the subject may be determined not likely to achieve response and/or durable response. In some instances, very high expansion or excessive expansion is also associated with high tumor burden and inflammatory cytokine production. In some instances, an agent that can reduce, decrease and/or dampen CAR+ T cell expansion and/or proliferation can be administered to such subjects.

In some contexts, optimal efficacy of an administered cell therapy, e.g., CAR+ T cell therapy, can depend on the ability of the administered cells to become activated, expand, to exert various effector functions, to persist, including long-term, to differentiate, transition or engage in reprogramming into certain phenotypic states (such as long-lived memory, less-differentiated, and effector states), to avoid or reduce immunosuppressive conditions in the local microenvironment of a disease, to provide effective and robust recall responses following clearance and re-exposure to target ligand or antigen, and avoid or reduce exhaustion, anergy, peripheral tolerance, terminal differentiation, and/or differentiation into a suppressive state. In some aspects, excessive or very high expansion or proliferation of the administered T cells may result in exhaustion, anergy, peripheral tolerance, terminal differentiation, and/or differentiation into a suppressive state. In some aspects, an agent that can reduce, decrease and/or dampen CAR+ T cell expansion and/or proliferation can prevent or reduce such exhaustion or differentiation.

In some instances, the administration of the agent is capable of reducing, decreasing, and/or dampening CAR+ T cell expansion and/or proliferation, such as a steroid, can result in reduced expansion of the administered CAR+ T cells. In some instances, administration of the agent can result in changes in parameters, e.g., reduced volumetric measures, e.g., SPD, or expression of inflammatory markers, e.g., LDH.

In some instances, the agent capable of reducing, decreasing, and/or dampening CAR+ T cell expansion and/or proliferation is a steroid, is an antagonist or inhibitor of a cytokine receptor, such as IL-6 receptor, CD122 receptor (IL-2Rbeta receptor), or CCR2, or is an inhibitor of a cytokine, such as IL-6, MCP-1, IL-10, IFN-γ, IL-8, or IL-18. In some instances, the agent is an agonist of a cytokine receptor and/or cytokine, such as TGF-β. In some instances, the agent, e.g., agonist, antagonist or inhibitor, is an antibody or antigen-binding fragment, a small molecule, a protein or peptide, or a nucleic acid.

In some instances, the agent capable of reducing, decreasing, and/or dampening CAR+ T cell expansion and/or proliferation is a steroid, e.g., corticosteroid. Corticosteroids typically include glucocorticoids and mineralocorticoids.

Any corticosteroid, e.g., glucocorticoid, can be used in the methods described herein. In some instances, glucocorticoids include synthetic and non-synthetic glucocorticoids. Exemplary glucocorticoids include, but are not limited to: alclomethasones, algestones, beclomethasones (e.g. beclomethasone dipropionate), betamethasones (e.g. betamethasone 17-valerate, betamethasone sodium acetate, betamethasone sodium phosphate, betamethasone valerate), budesonides, clobetasols (e.g. clobetasol propionate), clobetasones, clocortolones (e.g. clocortolone pivalate), cloprednols, corticosterones, cortisones and hydrocortisones (e.g. hydrocortisone acetate), cortivazols, deflazacorts, desonides, desoximethasones, dexamethasones (e.g. dexamethasone 21-phosphate, dexamethasone acetate, dexamethasone sodium phosphate), diflorasones (e.g. diflorasone diacetate), diflucortolones, difluprednates, enoxolones, fluazacorts, flucloronides, fludrocortisones (e.g., fludrocortisone acetate), flumethasones (e.g. flumethasone pivalate), flunisolides, fluocinolones (e.g. fluocinolone acetonide), fluocinonides, fluocortins, fluocortolones, fluorometholones (e.g. fluorometholone acetate), fluperolones (e.g., fluperolone acetate), fluprednidenes, fluprednisolones, flurandrenolides, fluticasones (e.g. fluticasone propionate), formocortals, halcinonides, halobetasols, halometasones, halopredones, hydrocortamates, hydrocortisones (e.g. hydrocortisone 21-butyrate, hydrocortisone aceponate, hydrocortisone acetate, hydrocortisone buteprate, hydrocortisone butyrate, hydrocortisone cypionate, hydrocortisone hemisuccinate, hydrocortisone probutate, hydrocortisone sodium phosphate, hydrocortisone sodium succinate, hydrocortisone valerate), loteprednol etabonate, mazipredones, medrysones, meprednisones, methylprednisolones (methylprednisolone aceponate, methylprednisolone acetate, methylprednisolone hemisuccinate, methylprednisolone sodium succinate), mometasones (e.g., mometasone furoate), paramethasones (e.g., paramethasone acetate), prednicarbates, prednisolones (e.g. prednisolone 25-diethylaminoacetate, prednisolone sodium phosphate, prednisolone 21-hemisuccinate, prednisolone acetate; prednisolone famesylate, prednisolone hemisuccinate, prednisolone-21 (beta-D-glucuronide), prednisolone metasulphobenzoate, prednisolone steaglate, prednisolone tebutate, prednisolone tetrahydrophthalate), prednisones, prednivals, prednylidenes, rimexolones, tixocortols, triamcinolones (e.g. triamcinolone acetonide, triamcinolone benetonide, triamcinolone hexacetonide, triamcinolone acetonide 21-palmitate, triamcinolone diacetate). These glucocorticoids and the salts thereof are discussed in detail, for example, in Remington's Pharmaceutical Sciences, A. Osol, ed., Mack Pub. Co., Easton, Pa. (16th ed. 1980).

In some instances, the steroid is administered after administration of the immunotherapy and/or cell therapy, or a first administration or dose thereof, or after the initiation of any of the foregoing. In some instances, the steroid is administered within 12, 18, 24, 36 or 48 hours, 1, 2, 3, 4, 5, 6, 7, 8, 9, 10, 11, 12, 13, 14 days or more, or 2, 3, 4, 5, 6, 7, 8, 9, 10 weeks or more, after administration of the immunotherapy and/or cell therapy, or a first administration or dose thereof, or after the initiation of any of the foregoing. In some instances, the steroid is administered within 12, 24, 36 or 48 hours, or within 2, 3, or 4 days after administration of the immunotherapy and/or cell therapy, or a first administration or dose thereof, or after the initiation of any of the foregoing.

In some examples, the glucocorticoid is selected from among cortisones, dexamethasones, hydrocortisones, methylprednisolones, prednisolones and prednisones. In a particular example, the glucocorticoid is dexamethasone.

In some instances, the agent is a corticosteroid and is administered in an amount that is therapeutically effective to reduce, decrease and/or dampen CAR+ T cell expansion and/or proliferation. In some instances, indicators of improvement or successful treatment include determination of pharmacokinetic parameters, e.g., any described herein, such as peak CAR+ T cell concentration and/or AUC.

In some aspects, the corticosteroid is provided in a therapeutically effective dose. Therapeutically effective concentration can be determined empirically by testing in known in vitro or in vivo (e.g. animal model) systems. In addition, animal models can be employed to help identify optimal dosage ranges. The precise dosage, which can be determined empirically, can depend on the particular therapeutic preparation, the regime and dosing schedule, the route of administration and the seriousness of the disease.

The corticosteroid can be administered in any amount that is effective reduce, decrease and/or dampen CAR+ T cell expansion and/or proliferation. The corticosteroid, e.g., glucocorticoid, can be administered, for example, at an amount between at or about 0.1 and 100 mg, per dose, 0.1 to 80 mg, 0.1 to 60 mg, 0.1 to 40 mg, 0.1 to 30 mg, 0.1 to 20 mg, 0.1 to 15 mg, 0.1 to 10 mg, 0.1 to 5 mg, 0.2 to 40 mg, 0.2 to 30 mg, 0.2 to 20 mg, 0.2 to 15 mg, 0.2 to 10 mg, 0.2 to 5 mg, 0.4 to 40 mg, 0.4 to 30 mg, 0.4 to 20 mg, 0.4 to 15 mg, 0.4 to 10 mg, 0.4 to 5 mg, 0.4 to 4 mg, 1 to 20 mg, 1 to 15 mg or 1 to 10 mg, to a 70 kg adult human subject. Typically, the corticosteroid, such as a glucocorticoid is administered at an amount between at or about 0.4 and 20 mg, for example, at or about 0.4 mg, 0.5 mg, 0.6 mg, 0.7 mg, 0.75 mg, 0.8 mg, 0.9 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg or 20 mg per dose, to an average adult human subject.

In some instances, the corticosteroid can be administered, for example, at a dosage of at or about 0.001 mg/kg (of the subject), 0.002 mg/kg, 0.003 mg/kg, 0.004 mg/kg, 0.005 mg/kg, 0.006 mg/kg, 0.007 mg/kg, 0.008 mg/kg, 0.009 mg/kg, 0.01 mg/kg, 0.015 mg/kg, 0.02 mg/kg, 0.025 mg/kg, 0.03 mg/kg, 0.035 mg/kg, 0.04 mg/kg, 0.045 mg/kg, 0.05 mg/kg, 0.055 mg/kg, 0.06 mg/kg, 0.065 mg/kg, 0.07 mg/kg, 0.075 mg/kg, 0.08 mg/kg, 0.085 mg/kg, 0.09 mg/kg, 0.095 mg/kg, 0.1 mg/kg, 0.15 mg/kg, 0.2 mg/kg, 0.25 mg/kg, 0.30 mg/kg, 0.35 mg/kg, 0.40 mg/kg, 0.45 mg/kg, 0.50 mg/kg, 0.55 mg/kg, 0.60 mg/kg, 0.65 mg/kg, 0.70 mg/kg, 0.75 mg/kg, 0.80 mg/kg, 0.85 mg/kg, 0.90 mg/kg, 0.95 mg/kg, 1 mg/kg, 1.05 mg/kg, 1.1 mg/kg, 1.15 mg/kg, 1.20 mg/kg, 1.25 mg/kg, 1.3 mg/kg, 1.35 mg/kg or 1.4 mg/kg, to an average adult human subject, typically weighing about 70 kg to 75 kg.

The corticosteroid, or glucocorticoid, for example dexamethasone, can be administered orally (tablets, liquid or liquid concentrate), PO, intravenously (IV), intramuscularly or by any other known route or route described herein (e.g., with respect to pharmaceutical formulations). In some aspects, the corticosteroid is administered as a bolus, and in other aspects it may be administered over a period of time. In some aspects, the corticosteroid is administered as a bolus, and in other aspects it may be administered over a period of time, e.g., over 1, 2, 3, 4, 5, 6, 7, 8, 9, 10, 15, 20, 30, 40, 50, 60, 70, 80, 90, 120, 180, 240, 360, 480 or 720 minutes or more, or a range defined by any two of the foregoing values.

In some aspects, the glucocorticoid can be administered over a period of more than one day, such as over two days, over 3 days, or over 4 or more days. In some instances, the corticosteroid can be administered one per day, twice per day, or three times or more per day. For example, the corticosteroid, e.g., dexamethasone, may in some examples be administered at 10 mg (or equivalent) IV twice a day for three days.

In some instances, the steroid, e.g., corticosteroid, is administered in multiple doses over a period of time. In some aspects, the steroid, e.g., corticosteroid, can be administered over a period of more than 2, 3, 4, 5, 6, 7, 8, 9, 10, 11, 12, 13, 14 days or more, or more than 2, 3, 4, 5, 6, 7, 8, 9, 10 weeks or more. In some instances, the steroid, e.g., corticosteroid, can be administered in multiple or repeat doses over a total duration of about 6, 12, 18, 24 hours or more, or 2, 3, 4, 5, 6, 7, 8, 9, 10, 11, 12, 13, 14 days or more, or 2, 3, 4, 5, 6, 7, 8, 9, 10 weeks or more. In some instances, the steroid, e.g., corticosteroid, can be administered one per day, twice per day, or three times or more per day. In some instances, the steroid, e.g., corticosteroid, can be administered at least or at least about every 1, 2, 3, 4, 5, 6, 7, 8, 9, 10, 11, 12, 18, 24, 36, 48 hours, or every 3, 4, 5, 6, 7, 8, 9, 10, 11, 12, 13, 14 days, or every 2, 3, 4, 5, 6, 7, 8, 9 or 10 weeks or more. In some aspects, the steroid, e.g., glucocorticoid, can be administered in multiple or repeated doses, over a period of more than one day, such as over two days, over 3 days, or over 4 or more days. In some instances, the steroid, e.g., corticosteroid or glucocorticoid, can be administered for a total duration of 6, 12, 18, 24 hours or 2, 3, 4, 5, 6, 7, 8, 9 or 10 days or more. In some instances, the corticosteroid can be administered one per day, twice per day, or three times or four times or more per day. In some instances, the corticosteroid can be administered at least or at least about every 1, 2, 3, 4, 5, 6, 7, 8, 9, 10, 11, 12, 18, 24, 36, 48 hours or more.

In some instances, the steroid, e.g., corticosteroid or glucocorticoid, can be administered at a given dose per day, e.g., a specific dose per day. In some instances, exemplary dose per day includes 0.1, 0.2, 0.3, 0.4, 0.5, 0.6, 0.7, 0.8, 0.9, 1.0, 1.5, 2, 3, 4, 5, 6, 7, 8, 9 or 10 mg/kg per day, or a range defined by any two of the foregoing values and equivalents thereof. In some instances, the steroid, e.g., corticosteroid or glucocorticoid, can be administered at or about 0.25, 0.5, 0.75, 1, 1.5, 2.0, 2.5, 3.0, 3.5, 4.0, 4.5, 5.0, 5.5, 6.0, 7.0, 8.0, 9.0, 10.0, 11.0, 12.0, 13.0, 14.0, 15.0, 16.0, 17.0, 18.0, 19.0, 20.0, 25.0, 50.0 or 100.0 mg/kg/day, or a range defined by any two of the foregoing values and equivalents thereof. In some instances, exemplary dose per day includes 5, 10, 20, 30, 40, 50, 60, 70, 80, 90, 100, 150 or 200 mg per day, or a range defined by any two of the foregoing values and equivalents thereof. In some instances, the steroid, e.g., corticosteroid or glucocorticoid, can be administered at or about 5, 10, 20, 30, 40, 50, 60, 70, 80, 90, 100, 150 or 200 mg/day, or a range defined by any two of the foregoing values and equivalents thereof.

In some instances, the dosage of corticosteroid, e.g., glucocorticoid, is administered in successively lower dosages per treatment. Hence, in some such treatment regimes, the dose of corticosteroid is tapered. For example, the corticosteroid may be administered at an initial dose (or equivalent dose, such as with reference to dexamethasone) of 4 mg, and upon each successive administration the dose may be lowered, such that the dose is 3 mg for the next administration, 2 mg for the next administration, and 1 mg for the next administration

Generally, the dose of corticosteroid administered is dependent upon the specific corticosteroid, as a difference in potency exists between different corticosteroids. It is typically understood that drugs vary in potency, and that doses can therefore vary, in order to obtain equivalent effects. Table 4 shows equivalence in terms of potency for various glucocorticoids and routes of administration. Equivalent potency in clinical dosing is well known. Information relating to equivalent steroid dosing (in a non-chronotherapeutic manner) may be found in the British National Formulary (BNF) 37, March 1999.

| **Table 4: Glucocorticoid administration** | |
|---|---|
| **Glucocorticoid (Route)** | **Equivalency Potency** |
| Hydrocortisone (IV or PO) | 20 |
| Prednisone | 5 |
| Prednisolone (IV or PO) | 5 |
| Methylprednisolone sodium succinate (IV) | 4 |
| Dexamethasone (IV or PO) | 0.5-0.75 |

Thus, in some instances, the steroid is administered in an equivalent dosage amount of from or from about 1.0 mg to 20 mg dexamethasone per day, such as 1.0 mg to 15 mg dexamethasone per day, 1.0 mg to 10 mg dexamethasone per day, 2.0 mg to 8 mg dexamethasone per day, or 2.0 mg to 6.0 mg dexamethasone per day, each inclusive. In some cases, the steroid is administered in an equivalent dose of at or about 4 mg or at or about 8 mg dexamethasone per day.

In some instances, the steroid is administered if fever persists after treatment with tocilizumab. For example, in some instances, dexamethasone is administered orally or intravenously at a dosage of 5-10 mg up to every 6-12 hours with continued fevers. In some instances, tocilizumab is administered concurrently with or subsequent to oxygen supplementation.

In some instances, the agent capable of reducing, decreasing, and/or dampening CAR+ T cell expansion and/or proliferation is an inhibitor of a microglial cell activity. In some instances, the administration of the inhibitor modulates the activity of microglia. In some instances, the inhibitor is an antagonist that inhibits the activity of a signaling pathway in microglia. In some instances, the microglia inhibitor affects microglial homeostasis, survival, and/or proliferation. In some instances, the inhibitor targets the CSF1R signaling pathway. In some instances, the inhibitor is an inhibitor of CSF1R. In some instances, the inhibitor is a small molecule. In some cases, the inhibitor is an antibody.

In some aspects, administration of the inhibitor results in one or more effects selected from an alteration in microglial homeostasis and viability, a decrease or blockade of microglial cell proliferation, a reduction or elimination of microglial cells, a reduction in microglial activation, a reduction in nitric oxide production from microglia, a reduction in nitric oxide synthase activity in microglia, or protection of motor neurons affected by microglial activation. In some instances, the agent alters the level of a serum or blood biomarker of CSF1R inhibition, or a decrease in the level of urinary collagen type 1 cross-linked N-telopeptide (NTX) compared to at a time just prior to initiation of the administration of the inhibitor. In some instances, the administration of the agent transiently inhibits the activity of microglia activity and/or wherein the inhibition of microglia activity is not permanent. In some instances, the administration of the agent transiently inhibits the activity of CSF1R and/or wherein the inhibition of CSF1R activity is not permanent.

In some instances, the agent capable of reducing, decreasing, and/or dampening CAR+ T cell expansion and/or proliferation is selected from an anti-inflammatory agent, an inhibitor of NADPH oxidase (NOX2), a calcium channel blocker, a sodium channel blocker, inhibits GM-CSF, inhibits CSF1R, specifically binds CSF-1, specifically binds IL-34, inhibits the activation of nuclear factor kappa B (NF-κB), activates a CB₂ receptor and/or is a CB₂ agonist, a phosphodiesterase inhibitor, inhibits microRNA-155 (miR-155), upregulates microRNA-124 (miR-124), inhibits nitric oxide production in microglia, inhibits nitric oxide synthase, or activates the transcription factor NRF2 (also called nuclear factor (erythroid-derived 2)-like 2, or NFE2L2).

In some instances, the agent capable of reducing, decreasing, and/or dampening CAR+ T cell expansion and/or proliferation is one that targets a cytokine, e.g., is an antagonist or inhibitor of a cytokine, such as transforming growth factor beta (TGF-beta), interleukin 6 (IL-6), interleukin 10 (IL-10), IL-2, MIP1β (CCL4), TNF alpha, IL-1, interferon gamma (IFN-gamma), or monocyte chemoattractant protein-1 (MCP-1). In some instances, the agent capable of reducing, decreasing, and/or dampening CAR+ T cell expansion and/or proliferation is one that targets (e.g. inhibits or is an antagonist of) a cytokine receptor, such as IL-6 receptor (IL-6R), IL-2 receptor (IL-2R/CD25), MCP-1 (CCL2) receptor (CCR2 or CCR4), a TGF-beta receptor (TGF-beta I, II, or III), IFN-gamma receptor (IFNGR), MIP1β receptor (e.g., CCR5), TNF alpha receptor (e.g., TNFR1), IL-1 receptor (IL1-Rα/IL-1Rβ), or IL-10 receptor (IL-10R).

The amount of a selected agent capable of reducing, decreasing, and/or dampening CAR+ T cell expansion and/or proliferation can be determined by standard clinical techniques. Exemplary adverse events include, but are not limited to, an increase in alanine aminotransferase, an increase in aspartate aminotransferase, chills, febrile neutropenia, headache, hypotension, left ventricular dysfunction, encephalopathy, hydrocephalus, seizure, and/or tremor.

In some instances, the agent is administered in a dosage amount of from or from about 30 mg to 5000 mg, such as 50 mg to 1000 mg, 50 mg to 500 mg, 50 mg to 200 mg, 50 mg to 100 mg, 100 mg to 1000 mg, 100 mg to 500 mg, 100 mg to 200 mg, 200 mg to 1000 mg, 200 mg to 500 mg or 500 mg to 1000 mg.

In some instances, the agent is administered from or from about 0.5 mg/kg to 100 mg/kg, such as from or from about 1 mg/kg to 50 mg/kg, 1 mg/kg to 25 mg/kg, 1 mg/kg to 10 mg/kg, 1 mg/kg to 5 mg/kg, 5 mg/kg to 100 mg/kg, 5 mg/kg to 50 mg/kg, 5 mg/kg to 25 mg/kg, 5 mg/kg to 10 mg/kg, 10 mg/kg to 100 mg/kg, 10 mg/kg to 50 mg/kg, 10 mg/kg to 25 mg/kg, 25 mg/kg to 100 mg/kg, 25 mg/kg to 50 mg/kg to 50 mg/kg to 100 mg/kg. In some instances, the agent is administered in a dosage amount of from or from about 1 mg/kg to 10 mg/kg, 2 mg/kg to 8 mg/kg, 2 mg/kg to 6 mg/kg, 2 mg/kg to 4 mg/kg or 6 mg/kg to 8 mg/kg, each inclusive. In some aspects, the agent is administered in a dosage amount of at least or at least about or about 1 mg/kg, 2 mg/kg, 4 mg/kg, 6 mg/kg, 8 mg/kg, 10 mg/kg or more. In some instances, the agent is administered at a dose of 4 mg/kg or 8 mg/kg.

In some instances, the agent is administered by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some instances, they are administered by parenteral, intrapulmonary, and intranasal, and, if desired for local treatment, intralesional administration. Parenteral infusions include intramuscular, intravenous, intraarterial, intraperitoneal, or subcutaneous administration.

In some instances, the amount of the agent is administered about or approximately twice daily, daily, every other day, three times a week, weekly, every other week or once a month.

In some instances, the agent is administered as part of a composition or formulation, such as a pharmaceutical composition or formulation as described below. Thus, in some cases, the composition comprising the agent is administered as described below. In other aspects, the agent is administered alone and may be administered by any known acceptable route of administration or by one described herein, such as with respect to compositions and pharmaceutical formulations.

In some instances, the agent is a small molecule, peptide, protein, antibody or antigen-binding fragment thereof, an antibody mimetic, an aptamer, or a nucleic acid molecule. In some instances, the method involves administration of an inhibitor of microglia activity. In some instances, the agent is an antagonist that inhibits the activity of a signaling pathway in microglia. In some instances, the agent affects microglial homeostasis, survival, and/or proliferation.

In some instances, the agent capable of reducing, decreasing, and/or dampening CAR+ T cell expansion and/or proliferation, is an antibody or antigen binding fragment. In some instances, the agent is tocilizumab, siltuximab, sarilumab, olokizumab (CDP6038), elsilimomab, ALD518/BMS-945429, sirukumab (CNTO 136), CPSI-2634, ARGX-109, FE301, or FM101.

In some instances, the agent is an antagonist or inhibitor of IL-6 or the IL-6 receptor (IL-6R). In some aspects, the agent is an antibody that neutralizes IL-6 activity, such as an antibody or antigen-binding fragment that binds to IL-6 or IL-6R. For example, in some instances, the agent is or comprises tocilizumab (atlizumab) or sarilumab, anti-IL-6R antibodies. In some instances, the agent is an anti-IL-6R antibody described in U.S. Patent No: 8,562,991. In some cases, the agent that targets IL-6 is an anti-IL-6 antibody, such as siltuximab, elsilimomab, ALD518/BMS-945429, sirukumab (CNTO 136), CPSI-2634, ARGX-109, FE301, FM101, or olokizumab (CDP6038). In some aspects, the agent may neutralize IL-6 activity by inhibiting the ligand-receptor interactions. The feasibility of this general type of approach has been demonstrated with a natural occurring receptor antagonist for interleukin-1. *See* Harmurn, C. H. et al., Nature (1990) 343:336-340. In some aspects, the IL-6/IL-6R antagonist or inhibitor is an IL-6 mutein, such as one described in U.S. Patent No. 5591827. In some instances, the agent that is an antagonist or inhibitor of IL-6/IL-6R is a small molecule, a protein or peptide, or a nucleic acid.

In some instances, the agent is tocilizumab. In some instances, tocilizumab is administered as an early intervention in accord with the described methods, and/or with the described articles of manufacture or compositions, at a dosage of from or from about 1 mg/kg to 12 mg/kg, such as at or about 4 mg/kg, 8 mg/kg, or 10 mg/kg. In some instances, tocilizumab is administered by intravenous infusion. In some instances, tocilizumab is administered for a persistent fever of greater than 39°C lasting 10 hours that is unresponsive to acetaminophen. In some instances, a second administration of tocilizumab is provided if symptoms recur after 48 hours of the initial dose.

In some instances, the agent is an agonist or stimulator of TGF-β or a TGF-β receptor (e.g., TGF-β receptor I, II, or III). In some aspects, the agent is an antibody that increases TGF-β activity, such as an antibody or antigen-binding fragment that binds to TGF-β or one of its receptors. In some instances, the agent that is an agonist or stimulator of TGF-β and/or its receptor is a small molecule, a protein or peptide, or a nucleic acid.

In some instances, the agent is an antagonist or inhibitor of MCP-1 (CCL2) or a MCP-1 receptor (e.g., MCP-1 receptor CCR2 or CCR4). In some aspects, the agent is an antibody that neutralizes MCP-1 activity, such as an antibody or antigen-binding fragment that binds to MCP-1 or one of its receptors (CCR2 or CCR4). In some instances, the MCP-1 antagonist or inhibitor is any described in Gong et al. J Exp Med. 1997 Jul 7; 186(1): 131-137 or Shahrara et al. J Immunol 2008; 180:3447-3456. In some instances, the agent that is an antagonist or inhibitor of MCP-1 and/or its receptor (CCR2 or CCR4) is a small molecule, a protein or peptide, or a nucleic acid.

In some instances, the agent is an antagonist or inhibitor of IFN-γ or an IFN-γ receptor (IFNGR). In some aspects, the agent is an antibody that neutralizes IFN-γ activity, such as an antibody or antigen-binding fragment that binds to IFN-γ or its receptor (IFNGR). In some aspects, the IFN-gamma neutralizing antibody is any described in Dobber et al. Cell Immunol. 1995 Feb;160(2):185-92 or Ozmen et al. J Immunol. 1993 Apr 1;150(7):2698-705. In some instances, the agent that is an antagonist or inhibitor of IFN-y/IFNGR is a small molecule, a protein or peptide, or a nucleic acid.

In some instances, the agent is an antagonist or inhibitor of IL-10 or the IL-10 receptor (IL-10R). In some aspects, the agent is an antibody that neutralizes IL-10 activity, such as an antibody or antigen-binding fragment that binds to IL-10 or IL-10R. In some aspects, the IL-10 neutralizing antibody is any described in Dobber et al. Cell Immunol. 1995 Feb;160(2):185-92 or Hunter et al. J Immunol. 2005 Jun 1;174(11):7368-75. In some instances, the agent that is an antagonist or inhibitor of IL-10/IL-10R is a small molecule, a protein or peptide, or a nucleic acid.

In some instances, the agent is an antagonist or inhibitor of IL-1 or the IL-1 receptor (IL-1R). In some aspects, the agent is an IL-1 receptor antagonist, which is a modified form of IL-1R, such as anakinra (see, e.g., Fleischmann et al., (2006) Annals of the rheumatic diseases. 65(8): 1006-12). In some aspects, the agent is an antibody that neutralizes IL-1 activity, such as an antibody or antigen-binding fragment that binds to IL-1 or IL-1R, such as canakinumab (see also EP 2277543). In some instances, the agent that is an antagonist or inhibitor of IL-1/IL-1R is a small molecule, a protein or peptide, or a nucleic acid.

In some instances, the agent is an antagonist or inhibitor of a tumor necrosis factor (TNF) or a tumor necrosis factor receptor (TNFR). In some aspects, the agent is an antibody that blocks TNF activity, such as an antibody or antigen-binding fragment that binds to a TNF, such as TNFα, or its receptor (TNFR, e.g., TNFRp55 or TNFRp75). In some aspects, the agent is selected from among infliximab, adalimumab, certolizumab pegol, golimumab and etanercept. In some instances, the agent that is an antagonist or inhibitor of TNF/TNFR is a small molecule, a protein or peptide, or a nucleic acid.

In some instances, the agent is an antagonist or inhibitor of signaling through the Janus kinase (JAK) and two Signal Transducer and Activator of Transcription (STAT) signaling cascade. JAK/STAT proteins are common components of cytokine and cytokine receptor signaling. In some instances, the agent that is an antagonist or inhibitor of JAK/STAT, such as ruxolitinib (see, e.g., Mesa et al. (2012) Nature Reviews Drug Discovery. 11(2):103-104), tofacitinib (also known as Xeljanz, Jakvinus tasocitinib and CP-690550) , Baricitinib (also known as LY-3009104, INCB-28050), Filgotinib (G-146034, GLPG-0634), Gandotinib (LY-2784544), Lestaurtinib (CEP-701), Momelotinib (GS-0387, CYT-387), Pacritinib (SB1518), and Upadacitinib (ABT-494). In some instances, the agent is a small molecule, a protein or peptide, or a nucleic acid.

In some instances, the agent is a kinase inhibitor. Kinase inhibitors, such as a CDK4 kinase inhibitor, a BTK kinase inhibitor, a MNK kinase inhibitor, or a DGK kinase inhibitor, can regulate the constitutively active survival pathways that exist in tumor cells and/or modulate the function of immune cells. In some instances, the kinase inhibitor is a Bruton's tyrosine kinase (BTK) inhibitor, e.g., ibrutinib. In some instances, the kinase inhibitor is a phosphatidylinositol-4,5-bisphosphate 3-kinase (PI3K) inhibitor. In some instances, the kinase inhibitor is a CDK4 inhibitor, e.g., a CDK4/6 inhibitor. In some instances, the kinase inhibitor is an mTOR inhibitor, such as, e.g., rapamycin, a rapamycin analog, OSI-027. The mTOR inhibitor can be, e.g., an mTORC1 inhibitor and/or an mTORC2 inhibitor, e.g., an mTORC1 inhibitor and/or mTORC2 inhibitor. In some instances, the kinase inhibitor is an MNK inhibitor, or a dual PI3K/mTOR inhibitor. In some instances, other exemplary kinase inhibitors include the AKT inhibitor perifosine, the mTOR inhibitor temsirolimus, the Src kinase inhibitors dasatinib and fostamatinib, the JAK2 inhibitors pacritinib and ruxolitinib, the PKCβ inhibitors enzastaurin and bryostatin, and the AAK inhibitor alisertib.

In some instances, the kinase inhibitor is a BTK inhibitor selected from ibrutinib (PCI- 32765); GDC-0834; RN-486; CGI-560; CGI-1764; HM-71224; CC-292; ONO-4059; CNX-774; and LFM-A13. In some instances, the BTK inhibitor does not reduce or inhibit the kinase activity of interleukin-2-inducible kinase (ITK), and is selected from GDC-0834; RN-486; CGI-560; CGI-1764; HM-71224; CC-292; ONO-4059; CNX-774; and LFM-A13.

In some instances, the kinase inhibitor is a BTK inhibitor, e.g., ibrutinib (1-[(3R)-3-[4-Amino-3 -(4-phenoxyphenyl)-1H-pyrazolo[3,4-d]pyrimidin-1-yl]piperidin-1 -yl]prop-2-en-1-one; also known as PCI-32765). In some instances, the kinase inhibitor is a BTK inhibitor, e.g., ibrutinib (PCI-32765). In some instances, 1, 2, 3, 4, 5, 6, 7, 8, 9, 10, 11, 12 or more cycles of ibrutinib are administered. In some instances, the BTK inhibitor is a BTK inhibitor described in International Application WO 2015/079417.

In some instances, the kinase inhibitor is a PI3K inhibitor. PI3K is central to the PI3K/Akt/mTOR pathway involved in cell cycle regulation and lymphoma survival. Exemplary PI3K inhibitor includes idelalisib (PI3Kδ inhibitor). In some instances, the agent is idelalisib and rituximab.

In some instances, the agent is an inhibitor of mammalian target of rapamycin (mTOR). In some instances, the kinase inhibitor is an mTOR inhibitor selected from temsirolimus; ridaforolimus (also known as AP23573 and MK8669); everolimus (RAD001); rapamycin (AY22989); simapimod; AZD8055; PF04691502; SF1126; and XI,765. In some instances, the agent is an inhibitor of mitogen-activated protein kinase (MAPK), such as vemurafenib, dabrafenib, and trametinib.

In some instances, a device, such as absorbent resin technology with blood or plasma filtration, can be used to reduce cytokine levels. In some instances, the device used to reduce cytokine levels is a physical cytokine absorber, such as an extracorporeal cytokine absorber. In some instances, a physical cytokine absorber can be used to eliminate cytokines from the bloodstream in an *ex vivo,* extracorporeal manner. In some instances, the agent is a porous polymer. In some instances, the agent is CytoSorb (see, e.g., Basu et al. Indian J Crit Care Med. (2014) 18(12): 822-824).

### V. ENGINEERED CELLS

In some instances, the described methods are associated with the administration of a cell therapy, such as for the treatment of diseases or conditions including various tumors. In some instances, the T cell therapy for use in accord with the described methods includes administering engineered cells expressing recombinant receptors designed to recognize and/or specifically bind to molecules associated with the disease or condition and result in a response, such as an immune response against such molecules upon binding to such molecules. The receptors may include chimeric receptors, *e.g.,* chimeric antigen receptors (CARs), and other transgenic antigen receptors including transgenic T cell receptors (TCRs) or chimeric autoantibody receptors (CAARs).

In some instances, the cells contain or are engineered to contain an engineered receptor, *e.g.,* an engineered antigen receptor, such as a chimeric antigen receptor (CAR), or a T cell receptor (TCR). Also described are populations of such cells, compositions containing such cells and/or enriched for such cells, such as in which cells of a certain type such as T cells or CD8⁺ or CD4⁺ cells are enriched or selected. Among the compositions are pharmaceutical compositions and formulations for administration, such as for adoptive cell therapy. Also described are therapeutic methods for administering the cells and compositions to subjects, *e.g.,* patients.

Thus, in some instances, the cells include one or more nucleic acids introduced via genetic engineering, and thereby express recombinant or genetically engineered products of such nucleic acids. In some instances, gene transfer is accomplished by first stimulating the cells, such as by combining it with a stimulus that induces a response such as proliferation, survival, and/or activation, *e.g.,* as measured by expression of a cytokine or activation marker, followed by transduction of the activated cells, and expansion in culture to numbers sufficient for clinical applications.

### A. Recombinant Receptors

The cells generally express recombinant receptors, such as antigen receptors including functional non-TCR antigen receptors, e.g., chimeric antigen receptors (CARs), and other antigen-binding receptors such as transgenic T cell receptors (TCRs). Also among the receptors are other chimeric receptors, such as chimeric autoantibody receptors (CAARs).

### 1. Chimeric Antigen Receptors (CARs)

In some instances, the recombinant receptor includes a chimeric antigen receptor (CAR). In some instances, the CAR is specific for a particular antigen (or marker or ligand), such as an antigen expressed on the surface of a particular cell type. In some instances, the antigen is a polypeptide. In some instances, it is a carbohydrate or other molecule. In some instances, the antigen is selectively expressed or overexpressed on cells of the disease or condition, e.g., the tumor or pathogenic cells, as compared to normal or non-targeted cells or tissues. In other instances, the antigen is expressed on normal cells and/or is expressed on the engineered cells.

In particular instances, the recombinant receptor, such as a chimeric receptor, contains an intracellular signaling region, which includes a cytoplasmic signaling domain (also interchangeably called an intracellular signaling domain), such as a cytoplasmic (intracellular) region capable of inducing a primary activation signal in a T cell, for example, a cytoplasmic signaling domain of a T cell receptor (TCR) component (e.g. a cytoplasmic signaling domain of a zeta chain of a CD3-zeta (CD3ζ) chain or a functional variant or signaling portion thereof) and/or that comprises an immunoreceptor tyrosine-based activation motif (ITAM).

In some instances, the chimeric receptor further contains an extracellular binding domain that specifically binds to an antigen (or a ligand). In some instances, the chimeric receptor is a CAR that contains an extracellular antigen-recognition domain that specifically binds to an antigen. In some instances, the antigen (or a ligand), is a protein expressed on the surface of cells. In some instances, the CAR is a TCR-like CAR and the antigen is a processed peptide antigen, such as a peptide antigen of an intracellular protein, which, like a TCR, is recognized on the cell surface in the context of a major histocompatibility complex (MHC) molecule.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, , 8,339,645, 8,398,282, 7,446,179, 6,410,319, 7,070,995, 7,265,209, 7,354,762, 7,446,191, 8,324,353, and 8,479,118, and European patent application number EP2537416, and/or those described by Sadelain et al., Cancer Discov., 3(4): 388-398 (2013); Davila et al. PLoS ONE 8(4): e61338 (2013); Turtle et al., Curr. Opin. Immunol., 24(5): 633-39 (2012); Wu et al., Cancer, 18(2): 160-75 (2012). In some aspects, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, Kochenderfer et al., Nature Reviews Clinical Oncology, 10, 267-276 (2013); Wang et al., J. Immunother. 35(9): 689-701 (2012); and Brentjens et al., Sci Transl Med,. 5(177) (2013). See also WO2014031687, US 8,339,645, US 7,446,179, US 2013/0149337, U.S. Patent No.: 7,446,190, and US Patent No.: 8,389,282. The chimeric receptors, such as CARs, generally include an extracellular antigen binding domain, such as a portion of an antibody molecule, generally a variable heavy (VH) chain region and/or variable light (VL) chain region of the antibody, *e.g.,* an scFv antibody fragment.

In some instances, the antigen targeted by the receptor is a polypeptide. In some instances, it is a carbohydrate or other molecule. In some instances, the antigen is selectively expressed or overexpressed on cells of the disease or condition, e.g., the tumor or pathogenic cells, as compared to normal or non-targeted cells or tissues. In other instances, the antigen is expressed on normal cells and/or is expressed on the engineered cells.

In some instances, the CAR is constructed with a specificity for a particular antigen (or marker or ligand), such as an antigen expressed in a particular cell type to be targeted by adoptive therapy, *e.g.,* a cancer marker, and/or an antigen intended to induce a dampening response, such as an antigen expressed on a normal or non-diseased cell type. Thus, the CAR typically includes in its extracellular portion one or more antigen binding molecules, such as one or more antigen-binding fragment, domain, or portion, or one or more antibody variable domains, and/or antibody molecules. In some instances, the CAR includes an antigen-binding portion or portions of an antibody molecule, such as a single-chain antibody fragment (scFv) derived from the variable heavy (VH) and variable light (VL) chains of a monoclonal antibody (mAb).

In some instances, the antibody or antigen-binding portion thereof is expressed on cells as part of a recombinant receptor, such as an antigen receptor. Among the antigen receptors are functional non-TCR antigen receptors, such as chimeric antigen receptors (CARs). Generally, a CAR containing an antibody or antigen-binding fragment that exhibits TCR-like specificity directed against peptide-MHC complexes also may be referred to as a TCR-like CAR. In some instances, the extracellular antigen binding domain specific for an MHC-peptide complex of a TCR-like CAR is linked to one or more intracellular signaling components, in some aspects via linkers and/or transmembrane domain(s). In some instances, such molecules can typically mimic or approximate a signal through a natural antigen receptor, such as a TCR, and, optionally, a signal through such a receptor in combination with a costimulatory receptor.

In some instances, the recombinant receptor, such as a chimeric receptor (e.g. CAR), includes a ligand-binding domain that binds, such as specifically binds, to an antigen (or a ligand). Among the antigens targeted by the chimeric receptors are those expressed in the context of a disease, condition, or cell type to be targeted via the adoptive cell therapy. Among the diseases and conditions are proliferative, neoplastic, and malignant diseases and disorders, including cancers and tumors, including hematologic cancers, cancers of the immune system, such as lymphomas, leukemias, and/or myelomas, such as B, T, and myeloid leukemias, lymphomas, and multiple myelomas.

In some instances, the antigen (or a ligand) is a polypeptide. In some instances, it is a carbohydrate or other molecule. In some instances, the antigen (or a ligand) is selectively expressed or overexpressed on cells of the disease or condition, *e.g.,* the tumor or pathogenic cells, as compared to normal or non-targeted cells or tissues.

In some instances, the CAR contains an antibody or an antigen-binding fragment (*e.g.* scFv) that specifically recognizes an antigen, such as an intact antigen, expressed on the surface of a cell.

Antigens targeted by the receptors in some instances are or include αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD138, CD171, epidermal growth factor protein (EGFR), truncated epidermal growth factor protein (tEGFR), type III epidermal growth factor receptor mutation (EGFR vIII), epithelial glycoprotein 2 (EPG-2), epithelial glycoprotein 40 (EPG-40), ephrinB2, ephrine receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), G Protein Coupled Receptor 5D (GPCR5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(IL-22Ra), IL-13 receptor alpha 2 (IL-13Ra2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, mesothelin, c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some instances include antigens associated with a B cell malignancy, such as any of a number of known B cell marker. In some instances, the antigen is or includes CD20, CD19, CD22, ROR1, CD45, CD21, CD5, CD33, Igkappa, Iglambda, CD79a, CD79b or CD30.

Antigens targeted by the receptors in some instances are or include orphan tyrosine kinase receptor ROR1, tEGFR, Her2, L1-CAM, CD19, CD20, CD22, mesothelin, CEA, and hepatitis B surface antigen, anti-folate receptor, CD23, CD24, CD30, CD33, CD38, CD44, EGFR, EGP-2, EGP-4, EPHa2, ErbB2, 3, or 4, FBP, fetal acethycholine e receptor, GD2, GD3, HMW-MAA, IL-22R-alpha, IL-13R-alpha2, kdr, kappa light chain, Lewis Y, L1-cell adhesion molecule, MAGE-A1, mesothelin, MUC1, MUC16, PSCA, NKG2D Ligands, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, c-Met, GD-2, and MAGE A3, CE7, Wilms Tumor 1 (WT-1), a cyclin, such as cyclin A1 (CCNA1), and/or biotinylated molecules, and/or molecules expressed by HIV, HCV, HBV or other pathogens. In some instances, the CAR binds a pathogen-specific or pathogen-expressed antigen. In some instances, the CAR is specific for viral antigens (such as HIV, HCV, HBV, etc.), bacterial antigens, and/or parasitic antigens.

In some instances, the CAR contains a TCR-like antibody, such as an antibody or an antigen-binding fragment (*e.g.* scFv) that specifically recognizes an intracellular antigen, such as a tumor-associated antigen, presented on the cell surface as a MHC-peptide complex. In some instances, an antibody or antigen-binding portion thereof that recognizes an MHC-peptide complex can be expressed on cells as part of a recombinant receptor, such as an antigen receptor. Among the antigen receptors are functional non-TCR antigen receptors, such as chimeric antigen receptors (CARs). Generally, a CAR containing an antibody or antigen-binding fragment that exhibits TCR-like specificity directed against peptide-MHC complexes also may be referred to as a TCR-like CAR.

Reference to "Major histocompatibility complex" (MHC) refers to a protein, generally a glycoprotein, that contains a polymorphic peptide binding site or binding groove that can, in some cases, complex with peptide antigens of polypeptides, including peptide antigens processed by the cell machinery. In some cases, MHC molecules can be displayed or expressed on the cell surface, including as a complex with peptide, *i.e.* MHC-peptide complex, for presentation of an antigen in a conformation recognizable by an antigen receptor on T cells, such as a TCRs or TCR-like antibody. Generally, MHC class I molecules are heterodimers having a membrane spanning α chain, in some cases with three α domains, and a non-covalently associated β2 microglobulin. Generally, MHC class II molecules are composed of two transmembrane glycoproteins, α and β, both of which typically span the membrane. An MHC molecule can include an effective portion of an MHC that contains an antigen binding site or sites for binding a peptide and the sequences necessary for recognition by the appropriate antigen receptor. In some instances, MHC class I molecules deliver peptides originating in the cytosol to the cell surface, where a MHC-peptide complex is recognized by T cells, such as generally CD8⁺ T cells, but in some cases CD4+ T cells. In some instances, MHC class II molecules deliver peptides originating in the vesicular system to the cell surface, where they are typically recognized by CD4⁺ T cells. Generally, MHC molecules are encoded by a group of linked loci, which are collectively termed H-2 in the mouse and human leukocyte antigen (HLA) in humans. Hence, typically human MHC can also be referred to as human leukocyte antigen (HLA).

The term "MHC-peptide complex" or "peptide-MHC complex" or variations thereof, refers to a complex or association of a peptide antigen and an MHC molecule, such as, generally, by non-covalent interactions of the peptide in the binding groove or cleft of the MHC molecule. In some instances, the MHC-peptide complex is present or displayed on the surface of cells. In some instances, the MHC-peptide complex can be specifically recognized by an antigen receptor, such as a TCR, TCR-like CAR or antigen-binding portions thereof.

In some instances, a peptide, such as a peptide antigen or epitope, of a polypeptide can associate with an MHC molecule, such as for recognition by an antigen receptor. Generally, the peptide is derived from or based on a fragment of a longer biological molecule, such as a polypeptide or protein. In some instances, the peptide typically is about 8 to about 24 amino acids in length. In some instances, a peptide has a length of from or from about 9 to 22 amino acids for recognition in the MHC Class II complex. In some instances, a peptide has a length of from or from about 8 to 13 amino acids for recognition in the MHC Class I complex. In some instances, upon recognition of the peptide in the context of an MHC molecule, such as MHC-peptide complex, the antigen receptor, such as TCR or TCR-like CAR, produces or triggers an activation signal to the T cell that induces a T cell response, such as T cell proliferation, cytokine production, a cytotoxic T cell response or other response.

In some instances, a TCR-like antibody or antigen-binding portion, are known or can be produced by known methods (see e.g. US Published Application Nos. US 2002/0150914; US 2003/0223994; US 2004/0191260; US 2006/0034850; US 2007/00992530; US20090226474; US20090304679; and International PCT Publication No. WO 03/068201).

In some instances, an antibody or antigen-binding portion thereof that specifically binds to a MHC-peptide complex, can be produced by immunizing a host with an effective amount of an immunogen containing a specific MHC-peptide complex. In some cases, the peptide of the MHC-peptide complex is an epitope of antigen capable of binding to the MHC, such as a tumor antigen, for example a universal tumor antigen, myeloma antigen or other antigen as described below. In some instances, an effective amount of the immunogen is then administered to a host for eliciting an immune response, wherein the immunogen retains a three-dimensional form thereof for a period of time sufficient to elicit an immune response against the three-dimensional presentation of the peptide in the binding groove of the MHC molecule. Serum collected from the host is then assayed to determine if desired antibodies that recognize a three-dimensional presentation of the peptide in the binding groove of the MHC molecule is being produced. In some instances, the produced antibodies can be assessed to confirm that the antibody can differentiate the MHC-peptide complex from the MHC molecule alone, the peptide of interest alone, and a complex of MHC and irrelevant peptide. The desired antibodies can then be isolated.

In some instances, an antibody or antigen-binding portion thereof that specifically binds to an MHC-peptide complex can be produced by employing antibody library display methods, such as phage antibody libraries. In some instances, phage display libraries of mutant Fab, scFv or other antibody forms can be generated, for example, in which members of the library are mutated at one or more residues of a CDR or CDRs. See *e.g.* US published application No. US20020150914, US2014/0294841; and Cohen CJ. et al. (2003) J Mol. Recogn. 16:324-332.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, variable heavy chain (V_{H}) regions capable of specifically binding the antigen, single chain antibody fragments, including single chain variable fragments (scFv), and single domain antibodies (*e.g.,* sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, *e.g.,* bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD.

In some instances, the antigen-binding proteins, antibodies and antigen binding fragments thereof specifically recognize an antigen of a full-length antibody. In some instances, the heavy and light chains of an antibody can be full-length or can be an antigen-binding portion (a Fab, F(ab')2, Fv or a single chain Fv fragment (scFv)). In other instances, the antibody heavy chain constant region is chosen from, *e.g.,* IgG1, IgG2, IgG3, IgG4, IgM, IgA1, IgA2, IgD, and IgE, particularly chosen from, *e.g.,* IgG1, IgG2, IgG3, and IgG4, more particularly, IgG1 (*e.g.,* human IgG1). In another instance, the antibody light chain constant region is chosen from, *e.g.,* kappa or lambda, particularly kappa.

Among the described antibodies are antibody fragments. An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; variable heavy chain (V_{H}) regions, single-chain antibody molecules such as scFvs and single-domain V_{H} single antibodies; and multispecific antibodies formed from antibody fragments. In particular instances, the antibodies are single-chain antibody fragments comprising a variable heavy chain region and/or a variable light chain region, such as scFvs.

The term "variable region" or "variable domain" refers to the domain of an antibody heavy or light chain that is involved in binding the antibody to antigen. The variable domains of the heavy chain and light chain (V_{H} and V_{L}, respectively) of a native antibody generally have similar structures, with each domain comprising four conserved framework regions (FRs) and three CDRs. (See, *e.g.*, Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, *e.g.,* Portolano et al., J. Immunol. 150:880-887 (1993); Clarkson et al., Nature 352:624-628 (1991).

Single-domain antibodies are antibody fragments comprising all or a portion of the heavy chain variable domain or all or a portion of the light chain variable domain of an antibody. In certain instances, a single-domain antibody is a human single-domain antibody. In some instances, the CAR comprises an antibody heavy chain domain that specifically binds the antigen, such as a cancer marker or cell surface antigen of a cell or disease to be targeted, such as a tumor cell or a cancer cell, such as any of the target antigens described herein or known.

Antibody fragments can be made by various techniques, including but not limited to proteolytic digestion of an intact antibody as well as production by recombinant host cells. In some instances, the antibodies are recombinantly-produced fragments, such as fragments comprising arrangements that do not occur naturally, such as those with two or more antibody regions or chains joined by synthetic linkers, *e.g.,* peptide linkers, and/or that are may not be produced by enzyme digestion of a naturally-occurring intact antibody. In some instances, the antibody fragments are scFvs.

A "humanized" antibody is an antibody in which all or substantially all CDR amino acid residues are derived from non-human CDRs and all or substantially all FR amino acid residues are derived from human FRs. A humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some instances, some FR residues in a humanized antibody are substituted with corresponding residues from a non-human antibody (*e.g.,* the antibody from which the CDR residues are derived), *e.g.,* to restore or improve antibody specificity or affinity.

Thus, in some instances, the chimeric antigen receptor, including TCR-like CARs, includes an extracellular portion containing an antibody or antibody fragment. In some instances, the antibody or fragment includes an scFv. In some aspects, the chimeric antigen receptor includes an extracellular portion containing the antibody or fragment and an intracellular signaling region. In some instances, the intracellular signaling region comprises an intracellular signaling domain. In some instances, the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM).

In some instances, the recombinant receptor such as the CAR, including the antibody portion of the recombinant receptor, e.g., CAR, further includes at least a portion of an immunoglobulin constant region, such as a hinge region, e.g., an IgG4 hinge region, and/or a C_{H}1/C_{L} and/or Fc region. In some instances, the recombinant receptor such as the CAR, including the antibody portion thereof, further includes a spacer, which may be or include at least a portion of an immunoglobulin constant region or variant or modified version thereof, such as a hinge region, e.g., an IgG4 hinge region, and/or a C_{H}1/C_{L} and/or Fc region. In some instances, the recombinant receptor further comprises a spacer and/or a hinge region. In some instances, the constant region or portion is of a human IgG, such as IgG4 or IgG1. In some aspects, the portion of the constant region serves as a spacer region between the antigen-recognition component, e.g., scFv, and transmembrane domain. The spacer can be of a length that provides for increased responsiveness of the cell following antigen binding, as compared to in the absence of the spacer. Exemplary spacers, e.g., hinge regions, include those described in international patent application publication number WO2014031687. In some examples, the spacer is or is about 12 amino acids in length or is no more than 12 amino acids in length. Exemplary spacers include those having at least about 10 to 229 amino acids, about 10 to 200 amino acids, about 10 to 175 amino acids, about 10 to 150 amino acids, about 10 to 125 amino acids, about 10 to 100 amino acids, about 10 to 75 amino acids, about 10 to 50 amino acids, about 10 to 40 amino acids, about 10 to 30 amino acids, about 10 to 20 amino acids, or about 10 to 15 amino acids, and including any integer between the endpoints of any of the listed ranges. In some instances, a spacer region has about 12 amino acids or less, about 119 amino acids or less, or about 229 amino acids or less. Exemplary spacers include IgG4 hinge alone, IgG4 hinge linked to C_{H}2 and C_{H}3 domains, or IgG4 hinge linked to the C_{H}3 domain. Exemplary spacers include, but are not limited to, those described in Hudecek et al. Clin. Cancer Res., 19:3153 (2013), international patent application publication number WO2014031687, U.S. Patent No. 8,822,647 or published app. No. US2014/0271635.

In some instances, the constant region or portion is of a human IgG, such as IgG4 or IgG1. In some instances, the spacer has the sequence ESKYGPPCPPCP (set forth in SEQ ID NO: 1), and is encoded by the sequence set forth in SEQ ID NO: 2. In some instances, the spacer has the sequence set forth in SEQ ID NO: 3. In some instances, the spacer has the sequence set forth in SEQ ID NO: 4. In some instances, the constant region or portion is of IgD. In some instances, the spacer has the sequence set forth in SEQ ID NO: 5. In some instances, the spacer has a sequence of amino acids that exhibits at least or at least about 85%, 86%, 87%, 88%, 89%, 90%, 91%, 92%, 93%, 94%, 95%, 96%, 97%, 98%, 99% or more sequence identity to any of SEQ ID NOS: 1, 3, 4 and 5. In some instances, the spacer has the sequence set forth in SEQ ID NOS: 26-34. In some instances, the spacer has a sequence of amino acids that exhibits at least or at least about 85%, 86%, 87%, 88%, 89%, 90%, 91%, 92%, 93%, 94%, 95%, 96%, 97%, 98%, 99% or more sequence identity to any of SEQ ID NOS: 26-34.

The antigen recognition domain generally is linked to one or more intracellular signaling components, such as signaling components that mimic activation through an antigen receptor complex, such as a TCR complex, in the case of a CAR, and/or signal via another cell surface receptor. Thus, in some instances, the antigen-binding component (e.g., antibody) is linked to one or more transmembrane and intracellular signaling domains or regions. In some instances, the transmembrane domain is fused to the extracellular domain. In one instance, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some instances is derived either from a natural or from a synthetic source. Where the source is natural, the domain in some aspects is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154. Alternatively the transmembrane domain in some instances is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain. In some instances, the linkage is by linkers, spacers, and/or transmembrane domain(s).

Among the intracellular signaling domains or regions are those that mimic or approximate a signal through a natural antigen receptor, a signal through such a receptor in combination with a costimulatory receptor, and/or a signal through a costimulatory receptor alone. In some instances, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain or region of the CAR.

The receptor, e.g., the CAR, generally includes at least one intracellular signaling component or components. In some instances, the receptor includes an intracellular component of a TCR complex, such as a TCR CD3 chain that mediates T-cell activation and cytotoxicity, e.g., CD3 zeta chain. Thus, in some aspects, the antigen-binding portion is linked to one or more cell signaling modules. In some instances, cell signaling modules include CD3 transmembrane domain, CD3 intracellular signaling domains, and/or other CD transmembrane domains. In some instances, the receptor, e.g., CAR, further includes a portion of one or more additional molecules such as Fc receptor γ, CD8, CD4, CD25, or CD16. For example, in some aspects, the CAR or other chimeric receptor includes a chimeric molecule between CD3-zeta (CD3-ζ) or Fc receptor γ and CD8, CD4, CD25 or CD16.

In some instances, upon ligation of the CAR or other chimeric receptor, the cytoplasmic domain or intracellular signaling domains or regions of the receptor activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some instances, a truncated portion of an intracellular signaling domain or region of an antigen receptor component or costimulatory molecule is used in place of an intact immunostimulatory chain, for example, if it transduces the effector function signal. In some instances, the intracellular signaling domain or domains or regions include the cytoplasmic sequences of the T cell receptor (TCR), and in some aspects also those of co-receptors that in the natural context act in concert with such receptors to initiate signal transduction following antigen receptor engagement, and/or any derivative or variant of such molecules, and/or any synthetic sequence that has the same functional capability.

In the context of a natural TCR, full activation generally requires not only signaling through the TCR, but also a costimulatory signal. Thus, in some instances, to promote full activation, a component for generating secondary or co-stimulatory signal is also included in the CAR. In other instances, the CAR does not include a component for generating a costimulatory signal. In some aspects, an additional CAR is expressed in the same cell and provides the component for generating the secondary or costimulatory signal.

T cell activation is in some aspects described as being mediated by two classes of cytoplasmic signaling sequences: those that initiate antigen-dependent primary activation through the TCR (primary cytoplasmic signaling sequences), and those that act in an antigen-independent manner to provide a secondary or co-stimulatory signal (secondary cytoplasmic signaling sequences). In some aspects, the CAR includes one or both of such signaling components.

In some aspects, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from TCR zeta, FcR gamma, FcR beta, CD3 gamma, CD3 delta, CD3 epsilon, CD8, CD22, CD79a, CD79b, and CD66d. In some instances, cytoplasmic signaling molecule(s) in the CAR contain(s) a cytoplasmic signaling domain or region, portion thereof, or sequence derived from CD3 zeta.

In some instances, the CAR includes a signaling domain or region and/or transmembrane portion of a costimulatory receptor, such as CD28, 4-1BB, OX40, DAP10, and ICOS. In some aspects, the same CAR includes both the activating and costimulatory components.

In some instances, the activating domain is included within one CAR, whereas the costimulatory component is provided by another CAR recognizing another antigen. In some instances, the CARs include activating or stimulatory CARs, costimulatory CARs, both expressed on the same cell (see WO2014/055668). In some aspects, the cells include one or more stimulatory or activating CAR and/or a costimulatory CAR. In some instances, the cells further include inhibitory CARs (iCARs, see Fedorov et al., Sci. Transl. Medicine, 5(215) (2013), such as a CAR recognizing an antigen other than the one associated with and/or specific for the disease or condition whereby an activating signal delivered through the disease-targeting CAR is diminished or inhibited by binding of the inhibitory CAR to its ligand, e.g., to reduce off-target effects.

In certain instances, the intracellular signaling domain comprises a CD28 transmembrane and signaling domain linked to a CD3 (e.g., CD3-zeta) intracellular domain. In some instances, the intracellular signaling domain comprises a chimeric CD28 and CD137 (4-1BB, TNFRSF9) co-stimulatory domains, linked to a CD3 zeta intracellular domain.

In some instances, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some instances, the CAR or other antigen receptor further includes a marker, such as a cell surface marker, which may be used to confirm transduction or engineering of the cell to express the receptor, such as a truncated version of a cell surface receptor, such as truncated EGFR (tEGFR). In some aspects, the marker includes all or part (e.g., truncated form) of CD34, a NGFR, or epidermal growth factor receptor (e.g., tEGFR). In some instances, the nucleic acid encoding the marker is operably linked to a polynucleotide encoding for a linker sequence, such as a cleavable linker sequence, e.g., T2A. For example, a marker, and optionally a linker sequence, can be any as disclosed in published patent application No. WO2014031687. For example, the marker can be a truncated EGFR (tEGFR) that is, optionally, linked to a linker sequence, such as a T2A cleavable linker sequence. An exemplary polypeptide for a truncated EGFR (e.g. tEGFR) comprises the sequence of amino acids set forth in SEQ ID NO: 7 or 16 or a sequence of amino acids that exhibits at least or at least about 85%, 86%, 87%, 88%, 89%, 90%, 91%, 92%, 93%, 94%, 95%, 96%, 97%, 98%, 99% or more sequence identity to SEQ ID NO: 7 or 16. An exemplary T2A linker sequence comprises the sequence of amino acids set forth in SEQ ID NO: 6 or 17 or a sequence of amino acids that exhibits at least or at least about 85%, 86%, 87%, 88%, 89%, 90%, 91%, 92%, 93%, 94%, 95%, 96%, 97%, 98%, 99% or more sequence identity to SEQ ID NO: 6 or 17.

In some instances, the marker is a molecule, e.g., cell surface protein, not naturally found on T cells or not naturally found on the surface of T cells, or a portion thereof. In some instances, the molecule is a non-self molecule, e.g., non-self protein, i.e., one that is not recognized as "self' by the immune system of the host into which the cells will be adoptively transferred.

In some instances, the marker serves no therapeutic function and/or produces no effect other than to be used as a marker for genetic engineering, e.g., for selecting cells successfully engineered. In other instances, the marker may be a therapeutic molecule or molecule otherwise exerting some desired effect, such as a ligand for a cell to be encountered in vivo, such as a costimulatory or immune checkpoint molecule to enhance and/or dampen responses of the cells upon adoptive transfer and encounter with ligand.

In some cases, CARs are referred to as first, second, and/or third generation CARs. In some aspects, a first generation CAR is one that solely provides a CD3-chain induced signal upon antigen binding; in some aspects, a second-generation CARs is one that provides such a signal and costimulatory signal, such as one including an intracellular signaling domain from a costimulatory receptor such as CD28 or CD137; in some aspects, a third generation CAR is one that includes multiple costimulatory domains of different costimulatory receptors.

In some instances, the chimeric antigen receptor includes an extracellular portion containing an antibody or antibody fragment. In some aspects, the chimeric antigen receptor includes an extracellular portion containing the antibody or fragment and an intracellular signaling domain. In some instances, the antibody or fragment includes an scFv and the intracellular domain contains an ITAM. In some aspects, the intracellular signaling domain includes a signaling domain of a zeta chain of a CD3-zeta (CD3ζ) chain. In some instances, the chimeric antigen receptor includes a transmembrane domain linking the extracellular domain and the intracellular signaling domain. In some aspects, the transmembrane domain contains a transmembrane portion of CD28. In some instances, the chimeric antigen receptor contains an intracellular domain of a T cell costimulatory molecule. The extracellular domain and transmembrane domain can be linked directly or indirectly. In some instances, the extracellular domain and transmembrane are linked by a spacer, such as any described herein. In some instances, the receptor contains extracellular portion of the molecule from which the transmembrane domain is derived, such as a CD28 extracellular portion. In some instances, the chimeric antigen receptor contains an intracellular domain derived from a T cell costimulatory molecule or a functional variant thereof, such as between the transmembrane domain and intracellular signaling domain. In some aspects, the T cell costimulatory molecule is CD28 or 4-1BB.

In some instances the scFv is derived from FMC63. FMC63 is a mouse monoclonal IgG1 antibody raised against Nalm-1 and -16 cells expressing CD19 of human origin (Ling, N. R., et al. (1987). Leucocyte typing III. 302). The FMC63 antibody comprises CDRH1and H2 set forth in SEQ ID NOS: 38 and 39 respectively, and CDRH3 set forth in SEQ ID NOS: 40 or 54 and CDRL1 set forth in SEQ ID NOS: 35 and CDR L2 set forth in SEQ ID NOS: 36 or 55 and CDR L3 set forth in SEQ ID NOS: 37 or 56. The FMC63 antibody comprises the heavy chain variable region (V_{H}) comprising the amino acid sequence of SEQ ID NO: 41 and the light chain variable region (V_{L}) comprising the amino acid sequence of SEQ ID NO: 42. In some instances, the svFv comprises a variable light chain containing the CDRL1 set forth in SEQ ID NO: 35, a CDRL2 set forth in SEQ ID NO: 36 or 55, and a CDRL3 set forth in SEQ ID NO: 37 or 56 and/or a variable heavy chain containing a CDRH1 set forth in SEQ ID NO:38, a CDRH2 set forth in SEQ ID NO:39, and a CDRH3 set forth in SEQ ID NO:40 or 54. In some instances, the scFv comprises a variable heavy chain region of FMC63 set forth in SEQ ID NO:41 and a variable light chain region of FMC63 set forth in SEQ ID NO: 42. In some instances, the variable heavy and variable light chain are connected by a linker. In some instances, the linker is set forth in SEQ ID NO:24. In some instances, the scFv comprises, in order, a VH, a linker, and a VL. In some instances, the scFv comprises, in order, a VL, a linker, and a VH. In some instances, the svFc is encoded by a sequence of nucleotides set forth in SEQ ID NO:25 or a sequence that exhibits at least or at least about 85%, 86%, 87%, 88%, 89%, 90%, 91%, 92%, 93%, 94%, 95%, 96%, 97%, 98%, or 99% sequence identity to SEQ ID NO:25. In some instances, the scFv comprises the sequence of amino acids set forth in SEQ ID NO:43 or a sequence that exhibits at least or at least about 85%, 86%, 87%, 88%, 89%, 90%, 91%, 92%, 93%, 94%, 95%, 96%, 97%, 98%, or 99% sequence identity to SEQ ID NO:43.

In some instances the scFv is derived from SJ25C1. SJ25C1 is a mouse monoclonal IgG1 antibody raised against Nalm-1 and -16 cells expressing CD19 of human origin (Ling, N. R., et al. (1987). Leucocyte typing III. 302). The SJ25C1 antibody comprises CDRH1, H2 and H3 set forth in SEQ ID NOS: 47-49, respectively, and CDRL1, L2 and L3 sequences set forth in SEQ ID NOS: 44-46, respectively. The SJ25C1 antibody comprises the heavy chain variable region (V_{H}) comprising the amino acid sequence of SEQ ID NO: 50 and the light chain variable region (V_{L}) comprising the amino acid sequence of SEQ ID NO: 51. In some instances, the svFv comprises a variable light chain containing the CDRL1 set forth in SEQ ID NO: 44, a CDRL2 set forth in SEQ ID NO: 45, and a CDRL3 set forth in SEQ ID NO:46 and/or a variable heavy chain containing a CDRH1 set forth in SEQ ID NO: 47, a CDRH2 set forth in SEQ ID NO: 48, and a CDRH3 set forth in SEQ ID NO:49. In some instances, the scFv comprises a variable heavy chain region of SJ25C1 set forth in SEQ ID NO:50 and a variable light chain region of SJ25C1 set forth in SEQ ID NO: 51. In some instances, the variable heavy and variable light chain are connected by a linker. In some instances, the linker is set forth in SEQ ID NO:52. In some instances, the scFv comprises, in order, a VH, a linker, and a VL. In some instances, the scFv comprises, in order, a VL, a linker, and a VH. In some instances, the scFv comprises the sequence of amino acids set forth in SEQ ID NO:53 or a sequence that exhibits at least or at least about 85%, 86%, 87%, 88%, 89%, 90%, 91%, 92%, 93%, 94%, 95%, 96%, 97%, 98%, or 99% sequence identity to SEQ ID NO:53.

For example, in some instances, the CAR contains an antibody, e.g., an antibody fragment, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some instances, the CAR contains an antibody, e.g., antibody fragment, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such instances, the receptor further includes a spacer containing a portion of an Ig molecule, such as a human Ig molecule, such as an Ig hinge, e.g. an IgG4 hinge, such as a hinge-only spacer.

In some instances, the transmembrane domain of the recombinant receptor, e.g., the CAR, is or includes a transmembrane domain of human CD28(e.g. Accession No. P01747.1) or variant thereof, such as a transmembrane domain that comprises the sequence of amino acids set forth in SEQ ID NO: 8 or a sequence of amino acids that exhibits at least or at least about 85%, 86%, 87%, 88%, 89%, 90%, 91%, 92%, 93%, 94%, 95%, 96%, 97%, 98%, 99% or more sequence identity to SEQ ID NO: 8; in some instances, the transmembrane-domain containing portion of the recombinant receptor comprises the sequence of amino acids set forth in SEQ ID NO: 9 or a sequence of amino acids having at least at or about 85%, 86%, 87%, 88%, 89%, 90%, 91%, 92%, 93%, 94%, 95%, 96%, 97%, 98%, 99% or more sequence identity thereto, or such as a 27-amino acid transmembrane domain of a human CD28.

In some instances, the chimeric antigen receptor contains an intracellular domain of a T cell costimulatory molecule. In some aspects, the T cell costimulatory molecule is CD28 or 4-1BB.

In some instances, the intracellular signaling domain, region or component(s) of the recombinant receptor, e.g. the CAR, contains an intracellular costimulatory signaling domain of human CD28 or a functional variant or portion thereof, such as a domain with an LL to GG substitution at positions 186-187 of a native CD28 protein. For example, in some instances, the intracellular signaling domain or region can comprise the sequence of amino acids set forth in SEQ ID NO: 10 or 11 or a sequence of amino acids that exhibits at least or at least about 85%, 86%, 87%, 88%, 89%, 90%, 91%, 92%, 93%, 94%, 95%, 96%, 97%, 98%, 99% or more sequence identity to SEQ ID NO: 10 or 11. In some instances, the intracellular domain or region comprises an intracellular costimulatory signaling domain or region of 4-1BB (e.g., Accession No. Q07011.1) or functional variant or portion thereof, such as the sequence of amino acids set forth in SEQ ID NO: 12 or a sequence of amino acids that exhibits at least or at least about 85%, 86%, 87%, 88%, 89%, 90%, 91%, 92%, 93%, 94%, 95%, 96%, 97%, 98%, 99% or more sequence identity to SEQ ID NO: 12 or such as a 42-amino acid cytoplasmic domain of a human 4-1BB.

In some instances, the intracellular signaling domain or region of the recombinant receptor, e.g. the CAR, comprises a human CD3 chain, optionally a zeta stimulatory signaling domain or region or functional variant thereof, such as an 112 AA cytoplasmic domain or region of isoform 3 of human CD3ζ (Accession No.: P20963.2) or a CD3 zeta signaling domain or region as described in U.S. Patent No.: 7,446,190 or U.S. Patent No. 8,911,993. For example, in some instances, the intracellular signaling domain or region comprises the sequence of amino acids as set forth in SEQ ID NO: 13, 14 or 15 or a sequence of amino acids that exhibits at least or at least about 85%, 86%, 87%, 88%, 89%, 90%, 91%, 92%, 93%, 94%, 95%, 96%, 97%, 98%, 99% or more sequence identity to SEQ ID NO: 13, 14 or 15.

In some aspects, the spacer contains only a hinge region of an IgG, such as only a hinge of IgG4 or IgG1, such as the hinge only spacer set forth in SEQ ID NO: 1. In other instances, the spacer is or contains an Ig hinge, e.g., an IgG4-derived hinge, optionally linked to a C_{H}2 and/or C_{H}3 domains. In some instances, the spacer is an Ig hinge, e.g., an IgG4 hinge, linked to C_{H}2 and C_{H}3 domains, such as set forth in SEQ ID NO: 4. In some instances, the spacer is an Ig hinge, e.g., an IgG4 hinge, linked to a C_{H}3 domain only, such as set forth in SEQ ID NO: 3. In some instances, the spacer is or comprises a glycine-serine rich sequence or other flexible linker such as known flexible linkers.

For example, in some instances, the CAR includes an antibody such as an antibody fragment, including scFvs, a spacer, such as a spacer containing a portion of an immunoglobulin molecule, such as a hinge region and/or one or more constant regions of a heavy chain molecule, such as an Ig-hinge containing spacer, a transmembrane domain containing all or a portion of a CD28-derived transmembrane domain, a CD28-derived intracellular signaling domain, and a CD3 zeta signaling domain. In some instances, the CAR includes an antibody or fragment, such as scFv, a spacer such as any of the Ig-hinge containing spacers, a CD28-derived transmembrane domain, a 4-1BB-derived intracellular signaling domain, and a CD3 zeta-derived signaling domain.

In some instances, nucleic acid molecules encoding such CAR constructs further includes a sequence encoding a T2A ribosomal skip element and/or a tEGFR sequence, e.g., downstream of the sequence encoding the CAR. In some instances, the sequence encodes a T2A ribosomal skip element set forth in SEQ ID NO: 6 or 17, or a sequence of amino acids that exhibits at least or at least about 85%, 86%, 87%, 88%, 89%, 90%, 91%, 92%, 93%, 94%, 95%, 96%, 97%, 98%, 99% or more sequence identity to SEQ ID NO: 6 or 17. In some instances, T cells expressing an antigen receptor (e.g. CAR) can also be generated to express a truncated EGFR (EGFRt) as a non-immunogenic selection epitope (e.g. by introduction of a construct encoding the CAR and EGFRt separated by a T2A ribosome switch to express two proteins from the same construct), which then can be used as a marker to detect such cells (see e.g. U.S. Patent No. 8,802,374). In some instances, the sequence encodes an tEGFR sequence set forth in SEQ ID NO: 7 or 16, or a sequence of amino acids that exhibits at least or at least about 85%, 86%, 87%, 88%, 89%, 90%, 91%, 92%, 93%, 94%, 95%, 96%, 97%, 98%, 99% or more sequence identity to SEQ ID NO: 7 or 16.

The recombinant receptors, such as CARs, expressed by the cells administered to the subject generally recognize or specifically bind to a molecule that is expressed in, associated with, and/or specific for the disease or condition or cells thereof being treated. Upon specific binding to the molecule, e.g., antigen, the receptor generally delivers an immunostimulatory signal, such as an ITAM-transduced signal, into the cell, thereby promoting an immune response targeted to the disease or condition. For example, in some instances, the cells express a CAR that specifically binds to an antigen expressed by a cell or tissue of the disease or condition or associated with the disease or condition.

### 2. T Cell receptors (TCRs)

In some instances, engineered cells, such as T cells, are described that express a T cell receptor (TCR) or antigen-binding portion thereof that recognizes an peptide epitope or T cell epitope of a target polypeptide, such as an antigen of a tumor, viral or autoimmune protein.

In some instances, a "T cell receptor" or "TCR" is a molecule that contains a variable α and β chains (also known as TCRα and TCRβ, respectively) or a variable γ and δ chains (also known as TCRα and TCRβ, respectively), or antigen-binding portions thereof, and which is capable of specifically binding to a peptide bound to an MHC molecule. In some instances, the TCR is in the αβ form. Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules.

Unless otherwise stated, the term "TCR" should be understood to encompass full TCRs as well as antigen-binding portions or antigen-binding fragments thereof. In some instances, the TCR is an intact or full-length TCR, including TCRs in the αβ form or γδ form. In some instances, the TCR is an antigen-binding portion that is less than a full-length TCR but that binds to a specific peptide bound in an MHC molecule, such as binds to an MHC-peptide complex. In some cases, an antigen-binding portion or fragment of a TCR can contain only a portion of the structural domains of a full-length or intact TCR, but yet is able to bind the peptide epitope, such as MHC-peptide complex, to which the full TCR binds. In some cases, an antigen-binding portion contains the variable domains of a TCR, such as variable α chain and variable β chain of a TCR, sufficient to form a binding site for binding to a specific MHC-peptide complex. Generally, the variable chains of a TCR contain complementarity determining regions involved in recognition of the peptide, MHC and/or MHC-peptide complex.

In some instances, the variable domains of the TCR contain hypervariable loops, or complementarity determining regions (CDRs), which generally are the primary contributors to antigen recognition and binding capabilities and specificity. In some instances, a CDR of a TCR or combination thereof forms all or substantially all of the antigen-binding site of a given TCR molecule. The various CDRs within a variable region of a TCR chain generally are separated by framework regions (FRs), which generally display less variability among TCR molecules as compared to the CDRs (see, e.g., Jores et al., Proc. Nat'1 Acad. Sci. U.S.A. 87:9138, 1990; Chothia et al., EMBO J. 7:3745, 1988; see also Lefranc et al., Dev. Comp. Immunol. 27:55, 2003). In some instances, CDR3 is the main CDR responsible for antigen binding or specificity, or is the most important among the three CDRs on a given TCR variable region for antigen recognition, and/or for interaction with the processed peptide portion of the peptide-MHC complex. In some contexts, the CDR1 of the alpha chain can interact with the N-terminal part of certain antigenic peptides. In some contexts, CDR1 of the beta chain can interact with the C-terminal part of the peptide. In some contexts, CDR2 contributes most strongly to or is the primary CDR responsible for the interaction with or recognition of the MHC portion of the MHC-peptide complex. In some instances, the variable region of the β-chain can contain a further hypervariable region (CDR4 or HVR4), which generally is involved in superantigen binding and not antigen recognition (Kotb (1995) Clinical Microbiology Reviews, 8:411-426).

In some instances, a TCR also can contain a constant domain, a transmembrane domain and/or a short cytoplasmic tail (see, e.g., Janeway et al., Immunobiology: The Immune System in Health and Disease, 3rd Ed., Current Biology Publications, p. 4:33, 1997). In some aspects, each chain of the TCR can possess one N-terminal immunoglobulin variable domain, one immunoglobulin constant domain, a transmembrane region, and a short cytoplasmic tail at the C-terminal end. In some instances, a TCR is associated with invariant proteins of the CD3 complex involved in mediating signal transduction.

In some instances, a TCR chain contains one or more constant domain. For example, the extracellular portion of a given TCR chain (e.g., α-chain or β-chain) can contain two immunoglobulin-like domains, such as a variable domain (e.g., Vα or Vβ; typically amino acids 1 to 116 based on Kabat numbering Kabat et al., "Sequences of Proteins of Immunological Interest, US Dept. Health and Human Services, Public Health Service National Institutes of Health, 1991, 5th ed.) and a constant domain (e.g., α-chain constant domain or Cα, typically positions 117 to 259 of the chain based on Kabat numbering or β chain constant domain or Cβ, typically positions 117 to 295 of the chain based on Kabat) adjacent to the cell membrane. For example, in some cases, the extracellular portion of the TCR formed by the two chains contains two membrane-proximal constant domains, and two membrane-distal variable domains, which variable domains each contain CDRs. The constant domain of the TCR may contain short connecting sequences in which a cysteine residue forms a disulfide bond, thereby linking the two chains of the TCR. In some instances, a TCR may have an additional cysteine residue in each of the α and β chains, such that the TCR contains two disulfide bonds in the constant domains.

In some instances, the TCR chains contain a transmembrane domain. In some instances, the transmembrane domain is positively charged. In some cases, the TCR chain contains a cytoplasmic tail. In some cases, the structure allows the TCR to associate with other molecules like CD3 and subunits thereof. For example, a TCR containing constant domains with a transmembrane region may anchor the protein in the cell membrane and associate with invariant subunits of the CD3 signaling apparatus or complex. The intracellular tails of CD3 signaling subunits (e.g. CD3γ, CD3δ, CD3ε and CD3ζ chains) contain one or more immunoreceptor tyrosine-based activation motif or ITAM that are involved in the signaling capacity of the TCR complex.

In some instances, the TCR may be a heterodimer of two chains α and β (or optionally γ and δ) or it may be a single chain TCR construct. In some instances, the TCR is a heterodimer containing two separate chains (α and β chains or γ and δ chains) that are linked, such as by a disulfide bond or disulfide bonds.

In some instances, the TCR can be generated from a known TCR sequence(s), such as sequences of Vα,β chains, for which a substantially full-length coding sequence is readily available. Methods for obtaining full-length TCR sequences, including V chain sequences, from cell sources are well known. In some instances, nucleic acids encoding the TCR can be obtained from a variety of sources, such as by polymerase chain reaction (PCR) amplification of TCR-encoding nucleic acids within or isolated from a given cell or cells, or synthesis of publicly available TCR DNA sequences.

In some instances, the TCR is obtained from a biological source, such as from cells such as from a T cell (e.g. cytotoxic T cell), T-cell hybridomas or other publicly available source. In some instances, the T-cells can be obtained from in vivo isolated cells. In some instances, the TCR is a thymically selected TCR. In some instances, the TCR is a neoepitope-restricted TCR. In some instances, the T- cells can be a cultured T-cell hybridoma or clone. In some instances, the TCR or antigen-binding portion thereof can be synthetically generated from knowledge of the sequence of the TCR.

In some instances, the TCR is generated from a TCR identified or selected from screening a library of candidate TCRs against a target polypeptide antigen, or target T cell epitope thereof. TCR libraries can be generated by amplification of the repertoire of Vα and Vβ from T cells isolated from a subject, including cells present in PBMCs, spleen or other lymphoid organ. In some cases, T cells can be amplified from tumor-infiltrating lymphocytes (TILs). In some instances, TCR libraries can be generated from CD4+ or CD8+ cells. In some instances, the TCRs can be amplified from a T cell source of a normal of healthy subject, i.e. normal TCR libraries. In some instances, the TCRs can be amplified from a T cell source of a diseased subject, i.e. diseased TCR libraries. In some instances, degenerate primers are used to amplify the gene repertoire of Vα and Vβ, such as by RT-PCR in samples, such as T cells, obtained from humans. In some instances, scTv libraries can be assembled from naive Vα and Vβ libraries in which the amplified products are cloned or assembled to be separated by a linker. Depending on the source of the subject and cells, the libraries can be HLA allele-specific. Alternatively, in some instances, TCR libraries can be generated by mutagenesis or diversification of a parent or scaffold TCR molecule. In some aspects, the TCRs are subjected to directed evolution, such as by mutagenesis, e.g., of the α or β chain. In some aspects, particular residues within CDRs of the TCR are altered. In some instances, selected TCRs can be modified by affinity maturation. In some instances, antigen-specific T cells may be selected, such as by screening to assess CTL activity against the peptide. In some aspects, TCRs, e.g. present on the antigen-specific T cells, may be selected, such as by binding activity, e.g., particular affinity or avidity for the antigen.

In some instances, the genetically engineered antigen receptors include recombinant T cell receptors (TCRs) and/or TCRs cloned from naturally occurring T cells. In some instances, a high-affinity T cell clone for a target antigen (e.g., a cancer antigen) is identified, isolated from a patient, and introduced into the cells. In some instances, the TCR clone for a target antigen has been generated in transgenic mice engineered with human immune system genes (e.g., the human leukocyte antigen system, or HLA). See, e.g., tumor antigens (see, e.g., Parkhurst et al. (2009) Clin Cancer Res. 15:169-180 and Cohen et al. (2005) J Immunol. 175:5799-5808. In some instances, phage display is used to isolate TCRs against a target antigen (see, e.g., Varela-Rohena et al. (2008) Nat Med. 14:1390-1395 and Li (2005) Nat Biotechnol. 23:349-354.

In some instances, the TCR or antigen-binding portion thereof is one that has been modified or engineered. In some instances, directed evolution methods are used to generate TCRs with altered properties, such as with higher affinity for a specific MHC-peptide complex. In some instances, directed evolution is achieved by display methods including, but not limited to, yeast display (Holler et al. (2003) Nat Immunol, 4, 55-62; Holler et al. (2000) Proc Natl Acad Sci U S A, 97, 5387-92), phage display (Li et al. (2005) Nat Biotechnol, 23, 349-54), or T cell display (Chervin et al. (2008) J Immunol Methods, 339, 175-84). In some instances, display approaches involve engineering, or modifying, a known, parent or reference TCR. For example, in some cases, a wild-type TCR can be used as a template for producing mutagenized TCRs in which in one or more residues of the CDRs are mutated, and mutants with an desired altered property, such as higher affinity for a desired target antigen, are selected.

In some instances, peptides of a target polypeptide for use in producing or generating a TCR of interest are known or can be readily identified by a skilled artisan. In some instances, peptides suitable for use in generating TCRs or antigen-binding portions can be determined based on the presence of an HLA-restricted motif in a target polypeptide of interest, such as a target polypeptide described below. In some instances, peptides are identified using computer prediction models known to those of skill in the art. In some instances, for predicting MHC class I binding sites, such models include, but are not limited to, ProPred1 (Singh and Raghava (2001) Bioinformatics 17(12): 1236-1237, and SYFPEITHI (see Schuler et al. (2007) Immunoinformatics Methods in Molecular Biology, 409(1): 75-93 2007). In some instances, the MHC-restricted epitope is HLA-A0201, which is expressed in approximately 39-46% of all Caucasians and therefore, represents a suitable choice of MHC antigen for use preparing a TCR or other MHC-peptide binding molecule.

HLA-A0201-binding motifs and the cleavage sites for proteasomes and immune-proteasomes using computer prediction models are known to those of skill in the art. For predicting MHC class I binding sites, such models include, but are not limited to, ProPred1 (described in more detail in Singh and Raghava, ProPred: prediction of HLA-DR binding sites. *BIOINFORMATICS* 17(12):1236-1237 2001), and SYFPEITHI (see Schuler et al. SYFPEITHI, Database for Searching and T-Cell Epitope Prediction. in Immunoinformatics Methods in Molecular Biology, vol 409(1): 75-93 2007)

In some instances, the TCR or antigen binding portion thereof may be a recombinantly produced natural protein or mutated form thereof in which one or more property, such as binding characteristic, has been altered. In some instances, a TCR may be derived from one of various animal species, such as human, mouse, rat, or other mammal. A TCR may be cell-bound or in soluble form. In some instances, for purposes of the described methods, the TCR is in cell-bound form expressed on the surface of a cell.

In some instances, the TCR is a full-length TCR. In some instances, the TCR is an antigen-binding portion. In some instances, the TCR is a dimeric TCR (dTCR). In some instances, the TCR is a single-chain TCR (sc-TCR). In some instances, a dTCR or scTCR have the structures as described in WO 03/020763, WO 04/033685, WO2011/044186.

In some instances, the TCR contains a sequence corresponding to the transmembrane sequence. In some instances, the TCR does contain a sequence corresponding to cytoplasmic sequences. In some instances, the TCR is capable of forming a TCR complex with CD3. In some instances, any of the TCRs, including a dTCR or scTCR, can be linked to signaling domains that yield an active TCR on the surface of a T cell. In some instances, the TCR is expressed on the surface of cells.

In some instances a dTCR contains a first polypeptide wherein a sequence corresponding to a TCR α chain variable region sequence is fused to the N terminus of a sequence corresponding to a TCR α chain constant region extracellular sequence, and a second polypeptide wherein a sequence corresponding to a TCR β chain variable region sequence is fused to the N terminus a sequence corresponding to a TCR β chain constant region extracellular sequence, the first and second polypeptides being linked by a disulfide bond. In some instances, the bond can correspond to the native inter-chain disulfide bond present in native dimeric αβ TCRs. In some instances, the interchain disulfide bonds are not present in a native TCR. For example, in some instances, one or more cysteines can be incorporated into the constant region extracellular sequences of dTCR polypeptide pair. In some cases, both a native and a non-native disulfide bond may be desirable. In some instances, the TCR contains a transmembrane sequence to anchor to the membrane.

In some instances, a dTCR contains a TCR α chain containing a variable α domain, a constant α domain and a first dimerization motif attached to the C-terminus of the constant α domain, and a TCR β chain comprising a variable β domain, a constant β domain and a first dimerization motif attached to the C-terminus of the constant β domain, wherein the first and second dimerization motifs easily interact to form a covalent bond between an amino acid in the first dimerization motif and an amino acid in the second dimerization motif linking the TCR α chain and TCR β chain together.

In some instances, the TCR is a scTCR. Typically, a scTCR can be generated using methods known to those of skill in the art, See e.g., Soo Hoo, W. F. et al. PNAS (USA) 89, 4759 (1992); Wülfing, C. and Plückthun, A., J. Mol. Biol. 242, 655 (1994); Kurucz, I. et al. PNAS (USA) 90 3830 (1993); International published PCT Nos. WO 96/13593, WO 96/18105, WO99/60120, WO99/18129, WO 03/020763, WO2011/044186; and Schlueter, C. J. et al. J. Mol. Biol. 256, 859 (1996). In some instances, a scTCR contains an introduced non-native disulfide interchain bond to facilitate the association of the TCR chains (see e.g. International published PCT No. WO 03/020763). In some instances, a scTCR is a non-disulfide linked truncated TCR in which heterologous leucine zippers fused to the C-termini thereof facilitate chain association (see e.g. International published PCT No. WO99/60120). In some instances, a scTCR contain a TCRα variable domain covalently linked to a TCRβ variable domain via a peptide linker (see e.g., International published PCT No. WO99/18129).

In some instances, a scTCR contains a first segment constituted by an amino acid sequence corresponding to a TCR α chain variable region, a second segment constituted by an amino acid sequence corresponding to a TCR β chain variable region sequence fused to the N terminus of an amino acid sequence corresponding to a TCR β chain constant domain extracellular sequence, and a linker sequence linking the C terminus of the first segment to the N terminus of the second segment.

In some instances, a scTCR contains a first segment constituted by an α chain variable region sequence fused to the N terminus of an α chain extracellular constant domain sequence, and a second segment constituted by a β chain variable region sequence fused to the N terminus of a sequence β chain extracellular constant and transmembrane sequence, and, optionally, a linker sequence linking the C terminus of the first segment to the N terminus of the second segment.

In some instances, a scTCR contains a first segment constituted by a TCR β chain variable region sequence fused to the N terminus of a β chain extracellular constant domain sequence, and a second segment constituted by an α chain variable region sequence fused to the N terminus of a sequence α chain extracellular constant and transmembrane sequence, and, optionally, a linker sequence linking the C terminus of the first segment to the N terminus of the second segment.

In some instances, the linker of a scTCRs that links the first and second TCR segments can be any linker capable of forming a single polypeptide strand, while retaining TCR binding specificity. In some instances, the linker sequence may, for example, have the formula - P-AA-P- wherein P is proline and AA represents an amino acid sequence wherein the amino acids are glycine and serine. In some instances, the first and second segments are paired so that the variable region sequences thereof are orientated for such binding. Hence, in some cases, the linker has a sufficient length to span the distance between the C terminus of the first segment and the N terminus of the second segment, or vice versa, but is not too long to block or reduces bonding of the scTCR to the target ligand. In some instances, the linker can contain from or from about 10 to 45 amino acids, such as 10 to 30 amino acids or 26 to 41 amino acids residues, for example 29, 30, 31 or 32 amino acids. In some instances, the linker has the formula -PGGG-(SGGGG)₅-P- wherein P is proline, G is glycine and S is serine (SEQ ID NO: 22). In some instances, the linker has the sequence GSADDAKKDAAKKDGKS (SEQ ID NO: 23)

In some instances, the scTCR contains a covalent disulfide bond linking a residue of the immunoglobulin region of the constant domain of the α chain to a residue of the immunoglobulin region of the constant domain of the β chain. In some instances, the interchain disulfide bond in a native TCR is not present. For example, in some instances, one or more cysteines can be incorporated into the constant region extracellular sequences of the first and second segments of the scTCR polypeptide. In some cases, both a native and a non-native disulfide bond may be desirable.

In some instances of a dTCR or scTCR containing introduced interchain disulfide bonds, the native disulfide bonds are not present. In some instances, the one or more of the native cysteines forming a native interchain disulfide bonds are substituted to another residue, such as to a serine or alanine. In some instances, an introduced disulfide bond can be formed by mutating non-cysteine residues on the first and second segments to cysteine. Exemplary non-native disulfide bonds of a TCR are described in published International PCT No. WO2006/000830.

In some instances, the TCR or antigen-binding fragment thereof exhibits an affinity with an equilibrium binding constant for a target antigen of between or between about 10-5 and 10-12 M and all individual values and ranges therein. In some instances, the target antigen is an MHC-peptide complex or ligand.

In some instances, nucleic acid or nucleic acids encoding a TCR, such as α and β chains, can be amplified by PCR, cloning or other suitable means and cloned into a suitable expression vector or vectors. The expression vector can be any suitable recombinant expression vector, and can be used to transform or transfect any suitable host. Suitable vectors include those designed for propagation and expansion or for expression or both, such as plasmids and viruses.

In some instances, the vector can a vector of the pUC series (Fermentas Life Sciences), the pBluescript series (Stratagene, LaJolla, Calif.), the pET series (Novagen, Madison, Wis.), the pGEX series (Pharmacia Biotech, Uppsala, Sweden), or the pEX series (Clontech, Palo Alto, Calif.). In some cases, bacteriophage vectors, such as λG10, λGT11, λZapII (Stratagene), λEMBL4, and λNM1149, also can be used. In some instances, plant expression vectors can be used and include pBI01, pBI101.2, pBI101.3, pBI121 and pBIN19 (Clontech). In some instances, animal expression vectors include pEUK-Cl, pMAM and pMAMneo (Clontech). In some instances, a viral vector is used, such as a retroviral vector.

In some instances, the recombinant expression vectors can be prepared using standard recombinant DNA techniques. In some instances, vectors can contain regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. In some instances, the vector can contain a nonnative promoter operably linked to the nucleotide sequence encoding the TCR or antigen-binding portion (or other MHC-peptide binding molecule). In some instances, the promoter can be a non-viral promoter or a viral promoter, such as a cytomegalovirus (CMV) promoter, an SV40 promoter, an RSV promoter, and a promoter found in the long-terminal repeat of the murine stem cell virus. Other promoters known to a skilled artisan also are contemplated.

In some instances, after the T-cell clone is obtained, the TCR alpha and beta chains are isolated and cloned into a gene expression vector. In some instances, the TCR alpha and beta genes are linked via a picornavirus 2A ribosomal skip peptide so that both chains are coexpression. In some instances, genetic transfer of the TCR is accomplished via retroviral or lentiviral vectors, or via transposons (see, e.g., Baum et al. (2006) Molecular Therapy: The Journal of the American Society of Gene Therapy. 13:1050-1063; Frecha et al. (2010) Molecular Therapy: The Journal of the American Society of Gene Therapy. 18:1748-1757; and Hackett et al. (2010) Molecular Therapy: The Journal of the American Society of Gene Therapy. 18:674-683.

In some instances, to generate a vector encoding a TCR, the α and β chains are PCR amplified from total cDNA isolated from a T cell clone expressing the TCR of interest and cloned into an expression vector. In some instances, the α and β chains are cloned into the same vector. In some instances, the α and β chains are cloned into different vectors. In some instances, the generated α and β chains are incorporated into a retroviral, e.g. lentiviral, vector.

### 3. Chimeric Auto-Antibody Receptors (CAARs)

In some instances, the recombinant receptor is a chimeric autoantibody receptor (CAAR). In some instances, the CAAR is specific for an autoantibody. In some instances, a cell expressing the CAAR, such as a T cell engineered to express a CAAR, can be used to specifically bind to and kill autoantibody-expressing cells, but not normal antibody expressing cells. In some instances, CAAR-expressing cells can be used to treat an autoimmune disease associated with expression of self-antigens, such as autoimmune diseases. In some instances, CAAR-expressing cells can target B cells that ultimately produce the autoantibodies and display the autoantibodies on their cell surfaces, mark these B cells as disease-specific targets for therapeutic intervention. In some instances, CAAR-expressing cells can be used to efficiently targeting and killing the pathogenic B cells in autoimmune diseases by targeting the disease-causing B cells using an antigen-specific chimeric autoantibody receptor. In some instances, the recombinant receptor is a CAAR, such as any described in U.S. Patent Application Pub. No. US 2017/0051035.

In some instances, the CAAR comprises an autoantibody binding domain, a transmembrane domain, and an intracellular signaling region. In some instances, the intracellular signaling region comprises an intracellular signaling domain. In some instances, the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM). In some instances, the intracellular signaling region comprises a secondary or costimulatory signaling region (secondary intracellular signaling regions).

In some instances, the autoantibody binding domain comprises an autoantigen or a fragment thereof. The choice of autoantigen can depend upon the type of autoantibody being targeted. For example, the autoantigen may be chosen because it recognizes an autoantibody on a target cell, such as a B cell, associated with a particular disease state, e.g. an autoimmune disease, such as an autoantibody-mediated autoimmune disease. In some instances, the autoimmune disease includes pemphigus vulgaris (PV). Exemplary autoantigens include desmoglein 1 (Dsg1) and Dsg3.

### 4. Multi-targeting

In some instances, the cells and methods include multi-targeting strategies, such as expression of two or more genetically engineered receptors on the cell, each recognizing the same of a different antigen and typically each including a different intracellular signaling component. Such multi-targeting strategies are described, for example, in International Patent Application, Publication No.: WO 2014055668 A1 (describing combinations of activating and costimulatory CARs, e.g., targeting two different antigens present individually on off-target, e.g., normal cells, but present together only on cells of the disease or condition to be treated) and Fedorov et al., Sci. Transl. Medicine, 5(215) (2013) (describing cells expressing an activating and an inhibitory CAR, such as those in which the activating CAR binds to one antigen expressed on both normal or non-diseased cells and cells of the disease or condition to be treated, and the inhibitory CAR binds to another antigen expressed only on the normal cells or cells which it is not desired to treat).

For example, in some instances, the cells include a receptor expressing a first genetically engineered antigen receptor (e.g., CAR or TCR) which is capable of inducing an activating or stimulatory signal to the cell, generally upon specific binding to the antigen recognized by the first receptor, e.g., the first antigen. In some instances, the cell further includes a second genetically engineered antigen receptor (e.g., CAR or TCR), e.g., a chimeric costimulatory receptor, which is capable of inducing a costimulatory signal to the immune cell, generally upon specific binding to a second antigen recognized by the second receptor. In some instances, the first antigen and second antigen are the same. In some instances, the first antigen and second antigen are different.

In some instances, the first and/or second genetically engineered antigen receptor (e.g. CAR or TCR) is capable of inducing an activating signal to the cell. In some instances, the receptor includes an intracellular signaling component containing ITAM or ITAM-like motifs. In some instances, the activation induced by the first receptor involves a signal transduction or change in protein expression in the cell resulting in initiation of an immune response, such as ITAM phosphorylation and/or initiation of ITAM-mediated signal transduction cascade, formation of an immunological synapse and/or clustering of molecules near the bound receptor (e.g. CD4 or CD8, etc.), activation of one or more transcription factors, such as NF-κB and/or AP-1, and/or induction of gene expression of factors such as cytokines, proliferation, and/or survival.

In some instances, the first and/or second receptor includes intracellular signaling domains or regions of costimulatory receptors such as CD28, CD137 (4-1BB), OX40, and/or ICOS. In some instances, the first and second receptor include an intracellular signaling domain of a costimulatory receptor that are different. In one instance, the first receptor contains a CD28 costimulatory signaling region and the second receptor contain a 4-1BB co-stimulatory signaling region or vice versa.

In some instances, the first and/or second receptor includes both an intracellular signaling domain containing ITAM or ITAM-like motifs and an intracellular signaling domain of a costimulatory receptor.

In some instances, the first receptor contains an intracellular signaling domain containing ITAM or ITAM-like motifs and the second receptor contains an intracellular signaling domain of a costimulatory receptor. The costimulatory signal in combination with the activating signal induced in the same cell is one that results in an immune response, such as a robust and sustained immune response, such as increased gene expression, secretion of cytokines and other factors, and T cell mediated effector functions such as cell killing.

In some instances, neither ligation of the first receptor alone nor ligation of the second receptor alone induces a robust immune response. In some aspects, if only one receptor is ligated, the cell becomes tolerized or unresponsive to antigen, or inhibited, and/or is not induced to proliferate or secrete factors or carry out effector functions. In some such instances, however, when the plurality of receptors are ligated, such as upon encounter of a cell expressing the first and second antigens, a desired response is achieved, such as full immune activation or stimulation, e.g., as indicated by secretion of one or more cytokine, proliferation, persistence, and/or carrying out an immune effector function such as cytotoxic killing of a target cell.

In some instances, the two receptors induce, respectively, an activating and an inhibitory signal to the cell, such that binding by one of the receptor to its antigen activates the cell or induces a response, but binding by the second inhibitory receptor to its antigen induces a signal that suppresses or dampens that response. Examples are combinations of activating CARs and inhibitory CARs or iCARs. Such a strategy may be used, for example, in which the activating CAR binds an antigen expressed in a disease or condition but which is also expressed on normal cells, and the inhibitory receptor binds to a separate antigen which is expressed on the normal cells but not cells of the disease or condition.

In some instances, the multi-targeting strategy is employed in a case where an antigen associated with a particular disease or condition is expressed on a non-diseased cell and/or is expressed on the engineered cell itself, either transiently (e.g., upon stimulation in association with genetic engineering) or permanently. In such cases, by requiring ligation of two separate and individually specific antigen receptors, specificity, selectivity, and/or efficacy may be improved.

In some instances, the plurality of antigens, e.g., the first and second antigens, are expressed on the cell, tissue, or disease or condition being targeted, such as on the cancer cell. In some aspects, the cell, tissue, disease or condition is multiple myeloma or a multiple myeloma cell. In some instances, one or more of the plurality of antigens generally also is expressed on a cell which it is not desired to target with the cell therapy, such as a normal or non-diseased cell or tissue, and/or the engineered cells themselves. In such instances, by requiring ligation of multiple receptors to achieve a response of the cell, specificity and/or efficacy is achieved.

### B. Vectors and Methods for Genetic Engineering

Various methods for the introduction of genetically engineered components, e.g., recombinant receptors, e.g., CARs or TCRs, are well known and may be used with the described methods and compositions. Exemplary methods include those for transfer of nucleic acids encoding the receptors, including via viral, e.g., retroviral or lentiviral, transduction, transposons, and electroporation.

In some instances, gene transfer is accomplished by first stimulating the cell, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications.

In some instances, recombinant nucleic acids are transferred into cells using recombinant infectious virus particles, such as, e.g., vectors derived from simian virus 40 (SV40), adenoviruses, adeno-associated virus (AAV). In some instances, recombinant nucleic acids are transferred into T cells using recombinant lentiviral vectors or retroviral vectors, such as gamma-retroviral vectors (see, e.g., Koste et al. Gene Therapy doi: 10.1038/gt.2014.25 (2014); Carlens et al. Exp Hematol., 28(10): 1137-46 (2000); Alonso-Camino et al. Mol Ther Nucl Acids, 2, e93 (2013); Park et al., Trends Biotechnol., November 29(11): 550-557 (2011).

In some instances, the retroviral vector has a long terminal repeat sequence (LTR), e.g., a retroviral vector derived from the Moloney murine leukemia virus (MoMI,V), myeloproliferative sarcoma virus (MPSV), murine embryonic stem cell virus (MESV), murine stem cell virus (MSCV), spleen focus forming virus (SFFV), or adeno-associated virus (AAV). Most retroviral vectors are derived from murine retroviruses. In some instances, the retroviruses include those derived from any avian or mammalian cell source. The retroviruses typically are amphotropic, meaning that they are capable of infecting host cells of several species, including humans. In one instance, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman, BioTechniques, 7:980-990 (1989); Miller, A. D. Human Gene Therapy, 1:5-14 (1990); Scarpa et al. Virology, 180:849-852 (1991); Burns et al. Proc. Natl. Acad. Sci. USA, 90:8033-8037 (1993); and Boris-Lawrie and Temin, Cur. Opin. Genet. Develop., 3:102-109 (1993).

Methods of lentiviral transduction are known. Exemplary methods are described in, e.g., Wang et al., J. Immunother., 35(9): 689-701 (2012); Cooper et al. Blood. 101:1637-1644 (2003); Verhoeyen et al., Methods Mol Biol., 506: 97-114 (2009); and Cavalieri et al., Blood., 102(2): 497-505 (2003).

In some instances, recombinant nucleic acids are transferred into T cells via electroporation (see, e.g., Chicaybam et al, PLoS ONE 8(3): e60298 (2013) and Van Tedeloo et al. Gene Therapy 7(16): 1431-1437 (2000)). In some instances, recombinant nucleic acids are transferred into T cells via transposition (see, e.g., Manuri et al. Hum Gene Ther 21(4): 427-437 (2010); Sharma et al. Molec Ther Nucl Acids 2, e74 (2013); and Huang et al. Methods Mol Biol 506: 115-126 (2009)). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA co-precipitation (Brash et al., Mol. Cell Biol., 7: 2031-2034 (1987)).

Other approaches and vectors for transfer of the nucleic acids encoding the recombinant products are those described, e.g., in international patent application, Publication No.: WO2014055668, and U.S. Patent No. 7,446,190.

In some instances, the cells, e.g., T cells, may be transfected either during or after expansion e.g. with a T cell receptor (TCR) or a chimeric antigen receptor (CAR). This transfection for the introduction of the gene of the desired receptor can be carried out with any suitable retroviral vector, for example. The genetically modified cell population can then be liberated from the initial stimulus (the CD3/CD28 stimulus, for example) and subsequently be stimulated with a second type of stimulus e.g. via a de novo introduced receptor). This second type of stimulus may include an antigenic stimulus in form of a peptide/MHC molecule, the cognate (cross-linking) ligand of the genetically introduced receptor (e.g. natural ligand of a CAR) or any ligand (such as an antibody) that directly binds within the framework of the new receptor (e.g. by recognizing constant regions within the receptor). See, for example, Cheadle et al, Methods Mol Biol. 907:645-66 (2012); or Barrett et al., Chimeric Antigen Receptor Therapy for Cancer Annual Review of Medicine, Vol. 65: 333-347 (2014).

In some cases, a vector may be used that does not require that the cells, e.g., T cells, are activated. In some such instances, the cells may be selected and/or transduced prior to activation. Thus, the cells may be engineered prior to, or subsequent to culturing of the cells, and in some cases at the same time as or during at least a portion of the culturing.

In some aspects, the cells further are engineered to promote expression of cytokines or other factors. Among additional nucleic acids, e.g., genes for introduction are those to improve the efficacy of therapy, such as by promoting viability and/or function of transferred cells; genes to provide a genetic marker for selection and/or evaluation of the cells, such as to assess in vivo survival or localization; genes to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also the publications of PCT/US91/08442 and PCT/US94/05601 by Lupton et al. describing the use of bifunctional selectable fusion genes derived from fusing a dominant positive selectable marker with a negative selectable marker. See, e.g., Riddell et al., US Patent No. 6,040,177, at columns 14-17.

In some instances, a single promoter may direct expression of an RNA that contains, in a single open reading frame (ORF), two or three genes (*e.g.* encoding the molecule involved in modulating a metabolic pathway and encoding the recombinant receptor) separated from one another by sequences encoding a self-cleavage peptide (*e.g.,* 2A sequences) or a protease recognition site (*e.g.,* furin). The ORF thus encodes a single polypeptide, which, either during (in the case of 2A) or after translation, is processed into the individual proteins. In some cases, the peptide, such as T2A, can cause the ribosome to skip (ribosome skipping) synthesis of a peptide bond at the C-terminus of a 2A element, leading to separation between the end of the 2A sequence and the next peptide downstream (*see,* for example, de Felipe. Genetic Vaccines and Ther. 2:13 (2004) and deFelipe et al. Traffic 5:616-626 (2004)). Many 2A elements are known. Examples of 2A sequences that can be used in the methods and nucleic acids disclosed herein, without limitation, 2A sequences from the foot-and-mouth disease virus (F2A, *e.g.,* SEQ ID NO: 21), equine rhinitis A virus (E2A, *e.g.,* SEQ ID NO: 20), Thosea asigna virus (T2A, *e.g.,* SEQ ID NO: 6 or 17), and porcine teschovirus-1 (P2A, *e.g.,* SEQ ID NO: 18 or 19) as described in U.S. Patent Publication No. 20070116690.

### C. Cells and Preparation of Cells for Genetic Engineering

Among the cells expressing the receptors and administered by the described methods are engineered cells. The genetic engineering generally involves introduction of a nucleic acid encoding the recombinant or engineered component into a composition containing the cells, such as by retroviral transduction, transfection, or transformation.

In some instances, the nucleic acids are heterologous, i.e., normally not present in a cell or sample obtained from the cell, such as one obtained from another organism or cell, which for example, is not ordinarily found in the cell being engineered and/or an organism from which such cell is derived. In some instances, the nucleic acids are not naturally occurring, such as a nucleic acid not found in nature, including one comprising chimeric combinations of nucleic acids encoding various domains from multiple different cell types.

The cells generally are eukaryotic cells, such as mammalian cells, and typically are human cells. In some instances, the cells are derived from the blood, bone marrow, lymph, or lymphoid organs, are cells of the immune system, such as cells of the innate or adaptive immunity, e.g., myeloid or lymphoid cells, including lymphocytes, typically T cells and/or NK cells. Other exemplary cells include stem cells, such as multipotent and pluripotent stem cells, including induced pluripotent stem cells (iPSCs). The cells typically are primary cells, such as those isolated directly from a subject and/or isolated from a subject and frozen. In some instances, the cells include one or more subsets of T cells or other cell types, such as whole T cell populations, CD4+ cells, CD8+ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. Among the methods include off-the-shelf methods. In some aspects, such as for off-the-shelf technologies, the cells are pluripotent and/or multipotent, such as stem cells, such as induced pluripotent stem cells (iPSCs). In some instances, the methods include isolating cells from the subject, preparing, processing, culturing, and/or engineering them, and re-introducing them into the same subject, before or after cryopreservation.

Among the sub-types and subpopulations of T cells and/or of CD4+ and/or of CD8+ T cells are naive T (T_{N}) cells, effector T cells (T_{EFF}), memory T cells and sub-types thereof, such as stem cell memory T (T_{SCM}), central memory T (T_{CM}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells.

In some instances, the cells are natural killer (NK) cells. In some instances, the cells are monocytes or granulocytes, e.g., myeloid cells, macrophages, neutrophils, dendritic cells, mast cells, eosinophils, and/or basophils.

In some instances, the cells include one or more nucleic acids introduced via genetic engineering, and thereby express recombinant or genetically engineered products of such nucleic acids. In some instances, the nucleic acids are heterologous, i.e., normally not present in a cell or sample obtained from the cell, such as one obtained from another organism or cell, which for example, is not ordinarily found in the cell being engineered and/or an organism from which such cell is derived. In some instances, the nucleic acids are not naturally occurring, such as a nucleic acid not found in nature, including one comprising chimeric combinations of nucleic acids encoding various domains from multiple different cell types.

In some instances, preparation of the engineered cells includes one or more culture and/or preparation steps. The cells for introduction of the nucleic acid encoding the transgenic receptor such as the CAR, may be isolated from a sample, such as a biological sample, e.g., one obtained from or derived from a subject. In some instances, the subject from which the cell is isolated is one having the disease or condition or in need of a cell therapy or to which cell therapy will be administered. The subject in some instances is a human in need of a particular therapeutic intervention, such as the adoptive cell therapy for which cells are being isolated, processed, and/or engineered.

Accordingly, the cells in some instances are primary cells, e.g., primary human cells. The samples include tissue, fluid, and other samples taken directly from the subject, as well as samples resulting from one or more processing steps, such as separation, centrifugation, genetic engineering (e.g. transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample from which the cells are derived or isolated is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some instances, the cells are derived from cell lines, e.g., T cell lines. The cells in some instances are obtained from a xenogeneic source, for example, from mouse, rat, non-human primate, and pig.

In some instances, isolation of the cells includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components.

In some examples, cells from the circulating blood of a subject are obtained, e.g., by apheresis or leukapheresis. The samples, in some aspects, contain lymphocytes, including T cells, monocytes, granulocytes, B cells, other nucleated white blood cells, red blood cells, and/or platelets, and in some aspects contains cells other than red blood cells and platelets.

In some instances, the blood cells collected from the subject are washed, e.g., to remove the plasma fraction and to place the cells in an appropriate buffer or media for subsequent processing steps. In some instances, the cells are washed with phosphate buffered saline (PBS). In some instances, the wash solution lacks calcium and/or magnesium and/or many or all divalent cations. In some aspects, a washing step is accomplished a semi-automated "flow-through" centrifuge (for example, the Cobe 2991 cell processor, Baxter) according to the manufacturer's instructions. In some aspects, a washing step is accomplished by tangential flow filtration (TFF) according to the manufacturer's instructions. In some instances, the cells are resuspended in a variety of biocompatible buffers after washing, such as, for example, Ca++/Mg++ free PBS. In certain instances, components of a blood cell sample are removed and the cells directly resuspended in culture media.

In some instances, the methods include density-based cell separation methods, such as the preparation of white blood cells from peripheral blood by lysing the red blood cells and centrifugation through a Percoll or Ficoll gradient.

In some instances, the isolation methods include the separation of different cell types based on the expression or presence in the cell of one or more specific molecules, such as surface markers, e.g., surface proteins, intracellular markers, or nucleic acid. In some instances, any known method for separation based on such markers may be used. In some instances, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100% enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some aspects, specific subpopulations of T cells, such as cells positive or expressing high levels of one or more surface markers, e.g., CD28+, CD62L+, CCR7+, CD27+, CD127+, CD4+, CD8+, CD45RA+, and/or CD45RO+ T cells, are isolated by positive or negative selection techniques.

For example, CD3+, CD28+ T cells can be positively selected using anti-CD3/anti-CD28 conjugated magnetic beads (e.g., DYNABEADS^{®} M-450 CD3/CD28 T Cell Expander).

In some instances, isolation is carried out by enrichment for a particular cell population by positive selection, or depletion of a particular cell population, by negative selection. In some instances, positive or negative selection is accomplished by incubating cells with one or more antibodies or other binding agent that specifically bind to one or more surface markers expressed or expressed (marker+) at a relatively higher level (marker^{high}) on the positively or negatively selected cells, respectively.

In some instances, T cells are separated from a PBMC sample by negative selection of markers expressed on non-T cells, such as B cells, monocytes, or other white blood cells, such as CD14. In some aspects, a CD4+ or CD8+ selection step is used to separate CD4+ helper and CD8+ cytotoxic T cells. Such CD4+ and CD8+ populations can be further sorted into subpopulations by positive or negative selection for markers expressed or expressed to a relatively higher degree on one or more naive, memory, and/or effector T cell subpopulations.

In some instances, CD8+ cells are further enriched for or depleted of naive, central memory, effector memory, and/or central memory stem cells, such as by positive or negative selection based on surface antigens associated with the respective subpopulation. In some instances, enrichment for central memory T (T_{CM}) cells is carried out to increase efficacy, such as to improve long-term survival, expansion, and/or engraftment following administration, which in some aspects is particularly robust in such sub-populations. See Terakura et al. Blood.1:72-82 (2012); Wang et al. J Immunother. 35(9):689-701 (2012). In some instances, combining TCM-enriched CD8+ T cells and CD4+ T cells further enhances efficacy.

In instances, memory T cells are present in both CD62L+ and CD62L- subsets of CD8+ peripheral blood lymphocytes. PBMC can be enriched for or depleted of CD62L-CD8+ and/or CD62L+CD8+ fractions, such as using anti-CD8 and anti-CD62L antibodies.

In some instances, the enrichment for central memory T (T_{CM}) cells is based on positive or high surface expression of CD45RO, CD62L, CCR7, CD28, CD3, and/or CD127; in some aspects, it is based on negative selection for cells expressing or highly expressing CD45RA and/or granzyme B. In some aspects, isolation of a CD8+ population enriched for TCM cells is carried out by depletion of cells expressing CD4, CD14, CD45RA, and positive selection or enrichment for cells expressing CD62L. In one aspect, enrichment for central memory T (TCM) cells is carried out starting with a negative fraction of cells selected based on CD4 expression, which is subjected to a negative selection based on expression of CD14 and CD45RA, and a positive selection based on CD62L. Such selections in some aspects are carried out simultaneously and in other aspects are carried out sequentially, in either order. In some aspects, the same CD4 expression-based selection step used in preparing the CD8+ cell population or subpopulation, also is used to generate the CD4+ cell population or subpopulation, such that both the positive and negative fractions from the CD4-based separation are retained and used in subsequent steps of the methods, optionally following one or more further positive or negative selection steps.

In a particular example, a sample of PBMCs or other white blood cell sample is subjected to selection of CD4+ cells, where both the negative and positive fractions are retained. The negative fraction then is subjected to negative selection based on expression of CD14 and CD45RA or CD 19, and positive selection based on a marker characteristic of central memory T cells, such as CD62L or CCR7, where the positive and negative selections are carried out in either order.

CD4+ T helper cells are sorted into naive, central memory, and effector cells by identifying cell populations that have cell surface antigens. CD4+ lymphocytes can be obtained by standard methods. In some instances, naive CD4+ T lymphocytes are CD45RO-, CD45RA+, CD62L+, CD4+ T cells. In some instances, central memory CD4+ cells are CD62L+ and CD45RO+. In some instances, effector CD4+ cells are CD62L- and CD45RO-.

In one example, to enrich for CD4+ cells by negative selection, a monoclonal antibody cocktail typically includes antibodies to CD 14, CD20, CD11b, CD 16, HLA-DR, and CD8. In some instances, the antibody or binding partner is bound to a solid support or matrix, such as a magnetic bead or paramagnetic bead, to allow for separation of cells for positive and/or negative selection. For example, in some instances, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In Vitro and In Vivo, p 17-25 Edited by: S. A. Brooks and U. Schumacher © Humana Press Inc., Totowa, NJ).

In some aspects, the sample or composition of cells to be separated is incubated with small, magnetizable or magnetically responsive material, such as magnetically responsive particles or microparticles, such as paramagnetic beads (e.g., such as Dynalbeads or MACS beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some instances, the magnetic particle or bead comprises a magnetically responsive material bound to a specific binding member, such as an antibody or other binding partner. There are many well-known magnetically responsive materials used in magnetic separation methods. Suitable magnetic particles include those described in Molday, U.S. Pat. No. 4,452,773, and in European Patent Specification EP 452342 B. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In certain instances, the magnetically responsive particles are coated in primary antibodies or other binding partners, secondary antibodies, lectins, enzymes, or streptavidin. In certain instances, the magnetic particles are attached to cells via a coating of primary antibodies specific for one or more markers. In certain instances, the cells, rather than the beads, are labeled with a primary antibody or binding partner, and then cell-type specific secondary antibody- or other binding partner (e.g., streptavidin)-coated magnetic particles, are added. In certain instances, streptavidin-coated magnetic particles are used in conjunction with biotinylated primary or secondary antibodies.

In some instances, the magnetically responsive particles are left attached to the cells that are to be subsequently incubated, cultured and/or engineered; in some aspects, the particles are left attached to the cells for administration to a patient. In some instances, the magnetizable or magnetically responsive particles are removed from the cells. Methods for removing magnetizable particles from cells are known and include, e.g., the use of competing non-labeled antibodies, and magnetizable particles or antibodies conjugated to cleavable linkers. In some instances, the magnetizable particles are biodegradable.

In some instances, the affinity-based selection is via magnetic-activated cell sorting (MACS) (Miltenyi Biotech, Auburn, CA). Magnetic Activated Cell Sorting (MACS) systems are capable of high-purity selection of cells having magnetized particles attached thereto. In certain instances, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain instances, the non-target cells are labelled and depleted from the heterogeneous population of cells.

In certain instances, the isolation or separation is carried out using a system, device, or apparatus that carries out one or more of the isolation, cell preparation, separation, processing, incubation, culture, and/or formulation steps of the methods. In some aspects, the system is used to carry out each of these steps in a closed or sterile environment, for example, to minimize error, user handling and/or contamination. In one example, the system is a system as described in International Patent Application, Publication Number WO2009/072003, or US 20110003380 A1.

In some instances, the system or apparatus carries out one or more, e.g., all, of the isolation, processing, engineering, and formulation steps in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some aspects, the separation and/or other steps is carried out using CliniMACS system (Miltenyi Biotec), for example, for automated separation of cells on a clinical-scale level in a closed and sterile system. Components can include an integrated microcomputer, magnetic separation unit, peristaltic pump, and various pinch valves. The integrated computer in some aspects controls all components of the instrument and directs the system to perform repeated procedures in a standardized sequence. The magnetic separation unit in some aspects includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some aspects uses antibody-coupled magnetizable particles that are supplied in a sterile, non-pyrogenic solution. In some instances, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some instances, the cell populations for use with the methods described herein are unlabeled and are not retained in the column. In some instances, the cell populations for use with the methods described herein are labeled and are retained in the column. In some instances, the cell populations for use with the methods described herein are eluted from the column after removal of the magnetic field, and are collected within the cell collection bag.

In certain instances, separation and/or other steps are carried out using the CliniMACS Prodigy system (Miltenyi Biotec). The CliniMACS Prodigy system in some aspects is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood is automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. J Immunother. 35(9): 651-660 (2012), Terakura et al. Blood. 1:72-82 (2012), and Wang et al. J Immunother. 35(9):689-701 (2012).

In some instances, a cell population described herein is collected and enriched (or depleted) via flow cytometry, in which cells stained for multiple cell surface markers are carried in a fluidic stream. In some instances, a cell population described herein is collected and enriched (or depleted) via preparative scale (FACS)-sorting. In certain instances, a cell population described herein is collected and enriched (or depleted) by use of microelectromechanical systems (MEMS) chips in combination with a FACS-based detection system (see, e.g., WO 2010/033140, Cho et al. Lab Chip 10, 1567-1573 (2010); and Godin et al. J Biophoton. 1(5):355-376 (2008). In both cases, cells can be labeled with multiple markers, allowing for the isolation of well-defined T cell subsets at high purity.

In some instances, the antibodies or binding partners are labeled with one or more detectable marker, to facilitate separation for positive and/or negative selection. For example, separation may be based on binding to fluorescently labeled antibodies. In some examples, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flow-cytometric detection system. Such methods allow for positive and negative selection based on multiple markers simultaneously.

In some instances, the preparation methods include steps for freezing, e.g., cryopreserving, the cells, either before or after isolation, incubation, and/or engineering. In some instances, the freeze and subsequent thaw step removes granulocytes and, to some extent, monocytes in the cell population. In some instances, the cells are suspended in a freezing solution, e.g., following a washing step to remove plasma and platelets. Any of a variety of known freezing solutions and parameters in some aspects may be used. One example involves using PBS containing 20% DMSO and 8% human serum albumin (HSA), or other suitable cell freezing media. This is then diluted 1:1 with media so that the final concentration of DMSO and HSA are 10% and 4%, respectively. The cells are generally then frozen to -80° C. at a rate of 1° per minute and stored in the vapor phase of a liquid nitrogen storage tank.

In some instances, the cells are incubated and/or cultured prior to or in connection with genetic engineering. The incubation steps can include culture, cultivation, stimulation, activation, and/or propagation. The incubation and/or engineering may be carried out in a culture vessel, such as a unit, chamber, well, column, tube, tubing set, valve, vial, culture dish, bag, or other container for culture or cultivating cells. In some instances, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some instances, the stimulating conditions or agents include one or more agent, e.g., ligand, which is capable of activating an intracellular signaling domain of a TCR complex. In some aspects, the agent turns on or initiates TCR/CD3 intracellular signaling cascade in a T cell. Such agents can include antibodies, such as those specific for a TCR, e.g. anti-CD3. In some instances, the stimulating conditions include one or more agent, e.g. ligand, which is capable of stimulating a costimulatory receptor, e.g., anti-CD28. In some instances, such agents and/or ligands may be, bound to solid support such as a bead, and/or one or more cytokines. Optionally, the expansion method may further comprise the step of adding anti-CD3 and/or anti CD28 antibody to the culture medium (e.g., at a concentration of at least about 0.5 ng/ml). In some instances, the stimulating agents include IL-2, IL-15 and/or IL-7. In some aspects, the IL-2 concentration is at least about 10 units/mL.

In some aspects, incubation is carried out in accordance with techniques such as those described in US Patent No. 6,040,177 to Riddell et al., Klebanoff et al. J Immunother. 35(9): 651-660 (2012), Terakura et al. Blood.1:72-82 (2012), and/or Wang et al. J Immunother. 35(9):689-701 (2012).

In some instances, the T cells are expanded by adding to a culture-initiating composition feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g. for a time sufficient to expand the numbers of T cells). In some aspects, the non-dividing feeder cells can comprise gamma-irradiated PBMC feeder cells. In some instances, the PBMC are irradiated with gamma rays in the range of about 3000 to 3600 rads to prevent cell division. In some aspects, the feeder cells are added to culture medium prior to the addition of the populations of T cells.

In some instances, the stimulating conditions include temperature suitable for the growth of human T lymphocytes, for example, at least about 25 degrees Celsius, generally at least about 30 degrees, and generally at or about 37 degrees Celsius. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some aspects is provided in any suitable amount, such as a ratio of LCL feeder cells to initial T lymphocytes of at least about 10:1.

In instances, antigen-specific T cells, such as antigen-specific CD4+ and/or CD8+ T cells, are obtained by stimulating naive or antigen specific T lymphocytes with antigen. For example, antigen-specific T cell lines or clones can be generated to cytomegalovirus antigens by isolating T cells from infected subjects and stimulating the cells in vitro with the same antigen.

### VI. COMPOSITIONS AND FORMULATIONS

In some instances, the cell therapy is provided as a composition or formulation, such as a pharmaceutical composition or formulation. Such compositions can be used in accord with the described methods, such as in the prevention or treatment of diseases, conditions, and disorders, or in detection, diagnostic, and prognostic methods.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some instances, the T cell therapy, such as engineered T cells (*e.g.* CAR T cells), are formulated with a pharmaceutically acceptable carrier. In some aspects, the choice of carrier is determined in part by the particular cell and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulations can include aqueous solutions. The formulation or composition may also contain more than one active ingredient useful for the particular indication, disease, or condition being prevented or treated with the cells, including one or more active ingredients where the activities are complementary to the cells and/or the respective activities do not adversely affect one another. Such active ingredients are suitably present in combination in amounts that are effective for the purpose intended. Thus, in some instances, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc.

The pharmaceutical composition in some instances contain cells in amounts effective to treat or prevent the disease or condition, such as a therapeutically effective or prophylactically effective amount. Therapeutic or prophylactic efficacy in some instances is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

The cells may be administered using standard administration techniques, formulations, and/or devices. Described are formulations and devices, such as syringes and vials, for storage and administration of the compositions. With respect to cells, administration can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell), it will generally be formulated in a unit dosage injectable form (solution, suspension, emulsion).

Formulations include those for oral, intravenous, intraperitoneal, subcutaneous, pulmonary, transdermal, intramuscular, intranasal, buccal, sublingual, or suppository administration. In some instances, the agent or cell populations are administered parenterally. The term "parenteral," as used herein, includes intravenous, intramuscular, subcutaneous, rectal, vaginal, and intraperitoneal administration. In some instances, the agent or cell populations are administered to a subject using peripheral systemic delivery by intravenous, intraperitoneal, or subcutaneous injection.

Compositions in some instances are provided as sterile liquid preparations, e.g., isotonic aqueous solutions, suspensions, emulsions, dispersions, or viscous compositions, which may in some aspects be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the cells in a solvent, such as in admixture with a suitable carrier, diluent, or excipient such as sterile water, physiological saline, glucose, dextrose, or the like. The compositions can also be lyophilized. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin.

The formulations to be used for in vivo administration are generally sterile. Sterility may be readily accomplished, e.g., by filtration through sterile filtration membranes.

For the prevention or treatment of disease, the appropriate dosage may depend on the type of disease to be treated, the type of agent or agents, the type of cells or recombinant receptors, the severity and course of the disease, whether the agent or cells are administered for preventive or therapeutic purposes, previous therapy, the subject's clinical history and response to the agent or the cells, and the discretion of the attending physician. The compositions are in some instances suitably administered to the subject at one time or over a series of treatments.

### VII.KITS AND ARTICLES OF MANUFACTURE

Also described are articles of manufacture or kit containing the described genetically engineered cells, and one or more agents for modulating the expansion, proliferation and/or activity of the engineered cells, and/or a further therapeutic agent and/or compositions comprising the same, optionally reagents for assessing and/or measuring one or more parameters, e.g., pharmacokinetic parameters and/or patient attributes and/or expression of biomarkers, and optionally instructions for use, for example, instructions for administering and/or assessment, according to the described methods. The articles of manufacture may include a container and a label or package insert on or associated with the container. Suitable containers include, for example, bottles, vials, syringes, test tubes, IV solution bags, etc. The containers may be formed from a variety of materials such as glass or plastic. In some instances, the container has a sterile access port. Exemplary containers include an intravenous solution bags, vials, including those with stoppers pierceable by a needle for injection.

The articles of manufacture described herein contain packaging materials. Packaging materials for use in packaging the described materials are well known to those of skill in the art. See, for example, U.S. Patent Nos. 5,323,907, 5,052,558 and 5,033,252, each of which is incorporated herein in its entirety. Examples of packaging materials include, but are not limited to, blister packs, bottles, tubes, inhalers, pumps, bags, vials, containers, syringes, disposable laboratory supplies, e.g., pipette tips and/or plastic plates, or bottles. The articles of manufacture or kits can include a device so as to facilitate dispensing of the materials or to facilitate use in a high-throughput or large-scale manner, e.g., to facilitate use in robotic equipment. Typically, the packaging is non-reactive with the compositions contained therein.

The article of manufacture or kit may further include a package insert indicating that the compositions can be used to treat a particular condition such as a condition described herein (e.g., multiple myeloma). Alternatively, or additionally, the article of manufacture or kit may further include another or the same container comprising a pharmaceutically-acceptable buffer. It may further include other materials such as other buffers, diluents, filters, needles, and/or syringes.

In some instances, the articles of manufacture or kits include one or more containers, typically a plurality of containers, packaging material, and a label or package insert on or associated with the container or containers and/or packaging, generally including instructions for use, e.g., instructions for nucleic acid assembly and/or introduction of the assembled nucleic acid molecules or sets of nucleic acid molecules into of cells, such as transfection or transduction of cells used in the described methods, such as T cells, T cell lines and/or T cell compositions.

The container in some instances holds a composition which is by itself or combined with another composition containing one or more agent(s) capable of modulating the expansion, proliferation and/or activity of the engineered cells, such as any described herein. The article of manufacture or kit may include one or more containers with a composition contained therein, wherein the composition includes one or more agent(s) capable of modulating the expansion, proliferation and/or activity of the engineered cells, such as any described herein; wherein the composition optionally includes a further therapeutic agent, and which article or kit further comprises instructions on the label or package insert for treating the subject in an effective amount.

In some instances, the articles of manufacture and/or kits further comprise an agent for lymphodepleting therapy, and optionally further includes instructions for administering the lymphodepleting therapy. In some instances, the instructions can be included as a label or package insert accompanying the compositions for administration.

In some instances, the articles of manufacture and/or kits further include one or more reagents for assaying biological samples, e.g., biological samples from subjects who are candidates for administration or who have been administered the therapy, and optionally instructions for use of the reagents or assays, e.g., assessment of one or more parameters, e.g., pharmacokinetic parameters and/or patient attributes and/or expression of biomarkers, and optionally instructions for use, for example, instructions for administering and/or assessment. In some instances, the biological sample is or is obtained from a blood, plasma or serum sample.

In some instances, the reagents can be used prior to the administration of the cell therapy or after the administration of cell therapy. For example, in some instances, the article of manufacture and/or kits further contain reagents for measuring the level of particular patient attributes and/or inflammatory markers, that are associated with certain pharmacokinetic parameters, response outcome and/or toxicity, and instructions for measuring. In some instances, the reagents include components for performing an *in vitro* assay to measure the parameters, such as an immunoassay, an aptamer-based assay, a histological or cytological assay, or an mRNA expression level assay. In some instances, the *in vitro* assay is selected from among an enzyme linked immunosorbent assay (ELISA), immunoblotting, immunoprecipitation, radioimmunoassay (RIA), immunostaining, flow cytometry assay, surface plasmon resonance (SPR), chemiluminescence assay, lateral flow immunoassay, inhibition assay and avidity assay. In some aspects, the reagent is a binding reagent that specifically binds the biomarkers (e.g. analytes). In some cases, the binding reagent is an antibody or antigen-binding fragment thereof, an aptamer or a nucleic acid probe. In some instances, the article of manufacture contains any reagents described herein for assessing the parameters.

In some instances, the articles of manufacture and/or kits comprise one or more reagent capable of detecting one or more parameters, e.g., pharmacokinetic parameters and/or patient attributes and/or expression of biomarkers, for example, instructions for administering and/or assessment, and instructions for using the reagent to assay a biological sample from a subject that is a candidate for treatment, wherein the one or more parameters is selected from among maximum (peak) plasma concentration (Cₘₐₓ) of CAR+ cells, e.g., CD3+, CD4+ and/or CD8+ CAR+ cells, the peak time (*i.e*. when maximum plasma concentration (Cₘₐₓ) occurs; Tₘₐₓ), the minimum plasma concentration (*i.e*. the minimum plasma concentration between doses of a therapeutic agent, e.g., CAR+ T cells; Cₘᵢₙ), the elimination half-life (T_{1/2}) and area under the curve (*i.e.* the area under the curve generated by plotting time versus plasma concentration of the therapeutic agent CAR+ T cells; AUC), volumetric measurements of a tumor, e.g., the sum of the products of diameters (SPD), longest tumor diameters (LD), sum of longest tumor diameters (SLD), necrosis, tumor volume, necrosis volume, necrosis-tumor ratio (NTR), peritumoral edema (PTE), and edema-tumor ratio (ETR), erythrocyte sedimentation rate (ESR), albumin, β2 microglobulin (β2-M), C-C Motif Chemokine Ligand 13 (CCL13), C-reactive protein (CRP), C-X-C motif chemokine 10 (CXCL10), IL-2, IL-5, IL-6, IL-7, IL-8, IL-10, IL-15, IL-16, interferon gamma (IFN-y), Lymphotoxin-alpha (LT-α), Monocyte chemoattractant protein-1 (MCP-1), macrophage inflammatory protein 1 alpha (MIP-1α), MIP-10, Serum Amyloid A1 (SAA-1), Transforming growth factor beta (TGF-β) and tumor necrosis factor alpha (TNF-α). In some instances, instructions for assaying presence or absence, level, amount, or concentration of an parameter in the subject compared to a threshold level of the analyte and/or parameters is also included.

### VIII. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, a "subject" is a mammal, such as a human or other animal, and typically is human. In some instances, the subject, e.g., patient, to whom the immunomodulatory polypeptides, engineered cells, or compositions are administered, is a mammal, typically a primate, such as a human. In some instances, the primate is a monkey or an ape. The subject can be male or female and can be any suitable age, including infant, juvenile, adolescent, adult, and geriatric subjects. In some instances, the subject is a non-primate mammal, such as a rodent.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The terms do not imply complete curing of a disease or complete elimination of any symptom or effect(s) on all symptoms or outcomes.

As used herein, "delaying development of a disease" means to defer, hinder, slow, retard, stabilize, suppress and/or postpone development of the disease (such as cancer). This delay can be of varying lengths of time, depending on the history of the disease and/or individual being treated. As is evident to one skilled in the art, a sufficient or significant delay can, in effect, encompass prevention, in that the individual does not develop the disease. For example, a late stage cancer, such as development of metastasis, may be delayed.

"Preventing," as used herein, includes providing prophylaxis with respect to the occurrence or recurrence of a disease in a subject that may be predisposed to the disease but has not yet been diagnosed with the disease. In some instances, the described cells and compositions are used to delay development of a disease or to slow the progression of a disease.

As used herein, to "suppress" a function or activity is to reduce the function or activity when compared to otherwise same conditions except for a condition or parameter of interest, or alternatively, as compared to another condition. For example, cells that suppress tumor growth reduce the rate of growth of the tumor compared to the rate of growth of the tumor in the absence of the cells.

An "effective amount" of an agent, e.g., a pharmaceutical formulation, cells, or composition, in the context of administration, refers to an amount effective, at dosages/amounts and for periods of time necessary, to achieve a desired result, such as a therapeutic or prophylactic result.

A "therapeutically effective amount" of an agent, e.g., a pharmaceutical formulation or engineered cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the immunomodulatory polypeptides or engineered cells administered. In some instances, the described methods involve administering the immunomodulatory polypeptides, engineered cells, or compositions at effective amounts, e.g., therapeutically effective amounts.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, recitation that nucleotides or amino acid positions "correspond to" nucleotides or amino acid positions in a disclosed sequence, such as set forth in the Sequence listing, refers to nucleotides or amino acid positions identified upon alignment with the disclosed sequence to maximize identity using a standard alignment algorithm, such as the GAP algorithm. By aligning the sequences, one skilled in the art can identify corresponding residues, for example, using conserved and identical amino acid residues as guides. In general, to identify corresponding positions, the sequences of amino acids are aligned so that the highest order match is obtained (see, e.g. : Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; Carrillo et al. (1988) SIAM J Applied Math 48: 1073).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors." Among the vectors are viral vectors, such as retroviral, e.g., gammaretroviral and lentiviral vectors.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

As used herein, "percent (%) amino acid sequence identity" and "percent identity" when used with respect to an amino acid sequence (reference polypeptide sequence) is defined as the percentage of amino acid residues in a candidate sequence (e.g., the subject antibody or fragment) that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known to the skilled person in this technical field. Reference to "about" a value or parameter herein includes (and describes) instances that are directed to that value or parameter per se. For example, description referring to "about X" includes description of "X".

As used herein, a composition refers to any mixture of two or more products, substances, or compounds, including cells. It may be a solution, a suspension, liquid, powder, a paste, aqueous, non-aqueous or any combination thereof.

If a definition set forth herein is contrary to or otherwise inconsistent with a definition set forth in the patents, applications, published applications and other publications that are referred to herein, the definition set forth herein prevails.

The section headings used herein are for organizational purposes only and are not to be construed as limiting the subject matter described.

### IX. EXAMPLES

The following examples are included for illustrative purposes only and are not intended to limit the scope of the invention.

### Example 1: Probability of Marrow Response Based on Peak CAR T Cell Expansion and Response and Neurotoxicity in High-Risk CLL Patients

Twenty-four (24) adult human subjects with relapsed or refractory (R/R) CD19+ chronic lymphocytic leukemia (CLL) were administered autologous T cells expressing a chimeric antigen-receptor (CAR) specific for CD19 and evaluated as described below.

The CAR included an scFv (in a V_{L}-linker-V_{H} orientation) specific for CD19, with variable regions derived from FMC63, an IgG hinge region, a transmembrane region, and intracellular signaling domains derived from human 41BB and CD3zeta. The construct further encoded a truncated EGFR (EGFRt), which served as a surrogate marker for CAR expression; the EGFRt-coding region was separated from the CAR sequence by a T2A skip sequence. Prior to administration of the cells, patients underwent leukapheresis; CD4+ and CD8+ populations were selected by immunoaffinity-based enrichment methods, transduced with a viral vector with the CAR construct, and expanded in culture over fifteen (15) days.

Beginning at least forty-eight (48) (and up to ninety-six (96)) hours prior to CAR+ T cell infusion, subjects received a lymphodepleting chemotherapy with either (a) cyclophosphamide (Cy, 60 mg/kg) with or without etoposide (2/13 subjects), or (b) cyclophosphamide (Cy, 60 mg/kg) in combination with fludarabine (flu, 25 mg/m² daily for 3-5 days (cy/flu, 11/13 subjects).

Cells for administration generally were formulated at a CAR+ CD4+ T cell to CAR+ CD8+ T cell ratio of approximately 1:1. Therapeutic compositions were successfully produced for all subjects. For 1/13 subjects, fewer than the target dose (2×10⁶/kg CAR+) of cells were produced.

Subjects were infused with a composition having approximately a 1:1 ratio of CD8+ CAR+ T cells to CD4+ CAR-T cells, at one of three different dose levels (2×10⁵ (N=4) 2×10⁶ (N=8) or 2×10⁷ (N=1) CAR+ T cells per kilogram (kg) weight of the subject). Lymphodepleting therapy and T cell infusions were administered out on an outpatient basis.

The incidence and grade of cytokine release syndrome (CRS) was determined according to Lee et al, Blood. 2014;124(2):188-95. Following treatment, subjects were assessed and monitored for neurotoxicity (neurological complications including symptoms of confusion, aphasia, seizures, convulsions, lethargy, and/or altered mental status), graded based on severity using a Grade 1-5 scale (see, *e.g.,* Guido Cavaletti & Paola Marmiroli Nature Reviews Neurology 6, 657-666 (December 2010). Grade 3 (severe symptoms), 4 (life-threatening symptoms) or 5 (death) indicated severe neurotoxicity.

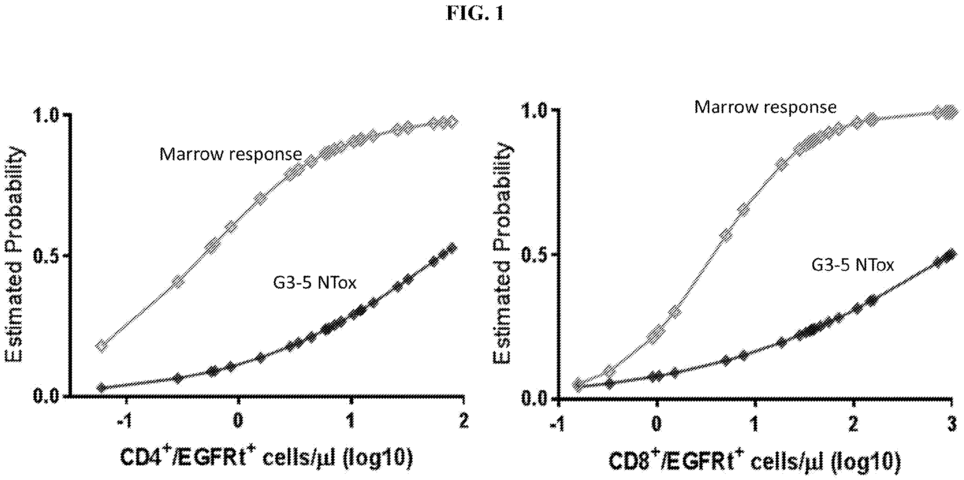

An estimated probability curve of response and an estimated probability of developing Grade 3-5 neurotoxicity were constructed based on the number of CD4+/EGFRt+ or CD8+/EGFRt+ CAR-T cells in the blood (**FIG. 1**). Generally, as the number of CAR- T cells increased, the probability of response increased then plateaued while the probability of developing Grade 3-5 neurotoxicity increased.

### Example 2: Administration of anti-CD19 CAR-Expressing Cells to Subjects

Twenty eight subjects with relapsed or refractory (R/R) non-Hodgkin lymphoma (NHL) were administered autologous T cells expressing an anti-CD19 chimeric antigen receptor (CAR). Subject demographics and baseline characteristics are set forth in **Table 3.** The CAR contained an anti-CD19 scFv derived from a murine antibody, an immunoglobulin-derived spacer, a transmembrane domain derived from CD28, a costimulatory region derived from 4-1BB, and a CD3-zeta intracellular signaling domain. To generate the autologous CAR-expressing T cells, T cells were isolated by immunoaffinity-based enrichment from leukapheresis samples from individual subjects, activated and transduced with a viral vector encoding an anti-CD 19 CAR, followed by expansion (at a target ratio of approximately 1:1 ratio of CD4+ to CD8+ CAR+ T cells).

| | | |
|---|---|---|
| **Table 3. Demographics and Baseline Characteristics** | | |

| **Characteristic** | | **N=28** |
|---|---|---|
| Median Age, years (range) | | 63 (37-79) |
| | ≥ 70 years, n (%) | 6 (21) |
| Male/Female, n (%) | | 19/9 (68/32) |

| B-NHL Subtype, n (%) | | |
|---|---|---|
| DLBCL, NOS | | 15 (54) |
| Transformed DLBCL | | 10 (36) |
| Follicular, Grade 3B | | 1 (4) |
| MCL | | 2 (7) |

| Disease Status, n (%) | | |
|---|---|---|
| Refractory^{∗} | | 24 (86) |
| Chemorefractory^{†} | | 23 (82) |

| Baseline ECOG score, n (%) | | |
|---|---|---|
| 0 | | 14 (50) |
| 1 | | 10 (36) |
| 2 | | 4 (14) |

| Prior Lines of Therapy | | |
|---|---|---|
| Median (range) | | 4 (1-8) |
| ≥ 5, n (%) | | 7 (25) |

| Prior Hematopoietic Stem Cell Transplant, n (%) | | |
|---|---|---|
| Any HSCT | | 16 (57) |
| | Allogeneic | 4 (14) |
| | Autologous | 13 (46) |

| | | |
|---|---|---|
| *<CR to last therapy ^{†}SD or PD to last chemo-containing regimen or relapse <12 months after autologous SCT | | |

Prior to administration of the CAR-expressing T cells, subjects were treated with 30 mg/m² fludarabine daily for 3 days and 300 mg/m² cyclophosphamide daily for 3 days. The cryopreserved cell compositions were thawed prior to intravenous administration. The therapeutic T cell dose was administered as a defined cell composition by administering a formulated CD4+ CAR+ cell population and a formulated CD8+ CAR+ population administered at a target ratio of approximately 1:1. At d=0, subjects were then treated with a single-dose or double-dose schedule of 5 × 10⁷ (DL1) or 1 × 10⁸ (DL2) CAR-expressing T cells by intravenous infusion (each single dose via separate infusions of CD4+ CAR-expressing T cells and CD8+ CAR-expressing T cells, respectively).

The presence or absence of various treatment-emergent adverse events was assessed in subjects treated with various dose schedules of CAR-T cell therapy (**Table 4** and **Table 5**). As shown in **Table 5,** no severe Cytokine Release Syndrome (sCRS) (Grade 3-5) was observed; Cytokine Release Syndrome (CRS) was observed in 36% (10/28) of the subjects. Grade 3-4 neurotoxicity was observed in 14% (4/28) of the subjects and 18% (5/28) of the subjects exhibited neurotoxicity of any grade. One subject was treated with tocilizumab and four patients received dexamethasone for early onset Grade 2 CRS or neurotoxicity. Six subjects received prophylactic anti-epileptics.

| **Table 4. Treatment-Emergent Adverse Events** | | | | |
|---|---|---|---|---|
| | **DL1-S N=22** | **DL1-D N=3** | **DL2-S N=3** | **Total N=28** |
| Any TEAE | 21 (96) | 3 (100) | 3 (100) | 27 (96) |
| Any Grade 3-5^{∗} TEAE | 16 (73) | 3 (100) | 0 | 19 (68) |
| Any Related TEAE | 14 (64) | 2 (67) | 1 (33) | 17 (61) |
| Any Related Grade 3-5^{∗} TEAE | 4 (18) | 1 (33) | 0 | 5 (18) |

| **All grade TEAEs reported in ≥15% patients** | | | | |
|---|---|---|---|---|
| **Preferred term, n (%)** | | | | |
| Fatigue | 7 (32) | 2 (67) | 2 (67) | 11 (39) |
| Cytokine release syndrome | 8 (36) | 2 (67) | 0 | 10 (36) |
| Decreased appetite | 6 (27) | 1 (33) | 1 (33) | 8 (29) |
| Constipation | 5 (23) | 1 (33) | 1 (33) | 7 (25) |
| Vomiting | 5 (23) | 1 (33) | 1 (33) | 7 (25) |
| Diarrhea | 5 (23) | 1 (33) | 0 | 6 (21) |
| Dizziness | 6 (27) | 0 | 0 | 6 (21) |
| Headache | 4 (18) | 1 (33) | 0 | 5 (18) |
| Hypertension | 4 (18) | 1 (33) | 0 | 5 (18) |
| Nausea | 3 (14) | 1 (33) | 1 (33) | 5 (18) |

| Peripheral edema | 5 (23) | 0 | 0 | 5 (18) |
|---|---|---|---|---|
| **Lab abnormalities** | | | | |
| Anemia | 16 (73) | 1 (33) | 1 (33) | 18 (64) |
| Neutropenia | 22 (100) | 3 (100) | 2 (67) | 27 (96) |
| Thrombocytopenia | 13 (59) | 3 (100) | 2 (67) | 18 (64) |

| | | | | |
|---|---|---|---|---|
| ^{∗}1 Grade 5 respiratory failure, assessed as possibly related to CAR-T cell therapy, in a patient with MCL who progressed and started on a subsequent therapy | | | | |

| **Table 5. Treatment-Emergent Adverse Events of Special Interest** | | | | | |
|---|---|---|---|---|---|
| Preferred Term, n (%) | | **DL1-S N=22** | **DL1-D N=3** | **DL2-S N=3** | **Total N=28** |
| Cytokine Release Syndrome (CRS), any | | 8 (36) | 2 (67) | 0 | 10 (36) |
| | Grade 3-4 | 0 | 0 | 0 | 0 |
| Neurotoxicity, any* | | 4 (18) | 1 (33) | 0 | 5 (18) |
| | Grade 3-4 | 3 (14) | 1 (33) | 0 | 4 (14) |

| | | | | | |
|---|---|---|---|---|---|
| * Includes: encephalopathy, confusional state, depressed level of consciousness, lethargy, or seizure | | | | | |

Subjects among the group were assessed for best overall response, observed over a period of up to a particular time-point in an ongoing study after the last CAR+ T cell infusion of single-dose of DL1. Results of overall responses are shown in **Table 6.** Of the 20 subjects that were treated with the single-dose of DL1 in the Diffuse Large B-Cell Lymphoma (DLBCL) cohort, an overall response rate (ORR) of 80% (16/20) was observed and 60% (12/20) of subjects showed evidence of complete remission (CR). 20% (4/20) of subjects showed evidence of partial response (PR) and 20% (4/20) of subjects showed evidence of progressive disease (PD). Of the subjects having been chemorefractory (having exhibited stable or progressive disease following last chemo-containing regimen or relapse less than 12 months after autologous SCT) prior to CAR+ T cell administration, the overall response rate was 83% (10 ORR, 7 CR, 3 PR, 2 PD, n=12). Among the subjects having been refractory (having exhibited less than complete remission following last treatment but not deemed chemorefractory), the overall response rate was 77% (13 ORR, 9 CR, 4 PR, 4 PD, n=17).

| **Table 6. Overall Response** | | | |
|---|---|---|---|
| | **DLBCL Cohort, DL1 single-dose schedule** | | |
| | **All (n=20)** | **Refractory* (n=17)** | **Chemorefractory**^{†} **(n=12)** |
| ORR, n (%) [95% CI] | 16 (80) [56, 94] | 13 (77) [50, 93] | 10 (83) [52, 98] |
| CR, n (%) [95% CI] | 12 (60) [36, 81] | 9 (53) [28, 77] | 7 (58) [28, 85] |
| PR | 4 (20) | 4 (24) | 3 (25) |
| PD | 4 (20) | 4 (24) | 2 (17) |

| | | | |
|---|---|---|---|
| ^{∗}<CR to last therapy ^{†}SD or PD to last chemo-containing regimen or relapse <12 months after autologous SCT | | | |

Of three DLBCL subjects that at the time of assessment had been treated with two doses of DL1, two (2) exhibited partial response (PR) and one (1) exhibited progressive disease (PD). Among 2 DLBCL subjects that at the time of assessment had been treated with a single-dose of DL2, both subjects were observed to achieve CR. Among a MCL cohort with a total of two subjects treated at the time of assessment with single-dose of DL1, 1 PR and 1 PD were observed. Two subjects with double-hit, three subjects with triple-hit, and four subjects with double-expressor DLBCL were treated and all achieved a response (7 CR, 2 PR).

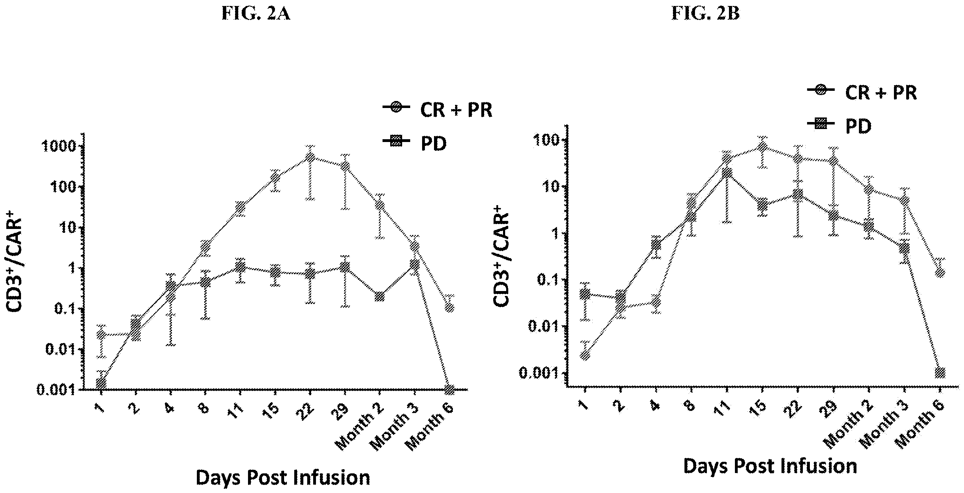

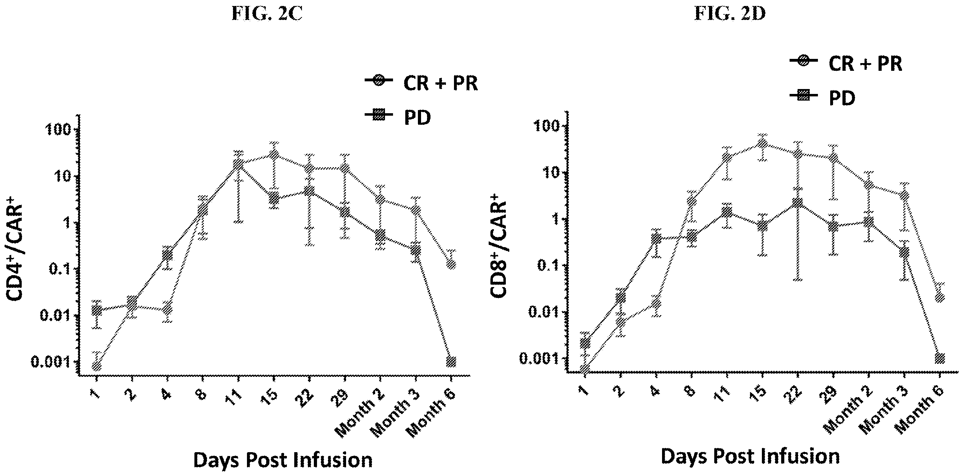

The number of CAR⁺ T cells in peripheral blood was determined at certain time points post-treatment by incubating cells with a transgene-specific reagent. The number of CD3⁺/CAR⁺ T cells in peripheral blood measured at certain time points post-infusion is shown for subjects treated with a single dose of DL1 grouped by best overall response in **FIG. 2A****.** Higher peak CD3⁺/CAR⁺ T cells were observed in responders (CR/PR) than PD. **FIGS. 2B-2D** shows CD3⁺/CAR⁺ T cells, CD4⁺/CAR⁺ T, and CD8⁺/CAR⁺ T cell levels (cells/µL blood; mean ± SEM) in subjects who achieved a response, grouped by continued response (CR/PR) or PD at 3 months.

The Cₘₐₓ (CAR⁺ cells/µL blood) and area under the curve (AUC) for responders (CR/PR) and PD were determined and shown in **Table 7.** The results were consistent with a conclusion that durable responses correlated with higher CD3⁺/CAR⁺ T cell levels in the blood, over time and at peak expansion.

| **Table 7. Cₘₐₓ and AUC₀₋₂₈ Higher in Patients with CR/PR vs PD** | | | | | | |
|---|---|---|---|---|---|---|
| | **CD3** | | **CD4** | | **CD8** | |
| | **CR/PR (n=16)** | **PD (n=4)** | **CR/PR (n=16)** | **PD (n=4)** | **CR/PR (n=16)** | **PD (n=4)** |
| **Cₘₐₓ (CAR⁺ cells/µL blood)** | | | | | | |
| Mean (SD) | 612 (1919) | 2 (1) | 220 (754) | 1 (0.6) | 426 (1314) | 0.5 (0.5) |
| Median (Min, Max) | 33 (1, 7726) | 1 (1, 3) | 8 (1, 3040) | 1 (0, 2) | 4 (0, 5238) | 0.3 (0, 1) |
| Q1, Q3 | 7, 123 | 0.7, 2 | 2, 46 | 0.6, 2 | 0.8, 104 | 0.1, 0.9 |

| **AUC₀₋₂₈** | | | | | | |
|---|---|---|---|---|---|---|
| Mean (SD) | 5883 (18821) | 16 (13) | 2369 (8388) | 10 (7) | 3873 (11963) | 6 (6) |
| Median (Min, Max) | 196 (11, 75773) | 14 (4, 31) | 47 (7, 33740) | 9 (3, 17) | 23 (1, 47834) | 4 (1, 14) |
| Q1, Q3 | 52, 781 | 5, 26 | 16, 261 | 4, 16 | 4, 761 | 1, 10 |

| | | | | | | |
|---|---|---|---|---|---|---|
| **AUC₀₋₂₈** = numbers per microliter for the indicated CAR+ cell population between days 0 and 28 | | | | | | |

### Example 3: Administration of Anti-CD19 CAR-Expressing Cells to Subjects with Relapsed and Refractory Non-Hodgkin's Lymphoma (NHL)

### A. Subjects and Treatment

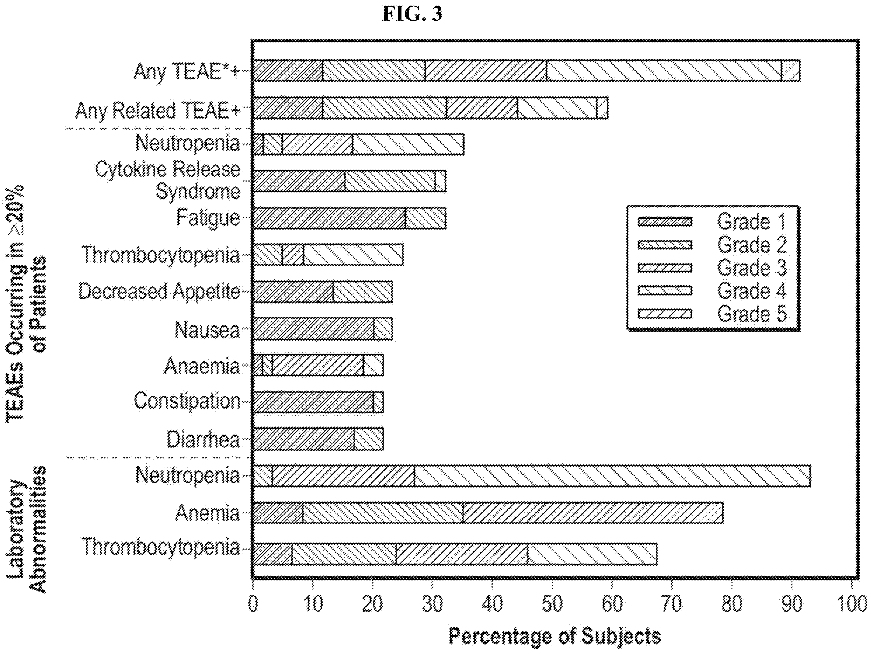

Therapeutic CAR+ T cell compositions containing autologous T cells expressing a chimeric antigen-receptor (CAR) specific for CD19 were administered to subjects with B cell malignancies. Results are described in this example for evaluation through a particular time-point in an ongoing study for cohort (full cohort) of fifty-five (55) adult human subjects with relapsed or refractory (R/R) aggressive non-Hodgkin's lymphoma (NHL), including diffuse large B-cell lymphoma (DLBCL), de novo or transformed from indolent lymphoma (NOS), primary mediastinal large b-cell lymphoma (PMBCL), and follicular lymphoma grade 3b (FLG3B) after failure of 2 lines of therapy. Among the subjects treated were those having Eastern Cooperative Oncology Group (ECOG) scores of between 0 and 2 (median follow-up 3.2 months). The 55 subjects did not include subjects with mantle cell lymphoma (MCL). No subjects were excluded based on prior allogenic stem cell transplantation (SCT) and there was no minimum absolute lymphocyte count (ALC) for apheresis required.

Outcomes at this time-point for a core subset of the 55 subjects (the subset excluding those subjects with a poor performance status (ECOG 2), DLBCL transformed from marginal zone lymphomas (MZL) and/or chronic lymphocytic leukemia (CLL, Richter's) (core cohort)) were separately assessed.

The demographics and baseline characteristics of the full and core cohort are set forth in **Table 8.**

| **Table 8. Demographics and Baseline Characteristics** | | | |
|---|---|---|---|
| **Characteristic** | | **FULL N=55** | **CORE N=44** |
| Median Age, years (range) | | 61 (29-82) | 61 (29-82) |
| | ≥ 65 years, n (%) | 22 (40) | 17 (39) |
| Male/Female, n (%) | | 38/17 (69/31) | 28/16 (64/36) |
| Months from diagnosis, median (range) | | 17 (3-259) | 20 (8-259) |

| B-NHL Subtype, n (%) | | | |
|---|---|---|---|
| DLBCL, NOS | | 40 (73) | 35 (80) |
| Transformed DLBCL | | 14 (26) | 8 (18) |
| Follicular, Grade 3B | | 1 (2) | 1 (2) |

| Molecular Subtype, n (%) | | | |
|---|---|---|---|
| Double/triple hit | | 15 (27) | 12 (27) |
| Double expressor | | 6 (11) | 4 (9) |

| Patient Characteristics, n (%) | | | |
|---|---|---|---|
| Chemorefractory^{†} | | 42 (76) | 34 (77) |
| ECOG 0-1 | | 48 (87) | 44 (100) |
| ECOG 2 | | 7 (13) | 0 |
| Prior lines of therapy, median (range) | | 3 (1-11) | 3 (1-8) |
| | < 5 lines of therapy | 44 (80) | 37 (84) |
| Any HSCT | | 27 (49) | 22 (50) |
| | Allogeneic | 4 (7) | 3 (7) |
| | Autologous | 24 (44) | 20 (45) |

| | | | |
|---|---|---|---|
| ^{∗}SD or PD to last chemo-containing regimen or relapse <12 months after autologous SCT | | | |

The therapeutic T cell compositions administered had been generated by a process including immunoaffinity-based enrichment of CD4+ and CD8+ cells from leukapheresis samples from the individual subjects to be treated. Isolated CD4+ and CD8+ T cells were activated and transduced with a viral vector encoding an anti-CD 19 CAR, followed by expansion and cryopreservation of the engineered cell populations. The CAR contained an anti-CD19 scFv derived from a murine antibody, an immunoglobulin-derived spacer, a transmembrane domain derived from CD28, a costimulatory region derived from 4-1BB, and a CD3-zeta intracellular signaling domain.

The cryopreserved cell compositions were thawed prior to intravenous administration. The therapeutic T cell dose was administered as a defined cell composition by admininstering a formulated CD4+ CAR+ cell population and a formulated CD8+ CAR+ population administered at a target ratio of approximately 1:1. Subjects were administered a single or double dose of CAR-expressing T cells ( each single dose via separate infusions of CD4+ CAR-expressing T cells and CD8+ CAR-expressing T cells, respectively) as follows: a single dose of dose level 1 (DL1) containing 5 × 10⁷ total CAR-expressing T cells (n=30), a double dose of DL1 in which each dose was administered approximately fourteen (14) days part (n=6, including one subject that inadvertently received two DL2 doses via the two-dose schedule, due to a dosing error), or a single dose of dose level 2 (DL2) containing 1 × 10⁸ (DL2) total CAR-expressing T cells (n=18). Beginning at three (3) days prior to CAR+ T cell infusion, subjects received a lymphodepleting chemotherapy with flurabine (flu, 30 mg/m² ) and cyclophosphamide (Cy, 300mg/m²).

### B. Safety

The presence or absence of treatment-emergent adverse events (TEAE) of the CAR-T cell therapy was assessed. **FIG. 3** depicts the percentage of subjects who were observed to have experienced laboratory abnormalities and TEAEs, which occurred in ≥20% of subjects. In addition to the TEAEs shown in **FIG. 3****,** the following event terms were observed at Grade 3-4 in ≥5% of patients: white blood cell count decreased (13.6%), encephalopathy (12%), hypertension (7%). Degree of toxicities observed were consistent between dose levels 1 and 2.

Subjects also were assessed and monitored for neurotoxicity (neurological complications including symptoms of confusion, aphasia, encephalophathy, myoclonus seizures, convulsions, lethargy, and/or altered mental status), graded on a 1-5 scale, according to the National Cancer Institute-Common Toxicity Criteria (CTCAE) scale, version 4.03 (NCI-CTCAE v4.03). Common Toxicity Criteria (CTCAE) scale, version 4.03 (NCI-CTCAE v4.03). See Common Terminology for Adverse Events (CTCAE) Version 4, U.S. Department of Health and Human Services, Published: May 28, 2009 (v4.03: June 14, 2010); and Guido Cavaletti & Paola Marmiroli Nature Reviews Neurology 6, 657-666 (December 2010). Cytokine release syndrome (CRS) also was determined and monitored, graded based on severity.

In 84% of the full cohort subjects, severe (grade 3 or higher) cytokine release syndrome (CRS) and severe neurotoxicity were not observed. Additionally, it was observed that 60% of the full cohort subjects did not develop any grade of CRS or neurotoxicity. No differences in incidence of CRS, neurotoxicity (NT), sCRS, or severe neurotoxicity (sNT) were observed between dose levels. **Table 9** summarizes the incidence of cytokine release syndrome (CRS) and neurotoxicity adverse events in patients 28 days after receiving at least one dose of CAR-T cells. As shown in **Table 9,** no sCRS (Grade 3-4) was observed in any subjects that received a single dose of DL2 or double dose of DL1. Severe neurotoxicity or severe CRS (grade 3-4) was observed in 16% (9/55) of the full cohort of subjects and in 18% (8/44) of the subjects in the core subset. 11% (n=6) of subjects received tocilizumab, 24% (n=13) of subjects received dexamethasone. Among the ECOG2 subjects within the full cohort, observed rates of CRS and neurotoxicity were 71% and 29%, respectively.

| **Table 9. Assessment of Presence or Absence of CRS and Neurotoxicity Adverse Events** | | | | | | |
|---|---|---|---|---|---|---|
| | **FULL** | | | | **CORE** | |
| | **All Dose Levels** | **DL1S** | **DL2S** | **DL1D^{†}** | | |
| Safety, N | 55 | 30 | 19 | 6 | 44 | |
| sCRS or sNT, n (%) | | 9 (16) | 6 (20) | 2 (11) | 1 (17) | 8 (18) |
| | CRS or NT, n (%) | 22 (40) | 12 (40) | 7 (37) | 3 (50) | 15 (34) |

| CRS | | | | | | |
|---|---|---|---|---|---|---|
| | Grade 1-2, n (%) | 18 (33) | 10 (33) | 5 (26) | 3 (50) | 12 (27) |
| | Grade 3-4, n (%) | 1 (2) | 1 (3) | 0 | 0 | 1 (2) |

| Neurotoxicity | | | | | | |
|---|---|---|---|---|---|---|
| | Grade 1-2, n (%) | 3 (6) | 1 (3) | 2 (11) | 0 | 2 (5) |
| | Grade 3-4, n (%) | 9 (16) | 6 (20) | 2 (11) | 1 (17) | 8 (18) |

| | | | | | | |
|---|---|---|---|---|---|---|
| Includes one patient treated at DL2 2-dose schedule due to dosing error | | | | | | |

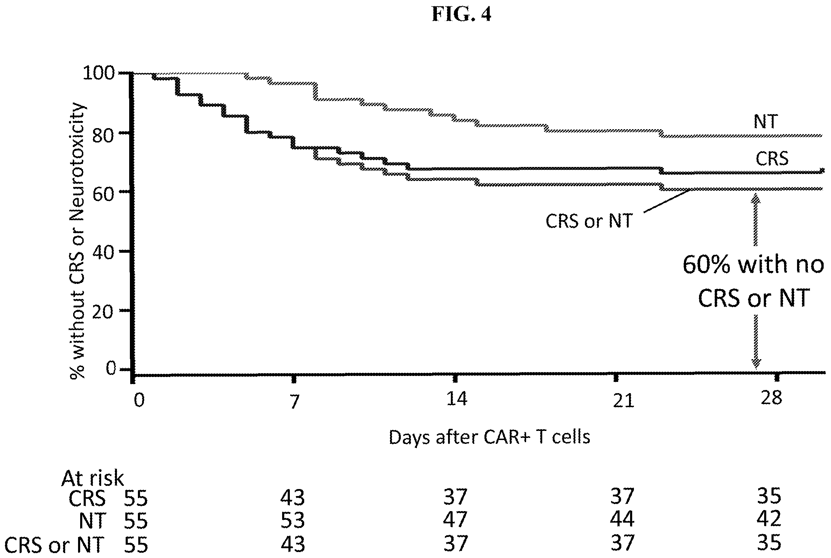

**FIG. 4** shows a Kaplan meier curve depicting observed time to onset of CRS and/or neurotoxicity. As shown, the observed median times to onset of CRS and to onset of neurotoxicity were 5 and 11 days, respectively, with only 11% of patients experiencing onset of CRS less than 72 hours after initiation of the administration of the cell therapy. The median time to resolution of CRS and neurotoxicity to Grade 1 or better was 5 and 7 days, respectively. The median time to complete resolution of CRS and neurotoxicity was 5 and 11 days, respectively. The results were consistent with a conclusion that there was a low rate of early onset of any CRS or neurotoxicity in the subjects.

### C. Response to Treatment

Subjects were monitored for response, including by assessing tumor burden at 1, 3, 6, 7, 12, 18, and 24 months after administration of the CAR+ T cells. Response rates are listed in **Table 10.** High durable response rates were observed in the cohort of subjects, which included subjects heavily pretreated or, with poor prognosis and/or with relapsed or refractory disease. For subjects across all doses in the Core (n=44) cohort, the observed overall response rate (ORR) was 86% and the observed complete response (CR) rate was 59%. At three months for the core cohort, the overall response rate (ORR) was 66%; the three-month CR rate was 50% among the core cohort. In the core cohort, the 3 month ORR was 58% (11/19) at dose level 1 and 78% at dose level 2; the 3 month CR rate was 42% (8/19) for dose level 1 and 56% (5/9) for dose level 2, consistent with a suggested dose response effect on treatment outcome. Additionally, the results were consistent with a relationship between dose and durability of response.

| **Table 10. Response** | | | | | | |
|---|---|---|---|---|---|---|
| | | **FULL** | | | | **CORE** |
| | | **All Dose Levels** | **DL1S** | **DL2S** | **DL1D^{c}** | **All Dose Levels** |
| Best Overall a Response, N^{a} | | 54 | 30 | 18 | 6 | 44 |
| | ORR, % (95% CI) | 76 (62, 87) | 80 (61, 92) | 72 (47, 90) | 67 (23, 96) | 86 (73, 95) |
| | CR, % (95% CI) | 52 (38, 66) | 53 (34, 72) | 50 (26, 74) | 50 (12, 88) | 59 (43, 74) |
| ≥ 3 mos f/u, n^{b} | | 41 | 24 | 11 | 6 | 32 |
| | 3 mo ORR, % (95% CI) | 51 (35, 67) | 46 (26, 67) | 64 (31, 89) | 50 (12, 88) | 66 (47, 81) |
| | 3 mo CR, % (95% CI) | 39 (24, 56) | 33 (16, 55) | 46 (17, 77) | 50 (12, 88) | 50 (32, 68) |

| | | | | | | |
|---|---|---|---|---|---|---|
| DL1S: DL1 1-dose schedule; DL2S: DL2 1-dose schedule; DL1D: DL1 2-dose schedule; ^{a}Included patients with event of PD, death, or 28 day restaging scans. Treated patients <28 days prior to data snapshot were not included. ^{b}The denominator is number of patients who received the CAR T-cell therapy ≥ 3 months age, prior date with an efficacy assessment at Month 3 or prior assessment of PD or death. ^{c}Includes one patient treated at DL2 2-dose schedule due to dosing error | | | | | | |

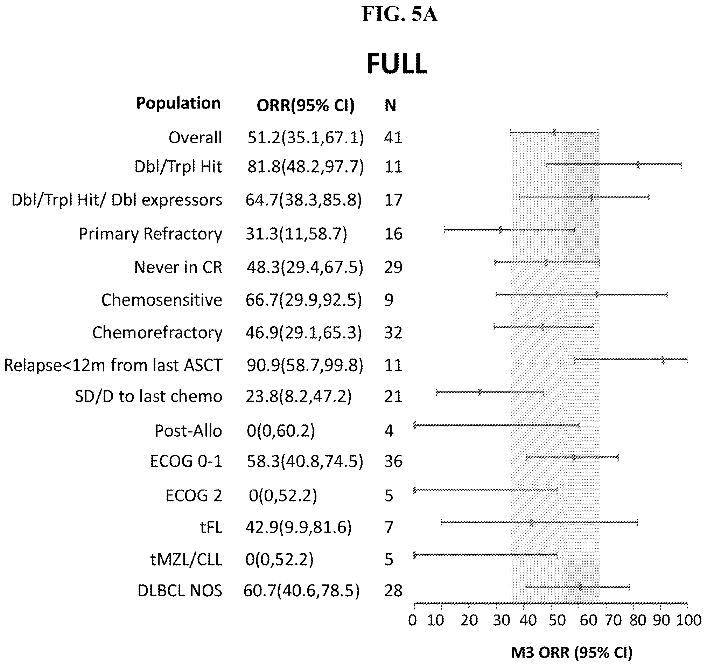

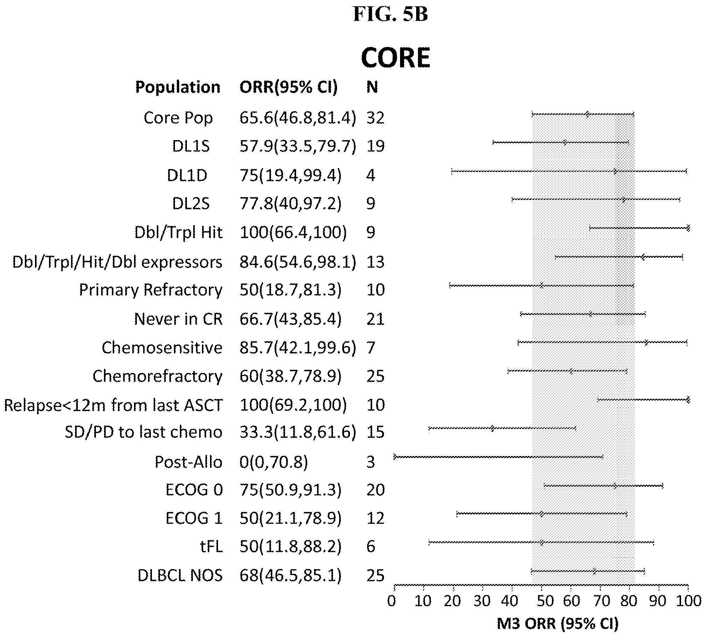

Overall response rates among various subgroups of subjects in the full and core cohorts are shown in **FIGS. 5A** and **5B****,** respectively. In poor-risk DLBCL subgroups, response rates were generally high. An ORR of greater than 50% was observed at 3 months in patients with double/triple hit molecular subtype, that had primary refractory or chemorefractory DLBCL or that never before had achieved a CR. Complete resolution of CNS involvement by lymphoma was observed in 2 patients.

Among the subjects treated six months or greater prior to the particular time-point of the evaluation, of the ten (10) patients that had been in response at three months, 9 (90%) remained in response at six months. At the evaluation time-point, 97 % of subjects in the core subset who had responded were alive and in follow-up, median follow-up time 3.2 months.

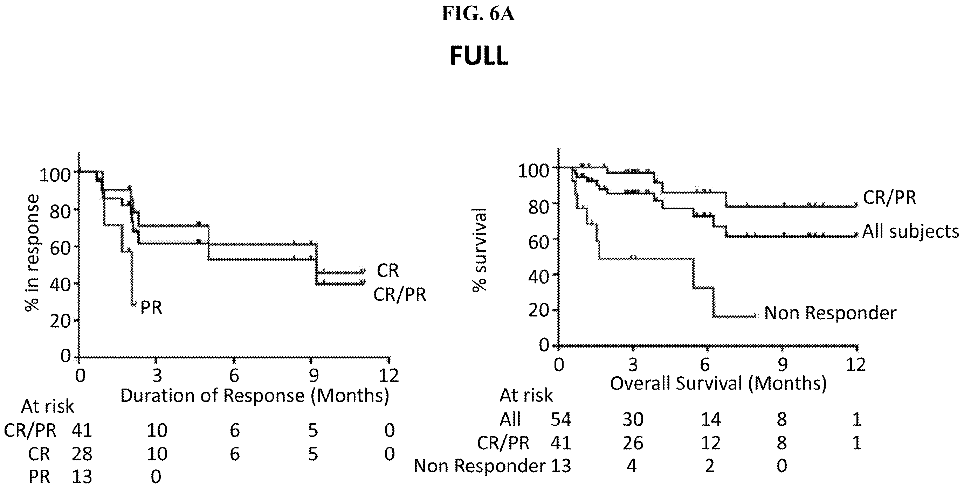

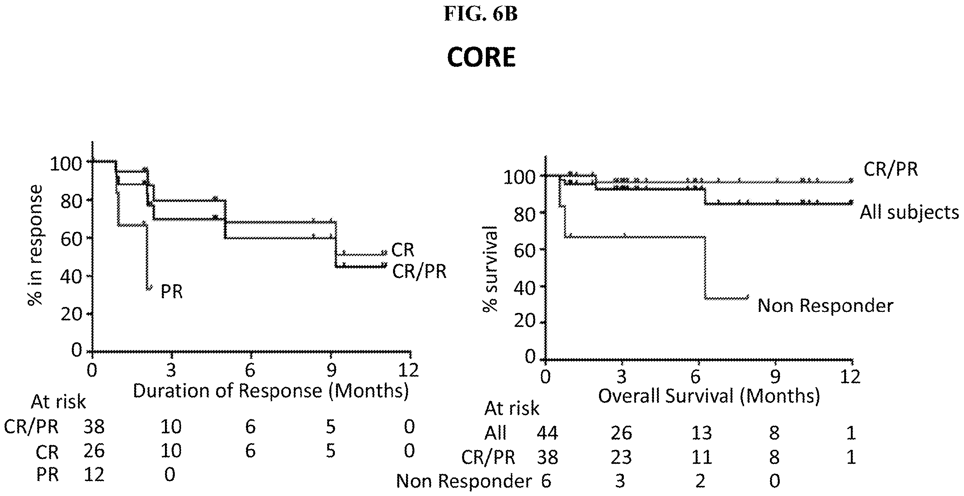

Results forof the duration of response and overall survival (grouped by best overall response (non-responder, CR/PR, CR and/or PR)) are shown for full and core cohorts of subjects, in **FIGS. 6A** and **6B****,** respectively. As shown, prolonged survival was observed in responders, with increased durability of response in subjects with CRs. All patients in response at three months remained alive at the time of evaluation, although 5/6 subjects with poor performance status (ECOG 2) had expired.

### C. Assessment of CAR+ T cells in Blood

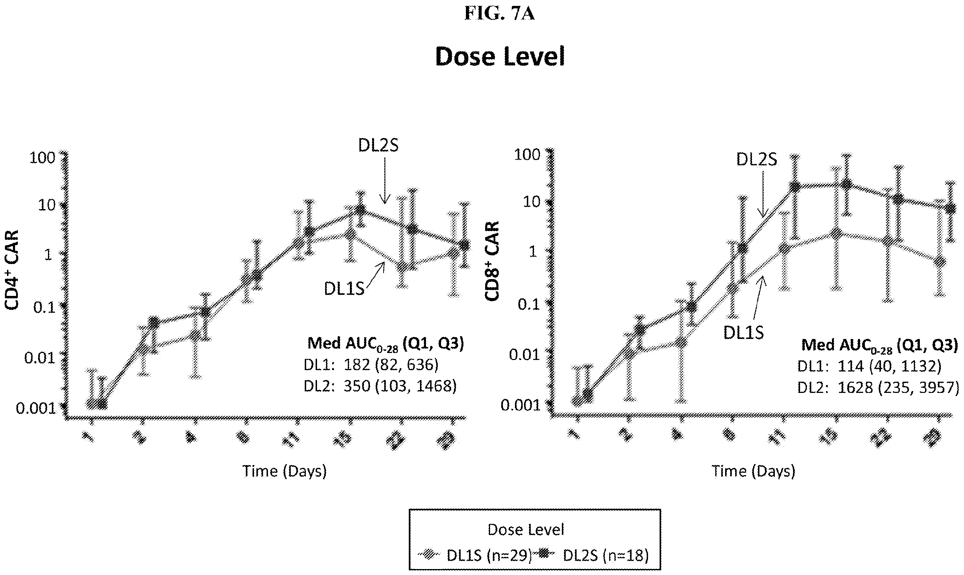

Pharmacokinetic analysis was carried out to assess numbers of CAR⁺ T cells in peripheral blood at various time points post-treatment. As shown in **FIG. 7A****,** CD4+ and CD8+ CAR-expressing cells, as measured by the number of cells/µL blood (median ± quartiles) plotted on a log scale, were detected throughout the course of assessment at both administered dose levels.

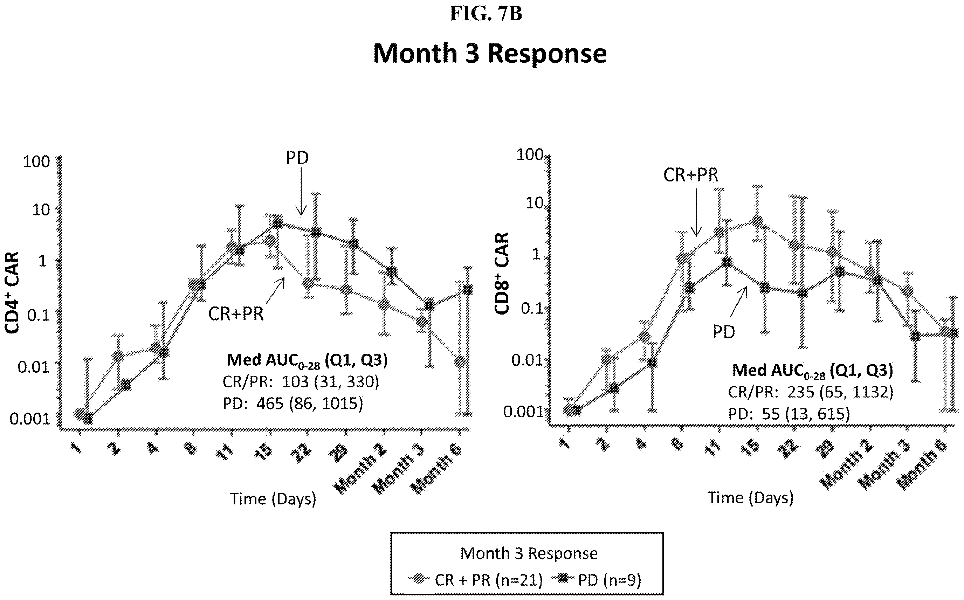

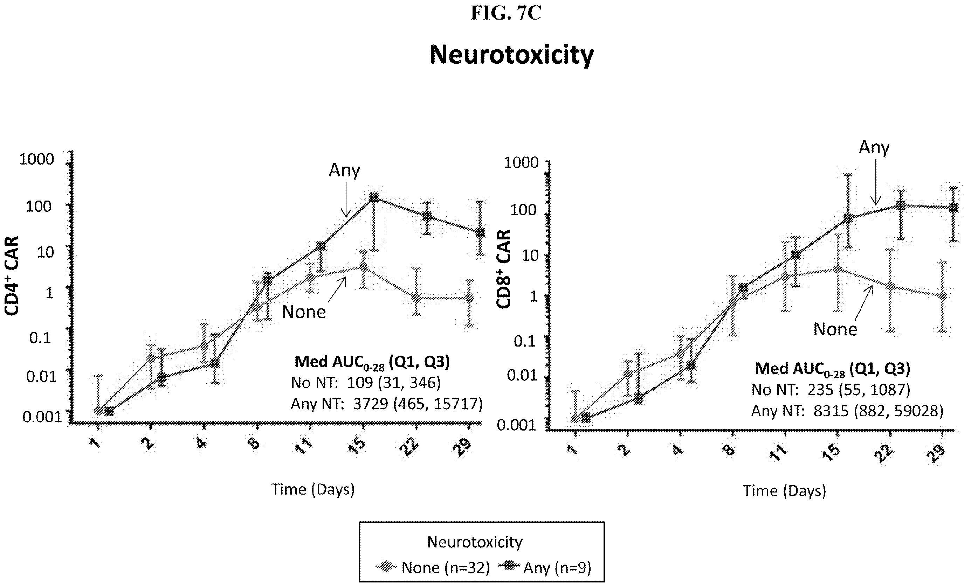

An increased median area under the curve (AUC) (CD8+ CAR+ cell numbers over time in the blood) was observed among subjects administered the higher dose level, as compared to the lower dose level, without an observed increase in toxicity. Higher peak CD8⁺/CAR⁺ T cell exposure was observed in responders (CR/PR) than non-responders (PD) ; persistence of cells over the time of assessment, including out to 3 and 6 months, was observed even in subjects whose disease had progressed (**FIG. 7B**). The results were consistent with a conclusion that treatment resulted in prolonged exposure and persistence of the engineered cells, even in subjects with poor responses. In some instances, combination approaches are used, such as administration of an immune checkpoint modulator or other immune modulatory agent, e.g., following relapse or disease progression, at a time at which engineered cells persist in the subject, e.g., as measured by levels of cells in peripheral blood. In some aspects, the cells, having persisted for a prolonged period, re-expand or become activated and/or exhibit antitumor function, following administration of the other agent or treatment. Higher median CD4+ and CD8+ CAR+ T cell numbers were generally observed over time in blood of subjects who developed neurotoxicity (**FIG. 7C**).

### D. Blood Analytes and Neurotoxicity

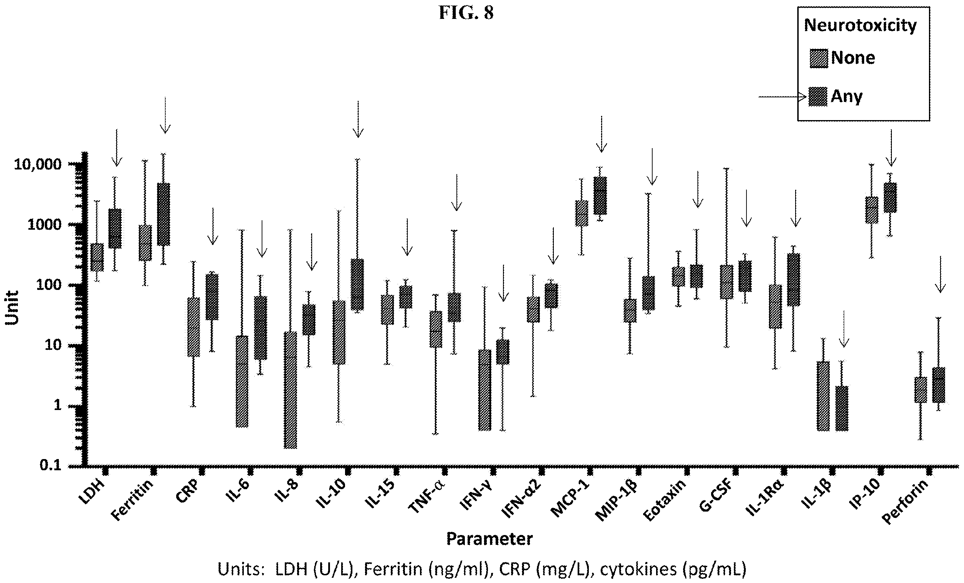

Various pre-treatment blood analytes, including cytokines, were measured in the blood of the subjects prior to administration of the CAR+ T cells. Potential correlations to risk of developing neurotoxicity were assessed using statistical analysis. **FIG. 8** shows median levels of the assessed analytes in units (LDH, U/L; ferritin, ng/mL; CRP, mg/L; cytokines, pg/mL) in subjects that did not develop a neurotoxicity versus subjects that did develop a neurotoxcity following CAR+ T cell therapy. Levels of certain blood analytes, including LDH, Ferritin, CRP, IL-6, IL-8, IL-10, TNF-α, IFN- α2, MCP-1 and MIP-1β, were observed to be associated with level of risk of developing neurotoxicity (Wilcoxon p values <0.05,without multiplicity adjustment). In particular, the results were consistent with a conclusion that pre-treatment levels of LDH, which in some instances is a surrogate for disease burden, may be useful for potential neurotoxicity risk assessment and/or risk-adapted dosing or adjustment of treatment of certain subjects. In addition, tumor burden measured before administration of the CAR-T cell composition correlated (Spearman p values <0.05) with the risk of developing neurotoxicity. In some aspects, LDH levels may be assessed alone and/or in combination with another pre-treatment parameter, such as another measure or indicator of disease burden, such as a volumetric tumor measurement such as sum of product dimensions (SPD) or other CT-based or MRI-based volumetric measurement of disease burden. In some aspects, one or more parameters indicative of disease burden are assessed, and in some contexts may indicate the presence, absence or degree of risk of developing neurotoxicity following the T cell therapy. In some aspects, the one or more parameters include LDH and/or a volumetric tumor measurement.

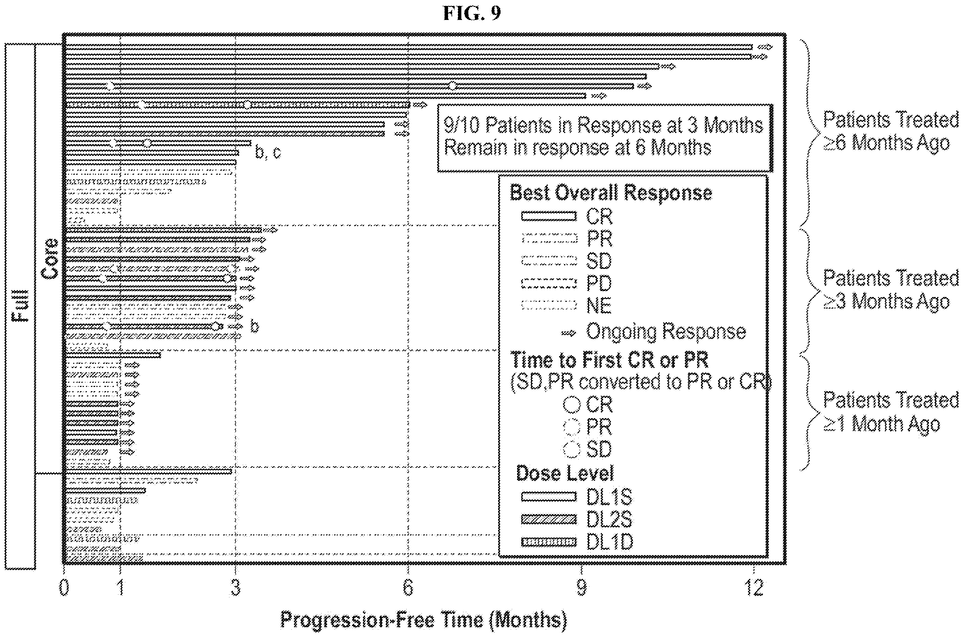

**FIG. 9** shows a graph plotting progression-free time (months) for individual subjects within the full and core cohorts. Each bar represents a single patient. Shading indicates best overall response (in each case, unless otherwise indicated, achieved at 1 month); texture indicates dose (solid=dose level 1 (DL1), single dose; cross-hatched, dose level 2 (DL2), single dose; vertical hatched=dose level 1 (DL1), two-dose). Horizontal arrows indicate an ongoing response. Certain individual subjects were initially assessed (e.g., at 1-month) as exhibiting stable disease (SD) or Partial Response (PR), and were later observed to have achieved a PR (e.g., conversion of SD to PR) or CR. In such cases, shading of the individual patient bar, as noted, indicates best overall response, and dots (same correspondence of shading to response achieved) along each individual subject bar, indicate when each SD, PR, and/or CR was observed to have occurred in the subject. Complete resolution of CNS involvement by lymphoma was observed in two patients. CAR+ cells in one subject were observed to have expanded following biopsy after relapse.

### Example 4: Administration of anti-CD19 CAR-Expressing Cells to Subjects with Mantle Cell Lymphoma (MCL)

Therapeutic CAR+ T cell compositions containing autologous T cells expressing a chimeric antigen-receptor (CAR) specific for CD19, generated as described in Example 1, were administered to four (4) human subjects with mantle cell lymphoma (MCL) that had failed 1 line of therapy. The cryopreserved cell compositions were thawed prior to intravenous administration. The therapeutic T cell composition was administered as a defined composition cell product with formulated CD4+ and CD8+ populations of CAR+ engineered T cells derived from the same subject administered at a target ratio of approximately 1:1. Subjects were administered a dose of CAR-expressing T cells (as a split dose of the CD4+ and CD8+ CAR-expressing T cells) at a single dose of dose level 1 (DL1) containing 5 × 10⁷ CAR-expressing T cells. Beginning at three (3) days prior to CAR+ T cell infusion, subjects received a lymphodepleting chemotherapy with flurabine (flu, 30 mg/m²) and cyclophosphamide (Cy, 300mg/m²).

Subjects were monitored for response and toxicities as described in Example 1. No CRS or neurotoxicity was observed in any of the subjects. Of the 4 subjects that were treated, two (2) subjects achieved PR (not durable) and two (2) patients had progressive disease.

### Example 5: Further Assessment of Pharmacokinetics, Pharmacodynamics and Blood Analytes in Subjects with Relapsed and Refractory Non-Hodgkin's Lymphoma (NHL) After Administration of Anti-CD19 CAR-Expressing Cells

Pharmacokinetic and pharmacodynamics parameters and blood analytes were assessed in patients at a subsequent point in time in the clinical study described in Example 3 above.

### A. Subjects, Response and Safety

The analysis at this time point presented in this example is based on assessment of a total of 91 subjects in the full DLBCL cohort (88 (34 from the CORE cohort) assessed for response and 91 assessed for safety) that had been administered the anti-CD 19 CAR-expressing cells. As shown in **Table 11.** The objective response rate (ORR) was 74%, including 52% subjects who showed a complete response (CR). The incidence of any grade of cytokine release syndrome (CRS) was 35%, with 1% severe CRS; and the incidence of any grade of neurotoxicity (NT) was 19%, with 1% severe NT.

| **Table 11. Response and Safety After CAR+ Cell Administration** | | | | |
|---|---|---|---|---|
| | **FULL** | **CORE** | | |
| | **All Dose Levels** | **All Dose Levels^{a}** | **DL1S** | **DL2S** |
| **Best Overall Response (BOR), n^{b}** | 88 | 65 | 34 | 27 |
| ORR, % (95% CI) | 74 (63,83) | 80(68, 89) | 77 (59,89) | 82 (62, 94) |
| CR, % (95% CI) | 52 (41,63) | 55(43, 68) | 47 (30, 65) | 63 (42, 81) |
| **Safety, n^{c}** | 91 | 67 | 34 | 29 |
| Any CRS, % (95% CI) | 35 (25, 46) | 36 (24, 48) | 41 (25, 59) | 24 (10, 44) |
| sCRS(grade 3-4), % (95% CI) | 1 (0, 6) | 1 (0, 8) | 38 (0, 15) | 0 |
| Any NTx, % (95% CI) | 19 (11,28) | 21 (12,33) | 24 (11, 41) | 17 (6, 36) |
| sNTx(grade 3-4), % (95% CI) | 12 (6, 21) | 15 (7, 26) | 21 (9, 38) | 7 (1, 23) |

| | | | | |
|---|---|---|---|---|
| a four patients treated on DL1D (dose level 1, two-dose schedule) with similar outcomes. b Includes patients with event of PD, death, or 28-day restaging scans. One patient did not have restaging scans available. c Includes all subjects who have received at least one dose of conforming CAR-expressing cell product 28 days prior to data snapshot date or died. | | | | |

### B. Pharmacokinetic Assessment

Numbers of CAR⁺ T cells in peripheral blood and bone marrow at time points before administration (pre-treatment or pre-lymphodepleting chemotherapy (LDC)) and various time points post-treatment (with day of administration as day 1) in 86 subjects in the DLBCL cohort with evaluable PK, by flow cytometry using an antibody specific for the truncated receptor used as a surrogate marker, and quantitative polymerase chain reaction (qPCR) using primers specific for a woodchuck hepatitis virus post-transcriptional regulatory element (WPRE) present in the vector encoding the chimeric antigen receptor (CAR). The area under the curve plotting numbers per microliter for the indicated CAR+ cell population between days 0 and 28 (AUC₀₋₂₈) and the maximum or peak blood concentration of CAR+ cells (Cₘₐₓ; CAR⁺ cells/µL blood) were assessed. B-cell aplasia was assessed in peripheral blood by flow cytometry, by staining with CD19. Cytokines were measured using a multiplex cytokine assay. For safety analysis, the data from all subjects receiving different dose levels were pooled. For response analysis, data were stratified by dose level. Statistical analysis was two-sided without multiplicity adjustment.

**FIG. 10A** shows detected numbers of CAR T cells per microliter of blood at various indicated time-points, as assessed by qPCR or qPCR. **FIG 10B** shows CAR+ cells per microliter of blood versus microliter of bone marrow at day 11 + 3. As shown in **FIG. 10A****,** levels of CAR-expressing cells in samples from subjects were observed both by flow cytometry-based assays and qPCR-based assays. As shown in **FIG. 10B****,** all subjects (n= 87 and 85 for flow cytometry and qPCR, respectively) with PK results assessed, showed detectable numbers of the CAR-expressing cells in the blood and bone marrow. Results were consistent with an observation that CAR+ T cells had trafficked similarly to the bone marrow and blood.

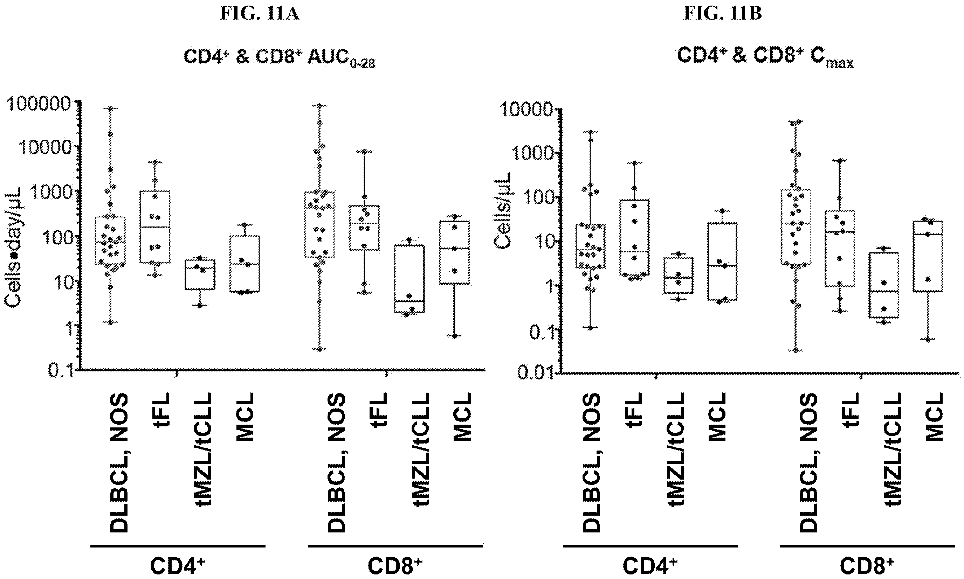

Levels over time of CD4+ and CD8+ CAR-expressing cells (as assessed by AUC₀₋₂₈ and Cₘₐₓ) were compared in different patient subgroups: diffuse large B-cell lymphoma de novo or transformed from follicular lymphoma (DLBCL, NOS; N=27), transformed follicular lymphoma (tFL; N=10), DLBCL transformed from marginal zone lymphoma or chronic lymphocytic leukemia (tMZL/tCLL; N=4), or mantle cell lymphoma (MCL; N-5), who had received CAR-expressing T cells at DL1. As shown in **FIGS. 11A** and **11B****,** AUC₀₋₂₈ and C_{max,}varied among subjects in different disease subgroups, with expansion of CD4+and CD8+ CAR-expressing cells trending lower in non-CORE subsets.

### C. Pharmacokinetic Assessment by Dose Level

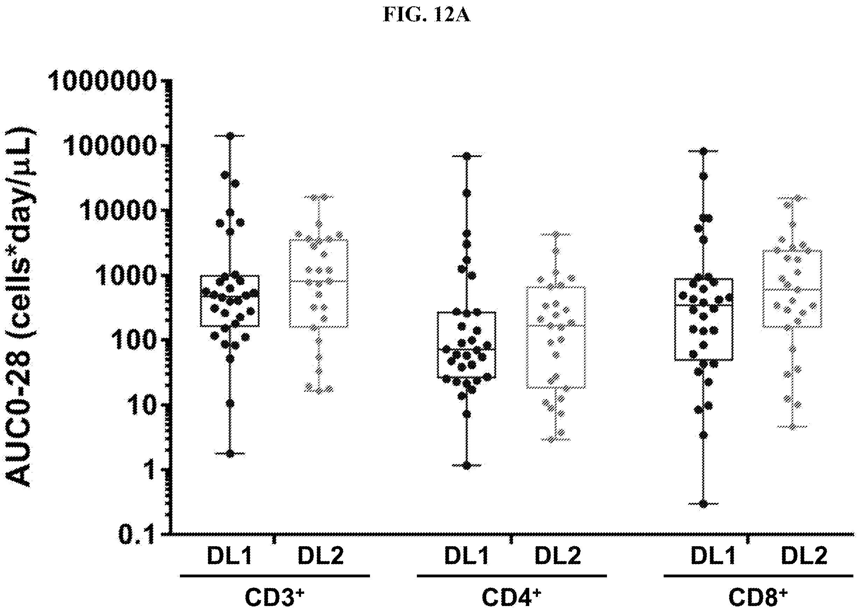

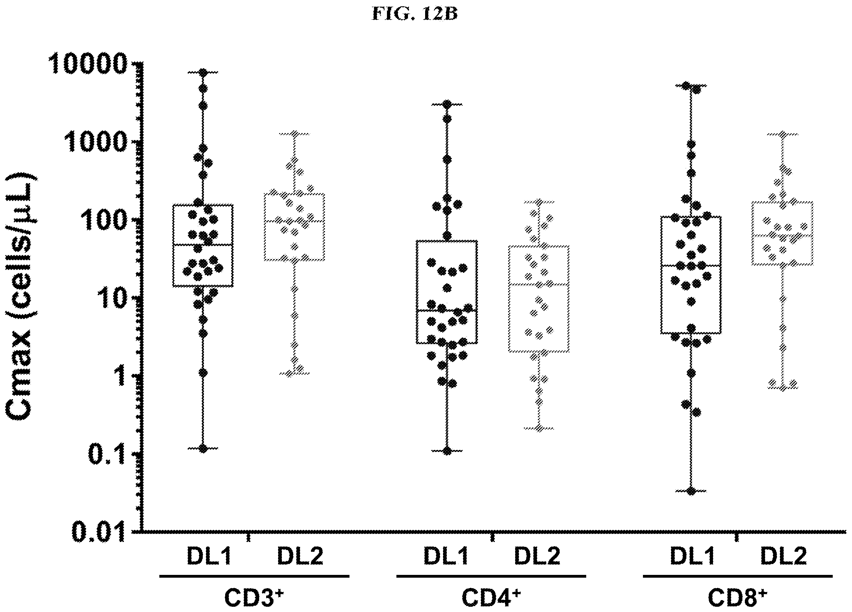

AUC₀₋₂₈ and Cₘₐₓ for CD3+, CD4+ and CD8+ CAR-expressing cells were also compared for subjects having received dose level 1 (DL1) and those having received dose level 2 (DL2), in the CORE cohort (subjects with DLBCL, NOS or high grade B-cell lymphoma (double/triple hit); N=65). As shown in **FIGS. 12A** and **12B** and in **Table 12,** a higher median AUC₀₋₂₈ was observed for CD3+, CD4+ and CD8+ CAR-expressing cells was observed in subjects that received DL2, compared to subjects who had received DL1. Similarly, a trend of higher expansion in subjects who had received DL2 was observed in the full DLBCL cohort. A higher durability of response (DOR) at 3 months also was observed among subjects who had received DL2 as compared to those having received DL1, without an increase in toxicity. The median time to Cₘₐₓ (Tₘₐₓ) for CD4+ and CD8+ CAR+ cells was similar between subjects who received DL1 and DL2.

Increased CAR+ T cell exposure was observed in DL2 versus DL1, corresponding to an increased durability of response without increased toxicity in DL2 subjects.

| **Table 12. Pharmacokinetics in Subjects Grouped by Dose Levels in Core cohort** | | | |
|---|---|---|---|
| | **DL1S (n = 32)** | **DL2S (n = 27)** | **Total, DL1S and DL2S (n = 59)** |
| **CD3⁺** | | | |
| **Cₘₐₓ, median (cells/µL)** | 48.2 | 96.2 | 65.8 |
| **Q1,** Q3 | 15.6, 151.3 | 302, 219.5 | 19.0, 204.2 |
| Min, max | 0.1, 7726.3 | 1.1, 1280.9 | 0.1, 7726.3 |
| **Tₘₐₓ, median (days)** | 14.5 | 15.0 | 15.0 |
| Q1, Q3 | 11, 15 | 11, 15 | 11, 15 |
| Min, max | 9, 24 | 8, 31 | 8, 31 |
| **AUC₀₋₂₈, median (cells*day/µL)** | 477.7 | 823.1 | 542.4 |
| Q1, Q3 | 165.9, 999.3 | 155.8, 3628.3 | 155.8, 3381.9 |
| Min, max | 1.8, 142816.7 | 16.5, 16087.8 | 1.8, 142816.7 |

| **CD4⁺** | | | |
|---|---|---|---|
| **Cₘₐₓ, median (cells/µL)** | 7.0 | 14.9 | 7.7 |
| Q1, Q3 | 2.6, 46.0 | 2.0,46.8 | 2.5, 46.8 |
| Min, max | 0.1, 3039.9 | 0.2, 169.4 | 0.1, 3039.9 |
| **Tₘₐₓ, median (days)** | 14.0 | 15.0 | 15.0 |
| Q1, Q3 | 11, 15 | 11, 15 | 11, 15 |
| Min, max | 8, 24 | 8, 31 | 8, 31 |
| **AUC₀₋₂₈, median (cells*day/µL)** | 71.1 | 166.1 | 91.5 |
| Q1, Q3 | 26.4, 274.7 | 18.1,679.0 | 23.9,368.8 |
| Min, max | 1.2, 68990.3 | 2.9, 4266.8 | 1.2, 68990.3 |

| **CD8⁺** | | | |
|---|---|---|---|
| **Cₘₐₓ, median (cells/µL)** | 26.1 | 62.8 | 43.6 |
| Q1, Q3 | 3.7, 111.2 | 26.2,171.7 | 9.1,151.6 |
| Min, max | 0.0, 5237.6 | 0.7,1261.8 | 0.0, 5237.6 |
| **Tₘₐₓ, median (days)** | 15.0 | 15.0 | 15.0 |
| Q1, Q3 | 11, 16 | 11, 17 | 11, 16 |
| Min, max | 4, 28 | 8, 31 | 4,31 |
| **AUC₀₋₂₈, median (cells*day/µL)** | 347.2 | 606.6 | 412.2 |
| Q1, Q3 | 52.1,871.4 | 155.7,2463.4 | 72.1,1852.5 |
| Min, max | 0.3,81865.9 | 4.7, 15570.0 | 0.3, 81865.9 |

### D. Persistence

Persistence of CAR-expressing cells and CD19+ B cell aplasia (low numbers or absence of CD19+ B cells) was assessed at various time points in evaluable subjects with DLBCL that had been administered CAR+ T cells, based on detectable CD3⁺, CD4⁺ or CD8⁺ CAR-expressing cell levels and levels of CD19⁺ B-cells detected in the blood, respectively. The results are set forth in **Table 13.** Among subjects evaluated at progression (n=37), a median of 0.17 CD4+ CAR+ cells/µL (range, 0-65.5 cells/µL) -expressing cell at progression was and a median of CD8+ CAR+ 0.15 cells/µL (range, 0-131.8 cells/µL) were observed at progression. Among subjects evaluated at relapse (progression after achieving CR/PR) (n=12), a median of 0.17/µL (range, 0-35.1 cells/µL) CD4+ CAR-expressing cells and a median of 0.20 cells/µL (range, 0-131.8 cells/µL). CD8+ CAR-expressing cells were observed at relapse Long-term persistence of CAR-expressing cells was observed in 75% of evaluable subjects with DLBCL at 12 months. Long-term persistence of B cell aplasia also was observed in 75% of the subjects at 12 months, and in subjects regardless of relapse status. The results are consistent with a conclusion that the anti-CD19 CAR-expressing cells exhibited long-term persistence in most subjects, and suggest the potential for ongoing, low-level disease control even in relapsed patients.

Of subjects who relapsed, 91.7% (11/12) had detectable CAR-expressing cells in the blood at the time of relapse. This result is consistent with a conclusion that a combination therapy or other intervention in some instances may be used to augment and/or boost CAR-expressing cells such as those that may be exhausted.

| **Table 13. CAR+ Cell Long-Term Persistence and CD19 Aplasia** | | | | | | |
|---|---|---|---|---|---|---|
| | **Month 3** | **Month 6** | **Month 9** | **Month 12** | **At Progression** | **At Relapse** |
| CAR T persistence in evaluable patients, n | 50 | 30 | 18 | 12 | 37 | 12 |
| CD3⁺ % | 100 | 80.0 | 77.8 | 75.0 | 91.9 | 91.7 |
| CD4⁺, % | 88.0 | 63.3 | 50.0 | 41.7 | 83.8 | 83.3 |
| CD8⁺, % | 90.0 | 70.0 | 55.6 | 50.0 | 83.8 | 75.0 |
| CD19⁺ B-cell aplasia (< 1 cell/µL), % | 96.0 | 93.3 | 77.8 | 75.0 | 97.3 | 100 |

### E. Pharmacokinetic Assessment and Toxicity

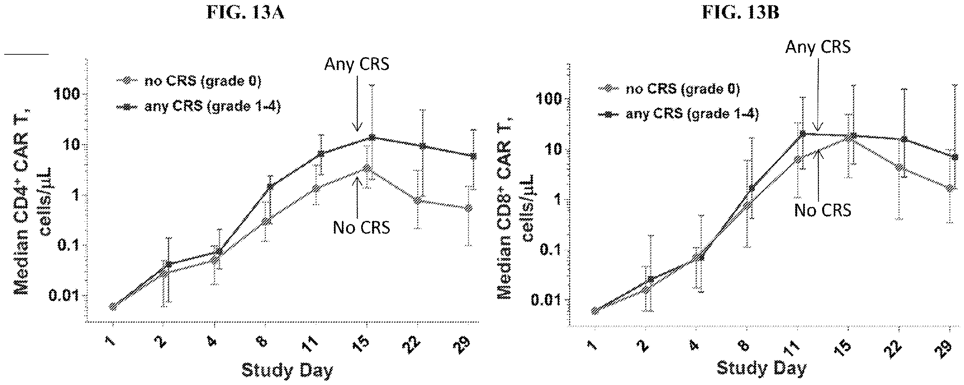

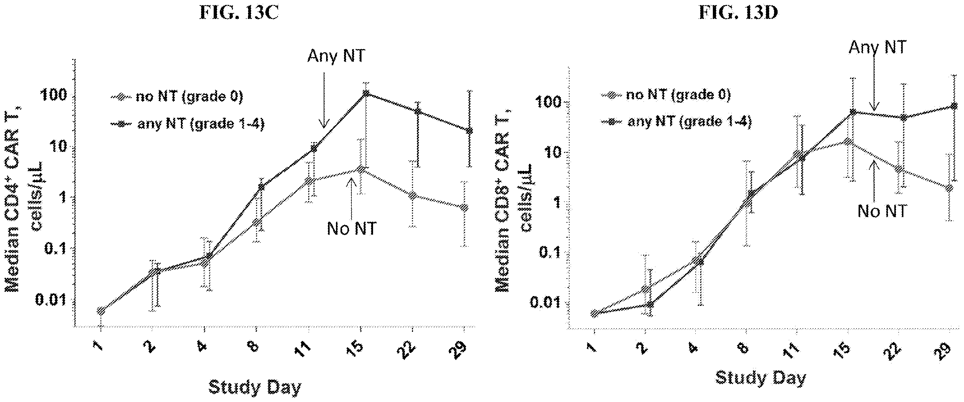

AUC₀₋₂₈ and Cₘₐₓ of CD4⁺ and CD8⁺ CAR-expressing cells was also compared for subjects with any grade (in this assessment, any of grade 1-4; no grade 5 CRS or NT observed) cytokine release syndrome (CRS) or neurotoxicity (NT) to subjects that were not assessed as exhibiting any grade of CRS or NT. The median CD4⁺ CAR⁺ AUC₀₋₂₈ (Q1, Q3) was 59 (18, 210) for no CRS (grade 0), and 267 (91, 1510) for any CRS (grades 1-4) (p = 0.001); the median CD8⁺ CAR⁺ AUC₀₋₂₈ (Q1, Q3) was 310 (36, 900) for no CRS (grade 0), and 605 (174, 5619) for any CRS (grades 1-4) (p = 0.021); the median CD4⁺ CAR⁺ AUC₀₋₂₈ (Q1, Q3) was 71 (23, 244) for no NT (grade 0), and 1269 (184, 3057) for any NT (grades 1-4) (p = 0.003); the median CD8⁺ CAR⁺ AUC₀₋₂₈ (Q1, Q3) was 304 (43, 799) for no NT (grade 0), and 2463 (607, 7691) for any NT (grades 1-4) (p = 0.004). As described above and shown in **FIGS. 13A-13D****,** higher of CD4⁺ and CD8⁺ CAR-expressing cell levels over time were associated with CRS and NT.

### F. Pharmacokinetic Assessment and Response

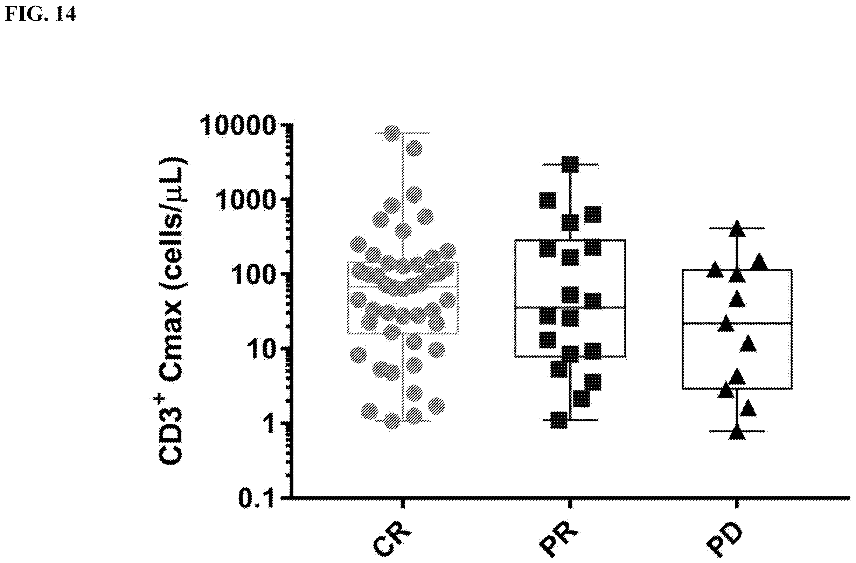

The number of peak CD3⁺ CAR⁺ cells/µL (CD3+ Cₘₐₓ) was assessed over time in subjects who had a best overall response (BOR) of CR, PR or PD. As shown in **FIG. 14****,** a trend towards better BOR was observed in subjects with higher expansion, with variability among subj ects.

### G. Pharmacokinetic Assessment by Blood Analytes and Patient Parameters

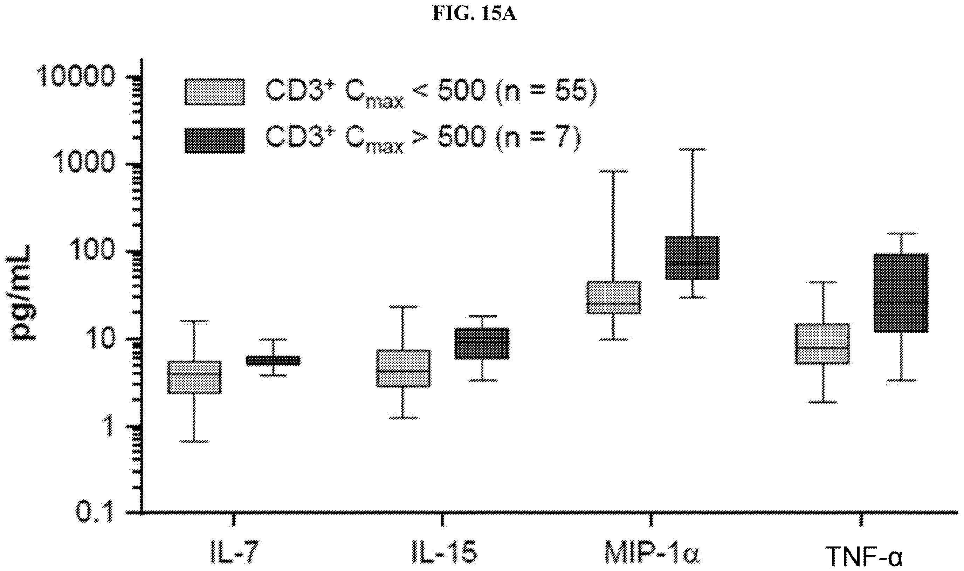

Pre-CAR+ T cell treatment (pre-lymphodepleting chemotherapy) plasma cytokine levels, including interleukin-7 (IL-7), IL-15, macrophage inflammatory protein (MIP-1α), were assessed in subjects that exhibited a CAR+CD3+ blood Cₘₐₓ > 500 (N=55) as compared to in subjects that exhibited CAR+CD3+ blood Cₘₐₓ < 500 (N=7). As shown in **FIG. 15A****,** elevated pre-CAR+ T cell treatment cytokine plasma levels were observed to be associated with CAR+CD3+ Cₘₐₓ > 500.

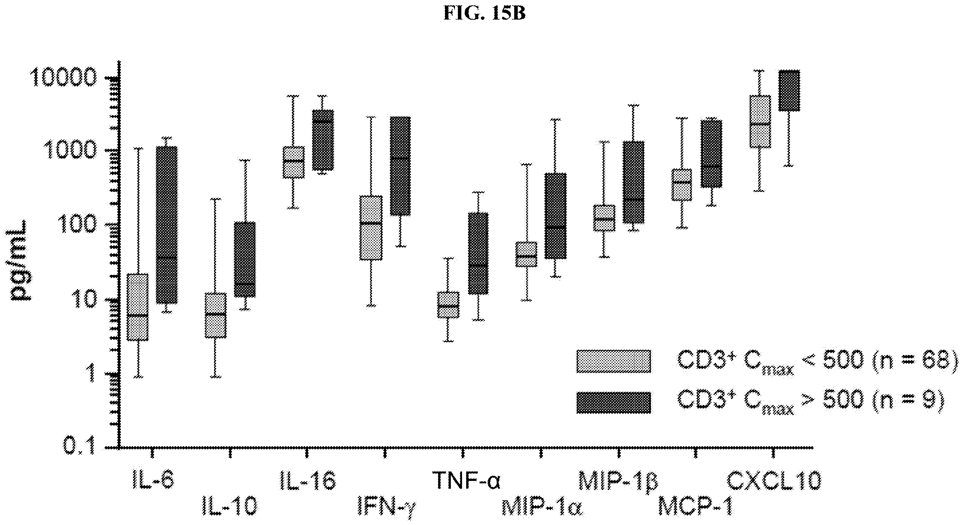

Peak levels of various plasma cytokines (IL-6, IL-10, IL-16, interferon gamma (IFN-y), tumor necrosis factor alpha (TNF-α), MIP-1α, MIP-1β, Monocyte chemoattractant protein-1 (MCP-1), and C-X-C motif chemokine 10 (CXCL10)) were also assessed in subjects that exhibited CAR+ CD3+ blood Cₘₐₓ > 500 (N=68) as compared to subjects that exhibited CAR+ CD3+ blood Cₘₐₓ < 500; N=9). As shown in **FIG. 15B****,** higher peak cytokine levels were observed to be associated with CAR+CD3+ Cₘₐₓ > 500 (Wilcoxon P values < 0.05; without multiplicity of adjustment).

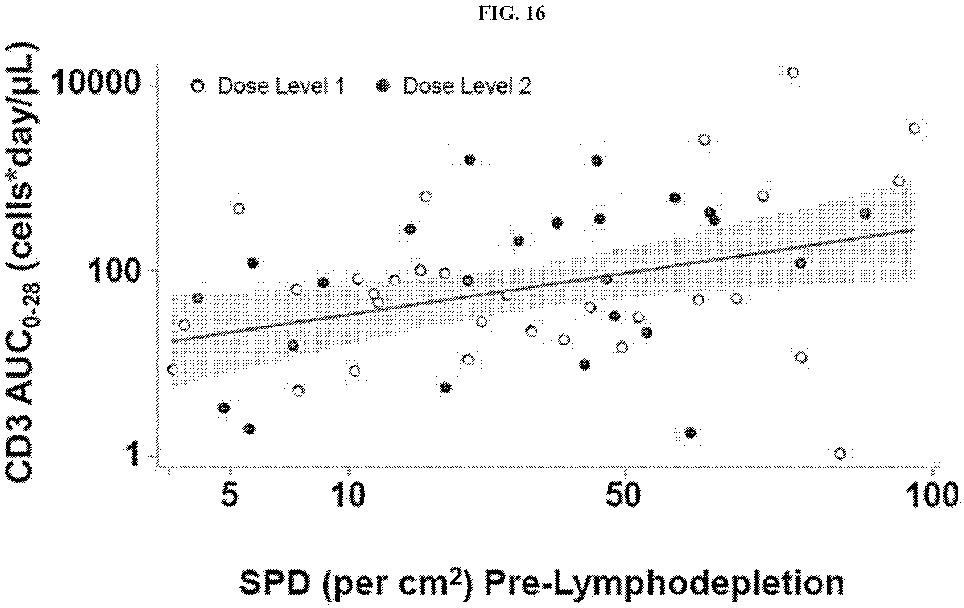

Relationship between pre-CAR+ T cell treatment (pre-lymphodepleting chemotherapy (LDC)) volumetric tumor measurement sum of product dimensions (SPD), as an indicator of tumor burden, and AUC₀₋₂₈ of CD3⁺ CAR+ T cells, representing CAR+ T exposure over time, was assessed. As shown in **FIG. 16****,** a positive correlation was observed between baseline SPD and CD3⁺ AUC₀₋₂₈, with a Spearman correlation of 0.32 and p = 0.019.

### H. Pre-treatmentpatient parameters and response and toxicity outcomes

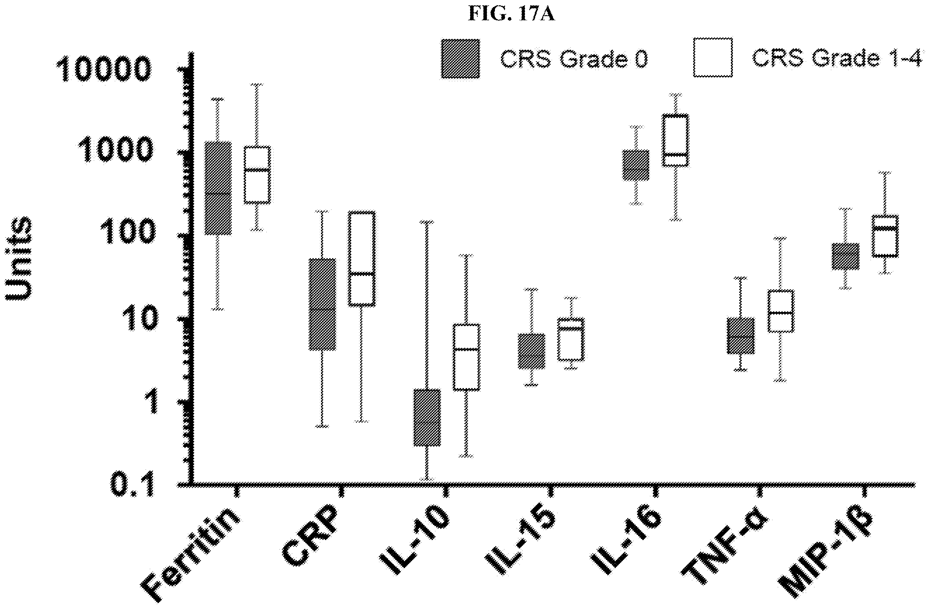

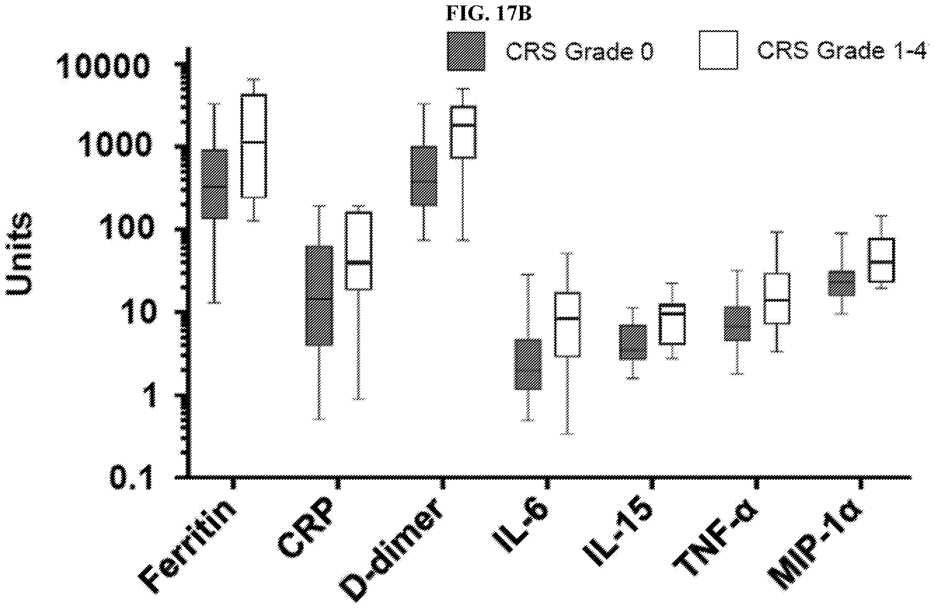

Pre-CAR+ T cell treatment (pre-LDC) analyte levels, including cytokines and inflammatory markers such as Ferritin, C-reactive protein (CRP), D-dimer (fibrin degradation product), IL-6, IL-10, IL-15, IL-16 TNF-α, MIP-1α, and MIP-1β, were compared for subjects with any grade (here, grade 1-4) cytokine release syndrome (CRS) or neurotoxicity (NT) to subjects that did not have any CRS or NT (grade 0). In this cohort, among subjects with CRS grade 1-4, all but one CRS events were determined to be grade 1 or 2. As shown in **FIG. 17A** (CRS) and **FIG. 17B** (NT), higher peak plasma cytokine levels and inflammatory marker levels were observed to be associated with CRS and NT, based on univariate analysis (Wilcoxon P values < 0.05 for all analytes except ferritin for CRS (p = 0.14) and CRP for CRS (p = 0.09)).

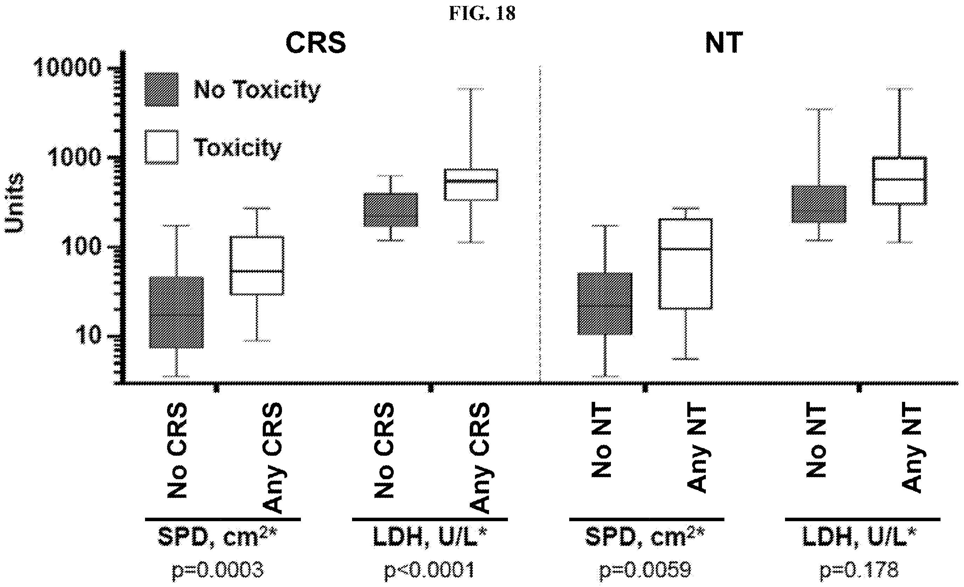

Pre-treatment (pre-LDC) patient parameters, such as levels of lactate dehydrogenase (LDH) and a volumetric tumor measurement such as sum of product dimensions (SPD), as an indicator of tumor burden, were compared between subjects that were not observed to have developed CRS or neurotoxicity versus subjects that were observed to have developed CRS or NT. As shown in **FIG. 18****,** subjects with CRS or NT exhibited higher levels of pre-treatment patient parameters such as SPD and LDH levels; such levels were observed to be correlated with CRS or NT, with univariate statistical analysis. Other patient parameters that were observed to be associated with CRS and NT include shorter time since diagnosis (p=0.05 and p=0.09, for CRS and NT, respectively). Patient parameters that were observed not to be associated with CRS or NT included age (p=0.19 and p=0.54, respectively) and prior numbers of therapies (p=0.67 and p=0.59, respectively) and patient weight (p=0.35 and p=0.44, respectively).

**FIG. 19A** shows pre-treatment SPD and LDH levels among individual patients (dots; with shading of individual dots indicating whether the individual patients did or did and did not exhibit any grade neurotoxicity (left-hand panel) or did or did not exhibit any grade CRS (righthand panel). In **FIG. 19A****,** dotted lines on the y and x axes delineate SPD ≥50 cm² and LDH ≥500, respectively. As shown in **FIG. 19A****,** an SPD of approximately 50 cm² or higher, and/or an LDH of approximately 500 or higher, were observed to be associated with risk of NT and CRS. Calculated odds ratio estimates for developing CRS or NT in subjects above or below the SPD and LDH levels indicated by dotted lines in **FIG. 19A****,** with 95% confidence intervals (CI), are depicted in **FIG. 19B****.** An odds ratio over 1 indicated an increased probability or likelihood of developing CRS or NT. As shown, SPD of 50 cm² or higher, and LDH of 500 or higher, were observed to be associated with increased risk of developing CRS or NT. SPD of 50 cm² or higher and LDH of 500 or higher was observed to be associated with an approximately 8-fold increased risk in developing any grade CRS and NT.

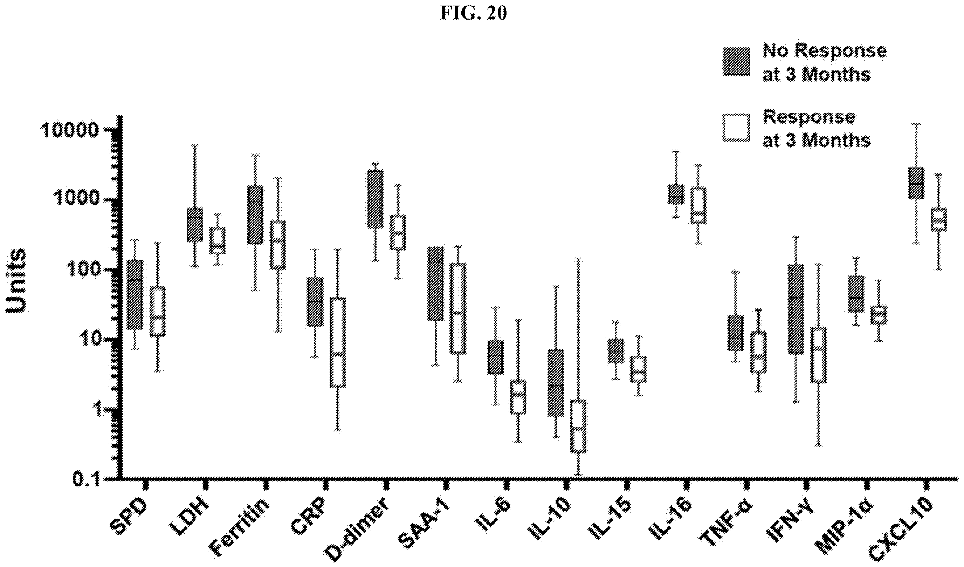

Various pre-treatment (pre-LDC) patient parameters, including markers associated with tumor burden (SPD), inflammatory cytokines and other blood analytes, including LDH, ferritin, CRP, D-dimer, SAA-1, IL-6, IL-10, IL-15, IL-16, TNF-α, IFN-γ and MIP-1α, were compared for subjects with and without a durable response at 3 months, with univariate statistical analysis. As shown in **FIG. 20****,** certain markers of tumor burden, markers of inflammation or inflammatory cytokines were observed to be lower in subjects that exhibited a durable response (p value < 0.05 for all parameters except SPD (p = 0.1274)).

### I. Peak Blood Analytes, Response and Toxicity

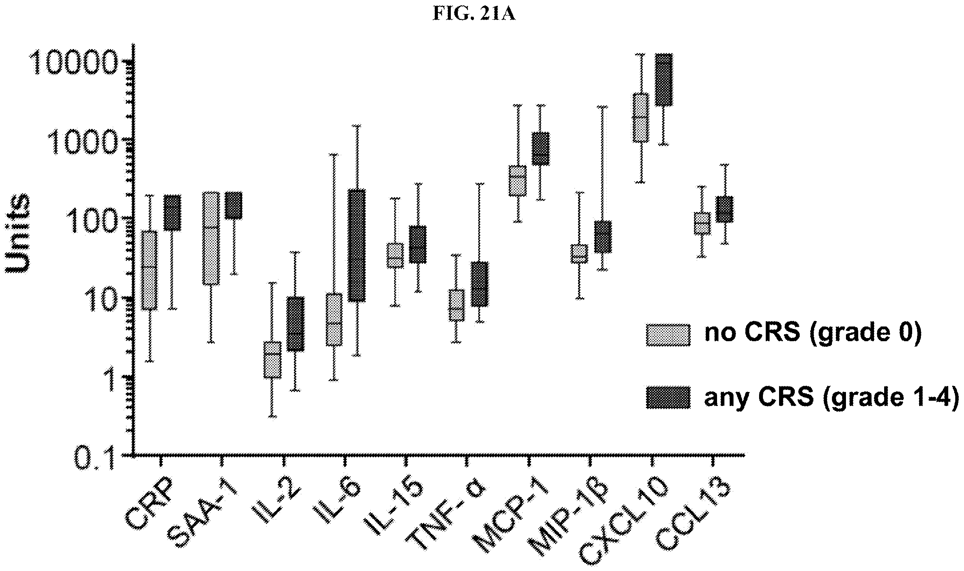

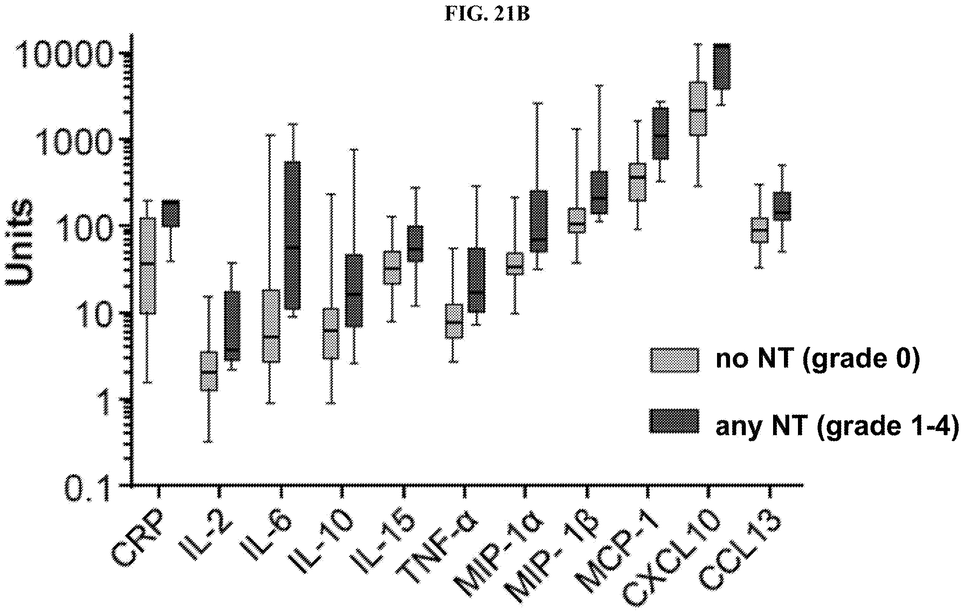

Peak post-treatment plasma levels of blood analytes, including cytokines and inflammatory markers such as CRP, Serum Amyloid A1 (SAA-1), IL-2, IL-6, IL-10, IL-15, TNF-α, MIP-1α, MIP-1β, MCP-1, CXCL10 and C-C Motif Chemokine Ligand 13 (CCL13) were compared for subjects with grade 1-4 cytokine release syndrome (CRS) or neurotoxicity (NT) to subjects that were not observed to have any CRS or NT. As shown in **FIG. 21A** (CRS) and **FIG. 21B** (NT), higher peak plasma cytokine levels and inflammatory marker levels were observed to be associated with CRS and NT (Wilcoxon P values < 0.001 for no CRS vs. any CRS and for no NT vs. any NT, except IL-15 (P= 0.05 and 0.006, respectively)).

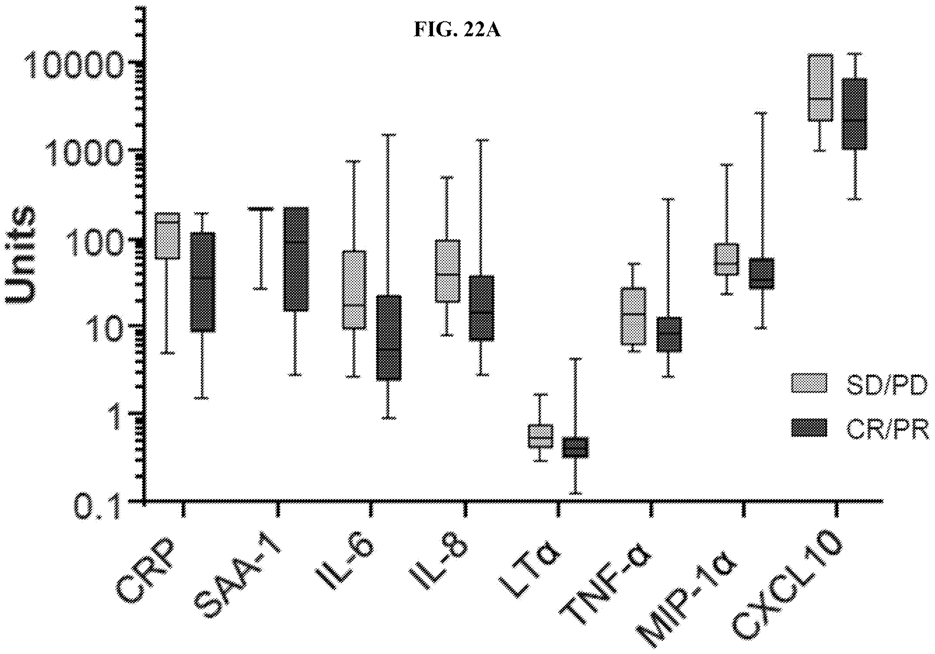

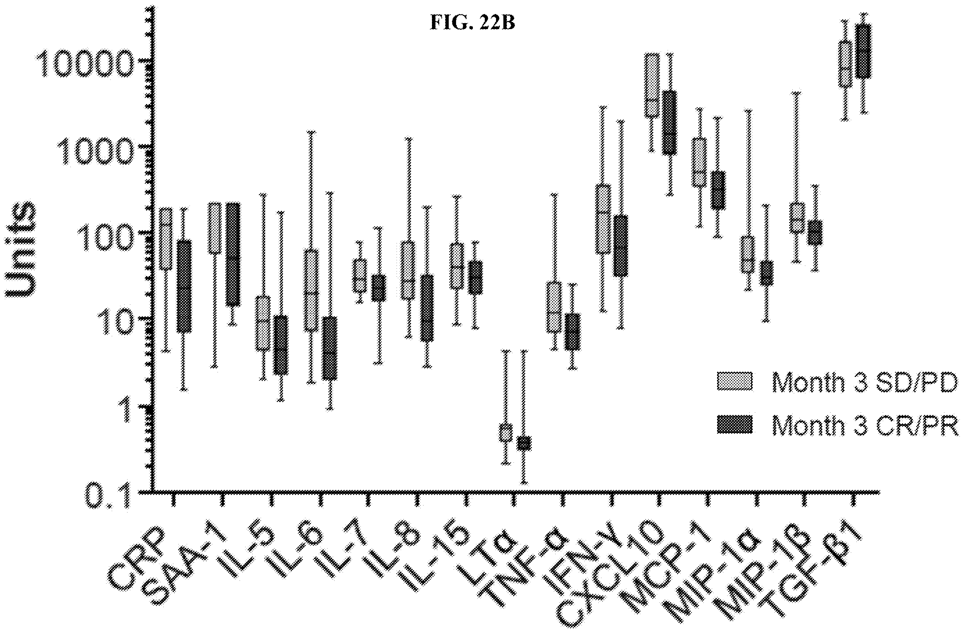

Peak plasma levels of blood analytes, including cytokines and inflammatory markers such as CRP, SAA-1, IL-5, IL-6, IL-7, IL-8, IL-15, Lymphotoxin-alpha (LT-α), TNF-α, IFN-y, MIP-1α, MIP-1β, MCP-1, CXCL10, and Transforming growth factor beta (TGF-β), were assessed for subjects with a best overall response (BOR) of complete response (CR) or partial response (PR) (N=57) compared to levels in subjects with stable disease (SD) or progressive disease (PD) (N=17); or for subjects with a 3-month SD or PD (SD/PD) (N=31), compared to subjects who exhibited CR/PR at 3-months (N=35). As shown in **FIG. 22A** (best overall response (BOR)) and **FIG. 22B** (month 3 response), lower peak plasma cytokine levels and inflammatory marker levels were observed to be associated with better BOR and response at month 3 (Wilcoxon P values < 0.05 without multiplicity of adjustment).

### Example 6: Probability of Response, Durable Response and Toxicity Based on Peak CAR T Cell Numbers

Probabilities of response, durable response and toxicity were calculated based on the peak number of CAR+ expressing cells, in evaluable subjects in the core DLBCL population, after administration of anti-CD 19 CAR-expressing cells, from the clinical study described in Examples 3-5 above. Subjects included those analyzed in the time point in Example 5.

An estimated probability curve of response (overall response rate, ORR; including subjects with complete response (CR) and partial response (PR)), 3-month response (M3 response; including CR and PR at month 3 after administration), any NT, any CRS, Grade 3-4 NT, Grade 3-5 NT or Grade 2-5 CRS, based on the maximum blood concentration of CD3+, CD4+ or CD8+ CAR-expressing cells (Cₘₐₓ; cells/µL blood). For the probability curves, linear logistic regression model fit was used, except CR/PR at month 3 for CD3+ and CR/PR at month 3 for CD8+, where quadratic model fit was used.

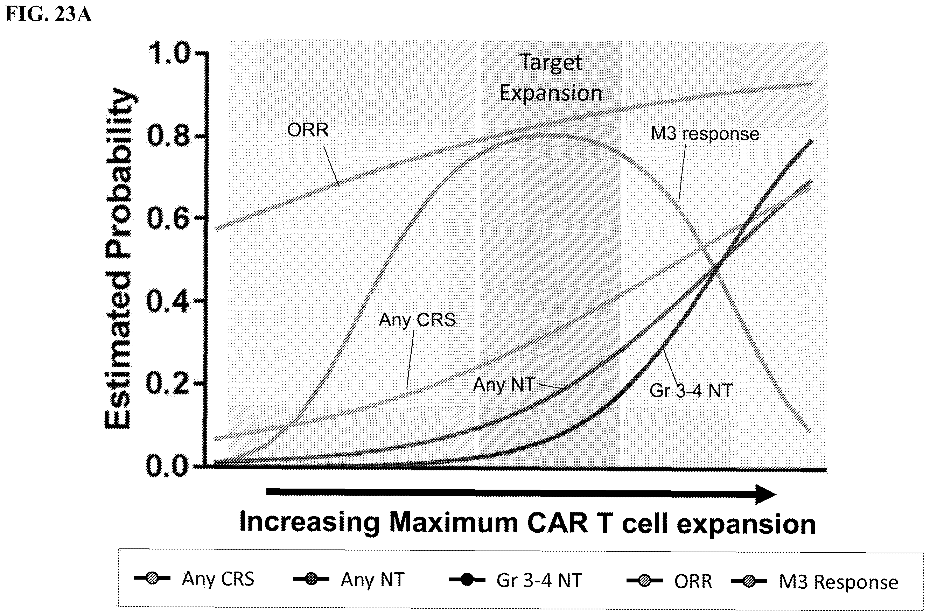

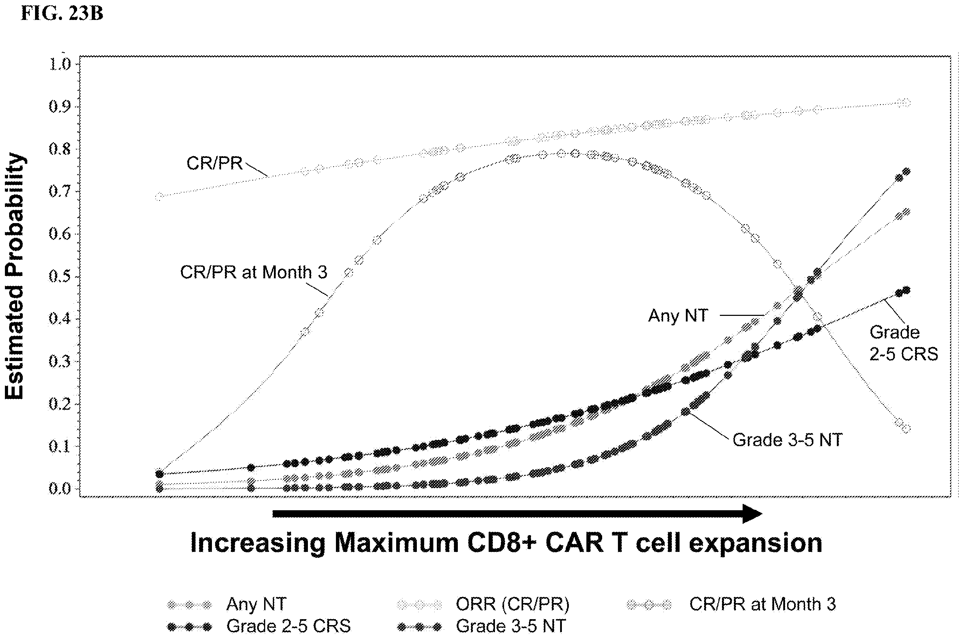

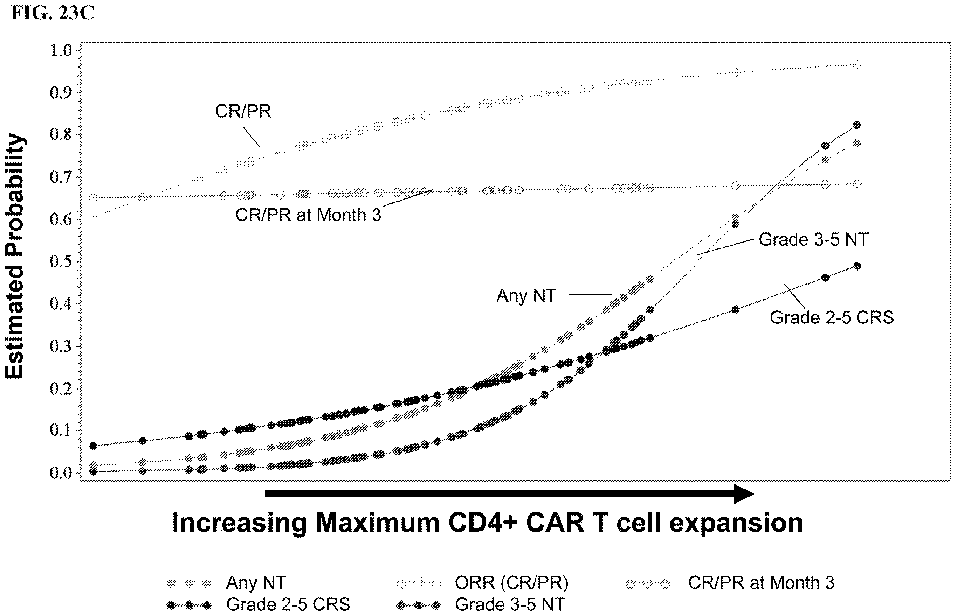

As shown in **FIG. 23A** (CD3+), **FIG. 23B** (CD8+) and **FIG. 23C** (CD4+), higher CD3+, CD8+ and CD4+ expansion was observed to correlate with increased rates of CRS, NT and response (ORR). Higher CD3+ and CD8+ expansion, was observed to result in a reduced probability of durable response (CR/PR at 3 months) at high Cₘₐₓ.

The results are consistent with a conclusion that certain pre-treatment patient characteristics, including high tumor burden and high levels of inflammatory biomarkers, were associated with increased CRS and neurotoxicity, and increased CAR T cell expansion. Lower durable response was associated with very high levels of CAR+ cell numbers or expansion, consistent with an observation that certain high degrees of CAR expansion may lead to exhaustion of highly expanding CAR+ cells.

In some instances described herein, a therapeutic range or window of CAR+ T cell exposure or peak CAR+ T cell levels, is targeted and/or achieved by the methods or compositions or dosages administered, that does or is designed to minimize risk of toxicity and/or maximize or optimize likelihood of response and/or durability of response. In some instances, subjects with expansion or exposure below a certain level may be administered one or more additional interventions, such as to boost CAR-T function; in some instances, subjects exhibiting high levels of exposure or expansion (such as those associated with risk of toxicity and/or decreased likelihood of durability of response) may be administered one or more interventions, such as early or prophylactic measures, such as those for the purpose of reducing or limiting CAR+ T cell expansion and/or reduce toxicity or improve durability of response, such as based on one or more of the observed parameters.

In this study at this time-point, increased CAR+ T cell exposure and higher median expansion was observed in DL2 versus DL1, corresponding to an increased durability of response (DOR) without increased toxicity in DL2 subjects. The results are consistent with the conclusion that increased CAR+ cell expansion over a range correlated with durable responses, but that a very high degree of expansion can be associated with a higher risk of toxicity and/or lower durability of response. Certain patient-specific factors such as baseline patient factors, such as homeostatic and inflammatory cytokine levels and parameters indicative of tumor burden, can in some instances be associated with higher degrees of expansion and with increased risk of toxicity.

The present invention is not intended to be limited in scope to the particular disclosed embodiments, which are provided, for example, to illustrate various aspects of the invention. Various modifications to the compositions and methods described will become apparent from the description and teachings herein.

### SEQUENCES

| **SEQ ID NO.** | **SEQUENCE** | **DESCRIPTION** |
|---|---|---|
| **1** | ESKYGPPCPPCP | spacer (IgG4hinge) (aa) |
| | | Homo sapiens |
| **2** | GAATCTAAGTACGGACCGCCCTGCCCCCCTTGCCCT | spacer (IgG4hinge) (nt) |
| | | homo sapiens |
| **3** | ESKYGPPCPPCPGQPREPQVYTLPPSQEEMTKNQVSLTCLVKGFYPSDIAVEWE SNGQPENNYKTTPPVLDSDGSFFLYSRLTVDKSRWQEGNVFSCSVMHEALHNHY TQKSLSLSLGK | Hinge-CH3 spacer |
| | | Homo sapiens |
| **4** | ESKYGPPCPPCPAPEFLGGPSVFLFPPKPKDTLMISRTPEVTCVVVDVSQEDPE VQFNWYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEALHN HYTQKSLSLSLGK | Hinge-CH2-CH3 spacer |
| | | Homo sapiens |
| **5** | RWPESPKAQASSVPTAQPQAEGSLAKATTAPATTRNTGRGGEEKKKEKEKEEQE ERETKTPECPSHTQPLGVYLLTPAVQDLWLRDKATFTCFVVGSDLKDAHLTWEV AGKVPTGGVEEGLLERHSNGSQSQHSRLTLPRSLWNAGTSVTCTLNHPSLPPQR LMALREPAAQAPVKLSLNLLASSDPPEAASWLLCEVSGFSPPNILLMWLEDQRE VNTSGFAPARPPPQPGSTTFWAWSVLRVPAPPSPQPATYTCVVSHEDSRTLLNA SRSLEVSYVTDH | IgD-hinge-Fc |
| | | Homo sapiens |
| **6** | LEGGGEGRGSLLTCGDVEENPGPR | T2A |
| | | artificial |
| **7** | MLLLVTSLLLCELPHPAFLLIPRKVCNGIGIGEFKDSLSINATNIKHFKNCTSI SGDLHILPVAFRGDSFTHTPPLDPQELDILKTVKEITGFLLIQAWPENRTDLHA FENLEIIRGRTKQHGQFSLAVVSLNITSLGLRSLKEISDGDVIISGNKNLCYAN TINWKKLFGTSGQKTKIISNRGENSCKATGQVCHALCSPEGCWGPEPRDCVSCR NVSRGRECVDKCNLLEGEPREFVENSECIQCHPECLPQAMNITCTGRGPDNCIQ CAHYIDGPHCVKTCPAGVMGENNTLVWKYADAGHVCHLCHPNCTYGCTGPGLEG CPTNGPKIPSIATGMVGALLLLLVVALGIGLFM | tEGFR |
| | | artificial |
| **8** | FWVLVWGGVLACYS LLVTVAFII FWV | CD28 (amino acids 153-179 of Accession No. P10747) |
| | | Homo sapiens |
| **9** | IEVMYPPPYLDNEKSNGTIIHVKGKHLCPSPLFPGPSKP FWVLVVVGGVLACYSLLVTVAFIIFWV | CD28 (amino acids 114-179 of Accession No. P10747) |
| | | Homo sapiens |
| **10** | RSKRSRLLHSDYMNMTPRRPGPTRKHYQPYAPPRDFAAYRS | CD28 (amino acids 180-220 of P10747) |
| | | Homo sapiens |
| **11** | RSKRSRGGHSDYMNMTPRRPGPTRKHYQPYAPPRDFAAYRS | CD28 (LL to GG) |
| | | Homo sapiens |
| **12** | KRGRKKLLYIFKQPFMRPVQTTQEEDGCSCRFPEEEEGGCEL | 4-1BB (amino acids 214-255 of Q07011.1) |
| | | Homo sapiens |
| **13** | RVKFSRSADAPAYQQGQNQLYNELNLGRREEYDVLDKRRGRDPEMGGKPRRKNP QEGLYNELQKDKMAEAYSEIGMKGERRRGKGHDGLYQGLSTATKDTYDALHMQA LPPR | CD3 zeta |
| | | Homo sapiens |
| **14** | RVKFSRSAEPPAYQQGQNQLYNELNLGRREEYDVLDKRRGRDPEMGGKPRRKNP QEGLYNELQKDKMAEAYSEIGMKGERRRGKGHDGLYQGLSTATKDTYDALHMQA LPPR | CD3 zeta |
| | | Homo sapiens |
| **15** | RVKFSRSADAPAYKQGQNQLYNELNLGRREEYDVLDKRRGRDPEMGGKPRRKNP QEGLYNELQKDKMAEAYSEIGMKGERRRGKGHDGLYQGLSTATKDTYDALHMQA LPPR | CD3 zeta |
| | | Homo sapiens |
| **16** | RKVCNGIGIGEFKDSLSINATNIKHFKNCTSISGDLHILPVAFRGDSFTHTPPL DPQELDILKTVKEITGFLLIQAWPENRTDLHAFENLEIIRGRTKQHGQFSLAVV SLNITSLGLRSLKEISDGDVIISGNKNLCYANTINWKKLFGTSGQKTKIISNRG ENSCKATGQVCHALCSPEGCWGPEPRDCVSCRNVSRGRECVDKCNLLEGEPREF VENSECIQCHPECLPQAMNITCTGRGPDNCIQCAHYIDGPHCVKTCPAGVMGEN NTLVWKYADAGHVCHLCHPNCTYGCTGPGLEGCPTNGPKIPSIATGMVGALLLL LVVALGIGLFM | tEGFR |
| | | artificial |
| **17** | EGRGSLLTCGDVEENPGP | T2A artificial |
| **18** | GSGATNFSLLKQAGDVEENPGP | P2A |
| **19** | ATNFSLLKQAGDVEENPGP | P2A |
| **20** | QCTNYALLKLAGDVESNPGP | E2A |
| **21** | VKQTLNFDLLKLAGDVESNPGP | F2A |
| **22** | PGGG-(SGGGG)₅-P-wherein P is proline, G is glycine and S is serine | linker |
| **23** | GSADDAKKDAAKKDGKS | Linker |
| **24** | GSTSGSGKPGSGEGSTKG | Linker |
| **25** | gacatccagatgacccagaccacctccagcctgagcgccagcctgggcgaccgg gtgaccatcagctgccgggccagccaggacatcagcaagtacctgaactggtat cagcagaagcccgacggcaccgtcaagctgctgatctaccacaccagccggctg cacagcggcgtgcccagccggtttagcggcagcggctccggcaccgactacagc ctgaccatctccaacctggaacaggaagatatcgccacctacttttgccagcag ggcaacacactgccctacacctttggcggcggaacaaagctggaaatcaccggc agcacctccggcagcggcaagcctggcagcggcgagggcagcaccaagggcgag gtgaagctgcaggaaagcggccctggcctggtggcccccagccagagcctgagc gtgacctgcaccgtgagcggcgtgagcctgcccgactacggcgtgagctggatc cggcagccccccaggaagggcctggaatggctgggcgtgatctggggcagcgag accacctactacaacagcgccctgaagagccggctgaccatcatcaaggacaac agcaagagccaggtgttcctgaagatgaacagcctgcagaccgacgacaccgcc atctactactgcgccaagcactactactacggcggcagctacgccatggactac tggggccagggcaccagc gtgaccgtgagcagc | Sequence encoding scFv |
| **26** | X₁PPX₂P | Hinge |
| | X₁ is glycine, cysteine or arginine | |
| | X₂ is cysteine or threonine | |
| **27** | Glu Pro Lys Ser Cys Asp Lys Thr His Thr Cys Pro Pro Cys Pro | Hinge |
| **28** | Glu Arg Lys Cys Cys Val Glu Cys Pro Pro Cys Pro | Hinge |
| **29** | ELKTPLGDTHTCPRCPEPKSCDTPPPCPRCPEPKSCDTPPPCPRCPEPKSCDTP PPCPRCP | Hinge |
| **30** | Glu Ser Lys Tyr Gly Pro Pro Cys Pro Ser Cys Pro | Hinge |
| **31** | Glu Ser Lys Tyr Gly Pro Pro Cys Pro Pro Cys Pro | Hinge |
| **32** | Tyr Gly Pro Pro Cys Pro Pro Cys Pro | Hinge |
| **33** | Lys Tyr Gly Pro Pro Cys Pro Pro Cys Pro | Hinge |
| **34** | Glu Val Val Val Lys Tyr Gly Pro Pro Cys Pro Pro Cys Pro | Hinge |
| **35** | RASQDISKYLN | FMC63 CDR L1 |
| **36** | SRLHSGV | FMC63 CDR L2 |
| **37** | GNTLPYTFG | FMC63 CDR L3 |
| **38** | DYGVS | FMC63 CDR H1 |
| **39** | VIWGSETTYYNSALKS | FMC63 CDR H2 |
| **40** | YAMDYWG | FMC63 CDR H3 |
| **41** | EVKLQESGPGLVAPSQSLSVTCTVSGVSLPDYGVSWIRQPPRKGLEWLGVIWGS ETTYYNSALKSRLTIIKDNSKSQVFLKMNSLQTDDTAIYYCAKHYYYGGSYAMD YWGQGTSVTVSS | FMC63 VH |
| **42** | DIQMTQTTSSLSASLGDRVTISCRASQDISKYLNWYQQKPDGTVKLLIYHTSRL HSGVPSRFSGSGSGTDYSLTISNLEQEDIATYFCQQGNTLPYTFGGGTKLEIT | FMC63 VL |
| **43** | DIQMTQTTSSLSASLGDRVTISCRASQDISKYLNWYQQKPDGTVKLLIYHTSRL HSGVPSRFSGSGSGTDYSLTISNLEQEDIATYFCQQGNTLPYTFGGGTKLEITG STSGSGKPGSGEGSTKGEVKLQESGPGLVAPSQSLSVTCTVSGVSLPDYGVSWI RQPPRKGLEWLGVIWGSETTYYNSALKSRLTIIKDNSKSQVFLKMNSLQTDDTA IYYCAKHYYYGGSYAMDYWGQGTSVTVSS | FMC63 scFv |
| **44** | KASQNVGTNVA | SJ25C1 CDR L1 |
| **45** | SATYRNS | SJ25C1 CDR L2 |
| **46** | QQYNRYPYT | SJ25C1 CDR L3 |
| **47** | SYWMN | SJ25C1 CDR H1 |
| **48** | QIYPGDGDTNYNGKFKG | SJ25C1 CDR H2 |
| **49** | KTISSVVDFYFDY | SJ25C1 CDR H3 |
| **50** | EVKLQQSGAELVRPGSSVKISCKASGYAFSSYWMNWVKQRPGQGLEWIGQIYPG DGDTNYNGKFKGQATLTADKSSSTAYMQLSGLTSEDSAVYFCARKTISSVVDFY FDYWGQGTTVTVSS | SJ25C1 VH |
| **51** | DIELTQSPKFMSTSVGDRVSVTCKASQNVGTNVAWYQQKPGQSPKPLIYSATYR NSGVPDRFTGSGSGTDFTLTITNVQSKDLADYFCQQYNRYPYTSGGGTKLEIKR | SJ25C1 VL |
| **52** | GGGGSGGGGSGGGGS | Linker |
| **53** | EVKLQQSGAELVRPGSSVKISCKASGYAFSSYWMNWVKQRPGQGLEWIGQIYPG DGDTNYNGKFKGQATLTADKSSSTAYMQLSGLTSEDSAVYFCARKTISSVVDFY FDYWGQGTTVTVSSGGGGSGGGGSGGGGSDIELTQSPKFMSTSVGDRVSVTCKA SQNVGTNVAWYQQKPGQSPKPLIYSATYRNSGVPDRFTGSGSGTDFTLTITNVQ SKDLADYFCQQYNRYPYTSGGGTKLEIKR | SJ25C1 scFv |
| **54** | HYYYGGSYAMDY | FMC63 CDR H3 |
| **55** | HTSRLHS | FMC63 CDR L2 |
| **56** | QQGNTLPYT | FMC63 CDR L3 |

## Claims

1. An agent that is capable of decreasing expansion or proliferation of genetically engineered T cells expressing a chimeric antigen receptor (CAR) in a subject for use in a method of modulating activity of engineered T cells expressing a CAR in treating cancer, wherein the method comprises:
administering the agent to the subject;
wherein the subject is one in which the level, amount or concentration of a volumetric measure of tumor burden or an inflammatory marker in a sample from the subject is at or above a threshold level;
wherein the sample is obtained from the subject prior to receiving administration of genetically engineered T cells expressing a CAR;
wherein the volumetric measure is a sum of the products of diameters (SPD) and the threshold value is greater than or equal to 50 cm²;
wherein the inflammatory marker is lactate dehydrogenase (LDH) and the threshold value is greater than or equal to 500 units per liter; and
wherein the agent is a steroid.

2. An agent that is capable of decreasing expansion or proliferation of genetically engineered T cells expressing a chimeric antigen receptor (CAR) in a subject and genetically engineered cells comprising T cells expressing a CAR for use in a method of modulating activity of engineered T cells expressing a CAR and treating cancer, wherein the engineered T cells are administered to treat cancer and the agent is administered to modulate activity of the engineered T cells, and wherein the method comprises:
a) administering the agent to the subject; and
b) administering a dose of genetically engineered T cells expressing a CAR,
wherein:
(i) the subject is one in which the level, amount or concentration of a volumetric measure of tumor burden or an inflammatory marker in a sample from the subject is at or above a threshold level;
(ii) the sample is obtained from the subject prior to receiving administration of genetically engineered T cells expressing a CAR;
(iii) the volumetric measure is a sum of the products of diameters (SPD) and the threshold value is greater than or equal to 50 cm²;
(iv) the inflammatory marker is lactate dehydrogenase (LDH) and the threshold value is greater than or equal to 500 units per liter; and
(v) the agent is a steroid.

3. The agent for use of claim 1, or the agent and genetically engineered cells for use of claim 2, wherein the method further comprises selecting a subject in which the level, amount or concentration of the volumetric measure of tumor burden or the inflammatory marker in the sample from the subject is at or above a threshold level.

4. The agent for use of claim 1 or claim 3, or the agent and genetically engineered cells for use of claim 2 or claim 3, wherein the agent is administered before, simultaneously with, intermittently with, during, during the course of or after administration of the genetically engineered T cells expressing a CAR.

5. The agent for use of any one of claims 1, 3 or 4, or the agent and genetically engineered cells for use of any one of claims 2-4, wherein the agent is administered prior to or concurrently with initiation of administration of the genetically engineered T cells expressing a CAR.

6. The agent for use of any one of claims 1 or 3-5, wherein the method further comprises administering a dose of the genetically engineered T cells expressing a CAR.

7. The agent for use of any one of claims 3-6, or the agent and genetically engineered cells for use of any one of claims 3-6, wherein, prior to administering the agent, the selected subject is at risk of developing cytokine release syndrome (CRS) or a neurotoxicity following administration of the genetically engineered T cells expressing a CAR.

8. The agent for use of any one of claims 1 or 3-7, or the agent and genetically engineered cells for use of any one of claims 2-7, wherein SPD is measured using computed tomography (CT), positron emission tomography (PET), and/or magnetic resonance imaging (MRI) of the subject.

9. The agent for use of any one of claims 1 or 3-8, or the agent and genetically engineered cells for use of any one of claims 2-8, wherein the sample is or comprises a blood sample, plasma sample, or serum sample.

10. The agent for use of any one of claims 1 or 3-9, or the agent and genetically engineered cells for use of any one of claims 2-9, wherein the steroid is a corticosteroid and/or the steroid is dexamethasone or methylprednisolone.

11. The agent for use of any one of claims 1 or 3-10, or the agent and genetically engineered cells for use of any one of claims 2-10, wherein the steroid is administered in an amount that is between or between about 1.0 mg and about 40 mg, between or between about 1.0 mg and about 20 mg, between or between about 2.0 mg and about 20 mg, between or between about 5.0 mg and about 25.0 mg, between or between about 10 mg and about 20 mg dexamethasone or equivalent thereof, each inclusive.

12. The agent for use of any one of claims 1 or 3-11, or the agent and genetically engineered cells for use of any one of claims 2-11, wherein SPD or LDH is measured in the subject within 1 day, 2 days, 3 days, 4 days, 6 days, 8 days, 12 days, 16 days, 20 days, 24 days, 28 days or more prior to initiation of administration of the genetically engineered T cells expressing a CAR.

13. The agent for use of any one of claims 1 or 3-12, or the agent and genetically engineered cells for use of any one of claims 2-12, wherein the level, amount or concentration of SPD or LDH is assessed prior to lymphodepletion.

14. The agent for use of any one of claims 1 or 3-13, or the agent and genetically engineered cells for use of any one of claims 2-13, wherein the cancer is a B cell malignancy, such as a cancer selected from the group consisting of sarcomas, carcinomas, lymphomas, non-Hodgkin lymphomas (NHLs), diffuse large B cell lymphoma (DLBCL), leukemia, chronic lymphocytic leukemia (CLL), acute lymphoblastic leukemia (ALL), acute myeloid leukemia (AML) and myeloma, pancreatic cancer, bladder cancer, colorectal cancer, breast cancer, prostate cancer, renal cancer, hepatocellular cancer, lung cancer, ovarian cancer, cervical cancer, rectal cancer, thyroid cancer, uterine cancer, gastric cancer, esophageal cancer, head and neck cancer, melanoma, neuroendocrine cancers, central nervous system (CNS) cancers, brain tumors, bone cancer, or soft tissue sarcoma.

## Patentansprüche

1. Mittel, das dazu in der Lage ist, die Expansion oder Proliferation von gentechnisch veränderten T-Zellen, die einen chimären Antigenrezeptor (CAR) exprimieren, in einem Subjekt zu verringern, zur Verwendung in einem Verfahren zur Modulation der Aktivität von gentechnisch veränderten T-Zellen, die einen CAR exprimieren, bei der Behandlung von Krebs, wobei das Verfahren umfasst:
Verabreichen des Mittels an das Subjekt;
wobei es sich bei dem Subjekt um eines handelt, bei dem der Spiegel, die Menge oder die Konzentration eines volumetrischen Maßes einer Tumorlast oder eines Entzündungsmarkers in einer Probe des Subjekts auf oder über einem Schwellenwert liegt;
wobei die Probe vom Subjekt vor der Verabreichung gentechnisch veränderter T-Zellen, die einen CAR exprimieren, erhalten wird;
wobei das volumetrische Maß eine Summe der Produkte der Durchmesser (SPD) ist und der Schwellenwert größer oder gleich 50 cm² ist,
wobei der Entzündungsmarker Laktatdehydrogenase (LDH) ist und der Schwellenwert größer oder gleich 500 Einheiten pro Liter ist; und
wobei das Mittel ein Steroid ist.

2. Mittel, das dazu in der Lage ist, die Expansion oder Proliferation von gentechnisch veränderten T-Zellen, die einen chimären Antigenrezeptor (CAR) exprimieren, in einem Subjekt und von gentechnisch veränderten Zellen, umfassend T-Zellen, die einen CAR exprimieren, zu verringern, zur Verwendung in einem Verfahren zur Modulation der Aktivität von gentechnisch veränderten T-Zellen, die einen CAR exprimieren, sowie zum Behandeln von Krebs, wobei die veränderten T-Zellen zur Behandlung von Krebs verabreicht werden und das Mittel verabreicht wird, um die Aktivität der veränderten T-Zellen zu modulieren, und wobei das Verfahren umfasst:
a) Verabreichen des Mittels an das Subjekt; und
b) Verabreichen einer Dosis gentechnisch veränderter T-Zellen, die einen CAR exprimieren,
wobei:
(i) es sich bei dem Subjekt um eines handelt, bei dem der Spiegel, die Menge oder die Konzentration eines volumetrischen Maßes einer Tumorlast oder eines Entzündungsmarkers in einer Probe des Subjekts auf oder über einem Schwellenwert liegt;
(ii) die Probe vom Subjekt vor der Verabreichung gentechnisch veränderter T-Zellen, die einen CAR exprimieren, erhalten wird;
(iii) das volumetrische Maß eine Summe der Produkte der Durchmesser (SPD) ist und der Schwellenwert größer oder gleich 50 cm² ist,
(iv) der Entzündungsmarker Laktatdehydrogenase (LDH) ist und der Schwellenwert größer oder gleich 500 Einheiten pro Liter ist; und
(v) das Mittel ein Steroid ist.

3. Mittel zur Verwendung nach Anspruch 1 oder Mittel und gentechnisch veränderte Zellen zur Verwendung nach Anspruch 2, wobei das Verfahren ferner das Auswählen eines Subjekts umfasst, bei dem der Spiegel, die Menge oder die Konzentration des volumetrischen Maßes einer Tumorlast oder des Entzündungsmarkers in der Probe des Subjekts auf oder über einem Schwellenwert liegt.

4. Mittel zur Verwendung nach Anspruch 1 oder 3 oder Mittel und gentechnisch veränderte Zellen zur Verwendung nach Anspruch 2 oder 3, wobei das Mittel vor, gleichzeitig mit, intermittierend mit, während, im Verlauf oder nach der Verabreichung der gentechnisch veränderten T-Zellen, die einen CAR exprimieren, verabreicht wird.

5. Mittel zur Verwendung nach einem der Ansprüche 1, 3 und 4 oder Mittel und gentechnisch veränderte Zellen zur Verwendung nach einem der Ansprüche 2-4, wobei das Mittel vor oder gleichzeitig mit dem Beginn der Verabreichung der gentechnisch veränderten T-Zellen, die einen CAR exprimieren, verabreicht wird.

6. Mittel zur Verwendung nach einem der Ansprüche 1 und 3-5, wobei das Verfahren ferner das Verabreichen einer Dosis der gentechnisch veränderten T-Zellen umfasst, die einen CAR exprimieren.

7. Mittel zur Verwendung nach einem der Ansprüche 3-6 oder Mittel und gentechnisch veränderte Zellen zur Verwendung nach einem der Ansprüche 3-6, wobei das ausgewählte Subjekt vor dem Verabreichen des Mittels Gefahr läuft, nach der Verabreichung der gentechnisch veränderten T-Zellen, die einen CAR exprimieren, ein Zytokinfreisetzungssyndrom (CRS) oder eine Neurotoxizität zu entwickeln.

8. Mittel zur Verwendung nach einem der Ansprüche 1 und 3-7 oder Mittel und gentechnisch veränderte Zellen zur Verwendung nach einem der Ansprüche 2-7, wobei SPD mittels Computertomographie (CT), Positronenemissionstomographie (PET) und/oder Magnetresonanztomographie (MRT) des Subjekts gemessen wird.

9. Mittel zur Verwendung nach einem der Ansprüche 1 und 3-8 oder Mittel und gentechnisch veränderte Zellen zur Verwendung nach einem der Ansprüche 2-8, wobei die Probe eine Blutprobe, Plasmaprobe oder Serumprobe ist oder umfasst.

10. Mittel zur Verwendung nach einem der Ansprüche 1 und 3-9 oder Mittel und gentechnisch veränderte Zellen zur Verwendung nach einem der Ansprüche 2-9, wobei das Steroid ein Kortikosteroid ist und/oder das Steroid Dexamethason oder Methylprednisolon ist.

11. Mittel zur Verwendung nach einem der Ansprüche 1 und 3-10 oder Mittel und gentechnisch veränderte Zellen zur Verwendung nach einem der Ansprüche 2-10, wobei das Steroid in einer Menge verabreicht wird, die zwischen oder zwischen etwa 1,0 mg und etwa 40 mg, zwischen oder zwischen etwa 1,0 mg und etwa 20 mg, zwischen oder zwischen etwa 2,0 mg und etwa 20 mg, zwischen oder zwischen etwa 5,0 mg und etwa 25, 0 mg, zwischen oder zwischen etwa 10 mg und etwa 20 mg Dexamethason oder Äquivalent davon liegt, jeweils einschließlich.

12. Mittel zur Verwendung nach einem der Ansprüche 1 und 3-11 oder Mittel und gentechnisch veränderte Zellen zur Verwendung nach einem der Ansprüche 2-11, wobei SPD oder LDH im Subjekt innerhalb von 1 Tag, 2 Tagen, 3 Tagen, 4 Tagen, 6 Tagen, 8 Tagen, 12 Tagen, 16 Tagen, 20 Tagen, 24 Tagen, 28 Tagen oder mehr vor Beginn der Verabreichung der gentechnisch veränderten T-Zellen, die einen CAR exprimieren, gemessen wird.

13. Mittel zur Verwendung nach einem der Ansprüche 1 und 3-12 oder Mittel und gentechnisch veränderte Zellen zur Verwendung nach einem der Ansprüche 2-12, wobei der Spiegel, die Menge oder die Konzentration von SPD oder LDH vor der Lymphodepletion beurteilt wird.

14. Mittel zur Verwendung nach einem der Ansprüche 1 und 3-13 oder Mittel und gentechnisch veränderte Zellen zur Verwendung nach einem der Ansprüche 2-13, wobei es sich bei dem Krebs um einen bösartigen B-Zell-Krebs handelt, beispielsweise um einen Krebs, der ausgewählt ist aus der Gruppe, bestehend aus: Sarkomen, Karzinomen, Lymphomen, Non-Hodgkin-Lymphomen (NHLs), diffusem großzelligen B-Zell-Lymphom (DLBCL), Leukämie, chronischer lymphatischer Leukämie (CLL), akuter lymphatischer Leukämie (ALL), akuter myeloischer Leukämie (AML) und Myelom, Bauchspeicheldrüsenkrebs, Blasenkrebs, Darmkrebs, Brustkrebs, Prostatakrebs, Nierenkrebs, Leberzellkrebs, Lungenkrebs, Eierstockkrebs, Gebärmutterhalskrebs, Mastdarmkrebs, Schilddrüsenkrebs, Gebärmutterkrebs, Magenkrebs, Speiseröhrenkrebs, Kopf- und Halskrebs, Melanom, neuroendokrinen Krebserkrankungen, Krebserkrankungen des Zentralnervensystems (ZNS), Hirntumoren, Knochenkrebs und Weichteilsarkom.

## Revendications

1. Agent qui est capable de diminuer l'expansion ou la prolifération de lymphocytes T génétiquement modifiés exprimant un récepteur d'antigène chimérique (CAR) chez un sujet, pour utilisation dans une méthode de modulation de l'activité de lymphocytes T modifiés exprimant un CAR dans le traitement d'un cancer, la méthode comprenant :
l'administration de l'agent au sujet ;
dans lequel le sujet est un sujet dans lequel le niveau, la quantité ou la concentration d'une mesure volumétrique d'une charge tumorale ou d'un marqueur inflammatoire dans un échantillon provenant du sujet est égal ou supérieur à un niveau seuil ;
dans lequel l'échantillon est obtenu du sujet avant réception de l'administration des lymphocytes T génétiquement modifiés exprimant un CAR ;
dans lequel la mesure volumétrique est une somme des produits des diamètres (SPD), et la valeur seuil est supérieure ou égale à 50 cm² ;
dans lequel le marqueur inflammatoire est la lactate déshydrogénase (LDH) et la valeur seuil est supérieure ou égale à 500 unités par litre ; et
dans lequel l'agent est un stéroïde.

2. Agent qui est capable de diminuer l'expansion ou la prolifération de lymphocytes T génétiquement modifiés exprimant un récepteur d'antigène chimérique (CAR) chez un sujet et de cellules génétiquement modifiées comprenant des lymphocytes T exprimant un CAR pour une utilisation dans une méthode de modulation de l'activité de lymphocytes T modifiés exprimant un CAR et de traitement d'un cancer, dans lequel les lymphocytes T modifiés sont administrés pour traiter le cancer et l'agent est administré pour moduler l'activité des lymphocytes T modifiés, et dans lequel la méthode comprend :
a) l'administration de l'agent au sujet ; et
b) l'administration d'une dose des lymphocytes T génétiquement modifiés exprimant un CAR,
dans lequel :
(i) le sujet est un sujet chez lequel le niveau, la quantité ou la concentration d'une mesure volumétrique d'une charge tumorale ou d'un marqueur inflammatoire dans un échantillon provenant du sujet est égal ou supérieur à un niveau seuil ;
(ii) l'échantillon est obtenu du sujet avant réception de l'administration des lymphocytes T génétiquement modifiés exprimant un CAR ;
(iii) la mesure volumétrique est une somme des produits des diamètres (SPD) et la valeur seuil est supérieure ou égale à 50 cm² ;
(iv) le marqueur inflammatoire est la lactate déshydrogénase (LDH) et la valeur seuil est supérieure ou égale à 500 unités par litre ; et
(v) l'agent est un stéroïde.

3. Agent pour une utilisation selon la revendication 1, ou agent et cellules génétiquement modifiées pour une utilisation selon la revendication 2, dans lequel la méthode comprend en outre la sélection d'un sujet chez lequel le niveau, la quantité ou la concentration de la mesure volumétrique de la charge tumorale ou du marqueur inflammatoire dans un échantillon provenant du sujet est supérieur ou égal à un niveau seuil.

4. Agent pour une utilisation selon la revendication 1 ou 3, ou agent et cellules génétiquement modifiées pour une utilisation selon la revendication 2 ou la revendication 3, dans lequel l'agent est administré avant, en même temps, en intermittence avec, pendant, au cours de l'évolution ou après administration des lymphocytes T génétiquement modifiés exprimant un CAR.

5. Agent pour une utilisation selon l'une quelconque des revendications 1, 3 ou 4, ou agent et cellules génétiquement modifiées pour une utilisation selon l'une quelconque des revendications 2 à 4, dans lequel l'agent est administré avant ou en même temps que le déclenchement de l'administration des lymphocytes T génétiquement modifiés exprimant un CAR.

6. Agent pour une utilisation selon l'une quelconque des revendications 1 ou 3 à 5, dans lequel la méthode comprend en outre l'administration d'une dose des lymphocytes T génétiquement modifiés exprimant un CAR.

7. Agent pour une utilisation selon l'une quelconque des revendications 3 à 6, ou agent et cellules génétiquement modifiées pour une utilisation selon l'une quelconque des revendications 3 à 6, dans lequel, avant administration de l'agent, le sujet choisi présente un risque de développer un syndrome de libération des cytokines (CRS) ou une neurotoxicité après administration des lymphocytes T génétiquement modifiés exprimant un CAR.

8. Agent pour une utilisation selon l'une quelconque des revendications 1 ou 3 à 7, ou agent et cellules génétiquement modifiées pour une utilisation selon l'une quelconque des revendications 2 à 7, dans lequel le SPD est mesuré par utilisation d'une tomodensitométrie (CT), d'une tomographie par émission de positons (PET) et/ou d'une imagerie par résonance magnétique (IRM) du sujet.

9. Agent pour une utilisation selon l'une quelconque des revendications 1 ou 3 à 8, ou agent et cellules génétiquement modifiées pour une utilisation selon l'une quelconque des revendications 2 à 8, dans lequel l'échantillon est ou comprend un échantillon sanguin, un échantillon de plasma ou un échantillon de sérum.

10. Agent pour une utilisation selon l'une quelconque des revendications 1 ou 3 à 9, ou agent et cellules génétiquement modifiées pour une utilisation selon l'une quelconque des revendications 2 à 9, dans lequel le stéroïde est un corticostéroïde et/ou le stéroïde est la dexaméthasone ou la méthylprednisolone.

11. Agent pour une utilisation selon l'une quelconque des revendications 1 ou 3 à 10, ou agent et cellules génétiquement modifiées pour une utilisation selon l'une quelconque des revendications 2 à 10, dans lequel le stéroïde est administré en une quantité comprise entre ou entre environ 1,0 mg et environ 40 mg, entre ou entre environ 1,0 mg et environ 20 mg, entre ou entre environ 2,0 mg et environ 20 mg, entre ou entre environ 5,0 mg et environ 25,0 mg, entre ou entre environ 10 mg et environ 20 mg de dexaméthasone ou d'un équivalent de cette dernière, limites comprises dans tous les cas.

12. Agent pour une utilisation selon l'une quelconque des revendications 1 ou 3 à 11, ou agent et cellules génétiquement modifiées pour une utilisation selon l'une quelconque des revendications 2 à 11, dans lequel le SPD ou la LDH est mesuré chez un sujet dans les 1 jour, 2 jours, 3 jours, 4 jours, 6 jours, 8 jours, 12 jours, 16 jours, 20 jours, 24 jours, 28 jours ou plus avant déclenchement de l'administration des lymphocytes T génétiquement modifiés exprimant un CAR.

13. Agent pour une utilisation selon l'une quelconque des revendications 1 ou 3 à 12, ou agent et cellules génétiquement modifiées pour une utilisation selon l'une quelconque des revendications 2 à 12, dans lequel le niveau, la quantité ou la concentration de SPD ou de LDH est évalué avant une lymphodéplétion.

14. Agent pour une utilisation selon l'une quelconque des revendications 1 ou 3 à 13, ou agent et cellules génétiquement modifiées pour une utilisation selon l'une quelconque des revendications 2 à 13, dans lequel le cancer est une tumeur maligne à lymphocytes B, tel qu'un cancer choisi dans le groupe consistant en les sarcomes, les carcinomes, les lymphomes, les lymphomes non-Hodgkiniens (LNH), les lymphomes diffus à grandes cellules B (DLBCL), une leucémie, une leucémie lymphocytaire chronique (LLC), une leucémie lymphoblastique aiguë (LLA), une leucémie myéloïde aiguë (LMA) et un myélome, un cancer du pancréas, un cancer de la vessie, un cancer colorectal, un cancer du sein, un cancer de la prostate, un cancer du rein, un cancer hépatocellulaire, un cancer du poumon, un cancer des ovaires, un cancer du col de l'utérus, un cancer rectal, un cancer de la thyroïde, un cancer de l'utérus, un cancer gastrique, un cancer de l'œsophage, un cancer de la tête et du cou, un mélanome, des cancers neuroendocriniens, des cancers du système nerveux central (SNC), des tumeurs du cerveau, un cancer des os ou un sarcome des tissus mous.
